(19) Europäisches Patentamt
European Patent Office
Office européen des brevets

(11) **EP 0 948 483 B1**

(12) **FASCICULE DE BREVET EUROPEEN**

(45) Date de publication et mention de la délivrance du brevet:
**13.03.2002 Bulletin 2002/11**

(21) Numéro de dépôt: **97953000.3**

(22) Date de dépôt: **23.12.1997**

(51) Int Cl.[7]: **C07D 209/72**, A61K 31/40, C07D 401/12, C07D 405/04, C07D 405/14, C07D 409/14, C07D 409/04, C07D 403/04, C07D 409/12

(86) Numéro de dépôt international:
**PCT/FR97/02407**

(87) Numéro de publication internationale:
**WO 98/29390 (09.07.1998 Gazette 1998/27)**

(54) **INHIBITEURS DE FARNESYLE TRANSFERASE**

FARNESYL TRANSFERASE INHIBITOREN

FARNESYL TRANSFERASE INHIBITORS

(84) Etats contractants désignés:
**AT BE CH DE DK ES FI FR GB GR IE IT LI LU NL PT SE**
Etats d'extension désignés:
**RO SI**

(30) Priorité: **30.12.1996 FR 9616206**

(43) Date de publication de la demande:
**13.10.1999 Bulletin 1999/41**

(73) Titulaire: **Aventis Pharma S.A.**
**92160 Antony (FR)**

(72) Inventeurs:
- **BOURZAT, Jean-Dominique**
  **F-94300 Vincennes (FR)**
- **COMMERCON, Alain**
  **F-94400 Vitry sur Seine (FR)**
- **DEREU, Norbert**
  **F-91170 Viry-Chatillon (FR)**
- **MAILLIET, Patrick**
  **F-94120 Fontenay sous Bois (FR)**
- **SOUNIGO-THOMPSON, Fabienne**
  **F-75012 Paris (FR)**
- **MARTIN, Jean-Paul**
  **F-92700 Colombes (FR)**
- **CAPET, Marc**
  **F-91170 Viry-Chatillon (FR)**
- **CHEVE, Michel**
  **F-91450 Soisy sur Seine (FR)**

(56) Documents cités:
**WO-A-95/12612** **FR-A- 2 736 641**

Remarques:
Le dossier contient des informations techniques présentées postérieurement au dépôt de la demande et ne figurant pas dans le présent fascicule.

EP 0 948 483 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne de nouveaux dérivés de benzoperhydroisoindole, leur préparation, les compositions pharmaceutiques qui les contiennent et leur utilisation pour la préparation de médicaments.

La protéine farnésyle transférase est une enzyme qui catalyse le transfert du groupement farnésyle du pyrophosphate de farnésyle (FPP) au résidue cystéine terminal de la séquence tétrapeptidique CAAX d'un certain nombre de protéines et en particulier de la protéine p21Ras, exprimant l'oncogène *ras*.

L'oncogène *ras* (H-, N- ou K-*ras*) est connu pour jouer un rôle clé dans les voies de signalisation cellulaire et les processus de division cellulaire. La mutation de l'oncogène *ras* ou sa surexpression est souvent associée au cancer humain: la protéine p21Ras mutée se retrouve dans de nombreux cancers humains et notamment dans plus de 50 % des cancers du colon et 90 % des cancers du pancréas (Kohl et al., Science, 260, 1834-1837, 1993).

L'inhibition de la farnésyle transférase et par conséquent de la farnésylation de la protéine p21Ras bloque la capacité de la protéine p21Ras mutée à induire une prolifération cellulaire et à transformer les cellules normales en cellules cancéreuses. D'autre part, il a été démontré que les inhibiteurs de farnésyle transférase sont également actifs sur des lignées cellulaires tumorales n'exprimant pas de *ras* muté ou surexprimé, mais présentant la mutation d'un oncogène ou la surexpression d'une oncoprotéine dont la voie de signalisation utilise la farnésylation d'une protéine, telle qu'un *ras* normal (Nagasu et al., Cancer Research 55, 5310-5314, 1995 ; Sepp-Lorenzino et al., Cancer Research 55, 5302-5309, 1995).

Les inhibiteurs de la Farnésyle transférase sont des inhibiteurs de la prolifération cellulaire et par conséquent des agents anti-tumoraux et anti-leucémiques.

Il a maintenant été découvert, et c'est également l'objet de cette invention, que les nouveaux produits de formule générale (I) présentés ci-après possèdent, de façon tout à fait surprenante et inattendue une activité inhibitrice de la farnésyle transférase et se révèlent être des agents anti-tumoraux et anti-leucémiques remarquables.

En particulier, il a été découvert selon l'invention, que l'activité biologique des composés selon l'invention est très nettement améliorée, et ceci contre toute attente, par la présence de groupements particuliers (représentés par Ar, ci-après) substitués en position 9 du squelette benzoperhydroisoindole.

Notamment, les composés correspondants présentent des activités bien supérieures à celles obtenues jusque là avec des composés de structures proches mais ne présentant pas ces substituants Ar.

La présente invention concerne les nouveaux composés de formule générale I

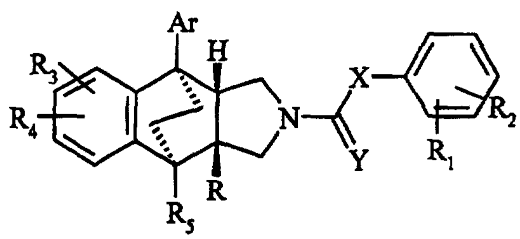 (I)

dans laquelle :

- Ar représente
  - un radical phényle substitué par un ou plusieurs atomes ou radicaux, identiques ou différents, choisis parmi les atomes d'halogène et les radicaux alcoyles contenant 1 à 4 atomes de carbone, tel que méthyle, alcényles contenant 2 à 4 atomes de carbone, hydroxy, mercapto, alcoylthio, alcoylsulfonyle ou alcoylsulfinyle, amino, alcoylamino ou dialcoylamino, formyle, alcoylcarbonyle, carboxy, alcoxycarbonyle, carbamoyle, alcoylcarbamoyle ou dialcoylcarbamoyle, cyano ou trifluorométhyle, alcoxy contenant 1 à 4 atomes de carbone, tel que méthoxy, dont la portion alcoyle est éventuellement perhalogénée, tel que trifluorométhoxy, ou
  - un radical phényle condensé à un hétérocycle de 4 à 7 chaînons contenant un ou plusieurs hétéroatomes choisis parmi les atomes d'oxygène, d'azote et de soufre,
  - un radical aromatique polycyclique,
  - un radical aromatique hétérocyclique de 5 à 12 chaînons incorporant un ou plusieurs hétéroatomes choisis parmi les atomes d'oxygène, d'azote et de soufre, lié au cycle condensé par une liaison carbone-carbone, avec pour l'ensemble de ces radicaux, alcoyl contenant 1 à 4 atomes de carbone,

- R représente un radical carboxy, ou un radical -COOMe, ou encore un radical $-CON(R_7)(R_8)$ pour lequel lorsque $R_7$ représente un atome d'hydrogène, $R_8$ représente un radical méthyle substitué par un radical 2-, 3- ou 4- pyridyle,

ou le N-oxyde de pyridine

- $R_1$ et $R_2$, représentent un atome d'hydrogène et l'autre des symboles représente un radical méthoxy.
- ou bien $R_3$ et $R_4$ représentent chacun un atome d'hydrogène, ou bien l'un des symboles $R_3$ ou $R_4$ représente un atome d'hydrogène et l'autre des symboles $R_3$ ou $R_4$ représente un radical méthoxy.
- $R_5$ représente un atome d'hydrogène ou un radical alcoyle, un radical alcoylthio, avec pour la définition de $R_5$, alcoyle contenant 1 à 4 atomes de carbone;
- X représente un groupement méthylène ou vinyldiyle et
- Y représente un atome d'oxygène ou de soufre,

sous forme racémique ou leurs isomères optiques ainsi que les sels du produit de formule générale (I).

Dans les définitions qui précédent et celles qui suivent,
les radicaux ou portions "alcoyle contenant 1 à 4 atomes de carbone", ou "alcoyle en C1 à C4" définit les radicaux méthyle, éthyle, propyle, butyle, et les isomères *iso, sec, tert* correspondants

- "alcényles contenant 2 à 4 atomes de carbone" définit les radicaux vinyle, allyle, propène-2 yle, butène-1 ou -2 ou-3 yle, et les isomères *iso* correspondants
- "alcoxy contenant 1 à 4 atomes de carbone" définit les radicaux méthoxy, éthoxy, propoxy, butoxy, et les isomères *iso, sec, tert* correspondants
- "alcoxycarbonyle en C2 à C4" définit les radicaux métoxycarbonyle, éthoxycarbonyle, propoxycarbonyle, et les isomères *iso, sec, tert* correspondants,

Selon l'invention, les composés de formule générale (I) se présentent de préférence sous forme dextrogyre.

Préférentiellement, Ar représente un radical 2,3-dihydro-1,4-benzodioxin-6-yle ou 2,3-dihydrobenzofuran-5-yl, ou un radical phényle substitué en position 4, de préférence par un radical méthyle, trifluorométhyle ou méthoxy ; en particulier, le radical 2,3-dihydro-1,4-benzodioxin-6-yle ; très avantageusement, Ar représente un radical phényle substitué en position 4 par un radical méthyle.

Préférentiellement, $R_5$ représente l'hydrogène ou un radical méthyle.

Préférentiellement, Y représente l'oxygène.

A titre illustratif et non limitatif des composés revendiqués on peut plus particulièrement citer les composés suivants :

4,9-éthano-2-[2-(2-méthoxyphényl)propènoyl]-9-(4-méthylphényl)-2,3,3a,4,9,9a-hexahydro-1H-benzo[f]isoindole-3a-carboxylate de méthyle-(3aRS,4SR,9SR,9aRS) acide 4,9-éthano-2-(2-méthoxyphényl)propènoyl-9-(4-méthylphényl)-2,3,3a,4,9,9a-hexahydro-1H-benzo[f]isoindole-3a-carboxylique-(3aRS,4SR,9SR,9aRS)

acide 4,9-éthano-2-[(2-méthoxyphényl)acétyl]-9-(4-méthylphényl)-2,3,3a,4,9,9a-hexahydro-1H-benzo[f]isoindole-3a-carboxylique-(3aRS,4SR,9SR,9aRS),

acide 4,9-éthano-9-(4-méthoxyphényl)-2-[(2-méthoxyphényl)acétyl]-2,3,3a,4,9,9a-hexahydro-1H-benzo[f]isoindole-3a-carboxylique (3aRS,4SR,9SR,9aRS),

acide 4,9-éthano-2-[(2-méthoxyphényl)acétyl]-9-(4-méthylsulfanylphényl)-2,3,3a,4, 9,9a-hexahydro-1H-benzo[f]isoindole-3a-carboxylique (3aRS,4SR,9SR,9aRS),

acide 4,9-éthano-9-(4-fluorophényl)-2-[(2-méthoxyphényl)acétyl]-2,3,3a,4,9,9a-hexahydro-1H-benzo[f]isoindole-3a-carboxylate de méthyle-(3aRS,4SR,9SR,9aRS),

acide 4,9-éthano-2-[(2-méthoxyphényl)acétyl]-9-(3-méthylphényl)-2,3,3a,4,9,9a-hexahydro-1H-benzo[f]isoindole-3a-carboxylique-(3aRS,4SR,9SR,9aRS),

acide 4,9-éthano-9-(3-méthoxyphényl)-2-[(2-méthoxyphényl)acétyl]-2,3,3a,4,9,9a-hexahydro-1H-benzo[f]isoindole-3a-carboxylique-(3aRS,4SR,9SR,9aRS),

9-(3,4-diméthylphényl)-4,9-éthano-2-[(2-méthoxyphényl)acétyl]-2,3,3a,4,9,9a-hexahydro-1H-benzo[f]isoindole-3a-carboxylate de méthyle-(3aRS,4SR,9SR,9aRS),

acide 9-(3,4-diméthylphényl)-4,9-éthano-2-[(2-méthoxyphényl)acétyl]-2,3,3a,4,9,9a-hexahydro-1H-benzo[f]isoindole-3a-carboxylique-(3aRS,4SR,9SR,9aRS),

3a-N-benzylcarbamoyl-4,9-éthano-2-[2-(2-méthoxyphényl)propènoyl]-9-(4-méthylphényl)-2,3,3a,4,9,9a-hexahydro-1H-benzo[f]isoindole-(3aRS,4SR,9SR,9aRS),

3a-carbamoyl-4,9-éthano-2-[2-(2-méthoxyphényl)propènoyl]-9-(4-méthylphényl)-2,3,3a,4,9,9a-hexahydro-1H-benzo[f]isoindole-(3aRS,4SR,9SR,9aRS),

4,9-éthano-2-[2-(2-méthoxyphényl)propènoyl]-9-(4-méthylphényl)-2,3,3a,4,9,9a-hexahydro-1H-benzo[f]isoindole-3a-hydroxamate de benzyle-(3aRS,4SR,9SR,9aRS),

acide 4,9-éthano-2-[2-(2-méthoxyphényl)propènoyl]-9-(4-méthylphényl)-,3,3a,4,9,9a-hexahydro-1H-benzo[f]isoindole-3a-hydroxamique-(3aRS,4SR,9SR,9aRS),

sous forme racémique ainsi que leurs isomères optiques.

On peut également citer selon l'invention, tout composé de formule générale (I) sélectionné individuellement parmi :

4,9-éthano-2-[2-(2-méthoxyphényl)propènoyl]-9-(4-méthylphènyl)-2,3,3a,4,9,9a-hexahydro-1H-benzo[f]isoindole-3a-carboxylate de méthyle-(3aRS,4SR,9SR,9aRS) acide 4,9-éthano-2-(2-méthoxyphényl)propènoyl-9-(4-méthylphényl)-2,3,3a,4,9,9a-hexahydro-1H-benzo[f]isoindole-3a-carboxylique-(3aRS,4SR,9SR,9aRS)

acide 4,9-éthano-2-[(2-méthoxyphényl)acétyl]-9-(4-méthylphényl)-2,3,3a,4,9,9a-hexahydro-1H-benzo[f]isoindole-3a-carboxylique-( 3aRS,4SR,9SR,9aRS),

acide 4,9-éthano-9-(4-méthoxyphényl)-2-[(2-méthoxyphényl)acétyl]-2,3,3a,4,9,9a-hexahydro-1H-benzo[f]isoindole-3a-carboxylique (3aRS,4SR,9SR,9aRS),

acide 4,9-éthano-2-[(2-méthoxyphényl)acétyl]-9-(4-méthylsulfanylphényl)-2,3,3a,4, 9,9a-hexahydro-1H-benzo[f]isoindole-3a-carboxylique (3aRS,4SR,9SR,9aRS),

acide 4,9-éthano-9-(4-fluorophényl)-2-[(2-méthoxyphényl)acétyl]-2,3,3a,4,9,9a-hexahydro-1H-benzo[f]isoindole-3a-carboxylique-(3aRS,4SR,9SR,9aRS),

acide 4,9-éthano-2-[(2-méthoxyphényl)acétyl]-9-(3-méthylphényl)-2,3,3a,4,9,9a-hexahydro-1H-benzo[f]isoindole-3a-carboxylique-(3aRS,4SR,9SR,9aRS),

acide 4,9-éthano-9-(3-méthoxyphényl)-2-[(2-méthoxyphényl)acétyl]-2,3,3a,4,9,9a-hexahydro-1H-benzo[f]isoindole-3a-carboxylique-(3aRS,4SR,9SR,9aRS),

9-(3,4-diméthylphényl)-4,9-éthano-2-[(2-méthoxyphényl)acétyl]-2,3,3a,4,9,9a-hexahydro-1H-benzo[f]isoindole-3a-carboxylate de méthyle-(3aRS,4SR,9SR,9aRS),

acide 9-(3,4-diméthylphényl)-4,9-éthano-2-[(2-méthoxyphényl)acétyl]-2,3,3a,4,9,9a-hexahydro-1H-benzo[f]isoindole-3a-carboxylique-(3aRS,4SR,9SR,9aRS),

3a-N-benzylcarbamoyl-4,9-éthano-2-[2-(2-méthoxyphényl)propènoyl]-9-(4-méthylphényl)-2,3,3a,4,9,9a-hexahydro-1H-benzo[f]isoindole-(3aRS,4SR,9SR,9aRS),

3a-carbamoyl-4,9-éthano-2-[2-(2-méthoxyphényl)propènoyl]-9-(4-méthylphényl)-2,3,3a,4,9,9a-hexahydro-1H-benzo[f]isoindole-(3aRS,4SR,9SR,9aRS),

4,9-éthano-2-[2-(2-méthoxyphényl)propènoyl]-9-(4-méthylphényl)-2,3,3a,4,9,9a-hexahydro-1H-benzo[f]isoindole-3a-hydroxamate de benzyle-(3aRS,4SR,9SR,9aRS),

acide 4,9-éthano-2-[2-(2-méthoxyphényl)propènoyl]-9-(4-méthylphényl)-,3,3a,4,9,9a-hexahydro- 1H-benzo[f]isoindole-3a-hydroxamique-(3aRS,4SR,9SR,9aRS)

4,9-éthano-2-[2-(2-méthoxyphényl)propènoyl]-9-(4-méthylphényl)-2,3,3a,4,9,9a-hexahydro-1H-benzo[f]isoindole-3a-N-(3-pyridylméthyl)-carboxamide-(3aRS,4SR, 9SR,9aRS)

4,9-éthano-2-[2-(2-méthoxyphényl )propènoyl]-9-(4-méthylphényl)-2,3,3a,4,9,9a-hexahydro-1H-benzo[f]isoindole-3a-N-(4-pyridylméthyl)-carboxamide-(3aRS,4SR,9SR,9aRS)

4,9-éthano-2-[2-(2-méthoxyphényl)propènoyl]-9-(4-méthylphényl)-2,3,3a,4,9,9a-hexahydro-1H-benzo[f]isoindole-3a-hydroxamate de méthyle-(3aRS,4SR,9SR,9aRS)

4,9-éthano-2-[2-(2-méthoxyphényl)propènoyl]-9-(4-méthylphényl)-2,3,3a,4,9,9a-hexahydro-1H-benzo[f]isoindole-3a-N',N'-diméthylcarbohydrazide-(3aRS,4SR,9SR,9aRS)

4,9-ethano-2-[2-(2-méthoxyphényl)propènoyl]-9-(4-méthylphényl)-2,3,3a,4,9,9a-hexahydro-1H-benzo[f]isoindole-3a-N'-phénylcarbohydrazide-(3aRS,4SR, 9SR,9aRS)

4,9-éthano-2-[2-(2-méthoxyphényl)propènoyl]-9-(4-méthylphényl)-2,3,3a,4,9,9a-hexahydro-1H-benzo[f]isoindole-3a-N',N'-pentaméthylènecarbohydrazide-(3aRS,4SR,9SR,9aRS)

4,9-éthano-2-[2-(méthoxyphényl)propènoyl]-9-(4-méthylphényl)-3a-phénylsulfonylaminocarbonyl-2,3,3a,4,9,9a-hexahydro-1H-benzo[f]isoindole-(3aRS,4SR,9SR,9aRS)

4,9-éthano-2-[2-(2-méthoxyphényl)propènoyl]-9-(4-méthylphényl)-2,3,3a,4,9,9a-hexahydro-1H-benzo[f]isoindole-3a-N-(N-oxo-3pyridyl)méthyl-carboxamide-(3aRS,4SR,9SR,9aRS)

4,9-éthano-2-[2-(2-méthoxyphényl)propènoyl]-9-(4-méthylphényl)-2,3,3a,4,9,9a-hexahydro-1H-benzo[f]isoindole-3a-N'-(4-méthoxyphènyl)carbohydrazide-(3aRS,4SR,9SR,9aRS)

4,9-éthano-2-[2-(2-méthoxyphényl)propènoyl]-9-(4-méthylphényl)-2,3,3a,4,9,9a-hexahydro-1H-bero[f]isoindole-3a-N'-méthyl,N'-phénylcarbohydrazide-(3aRS,4SR,9SR,9aRS)

4,9-éthano-2-[2-(2-méthoxyphényl)propènoyl]-9-(4-méthylphényl)-2,3,3a,4,9,9a-hexahydro-1H-benzo[f]isoindole-3a-N'-(2-méthylphényl)carbohydrazide-(3aRS,4SR,9SR,9aRS)

9-(2,3-dihydro-1,4-benzodioxin-6-yl)-4,9-éthano-2-[2-(2-méthoxy phényl)propènoyl]-2,3,3a,4,9,9a-hexahydro-1H-benzo[f]isoindole-3a-carboxylate de méthyle-(3aRS,4SR,9SR,9aRS)

acide 9-(2,3-dihydro-1,4-benzodioxin-6-yl)-4,9-éthano-2-[2-(2-méthoxyphényl) propènoyl]-2,3,3a,4,9,9a-hexahydro-1H-benzo[f]isoindole-3a-carboxylique-(3aRS.4SR,9SR,9aRS)

9-(2,3-dihydro-1,4-benzodioxin-6-yl)-4,9-éthano-2-[2-(2-méthoxy phényl)propènoyl)]-2,3,3a,4,9,9a-hexahydro-1H-benzo[f]isoindole-3a-[N-(3-pyridylméthyl)carboxamide-(3aRS,4SR,9SR,9aRS)

9-(2,3-dihydro-1,4-benzodioxin-6-yl)-4,9-éthano-2-[2-(2-méthoxy phényl)propènoyl)]- 2,3,3a,4,9,9a-hexahydro-1H-benzo[f]isoindole-3a-N-(2-thiénylméthyl)carboxamide-(3aRS,4SR,9SR,9aRS)

acide 4,9-éthano-2-[(2-méthoxyphényl)acétyl]-9-(3,4,5-triméthylphényl)-2,3,3a,4,9,9a-hexahydro-1H-benzo[f]isoindole-3a-carboxylique-(3a*RS*,4*SR*,9*SR*,9a*RS*)

acide 4,9-éthano-2-[2-(2-méthoxyphényl)propènoyl]-9-(2-méthyl-phényl)-2,3,3a,4,9,9a-hexahydro-1H-benzo[f]isoindole-3a-carboxylique-(3aRS, 4SR,9SR,9aRS)

4,9-éthano-2-[2-(2-méthoxyphényl)propènoyl]-9-(4-trifluorométhylphényl)-2,3,3a,4,9,9a-hexahydro-1H-benzo[f]isoindole-3a-carboxylate de méthyle-(3aRS,4SR,9SR,9aRS)

acide 4,9-éthano-2-[2-(2-méthoxyphényl)propènoyl]-9-(4-trifluorométhyl-phényl)-2,3,3a,4,9,9a-hexahydro-1H-benzo[f]isoindole-3a-carboxylique-(3aRS, 4SR,9SR,9aRS)

4,9-éthano-2-[2-(2-méthoxyphényl)propènoyl]-9-(4-trifluorométhylphényl)-2,3,3a,4,9,9a-hexahydro-1H-benzo[f]isoindole-3a-N-(4-pyridylméthyl)-carboxamide-(3aRS,4SR,9SR,9aRS)

4,9-éthano-2-[2-(2-méthoxyphényl)propènoyl]-9-(4-trifluorométhylphényl)-2,3,3a,4,9,9a-hexahydro-1H-benzo[f]isoindole-3a-N'-benzoyl-carbohydrazide-(3aRS,4SR,9SR,9aRS)

4,9-éthano-2-[2-(2-méthoxyphényl)propènoyl]-9-(4-trifluoromethylphényl)-2,3,3a,4,9,9a-hexahydro-1H-benzo[f]isoindole-3a-N'-phényl-carbohydrazide-(3aRS,4SR,9SR,9aRS)

4,9-éthano-2-[2-(2-méthoxyphényl)propènoyl]-9-(2-naphtyl)-2,3,3a,4,9,9a-hexahydra-1H-benzo[f]isoindole-3a-carboxylate de méthyle-(3aRS,4SR,9SR,9aRS)

acide 4,9-éthano-2-[2-(2-méthoxyphényl)propènoyl]-9-(2-naphtyl)-2,3,3a,4,9,9a-hexahydro-1H-benzo[f]isoindole-3a-carboxylique-(3aRS,4SR,9SR,9aRS)

4,9-éthano-2-[2-(2-méthoxyphényl)propénoyl]-9-(5-méthyl-2-thiényl)-2,3,3a,4,9,9a-hexahydro-1H-benzo[f]isoindole-3a-carboxylate de méthyle-(3aRS,4SR,9SR,9aRS)

acide 4,9-éthano-2-[2-(2-méthoxyphényl)propènoyl]-9-(5-méthyl-2-thiényl)-2,3,3a,4,9,9a-hexahydro-1H-benzo[f]isoindole-3a-carboxylique-(3aRS,4SR, 9SR,9aRS)

9-(4-bromophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS, 4SR, 9SR,9aRS)

acide 9-(4-bromophényl)-4,9-éthano-2-[2-(2-méthoxyphényl)propènoyl]-2,3,3a,4,9,9a-hexahydro-1H-benzo[f]isoindole-3a-carboxylique-(3aRS,4SR,9SR, 9aRS)

acide 9-(3,4-dichlorophényl)-4,9-éthano-2-[2-(2-méthoxyphényl)propènoyl]-2,3,3a,4,9,9a-hexahydro-1H-benzo[f]isoindole-3a-carboxylique-(3aRS, 4SR,9SR,9aRS)

4,9-éthano-2-[2-(2-méthoxyphényl)propènoyl]-9-(4-chlorophényl)-2,3,3a,4,9,9a-hexahydro-1H-benzo[f]isoindole-3a-carboxylate de méthyle-(3aRS,4SR,9SR,9aRS)

acide 9-(4-chlorophényl)-4,9-éthano-2-[2-(2-méthoxyphényl) propènoyl]-2,3,3a,4,9,9a-hexahydro-1H-benzo[f]isoindole-3a-carboxylique-(3aRS, 4SR,9SR,9aRS)

acide 4,9-éthano-2-[2-(2-méthoxyphényl)propènoyl]-9-(4-méthoxy-3-méthylphényl)-2,3,3a,4,9,9a-hexahydro-1H-benzo[f]isoindole-3a-carboxylique-(3aRS, 4SR,9SR,9aRS)

4,9-éthano-2-[2-(2-méthoxyphényl)propénoyl]-9-(3-indolyl)-2,3,3a,4,9,9a-hexahydro-1H-benzo[f]isoindole-3a-carboxylate de méthyle-(3aRS,4SR,9SR,9aRS)

acide 4,9-éthano-9-(4-isopropylphényl)-2-[(2-méthoxyphényl)acétyl]-2,3,3a,4,9,9a-hexahydro-1H-benzo[f]isoindole-3a-carboxylique-(3aRS,4SR,9SR,9aRS)

acide 4,9-éthano-9-(4-isopropylphényl)-2-[2-(2-méthoxyphényl) propènoyl]-2,3,3a,4,9,9a-hexahydro-1H-benzo[f]isoindole-3a-carboxylique-(3aRS,4SR,9SR, 9aRS)

4,9-éthano-2-[2-(2-méthoxyphényl)propènoyl]-9-(3-thiényl)-2,3,3a,4,9,9a-hexahydro-1H-benzo[f]isoindole-3a-carboxylate de méthyle-(3aRS,4SR,9SR,9aRS)

4,9-éthano-9-(4-éthylphényl)-2-[2-(2-méthoxyphényl)propènoyl]-2,3,3a,4,9,9a-hexahydro-1H-benzo[f]isoindole-3a-carboxylate de méthyle-(3aRS,4SR, 9SR,9aRS)

acide 4,9-éthano-9-(4-éthylphényl)-2-[2-(2-méthoxyphényl) propènoyl]-2,3,3a,4,9,9a-hexahydro-1H-benzo[f]isoindole-3a-carboxylique-(3aRS,4SR,9SR,9aRS)
9-(2,3-dihydrobenzofuran-5-yl)-4,9-éthano-2-[2-(2-méthoxy phényl)propènoyl]-2,3,3a,4,9,9a-hexahydro-1H-benzo[f]isoindole-3a-carboxylate de méthyle-(3aRS,4SR, 9SR, 9aRS)
acide 9-(2,3-dihydrobenzofuran-5-yl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR, 9SR, 9aRS)
9-(2,3-dihydrobenzofuran-5-yl)-4,9-éthano-2-[2-(2-méthoxy phényl)propènoyl)]-2,3,3a,4,9,9a-hexahydro-1H-benzo[f] isoindole-3a-N-(3-pyridylméthyl)carboxamide-(3aRS,4SR,9SR,9aRS)

4,9-éthano-9-(4-fluorophényl)-2-[2-(2-méthoxyphényl)propénoyl]-2,3,3a,4,9,9a-hexahydro-1H-benzo[f]isoindole-3a-carboxylate de méthyle-(3aRS,4SR,9SR,9aRS)

acide 4,9-éthano-9-(4-fluorophényl)-2-[2-(2-méthoxyphényl)propènoyl2,3,3a,4,9,9a-hexahydro-1H-benzo[f]isoindole-3a-carboxylique-(3aRS,4SR,9SR,9aRS)

9-(4-chloro-3-fluorophényl)-4,9-éthano-2-[2-(2-méthoxyphényl) propènoyl2,3,3a,4,9,9a-hexahydro-1H-benzo[f] isoindole-3a-carboxylate de méthyle-(3aRS, 4SR,9SR,9aRS)

acide 9-(4-chloro-3-fluorophényl)-4,9-éthano-2-[2-(2-mèthoxyphényl)propènoyl]-2,3,3a,4,9,9a-hexahydro-1H-benzo[f]isoindole-3a-carboxylique-(3aRS, 4SR,9SR,9aRS)

4,9-éthano-2-[2-(2-méthoxyphényl)propènoyl]-9-(3-méthylphényl)-2,3,3a,4,9,9a-hexahydro-1H-benzo[f]isoindole-3a-carboxylate de méthyle-(3aRS,4SR,9SR,9aRS)

acide 4,9-éthano-2-[2-(2-méthoxyphényl)propènoyl]-9-(3-méthylphényl)-2,3,3a,4,9,9a-hexahydro-1H-benzo[f] isoindole-3a-carboxylique-(3aRS, 4SR,9SR,9aRS)

9-( 1,3-benzodioxol-5-yl)-4,9-éthano-2-[2-(2-méthoxyphényl) propènoyl]2,3,3a,4,9,9a-hexahydro-1H-benzo[f] isoindole-3a-carboxylate de méthyle-(3aRS,4SR,9SR,9aRS)
9-(3,4-diméthylphényl)-4,9-éthano-2[2-(2-méthoxy phényl)propènoyl]-2,3,3a,4,9,9a-hexahydro-1H-benzo[f]isoindole-3a-carboxylate de méthyle-(3aRS,4SR,9SR,9aRS) acide-9-(3,4-diméthylphényl)-4,9-éthano-2[2-(2-méthoxyphényl)propènoyl]-2,3,3a,4,9,9a-hexahydro-1H-benzo[f]isoindole-3a-carboxylique-(3aRS, 4SR,9SR,9aRS)

4,9-éthano-9-(4-méthoxyphényl)-2-[2-(2-méthoxyphényl)propènoyl]-2,3,3a,4,9,9a-hexahydro-1H-benzo[f]isoindole-3a-carboxylate de méthyle-(3aRS, 4SR,9SR.9aRS)

acide 4,9-éthano-9-(4-méthoxyphényl)-2-[2-(2-méthoxyphényl)propènoyl]-2,3,3a,4,9,9a-hexahydro-1H-benzo[f] isoindole-3a-carboxylique-(3aRS, 4SR,9SR,9aRS)

4,9-éthano-9-(4-méthoxyphényl)-2-[2-(2-méthoxyphényl)propènoyl]-2,3,3a,4,9,9a-hexahydro-1H-benzo[f]isoindole-3a-N-(3-pyridylméthyl)-carboxamide-(3aRS,4SR,9SR,9aRS)

4.9-éthano-9-(4-méthoxyphényl)-2-[2-(2-méthoxyphényl)propènoyl]-2,3,3a,4,9,9a-hexahydro-1H-benzo[f]isoindole-3a-N'-phénylcarbohydrazide-(3aRS,4SR,9SR,9aRS)

acide 4,9-éthano-]-9-(4-méthoxyphényl)-2-[2-(2-methoxyphényl)propènoyl-2,3,3a,4,9,9a-hexahydro-1H-benzo[f] isoindole-3a-hydroxamique-(3aRS,4SR.9SR,9aRS)

4,9-éthano-9-(4-méthoxyphényl)-2-[2-(2-méthoxyphényl)propènoyl]- 2,3,3a,4,9,9a-hexahydro-1H-benzo[f]isoindole-3a-N'-(3-pyridyl)carbohydrazide-(3aRS,4SR,9SR,9aRS)

4,9-éthano-9-(4-méthoxyphényl)-2-[2-(2-méthoxyphényl)propènoyl]-2,3,3a,4,9,9a-hexahydro-1H-benzo[f]isoindole-3a-N-(3-thiénylméthyl)-carboxamide-(3aRS,4SR,9SR,9aRS)

acides 2-{4,9-éthano-9-(4-méthoxyphényl)-2-[2-(2-méthoxyphényl)propènoyl]-2,3,3a,4,9,9a-hexahydro-1H-benzo[f]isoindole-3a-carbonylamino-(3aRS,4SR,9SR,9aRS)}phénylacétiques-(RS) et (SR)

acide 4,9-éthano-2-[2-(2-méthoxyphényl)propènoyl]-9-(4-trifluorométhoxyphényl)-2,3,3a,4,9,9,9a-hexahydro-1H-benzo[f]isoindole-3a-carboxylique-(3aRS, 4SR, 9SR,9aRS)

9-(3-bromophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR, 9SR,9aRS)

acide 9-(3-bromophényl)-4,9-éthano-2-[2-(2-méthoxyphényl)propènoyl]-2,3,3a,4,9,9a-hexahydro-1H-benzo[f] isoindole-3a-carboxylique-(3aRS,4SR,9SR, 9aRS)
4,9-éthano-9-(3-fluorophényl-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS) acide 4,9-éthano-9-(3-fluorophényl)-2-[2-(2-méthoxyphényl)

propènoyl])-2,3,3a,4,9,9a-hexahydro-1H-benzo[f]-isoindole-3a-carboxylique-(3aRS, 4SR, 9SR, 9aRS)

4,9-éthano-9-(3-fluorophényl)-2-[2-(2-méthoxyphényl)propénoyl)]-2,3,3a,4,9,9a-hexahydro-1H-benzo[f]isoindole-3a-[N-(3-pyridylméthyl)carboxamide-(3aRS,4SR,9SR,9aRS)
9-(3-chlorophényl)-4,9-éthano-2-[2-(2-méthoxyphényl)propényl)propènoyl]-2,3,3a,4,9,9a-hexahydro-1H-benzo[f] isoindole-3a-carboxylate de méthyle-(3aRS,4SR,9SR,9aRS)

acide 9-(3-chlorophényl)-4,9-éthano-2-[2-(2-méthoxyphényl) propènoyl]-2,3,3a,4,9,9a-hexahydro-1H-benzo[f] isoindole-3a-carboxylique-(3aRS, 4SR,9SR,9aRS)
9-(3-chlorophényl)-4,9-éthano-2-[2-(2-méthoxyphényl)propènoyl)]-2,3,3a,4,9,9a-hexahydro-1H-benzo[f]isoindole 3a-[N-(3-pyridylméthyl)carboxamide-(3aRS,4SR,9SR,9aRS)

9-(3-N,N-diméthylaminophényl)-4,9-éthano-2-[2-(2-méthoxyphényl)propènoyl]-2,3,3a,4,9,9,9a-hexahydro-1H-benzo[f]isoindole-3a-carboxylate de méthyle-(3aRS, 4SR, 9SR,9aRS)

acide 9-(3-N,N-diméthylaminophényl)-4,9-éthano-2-[2-(2-méthoxyphényl) propènoyl]-2,3,3a,4,9,9,9a-hexahydro-1H-benzo[f]isoindole-3a-carboxylique-(3aRS,4SR,9SR, 9aRS)

chlorhydrate de 9-(3-aminophényl)-4,9-éthano-2-[2-(2-méthoxyphényl)propènoyl]-2,3,3a,4,9,9,9a-hexahydro-1H-benzo[f]isoindole-3a-carboxylate de méthyle-(3aRS, 4SR, 9SR,9aRS)

9-(4-N,N-diméthylaminophényl)-4,9-éthano-2-[2-(2-méthoxyphényl)propènoyl]-2,3,3a,4,9,9,9a-hexahydro-1H-benzo[f]isoindole-3a-carboxylate de méthyle-(3aRS, 4SR, 9SR,9aRS)

acide 9-(4-cyanophényl)-4,9-éthano-2-[2-(2-méthoxyphényl) propènoyl]-2,3,3a,4,9,9,9a-hexahydro-1H-benzo[f] isoindole-3a-carboxylique-(3aRS,4SR,9SR, 9aRS)

9-(3-cyanophényl)-4,9-éthano-2-[2-(2-méthoxyphényl) propènoyl]-2,3,3a,4,9,9,9a-hexahydro-1H-benzo[f]isoindole-3a-carboxylate de méthyle-(3aRS,4SR, 9SR,9aRS)

acide 4,9-éthano-9-(3-hydroxy-4-méthoxyphényl)-2-[2-(2-méthoxyphényl)propènoyl]-2,3,3a,4,9,9a-hexahydro-1H-benzo[f]isoindole-3a-carboxylique-(3aRS,4SR,9SR,9aRS)

4,9-éthano-5-méthoxy-2-[2-(2-méthoxyphényl)-propènoyl]-9-(4-méthylphényl)-2,3,3a,4,9,9a-hexahydro-1H-benzo[f]isoindole-3a-carboxylate de méthyle-(3aRS,4SR,9SR,9aRS)

acide 4,9-éthano-5-méthoxy-2-[2-(2-méthoxyphényl)-propènoyl]-9-(4-méthylphényl)-2,3,3a,4,9,9a-hexahydro-1H-benzo[f]isoindole-3a-carboxylique-(3aRS,4SR, 9SR,9aRS)

acide 4,9-éthano-2[2-(2-méthoxyphényl)-2-propènoyl]-4-méthyl-9-(4-méthoxyphényl)-2,3,3a,4,9,9a-hexahydro-1H-benzo[f]isoindole-3a-carboxylique-(3aRS,4SR,9SR,9aRS)

sous forme racémique ou leurs isomères optiques ainsi que leurs sels.

Dans ce qui précède et ce qui suit, l'expression "isomère optique" ou forme optiquement active définit la forme pure dudit isomère optique ou éventuellement le mélange des isomères optiques "enrichi", c'est-à-dire contenant majoritairement ledit isomère optique ou ladite forme.

Plus préférentiellement, on peut encore citer selon l'invention, tout composé de formule générale (I) sélectionné individuellement parmi :

acide 4,9-éthano-2[2-(2-méthoxyphényl)propènoyl]-9-(4-méthylphényl)-2,3,3a,4,9,9a-hexahydro-1H-benzo[f] isoindole-3a-carboxylique-(3aRS,4SR,9SR,9aRS)

4,9-éthano-2-[2-(2-méthoxyphényl )propènoyl]-9-(4-méthylphényl )-2,3,3a,4,9,9a-hexahydro-1H-benzo[f]isoindole-3a-N-(3-pyridylméthyl)-carboxamide-(3aRS,4SR, 9SR,9aRS)
9-(2,3-dihydro-1,4-benzodioxin-6-yl)-4,9-éthano-2-[2-(2-méthoxy phényl)propènoyl)]-2,3,3a,4,9,9a-hexahydro-1H-benzo[f]isoindole-3a-[N-(3-pyridylméthyl)carboxamide-(3aRS,4SR,9SR,9aRS)

acide 4,9-éthano-2-[2-(2-méthoxyphényl)propénoyl]-9-(4-trifluorométhyl-phényl)-2,3,3a,4,9,9a-hexahydro-1H-benzo[f]isoindole-3a-carboxylique-(3aRS, 4SR,9SR,9aRS)

9-(2,3-dihydrobenzofuran-5-yl)-4,9-éthano-2-[2-(2-méthoxyphényl)propènoyl]-2,3,3a,4,9,9a-hexahydro-1H-benzo[f]isoindole-3a-N-(3-pyridylméthyl)carboxamide-(3aRS,4SR,9SR, 9aRS)

acide 4,9-éthano-9-(4-méthoxyphényl)-2-[2-(2-méthoxyphényl)propènoyl]-2,3,3a,4,9,9a-hexahydro-1H-benzo[f]isoindole-3a-carboxylique-(3aRS, 4SR,9SR,9aRS)

acide 4,9-éthano-5-méthoxy-2-[2-(2-méthoxyphényl)-propènoyl]-9-(4-méthylphényl)-2,3,3a,4,9,9a-hexahydro-1H-benzo[f]isoindole-3a-carboxylique-(3aRS,4SR, 9SR,9aRS)

acide 4,9-éthano-2[2-(2-méthoxyphényl)-2-propènoyl]-4-méthyl-9-(4-méthoxyphényl)-2,3,3a,4,9,9a-hexahydro-1H-benzo[f]isoindole-3a-carboxylique-(3aRS,4SR,9SR,9aRS)

sous forme racémique ou leurs isomères optiques ainsi que leurs sels.

A titre d'isomère optique tout particulièrement avantageux des composés de formule générale (I) selon l'invention, on peut citer tout composé choisi isolément parmi :

énantiomère dextrogyre de l'acide 4,9-éthano-2[2-(2-méthoxyphényl)propènoyl]-9-(4-méthylphényl)-2,3,3a,4,9,9a-hexahydro-1H-benzo[f]isoindole-3a-carboxylique-(3aRS,4SR, 9SR,9aRS)

énantiomère dextrogyre du 4,9-éthano-2-[2-(2-méthoxyphényl) propènoyl]-9-(4-méthylphényl)- 2,3,3a,4,9,9a-hexahydro-1H-benzo[f]isoindole-3a-N-(3-pyridylméthyl)-carboxamide-(3aRS,4SR,9SR,9aRS)

énantiomére lévogyre du 4,9-éthano-2-[2-(2-méthoxyphényl)propènoyl]-9-(4-méthylphényl)-2,3,3a,4,9,9a-hexahydro-1H-benzo[f]isoindole-3a-N-(3-pyridylméthyl)-carboxamide-(3aRS,4SR,9SR,9aRS)

énantiomère dextrogyre du 9-(2,3-dihydro-1,4-benzodioxin-6-yl)-4,9-éthano-2-[2-(2-méthoxy phènyl)propènoyl)]-2,3,3a,4,9,9a-hexahydro-1H-benzo[f] isoindole-3a-N-(3-pyridylméthyl)carboxamide-(3aRS,4SR,9SR,9aRS)

énantiomère lévogyre du 9-(2,3-dihydro-1,4-benzodioxin-6-yl)-4,9-éthano-2-[2-(2-méthoxy phényl)propènoyl)]-2,3,3a,4,9,9a-hexahydro-1H-benzo[f] isoindole-3a-N-(3-pyridylméthyl)carboxamide-(3 aRS,4SR,9SR,9aRS)

énantiomère dextrogyre de l'acide 4,9-éthano-2-[2-(2-méthoxyphényl)propènoyl]-9-(4-trifluorométhylphényl)-2,3,3a,4,9,9a-hexahydro-1H-benzo[f]isoindole-3a-carboxylique-(3aRS,4SR,9SR,9aRS)

énantiomère dextrogyre de l'acide 4,9-éthano-9-(4-méthoxyphényl)-2-[2-(2-méthoxyphényl)propènoyl]-2,3,3a,4,9,9a-hexahydro-1H-benzo[f]isoindole-3a-carboxylique-(3aRS,4SR,9SR,9aRS)

énantiomère dextrogyre de l'acide 4,9-éthano-5-méthoxy-2-[2-(2-méthoxyphényl)-2-propènoyl]-9-(4-méthylphényl)-2,3,3a,4,9,9a-hexahydro-1H-benzo[f]isoindole-3a-carboxylique-(3aRS,4SR, 9SR,9aRS)

énantiomère dextrogyre de l'acide 4,9-éthano-2[2-(2-méthoxyphényl)-2-propènoyl]-4-méthyl-9-(4-méthoxyphényl)-2,3,3a,4,9,9a-hexahydro-1H-benzo[f]isoindole-3a-carboxylique-(3aRS,4SR,9SR,9aRS)

ou leurs sels.

En particulier on peut citer
énantiomère dextrogyre de l'acide 4,9-éthano-2[2-(2-méthoxyphényl)propènoyl]-9-(4-méthylphényl)-2,3,3a,4,9,9a-hexahydro-1H-benzo[f]isoindole-3a-carboxylique-(3aRS,4SR, 9SR,9aRS)
ou ses sels.

La Demanderesse propose en outre ci-après à titre non limitatif de la présente invention, divers protocoles opératoires ainsi que des intermédiaires réactionnels susceptibles d'être mis en oeuvre pour préparer les composés de formule générale (I). Bien entendu, il est à la portée de l'homme de l'art de s'inspirer de ces protocoles et ou produits intermédiaires pour mettre au point des procedés analogues en vue de conduire à ces mêmes composés.

Selon l'invention, les nouveaux produits de formule générale (I) revendiqués dans laquelle :

- Y représente un atome d'oxygène ou de soufre et
- X représente un groupement -CO-, méthylène, alcèn-1,1-diyle tel que vinyldiyle ou cycloalcan-1,1-diyle contenant

3 à 6 atomes de carbone,

peuvent être obtenus par action d'un acide de formule générale (II) :

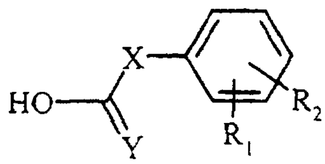

(II)

dans laquelle

- $R_1$, $R_2$ sont définis selon la formule générale I et X défini comme précédemment et
- Y représente un atome d'oxygène ou de soufre,

de son ester méthylique ou d'un dérivé de cet acide tel qu'un halogénure ou l'anhydride, sur un produit de formule générale (III) :

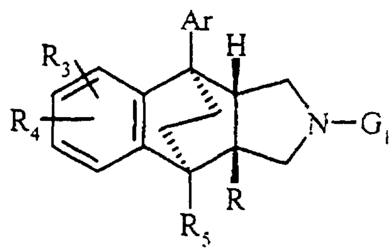

(III)

dans laquelle:

- Ar, R, $R_3$ $R_4$ et $R_5$ sont définis selon la formule générale I et
- $G_1$ représente un atome d'hydrogène (qui peut être obtenu à partir d'un produit de formule générale (III) dans laquelle $G_1$ représente un groupement protecteur d'une fonction amino tel qu'un radical benzyle, benzyloxycarbonyle, *tert*-butoxycarbonyle ou vinyloxycarbonyle par hydrogénolyse en présence d'un catalyseur tel que le palladium sur charbon, lorsque $G_1$ représente un radical benzyle ou benzytoxycarbonyle, ou par hydrolyse en milieu acide, lorsque $G_1$ représente un radical *tert*-butoxycarbonyle, vinyloxycarbonyle ou benzyloxycarbonyle).

Dans ce qui suit, la définition pour laquelle $G_1$ représente un radical benzyle n'est donnée qu'à titre illustratif, et il est évident à l'homme de l'art d'adapter les divers protocoles aux autres groupes protecteurs d'une fonction amino tel qu'un radical benzyle, benzyloxycarbonyle, *tert*-butoxycarbonyle ou vinyloxycarbonyle

Généralement, la réaction du produit de formule générale (II) sous forme acide sur le produit de formule générale (III) est effectuée en opérant dans un solvant organique tel qu'un hydrocarbure aliphatique halogéné tel que le dichlorométhane en présence d'un agent de condensation tel que le chlorhydrate de 1-éthyl-3-[3-(diméthylamino) propyl]carbodiimide, le 1,3-dicyclohexylcarbodiimide ou l'hexafluorophosphate de benzotriazol-1-yloxytris (diméthylamino)phosphonium et éventuellement d'un agent d'activation tel que l'hydroxybenzotriazole à une température comprise entre 0°C et la température de reflux du mélange réactionnel.

Généralement, la réaction du produit de formule générale (II) sous forme d'ester méthylique sur un produit de formule générale (III) est effectuée en opérant dans un solvant organique tel que le dioxane ou un hydrocarbure aliphatique halogéné tel que le dichlorométhane à une température comprise entre 0°C et la température de reflux du mélange réactionnel.

Généralement, la réaction du produit de formule générale (II) sous forme d'halogénure sur le produit de formule générale (III) est effectuée en opérant dans un solvant organique tel qu'un hydrocarbure aliphatique halogéné tel que le dichlorométhane en présence d'une base (amine aliphatique tertiaire) à une température comprise entre 0°C et la température de reflux du mélange réactionnel.

Généralement, la réaction du produit de formule générale (II) sous forme d'anhydride sur le produit de formule générale (III) est effectuée en opérant dans un solvant organique tel qu'un hydrocarbure aliphatique halogéné comme

le dichlorométhane en présence d'une base (amine aliphatique tertiaire, pyridine ou 4-diméthylaminopyridine) à une température comprise entre 0 et 50°C.

En outre , à l'issue de la réaction de II sur III, on peut éventuellement, lorsque R représente ou contient un radical $-COOR_6$ ou $-PO(OR_9)_2$ avec $R_6$ et $R_9$ représentant un radical alcoyle,

- procéder à une saponification du produit obtenu pour obtenir un produit de formule générale (I) dans laquelle R représente ou contient un radical carboxy ou
- procéder à la transformation au moyen d'agent nucléophile du produit obtenu pour obtenir un produit de formule générale (I) dans laquelle R représente ou contient un radical $-PO_3H_2$.

Généralement la saponification d'un produit de formule générale (I) dans laquelle R représente ou contient un ester de formule générale $-COOR_6$ en produit de formule générale (I) dans laquelle R représente ou contient un radical carboxy est effectuée au moyen d'une base minérale telle que la soude ou la potasse ou le carbonate de sodium dans un solvant organique tel qu'un alcool comme le méthanol ou l'éthanol ou tel qu'un éther comme le dioxane, à une température comprise entre 20°C et le reflux du solvant.

Généralement la transformation d'un produit de formule générale (I) dans laquelle R représente ou contient un radical $PO(OR_9)_2$ en produit de formule générale (I) dans laquelle R représente ou contient un radical $PO_3H_2$ s'effectue par action d'un agent nucléophile tel qu'un halogénure (iodure) de trialcoylsilyle (triméthylsilyle) ou d'un halogénure de sodium ou de lithium (iodure de sodium) en présence d'un trialcoylhalogénosilane (triméthylchlorosilane, triméthylbromosilane) en opérant dans un solvant tel que le tétrachlorure de carbone ou l'acétonitrile à une température comprise entre 0 et 50°C ou par chauffage avec un halogénure de métal alcalin (iodure de sodium), suivi d'une hydrolyse.

La présente invention concerne également les produits de formule générale (III) :

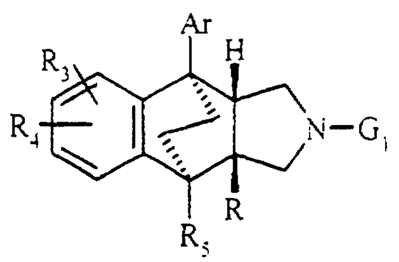

(III)

dans laquelle:

- Ar, R, $R_3$, $R_4$ et $R_5$ sont définis selon la formule générale I et
- $G_1$ représente un atome d'hydrogène ou un groupe protecteur d'une fonction amino, tel que benzyl.

Selon l'invention, les nouveaux produits de formule générale (I) dans laquelle:

- Y représente un atome d'oxygène ou de soufre et
- X représente un atome d'oxygène

peuvent être obtenus par action d'un halogénoformiate ou d'un halogénothioformiate de formule générale (IV) :

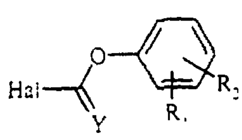

(IV)

dans laquelle :

- Y représente un atome d'oxygène ou de soufre,
- $R_1$ et $R_2$ sont définis comme précédemment et

- Hal représente un atome d'halogène,

sur un produit de formule générale (III).

Généralement, la réaction de l'halogénure de formule générale (IV) sur le produit de formule générale (III) est effectuée en milieu organique ou hydro-organique tel qu'un mélange dioxane-eau en présence d'une base minérale (soude) ou organique (triéthylamine) à une température comprise entre 0 et 50°C.

De la même façon que précédemment, lorsque R représente ou contient un radical $-COOR_6$ ou $-PO(OR_9)_2$, il est possible de saponifier le produit obtenu pour obtenir un produit de formule générale (I) dans laquelle R représente ou contient un radical carboxy ou de transformer au moyen d'agent nucléophile le produit obtenu en un produit de formule générale (I) dans laquelle R représente ou contient un radical $-PO_3H_2$.

Selon l'invention, les nouveaux produits de formule générale (I) dans laquelle :

- Y représente un atome d'oxygène ou de soufre et
- X représente un groupement NH

peuvent être obtenus par action d'un isocyanate ou d'un isothiocyanate de formule générale (V) :

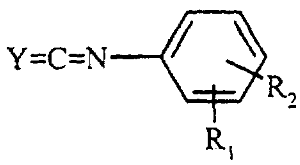

(V)

dans laquelle :

Y représente un atome d'oxygène ou de soufre et
$R_1$ et $R_2$ sont définis selon la formule générale I

sur un produit de formule générale (III) tel que défini précédemment.

Généralement, la réaction du produit de formule générale (V) sur le produit de formule générale (III) est effectuée dans un solvant organique inerte tel que le tétrahydrofurane ou le toluène en présence d'un agent d'activation tel que la 4-diméthylamino-pyridine ou la 4-pyrrolidino-pyridine à une température comprise entre 0 et 50°C.

La réaction de V sur II est suivie éventuellement, lorsque R représente ou contient un radical $-COOR_6$ ou $-PO(OR_9)_2$ dans lesquels $R_6$ ou $R_9$ représentent un radical alcoyle, de la saponification du produit obtenu pour obtenir un produit de formule générale (I) dans laquelle R représente ou contient un radical carboxy ou de la transformation au moyen d'agent nucléophile du produit obtenu pour obtenir un produit de formule générale (I) dans laquelle R représente ou contient un radical $-PO_3H_2$. La transformation éventuelle des radicaux $-COOR_6$ et $PO(OR_9)_2$ respectivement par des radicaux carboxy et $PO_3H_2$ s'effectue dans les conditions décrites précédemment.

Selon l'invention, les nouveaux produits de formule générale (I) dans laquelle :

- R représente un radical de formule générale

$$-(CH_2)_m-X_1-(CH_2)_n-Z$$

avec $X_1$, m et n définis comme précédemment et
Z représentant un radical $-COOR_6$ avec $R_6$ représentant un radical alcoyle droit ou ramifié contenant 1 à 3 atomes de carbone,
peuvent être obtenus par estérification d'un produit de formule générale (I) dans laquelle Z représente un radical carboxy.

Généralement, l'estérification est effectuée au moyen d'un alcool de formule générale $R_6$-OH dans laquelle $R_6$ est défini comme précédemment en opérant en milieu acide ou au moyen d'un halogénure d'alcoyle de formule générale $R_6$-Hal dans laquelle Hal représente un atome d'halogène (iode) en opérant en milieu alcalin (carbonate de métal alcalin ou alcalino-terreux tel que le carbonate de césium) en opérant dans un solvant organique tel que le diméthylformamide à une température comprise entre 0 et 50°C.

Selon l'invention, les nouveaux produits de formule générale (I) dans laquelle:

R représente un radical de formule générale

$$-(CH_2)_m-X_1-(CH_2)_n-Z$$

dans laquelle:
$X_1$, m et n sont définis comme précédemment et
Z représente un radical $-CON(R_7)(R_8)$
dans lequel :
$R_7$ représente un atome d'hydrogène ou un radical alcoyle droit ou ramifié contenant 1 à 6 atomes de carbone et
$R_8$ représente un atome d'hydrogène ou un radical alcoyle droit ou ramifié contenant 1 à 6 atomes de carbone éventuellement substitué par un radical amino, alcoylamino contenant 1 à 4 atomes de carbone, dialcoylamino dont chaque partie alcoyle contient 1 à 4 atomes de carbone, alcoxy contenant 1 à 4 atomes de carbone, alcoylthio contenant 1 à 4 atomes de carbone, alcoyloxycarbonyle dont la partie alcoyle contient 1 à 4 atomes de carbone, carboxy, cyano, phényle éventuellement substitué par un ou plusieurs radicaux identiques ou différents choisis parmi les radicaux alcoxy contenant 1 à 4 atomes de carbone ou trifluorométhyle, naphtyl-1 ou -2, furyl-2 ou -3, thiényl-2 ou -3, imidazolyl-4 ou -5 ou thiazolyl-4 ou -5, pyridyl-2, -3 ou -4, ou un radical indanyle ou chromanyle
ou bien
$R_7$ représente un atome d'hydrogène ou un radical alcoyle droit ou ramifié contenant 1 à 6 atomes de carbone et
$R_8$ représente un radical hydroxy, amino, alcoyloxy contenant 1 à 6 atomes de carbone en chaîne droite ou ramifiée éventuellement substitué par un radical phényl, alcoylamino ou dialcoylamino dont les parties alcoyles contiennent de 1 à 4 atomes de carbone,

ou bien préférentiellement dans lequel

- $R_7$ représente un atome d'hydrogène ou un radical alcoyle droit ou ramifié contenant 1 à 6 atomes de carbone et
- $R_8$ représente

  - un atome d'hydrogène,
  - un radical hydroxy,
  - un radical arylsulfonyle, tel que phénylsulfonyle, éventuellement substitué par un ou plusieurs atomes ou radicaux, identiques ou différents, choisis parmi les atomes d'halogène et les radicaux alcoyles, alcoyloxy, avec, pour ces radicaux alcoyl contenant 1 à 4 atomes de carbone,
  - un hétérocycle de 5 à 7 chaînons incorporant un ou plusieurs hétéroatomes choisis parmi les atomes d'azote, d'oxygène ou de soufre. ledit hétérocycle pouvant être lié par un hétéroatome,
  - un radical amino éventuellement substitué par un ou deux radicaux, identiques ou différents choisis parmi les radicaux

    - alcoyle contenant 1 à 4 atomes de carbone.
    - aryle, tel que phényle, éventuellement substitué par un ou plusieurs radicaux, identiques ou différents, choisis parmi les radicaux alcoyle, alcoyloxy avec pour ces radicaux, alcoyl contenant 1 à 4 atomes de carbone,
    - hétérocyclyle de 5 à 7 chaînons et contenant un ou plusieurs héléroatomes choisis parmi les atomes d'azote, d'oxygène et de soufre,
    - arylcarbonyle, tel que benzoyle, éventuellement substitué par un ou plusieurs radicaux, identiques ou différents, choisis parmi les radicaux alcoyle, alcoyloxy avec pour ces radicaux, alcoyl contenant 1 à 4 atomes de carbone,

  - un radical alcoyloxy contenant 1 à 6 atomes de carbone en chaîne droite ou ramifiée éventuellement substitué par un radical phényle,
  - un radical alcoyle droit ou ramifié contenant 1 à 6 atomes de carbone, tel que méthyle, éventuellement substitué par un radical amino, alcoylamino, dialcoylamino, hydroxy, alcoxy contenant 1 à 4 atomes de carbone, mercapto, alcoylthio, alcoyloxycarbonyle, carboxy, cyano, un radícal aromatique mono- ou polycyclique et ayant de 5 à 12 chaînons, incorporant ou non, un ou plusieurs hétéroatomes choisis parmi les atomes d'oxygène, d'azote et de soufre éventuellement substitué, ledit radical aromatique pouvant être notamment le radical 2-, ou 3-, ou 4- pyridyle, préférentiellement le 3-pyridyle ou le 4-pyridyle, ou le N-oxyde de pyridine, ou pouvant être également un radical phényle éventuellement substitué par un ou plusieurs atomes d'halogène ou par un ou plusieurs groupements hydroxy, amino, ou trifluorométhyle, ou par un ou plusieurs radicaux alcoyle ou

alcényle, alcoxy, alcoylthio, alcoylamino, alcoylcarbonyle ou alcoxycarbonyle en C2 à C4, carbamoyle, alcoylcarbamoyle ou dialcoylcarbamoyle dont la partie alcoyle est en C1 à C8, formyle ou encore un radical naphtyle-1 ou -2,
préférentiellement R- représente un atome d'hydrogène, $R_8$
représente un radical méthyle substitué par le radical 3-pyridyle,

peuvent être obtenus par action d'un produit de formule générale :

$$HN(R_7)(R_8)$$

dans laquelle $R_7$ et $R_8$ sont définis comme ci-dessus
sur un produit de formule générale (I) dans laquelle Z représente un radical carboxy.
Il est particulièrement avantageux :

- soit de faire réagir d'abord le chlorure d'oxalyle avec un composé de formule générale (I) dans lequel R représente un radical carboxy en solution dans du dichlorométhane pour former intermédiairement le chlorure d'acide, puis de faire réagir le composé de formule générale $HN(R_7)(R_8)$, éventuellement en présence d'une base telle que la triéthylamine,
- soit de faire réagir directement le composé de formule générale $HN(R_7)(R_8)$ avec un composé de formule générale (I) dans lequel R représente un radical carboxy dans un solvant organique tel qu'un alcool (éthanol) ou un solvant halogéné tel que le dichlorométhane en présence d'un agent de condensation tel que le N,N'-carbonyldiimidazole, le 1,1-dicyclohexylcarbodiimide, le chlorhydrate de 1-éthyl-3-[3-(diméthylamino)propyl]carbodiimide, l'hexafluorophosphate de benzo-triazol-1-yloxytris(diméthylamino) phosphonium à une température comprise entre 0 et 50°C.

Lorsque l'un au moins des symboles $R_7$ et $R_8$ est substitué par un radical amino, il est particulièrement avantageux de le protéger par un groupement protecteur tel qu'un radical *tert*-butoxycarbonyle, benzyloxycarbonyle ou benzyle préalablement à la condensation de l'amine de formule générale $HN(R_7)(R_8)$ sur l'acide approprié puis à remplacer le groupement protecteur par un atome d'hydrogène par exemple par hydrogénolyse au moyen d'hydrogène en présence d'un catalyseur tel que le palladium sur charbon, lorsqu'il représente un radical benzyle ou benzyloxycarbonyle, ou par hydrolyse en milieu acide, lorsqu'il représente un radical *tert*-butoxycarbonyle ou benzyloxycarbonyle.
Lorsque l'un au moins des symboles $R_7$ et $R_8$ est substitué par un radical carboxy, il est particulièrement avantageux de le protéger par un groupement protecteur tel qu'un radical alcoyle éventuellement substitué par un radical phényle tel que le radical benzyle préalablement à la condensation de l'amine de formule générale $HN(R_7)(R_8)$ sur l'acide approprié puis de remplacer le groupement protecteur par un atome d'hydrogène par exemple par hydrogénolyse au moyen d'hydrogène en présence d'un catalyseur tel que le palladium sur charbon ou par saponification dans les conditions décrites ci-dessus.
Lorsque, dans le produit de formule générale (I), $R_7$ représente un atome d'hydrogène ou un radical alcoyle contenant 1 à 6 atomes de carbone et $R_8$ représente un radical alcoyloxy substitué par un radical phényle, le remplacement du radical alcoyloxy, substitué par un radical phényle, par un radical hydroxy, effectué :

- soit par hydrogénolyse en présence d'un catalyseur tel que le palladium sur charbon,
- soit par traitement avec du chlorure d'aluminium en présence d'anisole dans un solvant organique tel que le nitrométhane à une température comprise entre -20°C et l'ambiante lorsque le radical alcoyle subtitué par un radical phényle est un radical benzyle,

permet d'obtenir un produit de formule générale (I) dans laquelle $R_7$ représente un atome d'hydrogène ou un radical alcoyle contenant 1 à 4 atomes de carbone et $R_8$ représente un radical hydroxy.
Selon l'invention, les nouveaux produits de formule générale (I) dans laquelle:

- R représente un radical de formule générale :

$$-NHCO-T$$

dans laquelle T représente un atome d'hydrogène ou un radical alcoyle (1 à 6 atomes de carbone) éventuellement substitué par un radical amino, carboxy. alcoyloxycarbonyle, hydroxy, alcoyloxy, mercapto ou alcoylthio,
peuvent être obtenus par action d'un acide de formule générale

T-CO-OH

dans laquelle T est défini comme ci-dessus
sur un produit de formule générale VI :

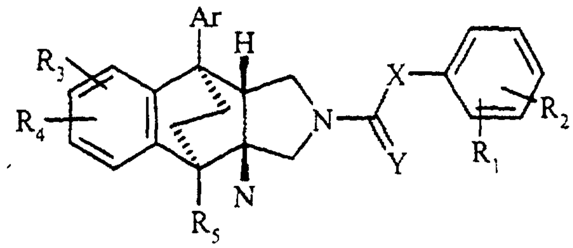

(VI)

dans laquelle :
Ar, $R_1$, $R_2$, $R_3$, $R_4$, $R_5$ X et Y sont définis selon la formule générale I.

Généralement, la réaction de l'acide de formule générale T-CO-OH sous forme acide sur le produit de formule générale (VI) est effectuée en opérant dans un solvant organique tel qu'un hydrocarbure aliphatique halogéné tel que le dichlorométhane en présence d'un agent de condensation tels que le chlorhydrate de 1-éthyl-3-[3-(diméthylamino) propyl]carbodiimide, le 1,1-dicyclohexylcarbodiimide ou l'hexafluorophosphate de benzotriazol-1-oxy tris(diméthylamino)phosphonium, éventuellement en présence d'un agent d'activation tel que l'hydroxybenzotriazole à une température comprise entre 0 et 50°C.

Généralement, la réaction de l'acide de formule générale T-CO-OH sous forme d'halogénure, lorsque T est différent d'un atome d'hydrogène, sur le produit de formule générale (VI) est effectuée en opérant dans un solvant organique tel qu'un hydrocarbure aliphatique halogéné tel que le dichlorométhane en présence d'une base (amine aliphatique tertiaire) à une température comprise entre 0 et 50°C

Généralement, la réaction de l'acide de formule générale T-CO-OH sous forme d'anhydride sur le produit de formule générale (VI) est effectuée en opérant dans un solvant organique tel qu'un hydrocarbure aliphatique halogéné comme le dichlorométhane en présence d'une base (amine aliphatique tertiaire, pyridine ou 4-diméthylaminopyridine) à une température comprise entre 0 et 50°C.

La réaction d'un composé T-CO-OH sur un composé VI peut être suivie éventuellement du remplacement des fonctions esters ou des fonctions amines protégées portées par T respectivement par des radicaux carboxy ou amino dans les conditions décrites précédemment.

Lorsque T est substitué par un radical amino, il est particulièrement avantageux de le protéger par un groupement protecteur tel qu'un radical benzyloxycarbonyle ou *tert*-butoxycarbonyle préalablement à la condensation de l'acide de formule générale T-CO-OH sur l'amine appropriée puis à remplacer le groupement protecteur par un atome d'hydrogène par exemple par hydrogénolyse au moyen d'hydrogène en présence d'un catalyseur tel que le palladium sur charbon ou par hydrolyse en milieu acide.

Lorsque T est substitué par un radical carboxy, il est particulièrement avantageux de le protéger par un groupement protecteur tel qu'un radical méthyle, éthyle ou benzyle préalablement à la condensation de l'acide de formule générale T-CO-OH sur l'amine appropriée puis à remplacer le groupement protecteur par un atome d'hydrogène par exemple par hydrogénolyse au moyen d'hydrogène en présence d'un catalyseur tel que le palladium sur charbon ou par saponification dans les conditions décrites précédemment.

Selon l'invention, les nouveaux produits de formule générale (I) dans laquelle: R représente un radical de formule générale

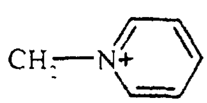

peuvent être obtenus par action d'un excès de pyridine et d'un acide fort ou d'un dérivé de cet acide sur un produit de formule générale :

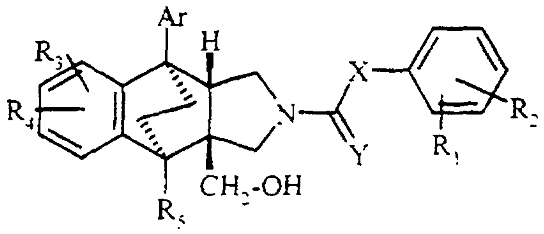

(VII)

dans laquelle Ar, $R_1$, $R_2$, $R_3$, $R_4$, $R_5$, X et Y sont définis selon la formule générale I.

De préférence l'acide fort est l'acide trifluorométhanesulfonique éventuellement en présence d'anhydride trifluorométhanesulfonique.

Selon l'invention, les nouveaux produits de formule générale (I) dans laquelle Y représente un atome de soufre peuvent être obtenus par thionation d'un produit de formule générale (I) dans laquelle Y représente un atome d'oxygène.

Généralement, la thionation est effectuée dans les conditions habituelles au moyen de pentasulfure de phosphore en opérant dans un solvant organique tel que le tétrahydrofurane à une température comprise entre 0 et 50°C.

Selon l'invention, les produits de formule générale (I) dans laquelle l'un des symboles $R_1$ ou $R_2$ représente un radical alcoylcarbonyloxy peuvent être obtenus par acylation d'un produit de formule générale (I) dans laquelle l'un des symboles $R_1$ ou $R_2$ représente un radical hydroxy au moyen d'un acide aliphatique ou d'un dérivé de cet acide tel qu'un halogénure ou l'anhydride dans les conditions habituelles d'estérification.

En ce qui concerne les produits intermédiaires décrits ci-dessus, nous proposons ci-après également des protocoles opératoires et des composés utiles pour les obtenir.

Classiquement, les produits de formule générale (III) dans laquelle R représente un radical carboxy ou un radical de formule générale $COOR_6$ peuvent être obtenus par action de l'acide trifluorométhanesulfonique sur un produit de formule générale (VIII) :

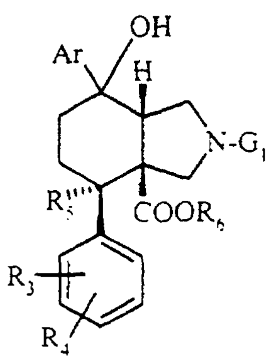

(VIII)

dans laquelle :

- Ar, $R_3$, $R_4$, et $R_5$ sont définis comme précédemment,
- $G_1$ représente un radical benzyle,
- $R_6$ représente un radical alcoyle contenant 1 à 3 atomes de carbone suivie du remplacement du groupe $G_1$ par un atome d'hydrogène

  - soit par hydrogénolyse dans les conditions décrites ci-dessus puis éventuellement selon le cas du remplacement de l'atome d'hydrogéne par un radical *tert*-butoxycarbonyle, par action de l'anhydride *tert*-butoxycarbonyle dans un solvant organique, ou par un radical benzyloxycarbonyle, par action du chlorure de benzyloxycarbonyle dans un solvant organique
  - soit par action d'un chloroformiate d'alcoyle, tel que le chloroformiate de vinyle ou le chloroformiate d'éthyle ou le chloroformiate de 2-chloroéthyle ou le chloroformiate de 2,2,2-trichloroéthyle, dans un solvant organique tel que le dichlorométhane à une température comprise entre 0°C et l'ambiante, suivie de l'hydrolyse acide du carbamate intermédiairement formé, généralement à l'aide d'une solution aqueuse 1 à 6 M d'acide chlorhydrique, éventuellement dans un solvant organique tel qu'un alcool, comme le méthanol ou l'éthanol, ou un

éther comme le tétrahydrofurane ou le dioxane.

Généralement la cyclisation intramoléculaire de type Friedel-Crafts du produit de formule générale (VIII) en produit de formule générale (III) peut être effectuée par l'action d'un excès, de 3 à 15 équivalents molaires, d'un acide fort tel que l'acide trifluorométhanesulfonique, éventuellement en présence d'anhydride trifluorométhanesulfonique en quantité catalytique ou éventuellement ajouté en ajouts successifs, en opérant dans un solvant organique tel que le dichlorométhane à une température comprise entre 0°C et le reflux de quelques minutes à plusieurs jours. Il est également possible d'effectuer la cyclisation intramoléculaire de type Friedel-Crafts par action d'un acide de Lewis, tel que le chlorure d'aluminium ou le tétrachlorure de titane ou le trifluorure de bore, éventuellement sous forme de complexe avec l'oxyde de diéthyle, dans un solvant organique tel que le dichlorométhane ou le nitrométhane ou le nitrobenzène.
Cette réaction peut éventuellement être suivie de la saponification du produit obtenu, et suivie éventuellement selon le cas du remplacement du radical benzyle par un atome d'hydrogène.
Le produit de formule générale (VIII) peut être obtenu quant à lui de manière classique par action d'un dérivé organomagnésien de formule générale Ar-Mg-X dans laquelle Ar est défini comme précédemment et X représente un atome d'halogène, ou d'un organolithien de formule générale Ar-Li dans laquelle Ar est défini comme précédemment sur un produit de formule générale (IX) :

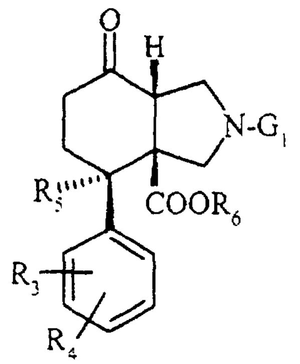

(IX)

dans laquelle $R_3$, $R_4$, $R_5$, $R_6$ et $G_1$ sont définis comme précédemment, dans les conditions habituelles.
Généralement la réaction d'un arylmagnésien, obtenu classiquement et éventuellement en présence de chlorure de cérium (III) anhydre dans les conditions décrites par Imamoto (Tetrahedron Lett., 1985 p.4763), sur le dérivé cétonique de formule générale (IX) est effectuée dans un solvant organique comme l'oxyde de diéthyle ou le tétrahydrofurane, en opérant à une température comprise entre 0°C et le reflux du mélange réactionnel de quelques minutes à 24 heures. Cependant il a été trouvé particulièrement avantageux d'opérer dans le toluène, éventuellement en mélange avec de l'oxyde de diéthyle ou du tétrahydrofurane.
Généralement la réaction d'un aryllithien, obtenu classiquement, sur le dérivé cétonique de formule générale (IX) est effectuée dans un solvant organique comme l'oxyde de diéthyle ou le tétrahydrofurane. en opérant à une température comprise entre -78° et -20°C de quelques minutes à 4 heures. Cependant il a été trouvé particulièrement avantageux d'opérer dans le toluène, éventuellement en mélange avec de l'oxyde de diéthyle ou du tétrahydrofurane.
Avantageusement, il a été développé dans le cadre de la présente invention, un procédé de préparation permettant d'obtenir les composés de formule générale (III) à partir des composés de formule générale (IX) via la formation d'un intermédiaire stable et caractérisable de formule générale (XV) caractérisé par la présence d'une fonction aryléthylénique en position 7.
Plus précisément, les composés de formule générale III

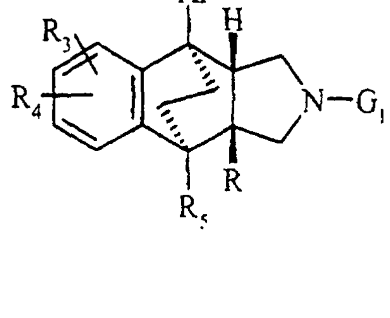

(III)

dans laquelle $R_3$, $R_4$, $R_5$, Ar et R sont tels que définis en formule générale I et G1 représente un radical benzyle peuvent être obtenus à partir de composés de formule générale (IX)

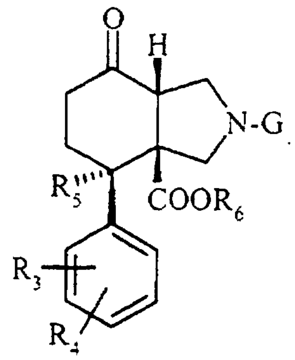

(IX)

dans laquelle $R_3$, $R_4$, $R_5$, $R_6$ et $G_1$ sont tels que définis précédemment via la formation d'un intermédiaire de formule générale (XV)

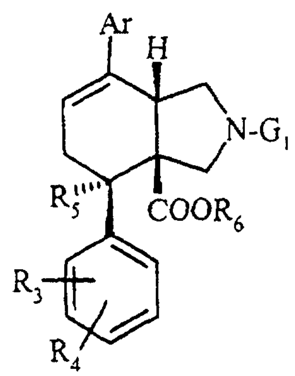

(XV)

dans laquelle $R_3$, $R_4$, $R_5$,$R_6$, Ar et $G_1$ sont tels que définis précédemment

Le protocole opératoire mis au point, implique successivement :

- soit la condensation, en position 7, d'un dérivé cétonique de formule générale (IX) avec de l'hydrazine, pour conduire à une hydrazone, suivie de l'action de l'iode pour conduire, selon la réaction de Barton (J. Chem. Soc. 1962 p.470), à un dérivé iodoéthylènique ; puis d'une réaction de couplage au palladium avec un acide arylboronique, réaction de Suzuki (Tetrahedron Lett. 1979 p.3437), de formule générale $Ar\text{-}B(OH)_2$, ou éventuellement avec l'anhydride trimérique de l'acide aryleboronique, dans laquelle Ar est défini comme précédemment, ou avec un arylstannane, réaction de Stille (Angew. Chem. Int. Ed. Engl., 1986 p.508), de formule générale $Ar\text{-}SnMe_3$ dans laquelle Ar est défini comme précédemment. pour conduire à cet intermédiaire aryléthylénique de formule générale (XV) dont la transformation par cyclisation intramoléculaire de type Friedel-Crafts conduit au produit (III) attendu,
- soit la réaction d'un dérivé cétonique de formule générale (IX) avec de l'anhydride trifluorométhanesulfonique, pour conduire à un triflate d'énol en position 7 (Org. Synth., 1990 p.116) ; puis d'une réaction de couplage au palladium avec un acide arylboronique, réaction de Suzuki (Tetrahedron Lett. 1979 p.3437), de formule générale $Ar\text{-}B(OH)_2$ dans laquelle Ar est défini comme précédemment, ou avec un arylstannane, réaction de Stille (Angew. Chem. Int. Ed. Engl., 1986 p.508), de formule générale $Ar\text{-}SnMe_3$ dans laquelle Ar est défini comme précédemment, pour conduire à cet intermédiaire aryléthylénique de formule générale (XV) dont la transformation par cyclisation intramoléculaire de type Friedel-Crafts conduit au produit (III) attendu.

Généralement le dérivé cétonique de formule générale (IX) est traité par un excès d'hydrate d'hydrazine, de 3 à 20 équivalents molaires, au reflux dans un solvant, tel que l'éthanol, de quelques minutes à quelques heures. L'hydrazone ainsi obtenue est alors agitée avec un excès d'iode, en présence d'une amine tertiaire aliphatique telle que la triéthylamine, à une température voisine de 20°C pendant quelques heures pour conduire à un dérivé iodoéthylénique.

Généralement le dérivé cétonique de formule générale (IX) est traité :

- soit par de l'anhydride trifluorométhanesulfonique en présence d'une base organique, telle que la 2,6-di-*tert*-butyl-4-méthylpyridine, dans un solvant organique tel que le dichlorométhane à une température voisine de l'ambiante pendant quelques heures, selon Stang (Synthesis, 1980 p283),

- soit par un bis(trifluorométhylsulfonyl)amide, comme la N,N-bis(trifluorométhylsulfonyl)aniline selon Mac Murry (Tetrahedron Lett., 1983 p979) ou la 2-[N,N-bis(trifluorométhylsulfonyl)amino]pyridine selon Comins (Tetrahedron Lett., 1992 p979), en présence d'une base comme le diisoprolylamidure de lithium dans un solvant organique comme le dichlorométhane ou le 1,2-diméthoxyéthane,

pour conduire à un triflate d'énol.

Généralement le couplage entre le dérivé iodoéthylènique, ou le triflate d'énol, obtenu précédemment et un acide arylboronique, obtenu classiquement et éventuellement isolé sous forme d'anhydride trimérique, est effectué par agitation dans un système biphasique constitué d'un solvant organique, préférentiellement un mélange de toluène et de méthanol, et d'une solution aqueuse basique, préférentiellement une solution 2N de carbonate de sodium, en présence d'une quantité catalytique de dérivé de palladium(0), préférentiellement du tétrakis(triphénylphosphine)palladium, à une température voisine du refiux pendant quelques heures pour conduire au composé aryléthylènique de formule générale (XV).

Généralement le couplage entre le dérivé iodoéthylènique, ou le triflate d'énol, obtenu précédemment avec un arylestannane, obtenu classiquement, est effectué par agitation dans un solvant organique aprotique polaire, préférentiellement le diméthylformamide ou la N-méthyl-pyrrolidone, en présence d'une quantité catalytique de dérivé de palladium(0), préférentiellement du tétrakis (triphénylphosphine)palladium, à une température comprise entre 50° et 100°C pendant quelques heures pour conduire au composé aryléthylènique de formule générale (XV).

Généralement la cyclisation intramoléculaire de type Friedel-Crafts du composé de formule générale (XV) en produit de formule générale (III) est effectuée dans les conditions décrites précédemment pour la cyclisation intramoléculaire des produits de formule générale (VIII).

Avantageusement, il a en outre été développé, dans le cadre de la présente invention, un second procédé de préparation permettant d'obtenir les composés de formule générale (III) tels que définis précédemment à partir des composés de formule générale (IX) via la formation de l'intermédiaire de formule générale (XV) éventuellement isolable. Ce second procédé est tout particulièrement avantageux lorsque le radical aryle Ar représente un noyau phényle susbtitué, en positions para ou méta-para ou méta-para-méta' par des groupements donneurs d'électrons, ou un radical hétérocyclique naturellement riche en électrons ou un radical hétérocyclique convenablement substitué par des groupements donneurs d'électrons, ce second procédé consiste à faire réagir directement, selon des réactions tandem de cyclisations intermoléculaire puis intramoléculaire de type Friedel-Crafts, un hydrocarbure aromatique ou hétérocyclique Ar-H avec un composé de formule générale (IX) dans un solvant organique en présence d'un excès d'acide fort, tel que l'acide trifluorométhanesulfonique, ou éventuellement d'un acide de Lewis, tel que le chlorure d'aluminium.

Le mode opératoire mis au point consiste à condenser le produit de formule générale (IX) avec un excès d'acide trifluorométhanesulfonique (de 5 à 20 équivalents molaires) dans un solvant organique tel que le dichlorométhane à une température voisine de l'ambiante de quelques heures à plusieurs jours. Selon le nombre d'équivalents molaires et la concentration de l'acide trifluorométhane-sulfonique ainsi que la nature du radical Ar et des substituants qu'il porte, cette réaction conduit soit directement aux composés de formule générale (III) soit intermédiairement aux composés de formule générale (XV) qui sont alors cyclisés comme décrit précédemment en composés de formule générale (III).

En outre, les composés de formule générale (I) ou (III) peuvent être obtenus par fonctionnalisation des substituants du cycle aromatique Ar des composés de formule générale (I) ou (III) correspondants, par application ou adaptation des méthodes connues de fonctionnalisations habituelles, telles que, et à titre non limitatif : les réactions de substitution fonctionnelle (par exemple le remplacement d'un atome d'halogène par un groupement cyano par un couplage au palladium), les réactions de déalkylation (par exemple par $BBr_3$), les réactions d'alkylation (notamment les réactions d'alkylation-cyclisation par action de $BBr_2$).

Un autre objet de la présente invention se rapporte également à ces composés de formule générale (XV):

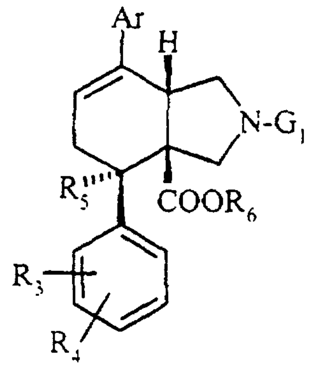

(XV)

dans laquelle $R_3$, $R_4$, $R_5$ et Ar sont tels que définis en formule générale I, $G_1$ représente un radical benzyle et $R_6$ représente un radical alcoyle contenant 1 à 3 atomes de carbone sous forme racémique ainsi que leurs isomères optiques.

Le produit de formule générale (IX), sous forme racémique ou ses isomères optiques, dans laquelle $R_3$, $R_4$, $R_5$, $R_6$ et Ar sont tels que définis en formule générale I, $G_1$ représente un radical benzyle peut être obtenu par action d'une N-trialcoylsilylméthyl-N-alcoxyméthyl-amine portant un groupement protecteur de la fonction amine tel qu'un radical benzyle, comme la N-triméthylsilylméthyl-N-n.butoxyméthyl-benzylamine qui peut être préparée dans les conditions décrites dans Chem. Pharm. Bull., 276 (1985), sur un dérivé de cyclohexènone de formule générale :

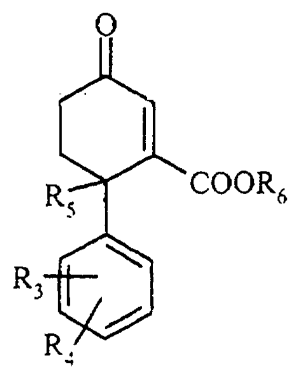

(X)

dans laquelle $R_3$, $R_4$, $R_5$ et $R_6$ sont définis comme ci-dessus, sous forme racémique ou ses isomères optiques.

Généralement, la réaction est effectuée dans un solvant organique tel qu'un hydrocarbure aliphatique halogéné comme le dichlorométhane en présence d'un acide fort tel que l'acide trifluoroacétique à une température comprise entre 0°C et le reflux du mélange réactionnel.

Les produits de formule générale (X) peuvent être obtenus par estérification des acides 3-oxo-6-phényl-cyclohex-1-ène-1-carboxyliques dans lesquels $R_3$, $R_4$ et $R_5$ sont définis comme ci-dessus au moyen d'un alcool aliphatique contenant 1 à 3 atomes de carbone en présence d'un acide minéral tel que l'acide chlorhydrique ou l'acide sulfurique à une température comprise entre 0°C et la température de reflux du mélange réactionnel, ou au moyen d'un halogénure d'alcoyle (iodure), en présence d'une base organique, telle que le 1,8-diazabicyclo[5.4.0]undéc-7-ène, ou minérale, telle que le carbonate de césium, en opérant dans un solvant choisi parmi le tétrahydrofurane, le diméthylformamide, l'acétone ou le dioxane.

Les acides 3-oxo-6-phényl-cyclohex-1-éne-1-carboxyliques, dans lesquels $R_3$, $R_4$ et $R_5$ sont définis comme ci-dessus, peuvent être obtenus à partir des acides 3-oxo-6-phényl-cyclohexane-1-ol-1-carboxyliques dans lesquels $R_3$, $R_4$ et $R_5$ sont définis comme ci-dessus, soit par deshydratation thermique, par chauffage à une température voisine de 190°C (selon J. Org. Chem., 1971 p.3707) ou par chauffage au reflux du toluène en présence d'acide p.toluènesulfonique, soit par action d'une base minérale telle que la soude à une température comprise entre 0 et 50°C.

Les acides 3-oxo-6-phényl-cyclohexane-1-ol-1-carboxyliques, dans lesquels $R_3$, $R_4$ et $R_5$ sont définis comme ci-dessus, peuvent être obtenus par action des acides phénylpyruviques, ou éventuellement des esters correspondants, plus particulièrement dans le cas où $R_5$ ne représente pas un atome d'hydrogène, dont le noyau phényle est éventuellement substitué par des substituants définis par $R_3$ et $R_4$ sur la méthylvinylcétone en opérant généralement en milieu hydro-alcoolique tel qu'un mélange méthanol-eau en présence d'une base minérale telle que la soude (selon

J. Org. Chem., 1971, 3707).

Les acides phénylpyruviques, dont le noyau phényle est éventuellement substitué par des substituants définis par $R_3$ et $R_4$, peuvent être obtenus, plus particulièrement dans le cas où $R_5$ représente un atome d'hydrogène, soit par hydrolyse des acides a-acétamidocinnamiques correspondants, par chauffage dans l'acide chlorhydrique selon Org. Synth. 1943 p.519, soit par hydrolyse des benzalhydantoines correspondantes, par chauffage dans la soude à 20 % selon Org. Synth. Coll. Vol. V p.627.

Les acides a-acétamidocinnamiques, dont le noyau phényle est éventuellement substitué par des substituants définis par $R_3$ et $R_4$, peuvent être obtenus, à partir des benzaldéhydes correspondants selon Org. Synth. 1939 pl. par action de la N-acétylglycine au reflux de l'anhydride acétique en présence d'acétate de sodium. Les azlactones intermédiaires ainsi obtenues sont ensuite hydrolysées en acides a-acétamidocinnamiques par chauffage au reflux dans l'acétone aqueux.

Les benzalhydantoines, dont le noyau phényle est éventuellement substitué par des substituants définis par $R_3$ et $R_4$, peuvent être obtenues par chauffage des benzaldéhydes correspondants. selon Org. Synth. Coll. Vol V p. 267, avec de l'hydantoine en présence d'une base organique telle que la pipéridine à une température voisine de 130°C.

Les phénylpyruvates, dont le noyau phényle est éventuellement substitué par des substituants définis par $R_3$ et $R_4$, peuvent être obtenus, plus particulièrement dans le cas où $R_5$ ne représente pas un atome d'hydrogène mais un radical alcoyle ou alcoylthio, à partir des acides 2-phénylalcanoiques, dont le noyau phényle est éventuellement substitué par des substituants définis par $R_3$ et $R_4$, par action du dianion correspondant, obtenu généralement par action d'une base organique telle que le n-butyllithium sur l'acide, sur l'oxalate d'éthyle à une température voisine de - 70°C, suivie de décarboxylation, en opérant dans les conditions décrites dans Tetrahedron Lett., 1981, 2439-42.

Les produits de formule générale (III)

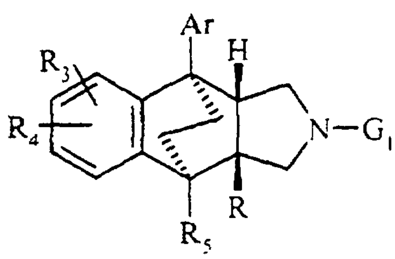

(III)

dans laquelle R représente un radical

$$(CH_2)_m\text{-}X_1(CH_2)_n\text{-}Z$$

dans lequel :

m est égal à 0,
X représente une liaison,
n est égal à 0 et
Z représente un radical $-COOR_6$ ou $-CON(R_7)(R_8)$

peuvent être obtenus à partir d'un produit de formule générale (VIII) dans laquelle R représente un radical carboxy par estérification et amidification dans les conditions décrites ci-dessus, suivie d'une cyclisation par action de l'acide trifluorométhanesulfonique dans les conditions décrites ci-dessus.

Les produits de formule générale (III) dans laquelle R représente un radical

$$(CH_2)_m\text{-}X_1(CH_2)_n\text{-}Z$$

dans lequel :

m est égal à 1
$X_1$ représente une liaison,
n est égal à 0 et
Z représente un radical $-COOR_6$, $-CON(R_7)(R_8)$ ou $PO(OR_9)_2$

peuvent être obtenus à partir d'un produit de formule générale :

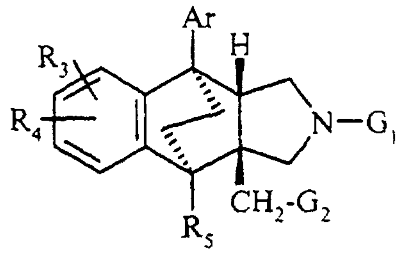

(XI)

dans laquelle Ar, $R_3$, $R_4$ et R sont définis comme précédemment et, $G_1$ représente un groupement protecteur de la fonction amine (benzyle, benzyloxycarbonyle ou *tert*-butoxycarbonyle et $G_2$ représente un groupement partant tel qu'un radical trifluorométhylsulfonyloxy.

Plus particulièrement, pour obtenir un produit de formule générale (III) dans laquelle R représente un radical

$$(CH_2)_m\text{-X: }(CH_2)_n\text{-Z}$$

dans lequel :

- m est égal à 1,
- $X_1$ représente une liaison,
- n est égal à 0 et
- Z représente un radical carboxy, $-COOR_6$ dans lequel $R_6$ représente un radical alcoyle ou $-CON(R_7)(R_8)$,

il est particulièrement avantageux de passer par l'intermédiaire du nitrile correspondant, qui peut être obtenu par action d'un cyanure de métal alcalin sur le produit de formule générale (XI) en opérant dans un solvant organique polaire tel que le diméthylsulfoxyde à une température comprise entre 0 et 50°C, qui est hydrolysé en acide correspondant qui peut alors être estérifié ou amidifié dans les conditions habituelles.

Plus particulièrement, pour obtenir un produit de formule générale (III) dans laquelle R représente un radical

$$(CH_2)_m\text{-}X_1(CH_2)_n\text{-Z}$$

dans lequel :

- m est égal à 1,
- $X_1$ représente une liaison,
- n est égal à 0 et
- Z représente un radical $-PO(OR_9)_2$,

il est particulièrement avantageux de faire réagir un trialcoylphosphite sur un produit de formule générale (XI), puis à transformer éventuellement le phosphonate obtenu en acide phosphonique correspondant.

Les produits de formule générale (III)

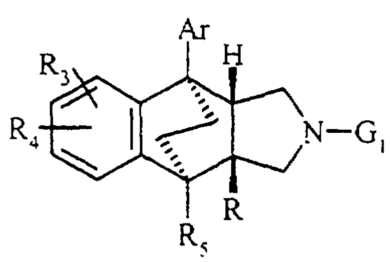

(III)

dans laquelle :

- R représente un radical $(CH_2)_m$-$X_1$-$(CH_2)_n$-Z avec m égal à 1, $X_1$ représentant un atome d'oxygène ou de soufre, n égal à 1 ou 2 et Z représentant un radical carboxy,-$COOR_6$ dans lequel $R_6$ représente un radical alcoyle ou -CON($R_7$)($R_8$) et
- $G_1$ représente un groupement protecteur de la fonction amine peuvent être obtenus par action d'un ester ou d'un amide de formule générale :

$$H\text{-}X_1\text{-}(CH_2)_n\text{-}Z$$

dans laquelle $X_1$, n et Z sont définis comme ci-dessus

sur un produit de formule générale (XI), en opérant dans un solvant organique anhydre tel que le dioxane en présence d'un hydrure de métal alcalin tel que l'hydrure de sodium, suivie éventuellement selon le cas de la saponification du produit de formule générale (III) ainsi obtenu.

Les produits de formule générale (III) dans laquelle :

- m est égal à 1,
- $X_1$ représente une liaison,
- n est égal à 1 avec le groupement méthylène pouvant être substitué par un radical carboxy ou alcoxycarbonyle ou carbamoyle ou alcoylcarbamoyle ou dialcoylcarbamoyle,
- $G_1$ représente un groupement protecteur de la fonction amine et
- Z représente un radical carboxy, -$COOR_6$ dans lequel $R_6$ représente un radical alcoyle ou -CON($R_7$)($R_8$),

peuvent être obtenus par action d'un acide malonique préalablement anionisé, ou d'un dérivé de l'acide malonique, de préférence un diester, sur un produit de formule générale (XI) en opérant dans un solvant organique anhydre tel que le dioxane en présence d'un hydrure de métal alcalin tel que l'hydrure de sodium, à une température comprise entre 0°C et la température de reflux du mélange réactionnel, suivie selon le cas de la saponification, de l'estérification, de l'amidification ou de la décarboxylation du produit de formule générale (III) ainsi obtenu.

Les produits de formule générale (III) dans laquelle :

R représente un radical -NH-CO-T dans lequel T est défini comme précédemment peuvent être obtenus par amidification d'un produit de formule générale (III) dans laquelle

- $R_3$ est défini comme précédemment,
- $G_1$ représente un groupement protecteur de la fonction amine et
- R représente un radical amino au moyen d'un acide de formule générale T-CO-OH

dans laquelle T est défini comme précédemment dans les conditions décrites ci-dessus pour l'amidification d'un produit de formule générale (VI).

Les produits de formule générale (III) dans laquelle R représente un radical amino ou les produits de formule générale (VI) peuvent être obtenus selon les méthodes qui permettent de transformer un radical carboxy en radical amino sans toucher au reste de la molécule.

Généralement, la fonction carboxy d'un produit de formule générale (III) ou (I) est transformée en radical amino en passant par un isocyanate qui peut être obtenu par pyrolyse de l'azoture d'acide qui peut lui-même être obtenu par action d'un azoture de métal alcalin sur l'halogénure d'acide correspondant. Classiquement l'isocyanate intermédiaire ainsi obtenu est condensé avec de l'alcool benzylique, puis le carbamate de benzyle obtenu est transformé en radical amino soit par hydrogénolyse soit par hydrolyse acide dans les conditions décrites précédemment.

Les produits de formule générale (III) dans laquelle R représente

- un radical

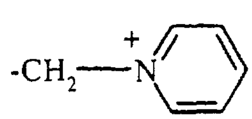

peuvent être obtenu par action dela pyridine et d'un acide fort ou d'un dérivé de cet acide sur un produit de formule générale (XII) :

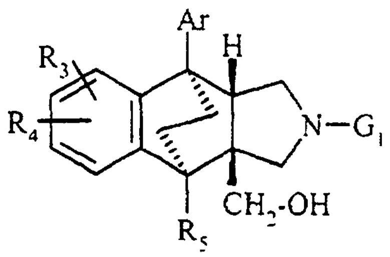

(XII)

dans laquelle Ar, $R_3$, $R_4$ et $R_5$ sont définis comme précédemment et $G_1$ représente un groupement protecteur de la fonction amine.

Les produits de formule générale (VII) ou de formule générale (XII) peuvent être obtenus respectivement par réduction d'un produit de formule générale (I) ou d'un produit de formule générale (III) dans laquelle R représente un radical de formule générale $-COOR_6$ dans laquelle $R_6$ représente de préférence un radical alcoyle contenant 1 à 3 atomes de carbone.

Généralement, la réduction est effectuée au moyen d'un hydrure double de lithium et d'aluminium en opérant dans un solvant organique tel qu'un éther comme le tétrahydrofurane à une température comprise entre 0 et 50°C.

Selon l'invention, les produits de formule générale (I) dans laquelle Ar, $R_1$, $R_2$, $R_3$, $R_4$, $R_5$, X et Y sont définis comme précédemment et R représente un radical $COOR_6$ dans lequel $R_6$ est défini comme précédemment peuvent être également obtenus à partir d'un produit de formule générale (IX) dans laquelle $R_3$, $R_4$, $R_5$ et $R_6$ sont définis comme précédemment et $G_1$ représente un atome d'hydrogène.

Selon l'invention, le produit de formule générale (IX)

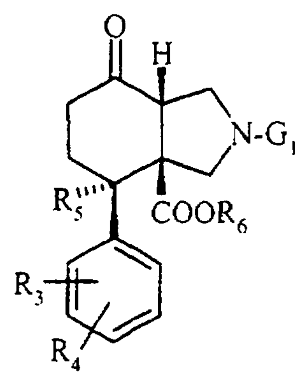

(IX)

dans laquelle $G_1$ représente un atome d'hydrogène est obtenu à partir du produit de formule générale (IX) dans laquelle $G_1$ représente un groupement protecteur de la fonction amino; dans les conditions décrites précédemment pour la préparation d'un produit de formule générale (III) dans laquelle $G_1$ représente un atome d'hydrogène.

Le produit de formule générale (IX) dans laquelle $G_1$ représente un atome d'hydrogène est transformé en produit de formule générale :

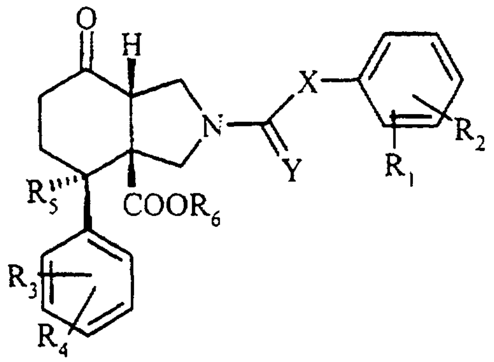

(XIII)

dans laquelle $R_1$, $R_2$, $R_3$, $R_4$, $R_5$, $R_6$, X et Y sont définis comme précédemment, en opérant, selon les significations de X et Y, de la manière suivante :

- les produits de formule générale (XIII) dans laquelle Y représente un atome d'oxygène ou de soufre et X représente un groupement -CO-, méthylène, vinyldiyle, alcèn-1,1-diyle ou cycloalcan-1,1-diyle, peuvent être obtenus par action d'un acide de formule générale (II), ou de son chlorure, ou de son anhydride sur un produit de formule générale (IX) dans laquelle $G_1$ représente un atome d'hydrogène en opérant dans les conditions décrites précédemment pour l'action d'un produit de formule générale (II) sur un produit de formule générale (III) dans laquelle $G_1$ représente un atome d'hydrogène,
- les produits de formule générale (XIII) dans laquelle Y représente un atome d'oxygène ou de soufre et X représente un atome d'oxygène peuvent être obtenus par action d'un halogénoformiate ou d'un halogénothioformiate de formule générale (IV) sur un produit de formule générale (IX) dans laquelle $G_1$ représente un atome d'hydrogène en opérant dans les conditions décrites précédemment pour l'action d'un produit de formule générale (IV) sur un produit de formule générale (III) dans laquelle $G_1$ représente un atome d'hydrogène,
- les produits de formule générale (XIII) dans laquelle Y représente un atome d'oxygène ou de soufre et X représente un groupement NH peuvent être obtenus par action d'un isocyanate ou d'un isothiocyanate de formule générale (V) sur un produit de formule générale (IX) dans laquelle $G_1$ représente un atome d'hydrogène en opérant dans les conditions décrites précédemment pour l'action d'un produit de formule générale (V) sur un produit de formule générale (III) dans laquelle $G_1$ représente un atome d'hydrogène.

Le produit de formule générale (XIII) est transformé en produit de formule générale :

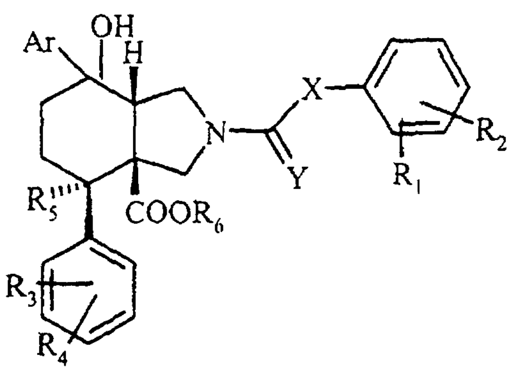

(XIV)

dans laquelle Ar, $R_1$, $R_2$, $R_3$, $R_4$, $R_5$, $R_6$, X et Y sont définis comme précédemment. par action d'un dérivé métallique de formule générale Ar-Mg-X ou Ar-Li dans laquelle X représente un atome d'halogène sur un produit de formule générale (XIII) en opérant dans les conditions décrites précédemment pour l'action d'un dérivé organo-magnésien ou organo-lithien de formule générale Ar-Mg-X ou Ar-Li sur un produit de formule générale (IX).

Le produit de formule générale (XIV) est transformé en produit de formule générale (I) dans laquelle Ar, $R_1$, $R_2$, $R_3$, $R_4$, $R_5$, $R_6$, X et Y sont définis comme précédemment, par action de l'acide trifluorométhanesulfonique, ou d'un acide de Lewis, sur le produit de formule générale (XIV) en opérant dans les conditions décrites précédemment pour l'action de l'acide trifluorométhanesulfonique, ou d'un acide de Lewis, sur un produit de formule générale (VIII).

L'action d'un hydrocarbure aromatique ou d'un hétérocycle aromatiqueAr-H, tel que défini précédemment, en présence d'acide trifluorométhanesulfonique ou d'un acide de Lewis, sur un produit de formule générale (XIII) dans laquelle $R_1$, $R_2$, $R_3$, $R_4$, $R_5$, $R_6$, X et Y sont définis comme précédemment, conduit à un produit de formule générale (I) dans laquelle Ar est défini comme précédemment et R represente un radical $COOR_6$ dans lequel $R_6$ représente un radical alcoyle de 1 à 3 atomes de carbone.

Selon l'invention, les produits de formule générale (I) dans laquelle :

- Ar, $R_1$, $R_2$, $R_3$, $R_4$, $R_5$, X et Y sont définis comme précédemment et
- R représente un radical

$$CH_2)_m\text{-}X_1\text{-}(CH_2)_n\text{-}Z$$

dans laquelle m, n, $X_1$ et Z sont définis comme précédemment peuvent aussi être préparés à partir d'un produit de formule générale (VII) dans les conditions décrites précédemment pour la préparation des produits de formule générale (III) à partir d'un produit de formule générale (XI), après remplacement du radical hydroxy du produit de formule générale (VII) par un groupement partant tel qu'un radical trifluorométhanesulfonyloxy.

Les mélanges réactionnels obtenus par les divers procédés décrits précédemment sont traités suivant des méthodes classiques physiques (évaporation, extraction, distillation, chromatographie, cristallisation par exemple) ou chimiques (formation de sels par exemple).

Les composés de formule (I) peuvent être éventuellement transformés en sels d'addition avec un acide minéral ou organique par action d'un tel acide au sein d'un solvant organique tel qu'un alcool, une cétone, un éther ou un solvant chloré. Ces sels font également partie de l'invention.

Les isomères optiques des produits de formule générale (I) peuvent être obtenus selon les méthodes habituelles de séparation à partir du produit racémique correspondant. Il est particulièrement avantageux d'effectuer la séparation par chromatographie en phase liquide à haute performance en utilisant une phase stationnaire chirale de type Pirkle modifiée en éluant avec un solvant convenable.

Comme phase stationnaire chirale peut être utilisée de préférence une phase dont le sélecteur chiral, qui est de préférence la 3,5-dinitro-phénylalanine, est éloigné de la silice par un bras aminoalcoyle contenant 3 à 14 atomes de carbone fixé sur les fonctions amines d'une silice aminopropyle et dont les fonctions silanols libres sont bloquées par des radicaux trialcoylsilyles.

Cette phase chirale peut être définie par la structure suivante :

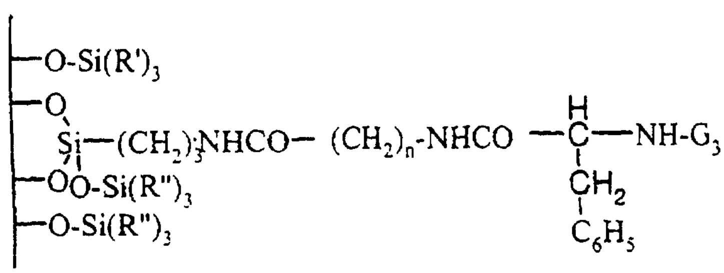

dans laquelle les symboles R', identiques ou différents, et R", identiques ou différents, représentent des radicaux alcoyles contenant 1 à 10 atomes de carbone, $G_3$ représente un groupe électro-attracteur et n représente un nombre entier compris entre 3 et 13 inclusivement, dont la porosité est voisine de 100 Å

La phase chirale peut être préparée par action sur une silice ammopropyle de l'anhydride d'un acide aminoalcanoïque contenant 4 à 14 atomes de carbone dont la fonction amine est protégée par un groupement protecteur tel que le radical *tert*-butoxycarbonyle, suivie du blocage d'une partie des fonctions silanols par des radicaux $Si(R')_3$ tels que définis précédemment, puis, après élimination des groupements protecteurs de la fonction amine, de l'amidification au moyen d'un aminoacide de formule générale :

$HO\text{-}CO \quad CH \cdot\cdot NH\text{-}G_3$

$CH_2$

$C_6H_5$

dans laquelle $G_3$ est défini comme précédemment, et enfin blocage des fonctions silanols résiduelles par des radicaux $Si(R'')_3$ tels que définis précédemment.

Généralement, l'action de l'anhydride d'un acide aminoalcanoique protégé sur la silice aminopropyle est effectuée en opérant dans un solvant organique anhydre tel que le diméthylformamide à une température voisine de 20°C.

Le blocage des fonctions silanols par des groupements $-Si(R'_3)$ tels que définis précédemment est effectuée par action d'un halogénotrialkylsilane sur la silice aminopropyle greffée par des restes aminoalcanoyles en opérant dans un solvant organique tel que le chlorure de méthylène en présence d'un agent basique tel que la pyridine.

L'élimination des groupements protecteurs des restes aminoalcanoyles s'effectue généralement, lorsque le groupement protecteur est un radical *tert*-butoxycarbonyle, par action de l'acide trifluoroacétique dans un solvant organique tel que le chlorure de méthylène.

L'amidification au moyen de phénylalanine dont la fonction amine est protégée est effectuée en présence d'un agent de condensation tel que la N-éthoxycarbonyl-2-éthoxy-1,2-dihydro-quinoléine en opérant dans un solvant organique anhydre tel que le diméthylformamide.

Le blocage des fonctions silanols résiduelles par des radicaux $-Si(R'')_3$ tels que définis précédemment est généralement effectué au moyen de trialkylsilylimidazole en opérant dans un solvant organique tel que le chlorure de méthylène.

La silice aminopropyle peut être préparée par action de l'aminopropyltriéthoxysilane sur une silice dont la porosité est voisine de 100 Å en opérant en présence d'imidazole dans un solvant organique anhydre tel qu'un hydrocarbure aromatique comme le toluène.

Comme le montrent les exemples ci-après, les nouveaux produits de formule générale (I), qui inhibent la farnésyle transférase et la farnésylation de la protéine Ras, présentent des propriétés antitumorales et antileucémiques remarquables.

La présente invention a également pour objet toute composition pharmaceutique contenant au moins un produit de formule générale en association avec un ou plusieurs diluants ou adjuvants pharmaceutiquement acceptables qu'ils soient inertes ou biologiquement actifs.

Les nouveaux produits de formule générale (I) peuvent se présenter sous forme de sels non toxiques et pharmaceutiquement acceptables. Ces sels non toxiques comprennent les sels avec les acides minéraux (acides chlorhydrique, sulfurique, bromhydrique, phosphorique, nitrique) ou avec les acides organiques (acides acétique, propionique, succinique, maléique, hydroxymaléique, benzoïque, fumarique, méthanesulfonique, trifluoroacétique ou oxalique) ou avec les bases minérales (soude, potasse, lithine, chaux) ou organiques (amines tertiaires comme la triéthylamine, la pipéridine, la benzylamine) selon la nature des composés de formule générale (1).

La présente invention concerne également l'utilisation des composés de formule générale (I) selon l'invention pour la préparation de compositions pharmaceutiques utiles pour inhiber la farnésyle transférase, et plus particulièrement pour inhiber la famésylation de l'oncogène *ras*.

Notamment, la présente invention concerne l'utilisation des composés de formule générale (I) selon l'invention pour la préparation de compositions pharmaceutiques utiles pour le traitement des maladies liées à des proliférations cellulaires par inhibition de la farnésyle transférase et en particulier pour le traitement des maladies liées à des proliférations cellulaires, surexprimant l'une quelconque des oncoprotéines H-Ras, N-Ras ou K-Ras, ou présentant une mutation de l'un quelconque des oncogènes *ras* correspondants.

L'invention concerne en particulier l'utilisation des composés de formule générale (1) selon l'invention pour la préparation de compositions pharmaceutiques utiles pour le traitement des maladies liées à des proliférations cellulaires, malignes ou bénignes. des cellules de divers tissus et/ou organes, comprenant les tissus musculaires, osseux ou conjonctifs, la peau, le cerveau, les poumons, les organes sexuels, les systèmes lymphatiques ou rénaux, les cellules mammaires ou sanguines, le foie, l'appareil digestif, le colon, le pancréas et les glandes thyroides ou adrénales. et incluant les pathologies suivantes : le psoriasis, la resténose, les tumeurs solides, le sarcome de Kaposi, les carcinomes, le cholangiocarcinome, le choriocarcinome, le neuroblastome, la tumeur de Wilms, la maladie de Hodgkin, les mélanomes, les tératocarcinomes, les gliomes, les myélomes multiples, les leucémies lymphocytaires chroniques, les lymphomes granulocytaires aigus ou chroniques, et

les cancers tels que les cancers du pancréas, du colon, du poumon, de l'ovaire, du sein, du cerveau, de la prostate, du foie, de l'estomac, de la vessie ou des testicules.

L'invention concerne tout particulièrement l'utilisation des composés de formule générale (I) selon l'invention, préférentiellement le composé décrit à l'exemple 3 pour la préparation de compositions pharmaceutiques utiles pour le traitement des cancers tels que les cancers du pancréas, du colon, du poumon, de l'ovaire, du sein, du cerveau, de la prostate, du foie, de l'estomac, de la vessie ou des testicules, et plus avantageusement le cancer du colon et du pancréas, en particulier du colon.

Les composés selon l'invention peuvent également être utiles pour la préparation de médicaments pour le traitement et/ou la prévention des conditions pathologiques liées aux voies de signalisations cellulaires, associées à la farnésyle transférase, ou à leurs conséquences ou symptômes.

Avantageusement, les composés selon la présente invention peuvent être utilisés pour pour la préparation de médicaments pour le traitement et/ou la prévention de maladies associées aux voies de signalisations cellulaires liées à Ras. Ainsi, les composés selon l'invention peuvent être utilisés pour la préparation de médicaments dans le traitement et/ou la prévention des rejets de greffes (telles que les allogreffes) (O'Donnel et al. 1995, Kidney International, vol.48, suppl.52, p.S.29-33).

Egalement, les composés selon l'invention peuvent être utiles pour la préparation de médicaments pour inhiber l'angiogénèse et agir sur la croissance tumorale (J.Rak et al., Cancer research, 55, 4575-4580, 1995), ces propriétés anti-angiogénèse pouvant être également utiles pour le traitement de certaines formes de cécité liées a la vascularisation rétinienne;

Les composés selon l'invention peuvent également être utiles pour la préparation de médicaments pour le traitement et/ou la prévention

- de maladies liées à un dysfonctionnement (pro- ou anti-) apoptotique comprenant en particulier les cancers précédemment cités;
- de l'hépatite delta et des virus associés (J.S.Glenn et al., Science, 256, 1331-1333, 1992), ainsi que, et de façon non limitative, les virus Herpès, Pox, Epstein-Barr, Sindbis, et adeno);
- des maladies inflammatoires et/ou auto-immunes, telles que à titre illustratif, les polyarthrites, les rhumatismes polyarticulaires, les inflammations intestinales, les oedèmes pulmonaires, les infarctus du myocarde, les fibroses, les lupus érythemateux, tels que la maladie de Kaposi , les immuno-glomérulonéphrites. les diabètes autoimmuns;
- les maladies cardiovasculaires (artériosclérose ou autres lésions artérielles), la resténose après angioplastie ou chirurgie vasculaire;
- des maladies osseuses, de la régulation du métabolisme osseux, par exemple la maladie de Paget, l'hypercalcémie, les métastases osseuses, l'ostéoporose;
- les maladies associées à un niveau de cholesterol elevé, telles l'hypercholestérolémie. l'hyperlipidémie, l'hyperpoprotéinémie, l'hyperlipidémie néphrotique, ou l'athérosclérose (Massy et al, Lancet, 347, 102-103, 1996);
- les désordres neurodégénératifs, tels que la maladie de Parkinson, d'Alzheimer, la sclérose latérale amyotrophique, les troubles neurologiques associés aux maladies virales telles que le sida, les rétinites, les atrophies ou dégénérescences spinocérébelleuses);

et pour la prévention et/ou le traitement des maladies suivantes : le SIDA en particulier et également, le rein polykystique (D.L.Schaffner et al., American Journal of Pathology, 142, 1051-1060, 1993), les neurofibromatoses, les fibroses pulmonaires, les arthrites, le psoriasis, les glomérulonéphrites, les formations de cicatrices hypertrophiques, les chocs endotoxiques;

ainsi que les syndrômes myéliodysplastiques, l'anémie aplastique, les lésions ischémiques associées aux infarctus, les lésions associées aux accidents vasculaires cérébraux et , les arrhytmies, les athéroscléroses, les maladies du foie dues à des toxines ou l'alcool, les maladies hématologiques, incluant notamment l'anémie chronique ou aplastique. les maladies dégénératives du système muscle-squelette, incluant notamment l'ostéoporose et l'arthrite, les fibroses kystiques, les scléroses multiples, les maladies du foie, les douleurs liées au cancer.

Le dit traitement peut être notamment effectué par inhibition de la croissance tumorale, notamment par inhibition de la farnésyle transférase, ou encore par inhibition de la croissance de tumeurs exprimant l'oncogène *ras* activé.

Le traitement thérapeutique peut aussi être effectué concurremment avec d'autres traitements thérapeutiques incluant des médicaments antinéoplastiques, des anticorps monoclonaux, des thérapies immunologiques ou des radiothérapies ou des modificateurs des réponses biologiques. Les modificateurs des réponses incluent, de manière non limitative, les lymphokines et les cytokines telles que les interleukines, les interférons ($\alpha$, $\beta$ ou $\delta$) et le TNF. D'autres agents chimiothérapeutiques utiles dans le traitement des désordres dus à la prolifération anormale des cellules incluent, de manière non limitative, les agents alkylants tels que les moutardes à l'azote comme la mechloretamine, le cyclophosphamide, le melphalan et le chlorambucil, des sulfonates d'alkyle comme le busulfan, les nitrosourées comme la carmustine, la lomustine, la sémustine et la streptozocine, les triazènes comme la dacarbazine, les antimétabolites comme les analogues de l'acide folique tel que le méthotrexate, les analogues de pyrimidine comme le fluorouracil et la cytarabine, des analogues de purines comme la mercaptopurine et la thioguanine, des produits naturels

tels que les alcaloïdes de vinca comme la vinblastine, la vincristine et la vendésine, des épipodophyllotoxines comme l'étoposide et le teniposide, des antibiotiques comme la dactinomycine, la daunorubicine, la doxorubicine, la bléomycine, la plicamycine et la mitomycine, des enzymes comme la L-asparaginase, des agents divers comme les complexes de coordination du platine tel que le cisplatine, les urées substituées telles que l'hydroxyurée, les dérivés de méthylhydrazine comme la procarbazine, les suppresseurs adrénocoticoïques comme le mitotane et l'aminoglutéthymide, les hormones et les antagonistes comme les adrénocorticostéroïdes comme la prednisone, les progestines comme le caproate d'hydroxyprogestérone, l'acétate de méthoxyprogestérone et l'acétate de megestrol, les oestrogènes comme le diéthylstilbestrol et l'éthynyiestradiol, les antioestrogènes comme le tamoxifène, les androgènes comme le propionate de testostérone et la fluoxymesterone.

Un autre objet de la présente invention est toute association d'un produit de formule générale (I) avec un ou plusieurs composés compatibles et pharmacologiquement actifs et/ou un traitement radiothérapique afin d'obtenir un effet thérapeutique synergique, lesdits composés étant préférentiellement des principes actifs connus pour leur activité inhibitrice de la prolifération cellulaire et particulièrement pour leur activité dans le traitement du cancer; ils peuvent être préférentiellement des composés antiprolifératifs agissant à l'un quelconque des stades de la voie de signalisation de l'oncogène *ras* comme un inhibiteur de tyrosine kinase, ou un autre inhibiteur de farnésyle transférase, ou un inhibiteur de HMG-Co-réductase, ou les composés cytotoxiques utilisés habituellement dans le traitement du cancer.

Les produits selon l'invention peuvent être utilisés pour prévenir ou retarder l'apparition ou la réapparition des conditions pathologiques ou pour traiter ces conditions pathologiques.

Les produits selon l'invention peuvent être administrés par voie orale, parentérale ou ou intrapéritonéale ou rectale, de préférence par voie orale.

Les compositions pour administration orale comprennent des comprimés, des pilules, des poudres ou des granulés. Dans ces compositions le produit actif selon l'invention est mélangé à un ou plusieurs diluants inertes tels que saccharose, lactose ou amidon. Ces compositions peuvent comprendre des substances autres que les diluants, par exemple un lubrifiant tel que le stéarate de magnésium.

Comme compositions liquides pour administration orale peuvent être utilisées des émulsions pharmaceutiquement acceptables, des solutions, des suspensions, des sirops, des élixirs contenant des diluants inertes tel que l'eau ou l'huile de paraffine. Ces compositions peuvent également comprendre des substances autres que les diluants, par exemple des produits mouillants, édulcorants ou aromatisants.

Les compositions selon l'invention pour administration parentérale peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive ou des esters organiques injectables par exemple l'oléate d'éthyle. Ces compositions peuvent également contenir des adjuvants, en particulier des agents mouillants, émulsifiants et dispersants. La stérilisation peut se faire de plusieurs façons, par exemple à l'aide d'un filtre bactériologique, en incorporant à la composition des agents stérilisants ou par chauffage. Elles peuvent être également préparées sous forme de compositions solides stériles qui peuvent être dissoutes au moment de l'emploi dans de l'eau stérile ou tout autre milieu stérile injectable.

Les compositions pour administration rectale sont des suppositoires qui peuvent contenir, outre le produit actif, des excipients tels que le beurre de cacao.

Les doses utilisées pour mettre en oeuvre les méthodes selon l'invention sont celles qui permettent un traitement prophylactique ou un maximum de réponse thérapeutique. Les doses varient selon la forme d'administration, le produit particulier sélectionné et les caractéristiques propres du sujet à traiter. En général, les doses sont celles qui sont thérapeutiquement efficaces pour le traitement des désordres dus à une prolifération cellulaire anormale, et notamment un traitement cytostatique. Les produits selon l'invention peuvent être administrés aussi souvent et aussi longtemps que nécessaire pour obtenir l'effet thérapeutique désiré.

Généralement, les doses sont comprises, chez l'homme, entre 0,1 et 10000 mg/kg par jour, préférentiellement comprises entre 100 et 2000 mg/kg par jour, de préférence par voie orale. Il est entendu que, pour choisir le dosage le plus approprié, devront être pris en compte la voie d'administration, le poids du malade, son état de santé général, son âge et tous les facteurs qui peuvent influer sur l'efficacité du traitement.

D'une façon générale, le médecin déterminera la posologie appropriée en fonction de l'âge, du poids et de tous les autres facteurs propres au sujet à traiter.

L'exemple 105 illustre des compositions selon l'invention.

Les exemples suivants sont présentés à titre illustratif et non limitatif de la présente invention.

**EXEMPLE 1**

Préparation du 4,9-éthano-2-[2-(2-méthoxyphényl)propènoyl]-9-(4-méthylphényl)-2,3,3a,4,9,9a-hexahydro-1H-benzo [f]isoindole-3a-carboxylate de méthyle-(3aRS,4SR,9SR,9aRS)

Etape A

A une solution de 428 g (1,98 mol) d'acide 3-oxo-6-phénylcyclohex-1-ène-1-carboxylique-(RS), qui peut être obtenu selon J. Org. Chem., 1971, 36, 3707, dans 4,5 $cm^3$ d'acétone, on ajoute successivement 358 g (2,52 mol) d'iodure de méthyle et 361 g (2,38 mol) de 1,8-diazabicyclo[5.4.0]undéc-7-ène, puis on porte au reflux pendant cinq heures. L'acétone est ensuite distillée, puis le résidu est agité avec 2,5 $dm^3$ d'eau. Après refroidissement à 10°C, le précipité formé est essoré, lavé à l'eau glacée, puis séché à 30°C. On obtient ainsi 423 g (93 %) de 3-oxo-6-phényl-cyclohex-1-éne-1-carboxylate de méthyle-(RS), sous forme d'une poudre jaune dont les caractéristiques sont les suivantes :

- point de fusion = 66°C
- spectre de R.M.N. (300 MHz, $CDCl_3$, d en ppm) : 2,1-2,4 (mt, 4H: H en 4 et en 5), 3,72 (s, 3H: $CH_3$) ; 4,25 (dt, 1H: H en 6) ; 6,88 (s, 1H: H en 2) ; 7,2-7,52 (mt, 5H: aromatiques).

Etape B

Une solution de 170 g (0,77 mol) de 3-oxo-6-phénylcyclohex-1-ène-1-carboxylate de méthyle-(RS) et de 0,9 $cm^3$ d'acide trifluoroacétique dans 880 $cm^3$ de dichlorométhane est portée au reflux. On ajoute alors, en 15 minutes au reflux. 256.3 g (0,913 mol) de N-n.butoxyméthyl-N-[(triméthylsilyl)méthyl-benzylamine, qui peut être obtenue selon Chem. Pharm. Bull., 1985, 276. Le milieu réactionnel est alors refroidi à 20°C. Après addition de 20 $cm^3$ d'une solution aqueuse saturée d'hydrogénocarbonate de sodium, la phase organique est décantée, lavée à l'eau, puis agitée avec 900 g de silice (63-200 Mesh) pendant 30 minutes. La silice est filtrée, puis lavée par 1,5 $cm^3$ de dichlorométhane. On obtient alors, après concentration du solvant sous pression réduite, 270 g d'une huile brune qui est reprise avec 800 $cm^3$ de cyclohexane et 770 $cm^3$ d'une solution aqueuse normale d'acide méthanesulfonique. Après décantation, la phase organique est lavée par deux fois 200 $cm^3$ d'eau. Les phases aqueuses jointes sont lavées par deux fois 200 $cm^3$ de cyclohexane, puis neutralisées par ajout d'hydrogénocarbonate de sodium et extraites avec 400 $cm^3$ puis deux fois 200 $cm^3$ d'acétate d'éthyle. Les phases organiques jointes sont ensuite lavées par une solution demi-saturée de chlorure de sodium, séchees sur sulfate de sodium puis concentrées sous pression réduite. On obtient alors 248 g (90 %) de 2-benzyl-7-oxo-4-phényloctahydroisoindole-3a-carboxylate de méthyle-(3aRS,4SR, 7aRS), sous forme d'une huile orangée, dont les caractéristiques sont les suivantes :

- spectre de R.M.N. (300 MHz, $CDCl_3$, d en ppm) : 2,4-2,65 (mt, 4H: H en 5 et en 6); 2,85 (2 dt, 2H: H en 1); 3,02 (2 dt, 2H: H en 3) ; 3,2 (2 dt, 1H: H en 4); 3,35 (mt, 1H: H en 7a) ; 3,5 (2 dt, 2H: H benzyliques) : 7-7,3 (mt, 10H: aromatiques).

Etape C

5,1 $cm^3$ de 4-bromotoluène et 1 g de magnésium en tournures dans 100 $cm^3$ d'oxyde de diéthyle sont chauffés au reflux pendant une heure. 100 $cm^3$ de toluène sont ajoutés et le milieu est chauffé à 60°C sous courant d'azote. Après refroidissement à une température voisine de 5°C, une solution de 10 g de 2-benzyl-7-oxo-4-phényloctahydroisoindole-3a-carboxylate de méthyle-(3aRS,4SR,7aRS) dans 80 $cm^3$ de toluène est additionnée. Le mélange réactionnel est agité pendant une heure à une température voisine -de 20°C puis hydrolysé par 100 $cm^3$ d'une solution aqueuse saturée en chlorure d'ammonium. La phase aqueuse est séparée par décantation et extraite par deux fois 75 $cm^3$ d'acétate d'éthyle, Les phases organiques sont réunies, lavées successivement par 100 $cm^3$ d'eau distillée et 100 $cm^3$ d'une solution aqueuse saturée en chlorure de sodium, séchées sur sulfate de magnésium et concentrées sous pression réduite. Après purification par flash-chromatographie sur gel de silice (230-400 mesh), en éluant avec un mélange cyclohexane-acétate d'éthyle (80-20 en volumes), on obtient 9 g de 2-benzyl-7-hydroxy-7-(4-méthylphényl)-4-phényloctahydroisoindole-3a-carboxylate de méthyle-(3aRS,4SR,7RS,7aRS) sous forme de solide blanc dont les caractéristiques sont les suivantes :

- point de fusion = 164°C
- spectre de R.M.N. $^1H$ (250 MHz, $CDCl_3$, d en ppm). ; 1,84 et 2,60 (d mt et mt, J = 12,5 Hz, 1H chacun : $CH_2$ en 5) ; de 2,05 à 2,20 (mt, 2H : 1H du $CH_2$ en 1 et 1H du $CH_2$ en 6) ; 2,32 (s, 3H : $ArCH_3$) ; 2,40 et 2,95 (2d, J = 10,5

Hz, 1H chacun : $CH_2$ en 3) ; 2,45 (mt, 1H : l'autre H du $CH_2$ en 6) ; 2,64 (mt, 1H : l'autre H du $CH_2$ en 1) ; 2,85 (mt, 1H : H en 7a) ; 3,32 (s, 3H : $COOCH_3$) ; 3,40 et 3,70 (2d, J = 12,5 Hz, 1H chacun : $NCH_2Ar$) ; 3,50 (dd, J = 12,5 et 3 Hz, 1H : H en 4) ; 6,68 (s; 1H : OH en 7) ; de 7,00 à 7,50 (mt, 10H : H aromatiques en 4 et H aromatiques du benzyle) : 7,12 et 7,40 (2d, J = 8 Hz, 2H chacun : H en ortho et méta de l'aromatique en 7)

Etape D

A une solution de 20 g de 2-benzyl-7-hydroxy-7-(4-méthylphényl)-4-phényloctahydroisoindole-3a-carboxylate de méthyle-(3aRS,4SR,7RS,7aRS) dans 200 $cm^3$ de dichlorométhane, maintenue à une température voisine de 0°C, on ajoute goutte à goutte un mélange de 29 $cm^3$ d'acide trifluorométhanesulfonique et de 100 $cm^3$ de dichlorométhane. Le mélange réactionnel est agité pendant 30 minutes à une température voisine de 0°C, 2 heures à une température voisine de 20°C puis refroidi à une température voisine de 0°C. On ajoute alors 50 $cm^3$ d'eau distillée puis le pH de la phase aqueuse est amené entre 8 et 9 par addition d'une solution aqueuse 4N d'hydroxyde de sodium. La phase organique est séparée par décantation, lavée successivement par 100 $cm^3$ d'eau distillée et 100 $cm^3$ d'une solution aqueuse saturée en chlorure de sodium, séchée sur sulfate de magnésium et concentrée sous pression réduite. On obtient ainsi, après recristallisation dans l'isopropanol, 18,2 g de 2-benzyl-4,9-éthano-9-(4-méthylphényl)-2,3,3a,4,9,9a-hexahydro-1H-benzo[f] isoindole-3a-carboxylate de méthyle-(3aRS,4SR,9SR,9aRS) sous forme de solide blanc dont les caractéristiques sont les suivantes :

- point de fusion = 158°C
- spectre de R.M.N. $^1H$ (300 MHz, CDCl3, d en ppm) : 1,61 - 1,90 et 2.67 (3 mts, respectivement 1H -1H et 2H : $CH_2CH_2$) ; de 2,50 à 2,60 (mt, 2H : 1H du $CH_2$ en 1 et 1H $CH_2$ en 3) ; 2,57 (s, 3H : $ArCH_3$) ; 2,95 (d, J = 10 Hz, l'autre H du $CH_2$ en 1) ; 3,37 (d, J = 10 Hz, l'autre H du $CH_2$ en 3) ; 3,44 (d large, J = 10 Hz. 1H : H en 9a) ; 3,54 et 3,85 (2d, J = 12,5 Hz, 1H chacun : $NCH_2Ar$) ; 3,57 (s large, 1H : H en 4) ; 3,72 (s, 3H : $COOCH_3$) ; 6,70 (d large, J = 7,5 Hz, 1H : H en 8) ; de 7,10 à 7,60 (mt, 12H : H en 5 - H en 6 - H en 7 - H en ortho et méta de l'aromatique en 9 et H aromatiques du benzyle).

Etape E

18 g de 2-benzyl-4,9-éthano-9-(4-méthylphényl)-2,3,3a,4,9,9a-hexahydro-1H-benzo[f]isoindole-3a-carboxylate de méthyle-(3aRS,4SR,9SR,9aRS), en solution dans 250 $cm^3$ de méthanol, sont réduits par 7,8 g de formiate d'ammonium en présence de 2 g de palladium sur charbon à 10% (p/p) en chauffant au reflux pendant deux heures. Après refroidissement, le catalyseur est séparé par filtration, rincé par trois fois 50 $cm^3$ de méthanol et le filtrat concentré sous pression réduite. Le résidu est dissout dans 200 $cm^3$ d'acétate d'éthyle et la phase organique lavée successivement par 100 $cm^3$ d'une solution aqueuse saturée en hydrogénocarbonate de sodium, 100 $cm^3$ d'eau distillée et 100 $cm^3$ d'une solution aqueuse saturée en chlorure de sodium, séchée sur sulfate de magnésium et concentrée sous pression réduite. Par agitation dans 100 $cm^3$ de pentane, l'huile obtenue cristallise. Par filtration, on obtient 13,2 g de 4,9-éthano-9-(4-méthylphényl)-2,3,3a,4,9,9a-hexahydro-1H-benzo[f]isoindole-3a-carboxylate de méthyle-(3aRS, 4SR,9SR,9aRS) sous forme de solide blanc dont les caractéristiques sont les suivantes :

- point de fusion = 118°C
- spectre de R.M.N. $^1H$ (250 MHz, $CDCl_3$, d en ppm) : 1,56 - 1,78 et 2,24 (3 mts, respectivement 1H -1H et 2H : $CH_2CH_2$) ; 2,42 (s, 3H : $ArCH_3$) ; 2,79 et 2,96 (2 dd, respectivement J = 12,5 et 5,5 Hz et J = 12,5 et 8 Hz, 1H chacun : $CH_2$ en 1) ; 3,10 et 3,39 (2 d, J = 12,5 Hz, 1H chacun : $CH_2$ en 3) ; 3,30 (mt, 1H : H en 9a) ; 3,51 (s large, 1H : H en 4) ; 3,60 (s, 3H : $COOCH_3$) ; 6,56 (d large, J = 7.5 Hz, 1H : H en 8); de 6,95 à 7,45 (mt, 7H : H en 5 - H en 6 - H en 7 et H en ortho et méta de l'aromatique en 9).

Etape F

36,6 g d'acide (2-méthoxyphényl)acétique en suspension dans 110 $cm^3$ de bis(diméthylamino)méthane sont refroidis à une température voisine de 0°C. 110 $cm^3$ d'anhydride acétique sont ajoutés goutte à goutte, la température du milieu réactionnel ne dépassant pas 40°C. Le mélange réactionnel est agité pendant 24 heures à une température voisine de 20°C puis refroidi à une température voisine de 0°C. 500 $cm^3$ d'eau distillée sont alors ajoutés et l'agitation poursuivie pendant 2 heures à une température voisine de 0°C. Le solide qui est apparu est séparé par filtration, lavé par trois fois 100 $cm^3$ d'eau distillée et seché sous pression réduite à 40°C. On obtient ainsi 23 g d'acide 2-(2-méthoxyphényl)propènoïque sous forme de solide crème dont les caractéristiques sont les suivantes :

- point de fusion = 145°C

- spectre de R.M.N. $^1H$ (200 MHz. DMSO d6, d en ppm) : 3,74 (s, 3H : $ArOCH_3$) ; 5.71 et 6,15 (2d, J = 0,5 Hz, 1H chacun : $=CH_2$) ; de 6,90 à 7,50 (mt, 4H : H aromatiques) ; de 11,5 à 13,5 (mf très étalé, 1H : COOH).

Etape G

A une solution de 6,31 g d'acide 2-(2-méthoxyphényl)propènoïque dans 150 $cm^3$ de dichlorométhane contenant 5 gouttes de N,N-diméthylformamide on ajoute goutte à goutte une solution de 3,05 $cm^3$ de chlorure d'oxalyle dans 150 $cm^3$ de dichlorométhane. Le mélange réactionnel est encore agité pendant deux heures à une température voisine de 20°C puis refroidi à une température voisine de 0°C et coulé goutte à goutte dans une solution de 12,3 g de 4,9-éthano-9-(4-méthylphényl)-2,3,3a,4,9,9a-hexahydro-1H-benzo[f]isoindole-3a-carboxylate de méthyle-(3aRS, 4SR,9SR,9aRS) dans 250 $cm^3$ de dichlorométhane et 10 $cm^3$ de triéthylamine en maintenant la température au voisinage de 0°C. Le mélange réactionnel est encore agité pendant une heure à une température voisine de 0°C puis pendant une heure à une température voisine de 20°C et versé dans 200 $cm^3$ d'eau distillée. La phase organique est séparée par décantation, lavée par deux fois 200 $cm^3$ d'eau distillée puis 200 $cm^3$ d'une solution aqueuse saturée en chlorure de sodium, séchée sur sulfate de magnésium et concentrée sous pression réduite. Après purification par flash-chromatographie sur gel de silice (230-400 mesh), en éluant avec un mélange cyclohexane-acétate d'éthyle (70-30 en volumes), on obtient 14,9 g de 4,9-éthano-2-[2-(2-méthoxyphényl)propènoyl]-9-(4-méthylphényl)-2,3,3a, 4,9,9a-hexahydro-1H-benzo[f]isoindole-3a-carboxylate de méthyle-(3aRS,4SR,9SR,9aRS) sous forme de solide blanc dont les caractéristiques sont les suivantes :

- point de fusion = 152°C
- spectre de R.M.N. $^1H$ (250 MHz, DMSO d6, à une tempèrature de 383 K, d en ppm) : 1,44 - 1,68 et de 2,00 à 2,30 (3 mts. respectivement 1H - 1H et 2H : $CH_2CH_2$) ; 2,40 (s, 3H : $ArCH_3$) ; de 3.35 à 3.50 (mt, 3H : $CH_2$ en 1 et H en 9a) ; 3.46 (mt, 1H : H en 4) ; 3.55 (s, 3H : $COOCH_3$) ; 3,60 et 4,10 (2d, J = 12.5 Hz, 1H chacun : $CH_2$ en 3) ; 3,73 (s, 3H : $ArOCH_3$) ; 5,54 et 5,70 (2s, 1H chacun : $=CH_2$) ; 6.46 (d large, J = 7,5 Hz, 1H : H en 8) ; de 6,90 à 7,40 (mt, 11H : H en 5 - H en 6 - H en 7 - H en ortho et méta de l'aromatique en 9 et H aromatiques en ortho - méta et para du $OCH_3$).

**EXEMPLE 2**

<u>Préparation de l'acide 4,9-éthano-2-[2-(2-méthoxyphényl)propènoyl]-9-(4-méthyl-phényl)-2,3,3a,4,9,9a-hexahydro-1H-benzo[f]isoindole-3a-carboxylique-(3aRS,4SR, 9SR,9aRS)</u>

14,7 g de 4,9-éthano-2-[2-(2-méthoxyphényl)propènoyl]-9-(4-méthylphényl)-2,3,3a,4,9,9a-hexahydro-1H-benzo[f]isoindole-3a-carboxylate de méthyle-(3aRS,4SR,9SR,9aRS) sont chauffés à reflux, pendant six heures, dans 400 $cm^3$ d'éthanol en présence de 43,5 $cm^3$ d'une solution aqueuse normale d'hydroxyde de sodium. Le mélange réactionnel est ensuite concentré sous pression réduite et le résidu dissout dans 250 $cm^3$ d'eau distillée. La phase aqueuse est lavée par trois fois 200 $cm^3$ d'oxyde de diéthyle puis acidifiée par une solution aqueuse 4 N d'acide chlorhydrique jusqu'à un pH voisin de 2. Les cristaux qui apparaissent sont séparés par filtration, lavés par trois fois 400 $cm^3$ d'eau distillée puis 200 $cm^3$ d'éther de pétrole et séchés sous pression réduite à 40°C. On obtient ainsi 12,6 g d'acide 4,9-éthano-2-[2-(2-méthoxyphényl)propènoyl]-9-(4-méthylphényl)-2,3,3a,4,9,9a-hexahydro-1H-benzo[f] isoindole-3a-carboxylique-(3aRS,4SR,9SR,9aRS) sous forme de solide blanc dont les caractéristiques sont les suivantes :

- point de fusion = 142°C
- spectre de R.M.N. $^1H$ (400 MHz, DMSO d6, à une température de 413 K, d en ppm) : 1,42 - 1,65 - 2,00 et 2,15 (4 mts, 1H chacun : $CH_2CH_2$) ; 2,40 (s, 3H : Ar $CH_3$) ; de 3,20 à 3,40 (mt, 3H : $CH_2$ en 1 et H en 9a) ; 3,55 (mt, 1H : H en 4) ; 3,60 et 4,06 (2 d, J = 12,5 Hz, 1H chacun : $CH_2$ en 3) ; 3,68 (s, 3H : $OCH_3$) ; 5,53 et 5,70 (2 s, 1H chacun : = $CH_2$) ; 6,45 (d large, J = 7,5 Hz, 1H : H en 8) ; 6.95 (t large, J = 7,5 Hz, 1H : H aromatique en para du $OCH_3$) ; 7,03 (d large, J = 7,5 Hz, 1H : H aromatique en ortho du $OCH_3$) ; de 6,90 à 7,30 (mt, 9H : H en 5 - H en 6 - H en 7 et H aromatiques en méta du $OCH_3$ et H aromatiques en ortho et méta de l'aromatique en 9).

**EXEMPLE 3**

<u>Isolement de l'énantiomère dextrogyre de l'acide 4,9-éthano-2-[2-(2-méthoxyphényl)propènoyl]-9-(4-méthylphényl)-2,3,3a,4,9,9a-hexahydro-1H-benzo [f]isoindole-3a-carboxylique-(3aRS,4SR,9SR,9aRS)</u>

500 mg d'acide 4,9-éthano-2-[2-(2-méthoxyphényl)propènoyl]-9-(4-méthylphényl)-2,3,3a,4,9,9a-hexahydro-

1H-benzo[f]isoindole-3a-carboxylique-(3aRS,4SR,9SR,9aRS) sont dédoublés sur une colonne de silice chirale, porteuse de greffons (3,5-dinitrobenzoyl) S-phénylalanine en éluant par un mélange de dichlorométhane, d'isopropanol et de n-heptane (85-10-5 en volumes). En recueillant la seconde fraction éluée (temps de rétention 60 minutes), on obtient après concentration sous pression réduite 220 mg de l'énantiomère dextrogyre dont les caractéristiques sont les suivantes :

- point de fusion = 225°C
- spectre de masse (IE) : M/Z= 493 ($M^+$) et 449 ($M^+$ - $CO_2$)
- pouvoir rotatoire: $[\alpha]_{365}^{20}$ = +63,3 +/- 1,0° (c = 0,5/ méthanol).

La silice chirale peut être préparée de la manière suivante :

Dans un tricol de 6 $dm^3$, on met en suspension 948 g de silice aminopropyle (100 Å - 10 µm - $NH_2$ ; Macherey-Nagel) dans 3 $dm^3$ de N,N-diméthylformamide. On ajoute 180 g d'anhydride de l'acide N-*tert*-butoxycarbonylamino-11-undécanoïque et on agite le mélange réactionnel pendant 18 heures à une température voisine de 20°C. La silice est séparée par filtration et lavée successivement par 2 fois 2500 $cm^3$ de dichlorométhane puis 2 fois 2500 $cm^3$ de N, N-diméthylformamide. La silice ainsi lavée est remise en suspension dans 3 litres de N,N-diméthylformamide et on ajoute 180 g d'anhydride de l'acide N-*tert*-butoxycarbonylamino-11-undécanoïque puis on agite le mélange réactionnel pendant 18 heures à une température voisine de 20°C. La silice est séparée par filtration, lavée successivement par 2 fois 2500 $cm^3$ de dichlorométhane, 2 fois 2500 $cm^3$ de tétrahydrofurane, 2 fois 2500 $cm^3$ de méthanol et 2 fois 2500 $cm^3$ d'oxyde de diéthyle puis séchée sous pression réduite à une température voisine de 20°C. On obtient ainsi 971 g de silice désignée par l'appellation "BOC-$C_{11}$-$C_3$-silice" sous forme de poudre blanche dont la structure est confirmée par le spectre infra-rouge et dont l'analyse élémentaire (trouvée) est : C % = 9,85 ; H % = 2,05 ; N % = 1,05.

Dans un tricol de 6 $dm^3$, on met en suspension 971 g de silice "BOC-$C_{11}$-$C_3$-silice" dans 2500 $cm^3$ de dichlorométhane et 470 g d'imidazole. On ajoute goutte à goutte 850 $cm^3$ de diméthyloctylchlorosilane et on agite le mélange réactionnel pendant 16 heures à une température voisine de 20°C. Le solide obtenu est séparé par filtration et lavé successivement par 2 fois 2500 $cm^3$ de dichlorométhane, deux fois 2500 $cm^3$ de méthanol, 2 fois 2500 $cm^3$ de tétrahydrofurane, deux fois 2500 $cm^3$ de dichlorométhane et 2 fois 2500 $cm^3$ d'oxyde de diéthyle puis séché sous pression réduite à une température voisine de 20°C. On obtient ainsi 1179 g de silice désignée par l'appellation "BOC-$C_{11}$-$C_3$-silice-O-$Si(CH_3)_2(CH_2)_7CH_3$" sous forme de poudre blanche dont la structure est confirmée par le spectre infra-rouge et dont l'analyse élémentaire (trouvée) est : C % = 13,9 ; H % = 2,83 ; N % = 1,16.

Dans un tricol de 6 $dm^3$, on met en suspension 1178 g de silice "BOC-$C_{11}$-$C_3$-silice-O-$Si(CH_3)_2(CH_2)_7CH_3$" dans 2500 $cm^3$ d'une solution à 5 % en volume d'acide trifluoroacétique dans le dichlorométhane. On agite à 20°C. La silice est séparée par filtration et lavée successivement par 2 fois 2500 $cm^3$ de dichlorométhane, 2 fois 2500 $cm^3$ d'un mélange dichlorométhane-diisopropyléthylamine (70-30 en volumes), 2500 $cm^3$ de dichlorométhane, 2 fois 2500 $cm^3$ de tétrahydrofurane, 2 fois 2000 $cm^3$ de méthanol et 2 fois 2000 $cm^3$ d'oxyde de diéthyle puis séchée sous pression réduite à une température voisine de 50°C. On obtient ainsi 1080,5g de silice désignée par l'appellation "$C_{11}$-$C_3$-silice-O-$Si(CH_3)_2(CH_2)_7CH_3$" sous forme de poudre blanche dont la structure est confirmée par le spectre infra-rouge et dont l'analyse élémentaire (trouvée) est : C % = 12,6 ; H % = 2,44 ; N % = 1,05.

Dans un ballon tricol de 6 $dm^3$, on met en suspension 1080 g de silice "$C_{11}$-$C_3$-(silice)-O-$Si(CH_3)_2(CH_2)_7CH_3$" dans 2500 $cm^3$ de N,N-diméthylformamide séché sur tamis moléculaire 4Å. On ajoute 108 g de N-benzoyl-3,5-dinitro-L-phénylalanine et 75 g de N-éthoxycarbonyl-2-éthoxy-1.2-dihydroquinoléine. On agite le mélange réactionnel pendant 1 nuit. La silice est séparée par filtration sur verre fritté puis est lavée par 2 fois 2500 $cm^3$ de dichlorométhane, 2 fois 2500 $cm^3$ de tetrahydrofurane, 2500 $cm^3$ de N,N-diméthylformamide et 2500 $cm^3$ de dichlorométhane. La silice ainsi lavée est remise en suspension dans 2500 $cm^3$ de N,N-diméthylformamide. On ajoute successivement 108 g de N-benzoyl-3.5-dinitro-L-phénylalanine et 75 g de N-éthoxycarbonyl-2-éthoxy-1,2-dihydro-quinoléine puis on agite pendant une nuit à 20°C. La silice est séparée par filtration sur verre fritté et est lavée successivement par 2 fois 2500 $cm^3$ de dichlorométhane, 2 fois 2500 $cm^3$ de tétrahydrofurane, 2 fois 2500 $cm^3$ de méthanol et 2 fois 2500 $cm^3$ d'oxyde de diéthyle. Après séchage à 60°C sous pression réduite (2,7 kPa), on obtient 1093,6 g de silice désignée par l'appellation "DNB-L-Phe-$C_{11}$-$C_3$ (silice)-O-$Si(CH_3)_2(CH_2)_7CH_3$", sous forme d'une poudre jaune pâle dont la structure est confirmée par son spectre infra-rouge, et dont l'analyse élémentaire (trouvée) est : C % = 14,5, H % = 2,4, N % = 1,68.

Dans un ballon tricol de 4 $dm^3$, on met en suspension 519 g de silice "DNB-L-Phe-$C_{11}$-$C_3$ (silice)-O-$Si(CH_3)_2$ $(CH_2)_7CH_3$" dans 3000 $cm^3$ de N,N-diméthylformamide séché sur tamis moléculaire 4 Å. On ajoute 450 $cm^3$ de triméthylsilylimidazole en 15 minutes, puis on agite pendant une nuit. La silice est séparée par filtration et est lavée successivement par 2 fois 1500 $cm^3$ de tétrahydrofurane, 2 fois 1500 $cm^3$ de méthanol, 2 fois 1500 $cm^3$ d'acétone et 2 fois 1500 $cm^3$ de dichlorométhane. Après séchage à une température de 60°C sous pression réduite (2,7 kPa), on obtient 519 g de silice désignée par l'appellation "DNB-L-Phe-$C_{11}$-$C_3$ (silice)-[O-$Si(CH_3)_2(CH_2)_7CH_3$]-[O-$Si(CH_3)_3$]" sous forme d'une poudre jaune pâle dont la structure est confirmée par son spectre infra-rouge, et dont l'analyse élémentaire (trouvée) est : C % = 15,3, H % = 1,8, N % = 2,6.

L'anhydride de l'acide N-*tert*-butoxycarbonyl-11-amino-undécanoique peut être préparé de la manière suivante :

On dissout 30,1 g d'acidc N-*tert*-butoxycarbonyl-11-amino-undécanoïque dans 480 $cm^3$ d'acétate d'éthyle. On refroidit cette solution à 5°C puis, en la maintenant à cette température, on ajoute en 10 minutes une solution de 10,63 g de 1,3-dicyclohexylcarbodiimide dans 120 $cm^3$ d'acétate d'èthyle. On agite le mélange réactionnel pendant 1 heure à 5°C puis pendant 16 heures à une température voisine de 20°C. On sépare le précipité forme par filtration et le lave par 30 $cm^3$ d'acétate d'éthyle. Le filtrat est concentré à sec sous pression réduite (2,7 kPa) à 30°C. Le solide obtenu est séché à 20°C sous pression réduite (2,7 kPa). On obtient ainsi, avec un rendement voisin de 100%, 31 g d'anhydride de l'acide N-*tert*-butoxycarbonyl-11-amino-undécanoïque.

L'acide N-*tert*-.butoxycarbonyl-11-amino-undécanoïque peut être préparé de selon J. Org. Chem., 41, 1350 (1976).

**EXEMPLE 4**

Isolement de l'énantiomère lévogyre de l'acide 4,9-éthano-2-[2-(2-méthoxyphényl)propènoyl]-9-(4-méthylphényl)-2,3,3a,4,9,9a-hexahydro-1H-benzo [f]isoindole-3a-carboxylique-(3aRS,4SR,9SR,9aRS)

500 mg d'acide 4,9-éthano-2-[2-(2-méthoxyphényl)propénoyl]-9-(4-méthylphényl)-2,3,3a,4,9,9a-hexahydro-1H-benzo[f]isoindole-3a-carboxylique-(3aRS,4SR,9SR,9aRS) sont dédoublés sur une colonne de silice chiralc, porteuse de greffons de (3,5-dinitrobenzoyl) S-phénylalanine en éluant par un mélange dichlorométhane-isopropanol-n-heptane (85-10-5 en volumes). En recueillant la première fraction éluée (temps de rétention 47 minutes), on obtient après concentration sous pression réduite 240 mg de l'énantiomère lévogyre dont les caractéristiques sont les suivantes :

- point de fusion = 225°C
- spectre de masse (IE): M/Z= 493 ($M^+$) et 449 ($M^+$ - $CO_2$)
- pouvoir rotatoire: $[\alpha]_{365}^{20}$ = -76,0 +/-1,2° (c = 0,5/ methanol).

**EXEMPLE 5**

Préparation de l'acide 4,9-éthano-2-[(2-méthoxyphényl)acétyl]-9-(4-méthylphényl)-2,3,3a,4,9,9a-hexahydro-1H-benzo[f]isoindole-3a-carboxylique-(3aRS,4SR,9SR,9aRS)

Etape A

A une solution de 755 mg (2,17 mmol) de 4,9-éthano-9-(4-méthylphenyl)-2,3,3a,4,9,9a-hexahydro-1H-benzo [f]isoindole-3a-carboxylate de méthyle-(3aRS, 4SR,9SR,9aRS), obtenu à l'étape E de l'exemple 1, dans 20 $cm^3$ de dichlorométhane, on ajoute successivement 362 mg (2,17 mmol) d'acide 2-méthoxyphénylacétique, 456 mg (2,38 mmol) de chlorhydrate de 1-éthyl-3-[(3-diméthylamino)propyl]carbodiimide et 59 mg (0,43 mmol) d'hydrate de N-hydroxybenzotriazole. Après une nuit d'agitation à température ambiante, on ajoute 15 $cm^3$ de dichlorométhane, on lave avec deux fois 15 $cm^3$ d'eau, on sèche sur sulfate de magnésium et on évapore le solvant sous pression réduite. Après purification par flash-chromatographie sur gel de silice (230-400 Mesh) en éluant par un mélange cyclohexane-acétate d'éthyle (90-10 en volumes), on obtient, après concentration du solvant sous pression réduite, 820 mg (76%) de 4,9-éthano-2-[(2-méthoxyphényl)acétyl]-9-(4-méthylphényl)-2,3,3a,4,9,9a-hexahydro-1H-benzo[f] isoindole-3a-carboxylate de méthyle-(3aRS,4SR,9SR,9aRS), sous forme d'une poudre blanche dont les caractéristiques sont les suivantes :

- point de fusion = 189°C
- spectre de masse (IE) : M/Z = 495 ($M^+$)

Etape B

En opérant comme à l'exemple 2, mais à partir de 819 mg (1,65 mmol) de 4,9-éthano-2-[(2-méthoxyphényl) acétyl]-9-(4-méthylphényl)-2,3,3a,4,9,9a-hexahydro-1H-benzo[f]isoindole-3a-carboxylate de méthyle-(3aRS,4SR, 9SR,9aRS), au reflux pendant 18 heures dans 17 $cm^3$ d'une solution aqueuse normale d'hydroxyde de sodium et 17 $cm^3$ de méthanol, on obtient, après recristallisation dans 25 $cm^3$ d'oxyde de diisopropyle, 720 mg (74%) d'acide 4,9-éthano-2-[(2-méthoxyphényl)acétyl]-9-(4-méthylphényl )-2,3,3a,4,9,9a-hexahydro-1H-benzo[f]isoindole-3a-carboxylique-(3aRS,4SR,9SR,9aRS), sous forme d'une poudre blanche dont les caractéristiques sont les suivantes :

- point de fusion = 182°C
- spectre de R.M.N. $^1H$ (400 MHz, DMSO d6, à une température de 423 K, d en ppm) : 1,40 - 1,64 - 1,95 et 2,10 (4 mts, 1H chacun : $CH_2CH_2$) : 2,41 (s, 3H : $ArCH_3$) : de 3,30 à 3,65 (mt, 6H : $CH_2$ en 1 - $NCOCH_2Ar$ - 1H du $CH_2$ en 3 et H en 9a) ; 3,48 (mt, 1H : H en 4) ; 3,75 (s, 3H : $OCH_3$) ; 4,18(d, J = 12,5 Hz, 1H : l'autre H du $CH_2$ en 3); 6,46 (d large, J = 7,5 Hz, 1H : H en 8) ; 6,90 (t large, J = 7,5 Hz, 1H : H aromatique en para du $OCH_3$) ; 6,96 (d large, J = 7,5 Hz, 1H : H aromatique en ortho du $OCH_3$) ; de 7,00 à 7,35 (mt, 5H : H en 5 - H en 6 - H en 7 et H aromatiques en méta du $OCH_3$) ; 7,30 (AB limite, 4H : H aromatiques en ortho et méta de l'aromatique en 9).

**EXEMPLE 6**

Préparation de l'acide 4,9-éthano-9-(4-méthoxyphényl)-2-[(2-méthoxyphényl)acétyl]-2,3,3a,4,9,9a-hexahydro-1H-benzo[f]isoindole-3a-carboxylique-(3aRS,4SR,9SR,9aRS)

Etape A

2,07 $cm^3$ (16,5 mmol) de 4-bromoanisole et 402 mg (16,5 mmol) de magnésium en tournures dans 20 $cm^3$ d'oxyde de diéthyle sont chauffés au reflux sous atmosphère d'argon pendant trois heures. Après refroidissement à 0°C, on ajoute 1,36 g (5,5 mmol) de chlorure de cérium anhydre, puis on coule, goutte à goutte à une température voisine de 5°C et sous atmosphère d'argon, une solution de 2 g (5,5 mmol) de 2-benzyl-7-oxo-4-phényloctahydroisoindole-3a-carboxylate de méthyle-(3aRS,4SR,7aRS), obtenu à l'étape B de l'exemple 1, dans 5 $cm^3$ d'oxyde de diéthyle sec. Le mélange réactionnel est agité pendant une heure à une température voisine de 0°C, puis la nuit à température ambiante. Après refroidissement à 0°C, le milieu réactionnel est hydrolysé par 15 $cm^3$ d'une solution aqueuse saturée en chlorure d'ammonium. La phase aqueuse est séparée par décantation et extraite par deux fois 50 $cm^3$ d'acétate d'éthyle. Les phases organiques sont réunies, lavées successivement par 25 $cm^3$ d'eau distillée et 25 $cm^3$ d'une solution aqueuse saturée en chlorure de sodium, séchées sur sulfate de magnésium et concentrées sous pression réduite. Après purification par flash-chromatographie sur gel de silice (230-400 mesh), en éluant avec un mélange cyclohexane-acétate d'éthyle (60-40 en volumes), on obtient 1,345 g (51%) de 2-benzyl-7-hydroxy-7-(4-méthoxyphényl)-4-phényloctahydroisoindole-3a-carboxylate de méthyle-(3aRS,4SR,7RS,7aRS) sous forme de solide blanc dont la caractéristique est la suivante :

- point de fusion = 136°C

Etape B

En opérant comme à l'étape D de l'exemple 1, mais à partir de 1,343 g (2,85 mmol) de 2-benzyl-7-hydroxy-7-(4-méthoxyphényl)-4-phényloctahydroisoindole-3a-carboxylate de méthyle-(3aRS,4SR,7RS,7aRS) et de 4,56 $cm^3$ d'acide trifluorométhanesulfonique dans 65 $cm^3$ de dichlorométhane, pendant 30 minutes à une température voisine de 0°C, puis la nuit à température ambiante, on obtient, après purification par flash-chromatographie sur gel de silice (230-400 mesh), en éluant avec un mélange cyclohexane-acétate d'éthyle (90-10 en volumes), 863 mg (67%) de 2-benzyl-4,9-éthano-9-(4-méthoxylphényl)-2,3,3a,4,9,9,9a-hexahydro-1H-benzo[f]isoindole-3a-carboxylate de méthyle-(3aRS,4SR,9SR,9aRS) sous forme de solide blanc dont la caractéristique est la suivante :

- point de fusion = 127°C

Etape C

En opérant comme à l'étape E de l'exemple 1, mais à partir de 839 mg (1,85 mmol) de 2-benzyl-4,9-éthano-9-(4-méthoxyphényl)-2,3,3a,4,9,9a-hexahydro-1H-benzo[f]isoindole-3a-carboxylate de méthyle-(3aRS,4SR,9SR,9aRS) et de 0,35g (5,5 mmol) de formiate d'ammonium en présence de 0,27 g de palladium sur charbon à 10% (p/p) dans 20 $cm^3$ de méthanol, en chauffant au reflux pendant 3 heures, on obtient, après agitation de la meringue obtenue dans 30 $cm^3$ de pentane, 559 mg (83%) de 4,9-éthano-9-(4-méthoxyphényl)-2,3,3a,4,9,9a-hexahydro-1H-benzo [f] isoindole-3a-carboxylate de méthyle-(3aRS,4SR,9SR,9aRS) sous forme de solide blanc dont la caractéristique est la suivante :

- point de fusion = 112°C

Etape D

En opérant comme à l'étape A de l'exemple 5, mais à partir de 548 mg (1,51 mmol) de 4,9-éthano-9-(4-méthoxyphényl)-2,3,3a,4,9,9a-hexahydro-1H-benzo [f]isoindole-3a-carboxylate de méthyle-(3aRS,4SR,9SR,9aRS), de 251 mg (1,51 mmol) d'acide 2-méthoxyphénylacétique, de 318 mg (1,66 mmol) de chlorhydrate de 1-éthyl-3-[(3-diméthylamino)propyl]carbodiimide et de 41 mg (0,3 mmol) d'hydrate de N-hydroxybenzotriazole dans 15 $cm^3$ de dichlorométhane, on obtient, après purification par flash-chromatographie sur gel de silice (230-400 Mesh) en éluant par un mélange cyclohexane-acétate d'éthyle (90-10 en volumes), 575 mg (75%) de 4,9-éthano-9-(4-méthoxyphényl)-2-[(2-méthoxyphényl)acétyl]-2,3,3a,4,9,9a-hexahydro-1H-benzo[f]isoindole-3a-carboxylate de méthyle-(3aRS,4SR, 9SR,9aRS), sous forme d'une poudre blanche dont les caractéristiques sont les suivantes :

- point de fusion = 169°
- spectre de masse (IE) : M/Z= 511 ($M^+$)

Etape E

En opérant comme à l'exemple 2, mais à partir de 566 mg (1,1 mmol) de 4,9-éthano-9-(4-méthoxylphényl)-2-[(2-méthoxyphényl)acétyl]-2,3,3a,4,9,9a-hexahydro-1H-benzo[f]isoindole-3a-carboxylate de méthyle-(3aRS,4SR, 9SR,9aRS), au reflux pendant dix-huit heures dans 11,5 $cm^3$ d'une solution aqueuse normale d'hydroxyde de sodium et 7,5 $cm^3$ de méthanol, on obtient, après recristallisation dans 10 $cm^3$ d'oxyde de diisopropyle, 385 mg (70%) d'acide 4,9-éthano-9-(4-méthoxyphényl)-2-[(2-méthoxyphényl)acétyl]-2,3,3a,4,9,9a-hexahydro-1H-benzo [f]isoindole-3a-carboxylique (3aRS,4SR,9SR,9aRS), sous forme d'une poudre blanche dont les caractéristiques sont les suivantes :

- point de fusion = 163°C
- spectre de R.M.N. $^1H$ (400 MHz, DMSO d6, à une température de 393 K, d en ppm) : 1,36 - 1,61 - 1,90 et 2,07 (4 mts, 1H chacun : $CH_2CH_2$) ; de 3,30 à 3,65 (mt, 6H : $CH_2$ en 1 - $NCOCH_2Ar$ - 1H du $CH_2$ en 3 et H en 9a) : 3,46 (mt, 1H : H en 4) ; 3,73 (s large, 3H : $OCH_3$) ; 3,80 (s, 3H : $OCH_3$ de l'aromatique en 9) ; 4,15 (d, J = 12,5 Hz, 1H : l'autre H du $CH_2$ en 3) ; 6,45 (d large, J = 7,5 Hz, 1H : H en 8) ; 6,90 (t large. J = 7,5 Hz, 1H : H aromatique en para du $OCH_3$) ; 6,96 (d large, J = 7.5 Hz, 1H : H aromatique en ortho du $OCH_3$) ; 7,06 (d, J = 8 Hz, 2H : H aromatiques en ortho du $OCH_3$ pour l'aromatique en 9) ; de 7,00 à 7,30 (mt, 5H : H en 5 - H en 6 - H en 7 et H aromatiques en méta du $OCH_3$) ; 7,30 (d, J = 8 Hz, 2H : H aromatiques en méta du $OCH_3$ pour l'aromatique en 9).

**EXEMPLE 7**

Préparation de l'acide 4,9-éthano-2-[(2-méthoxyphényl)acétyl]-9-(4-méthylsulfanylphényl)-2,3,3a,4,9,9a-hexahydro-1H-benzo[f]isoindole-3a-carboxylique (3aRS,4SR, 9SR,9aRS),

Etape A

En opérant comme à l'étape A de l'exemple 6, mais à partir de 1,34 g (6,6 mmol) de 4-bromothioanisole et 161 mg (6,6 mmol) de magnésium en tournures, au reflux pendant trois heures dans 4 $cm^3$ d'oxyde de diéthyle, puis successivement de 543 mg (2,2 mmol) de chlorure de cérium anhydre et d'une solution de 800 mg (2,2 mmol) de 2-benzyl-7-oxo-4-phényloctahydroisoindole-3a-carboxylate de méthyle-(3aRS,4SR,7aRS), obtenu à l'étape B de l'exemple 1, dans 2 $cm^3$ d'oxyde de diéthyle, on obtient, après purification par flash-chromatographie sur gel de silice (230-400 mesh), en éluant avec du cyclohexane puis avec un mélange cyclohexane-acétate d'éthyle (80-20 en volumes), 679 g (63%) de 2-benzyl-7-hydroxy-7-(4-méthylsulfanylphényl)-4-phényloctahydroisoindole-3a-carboxylate de méthyle-(3aRS,4SR,7RS,7aRS) sous forme de solide blanc dont la caractéristique est la suivante :

- point de fusion = 124-6°C

Etape B

En opérant comme à l'étape D de l'exemple 1, mais a partir de 676 mg (1,39 mmol) de 2-benzyl-7-hydroxy-7-(4-méthylsulfanylphényl)-4-phényloctahydroisoindole-3a-carboxylate de méthyle-(3aRS,4SR,7RS,7aRS) et de 2,22 $cm^3$ d'acide trifluorométhanesulfonique dans 34 $cm^3$ de dichlorométhane, pendant trente minutes à une température voisine de 0°C, puis soixante-dix heures à température ambiante, on obtient, après purification par flash-chromatographie sur gel de silice (230-400 mesh), en éluant avec du cyclohexane puis avec un mélange cyclohexane-acétate d'éthyle (90-10 en volumes), 366 mg (56%) de 2-benzyl-4,9-éthano-9-(4-méthylsulfanylphényl)-2,3,3a,4,9,9a-hexahydro-1H-benzo[f]isoindole-3a-carboxylate de méthyle-(3aRS,4SR,9SR,9aRS) sous forme de solide blanc dont la caractéristique est la suivante :

- point de fusion = 105-7°C

Etape C

Une solution de 246 mg (0,52 mmol) de 2-benzyl-4,9-éthano-9-(4-méthylsulfanylphényl)-2,3,3a,4,9,9a-hexahydro-1H-benzo[f]isoindole-3a-carboxylate de méthyle-(3aRS,4SR,9SR,9aRS) dans 10 $cm^3$ de dichlorométhane est refroidie à 0°C sous atmosphère d'argon. On ajoute alors 115 mg (0,79 mmol) de chloroformiate de 2-chloroéthyle puis on agite la nuit à température ambiante. Après concentration du solvant sous pression réduite, le résidu est chauffé à reflux pendant quatre heures dans 10 $cm^3$ de méthanol contenant 1,5 $cm^3$ d'une solution 1M d'acide chlorhydrique dans le méthanol. Les cristaux formés sont essorés, lavés avec deux fois 1 $cm^3$ de méthanol glacé, puis neutralisés par agitation dans 10 $cm^3$ d'une solution décinormale d'hydroxyde de sodium. Après extraction au dichlorométhane et concentration à sec, on obtient 145 mg (62%) de 4,9-éthano-9-(4-méthylsulfanylphényl)-2,3,3a,4,9,9a-hexahydro-1H-benzo[f]isoindole-3a-carboxylate de méthyle-(3aRS,4SR,9SR,9aRS) sous forme d'une meringue blanche dont la caractéristique est la suivante :

- point de fusion = 90-4°C

Etape D

En opérant comme à l'étape A de l'exemple 5, mais à partir de 145 mg (0,38 mmol de 4,9-éthano-9-(4-méthylsulfanylphényl)-2,3,3a,4,9,9a-hexahydro-1H-benzo [f]isoindole-3a-carboxylate de méthyle-(3aRS,4SR,9SR,9aRS), de 64 mg (0,38 mmol) d'acide 2-méthoxyphénylacétique, de 91 mg (0,42 mmol) de chlorhydrate de 1-éthyl-3-[(3-diméthylamino)propyl]carbodiimide et de 10 mg (0,076 mmol) d'hydrate de N-hydroxybenzotriazole dans 5 $cm^3$ de dichlorométhane, on obtient, après purification par flash-chromatographie sur gel de silice (230-400 Mesh) en éluant par des mélanges cyclohexane-acétate d'éthyle (80-20 puis 70-30 en volumes), 182 mg (90%) de 4,9-éthano-2-[(2-méthoxyphényl)acétyl]-9-(4-méthylsulfanylphényl)-2,3,3a,4,9,9a-hexahydro-1H-benzo[f]isoindole-3a-carboxylate de méthyle-(3aRS,4SR,9SR,9aRS), sous forme d'une poudre blanche dont les caractéristiques sont les suivantes :

- point de fusion = 137°C.
- spectre de masse (IE) : M/Z= 527 ($M^+$)

Etape E

En opérant comme à l'exemple 2, mais à partir de 180 mg (0,34 mmol) de 4,9-éthano-2-[(2-méthoxyphényl) acétyl]-9-(4-méthylsulfanylphényl)-2,3,3a,4,9,9a-hexahydro-1H-benzo[f]isoindole-3a-carboxylate de méthyle-(3aRS, 4SR,9SR,9aRS), au reflux pendant dix-huit heures dans 3,7 $cm^3$ d'une solution aqueuse normale d'hydroxyde de sodium et 3,7 $cm^3$ de méthanol, on obtient, après recristallisation dans 5 $cm^3$ d'oxyde de diisopropyle, 144 mg (81%) d'acide 4,9-éthano-2-[(2-méthoxyphényl)acétyl]-9-(4-méthylsulfanylphényl)-2,3,3a,4,9,9a-hexahydro- 1H-benzo[f] isoindole-3a-carboxylique-(3aRS,4SR,9SR,9aRS), sous fomie d'une poudre blanche dont les caractéristiques sont les suivantes :

- point de fusion = 145°C
- spectre de R.M.N. $^1H$ (250 MHz, DMSO d6, à une température de 393 K, d en ppm) : 1,40 - 1,63 - 1,92 et 2,10 (4 mts, 1H chacun : $CH_2CH_2$); 2,59 (s, 3H : $SCH_3$); de 3,30 à 3,70 (mt, 6H : $CH_2$ en 1-$NCOCH_2Ar$ - 1H du $CH_2$ en 3 et H en 9a); 3,50 (mt, 1H : H en 4) ; 3,73 (s large, 3H : $OCH_3$) ; 4,18 (d, J = 12.5 Hz, 1H : l'autre H du $CH_2$ en 3) ; 6,48 (d large, J = 7,5 Hz, 1H : H en 8) ; 6,92 (t large, J = 7,5 Hz, 1H : H aromatique en para du $OCH_3$); 7.00 (d large, J = 7,5 Hz. 1 H : H aromatique en ortho du $OCH_3$) ; de 7,05 à 7,35 (mt, 5H : H en 5 - H en 6 - H en 7 et H aromatiques en méta du $OCH_3$) ; 7,35 et 7,45 (2 d larges, J = 8 Hz, 2H chacun : H aromatiques en ortho et méta de l'aromatique en 9).

**EXEMPLE 8**

Préparation de l'acide 4,9-éthano-9-(4-fluorophenyl)-2-[(2-méthoxyphényl)acétyl]-2,3,3a,4,9,9a-hexahydro-1H-benzo [f]isoindole-3a-carboxylique-(3aRS,4SR,9SR,9aRS)

Etape A

En opérant comme à l'étape A de l'exemple 6, à partir de 6,1 $cm^3$ d'une solution 1M de bromure de 4-fluorophénylmagnésium dans l'oxyde de diéthyle, puis successivement de 1,36 g (5,5 mmol) de chlorure de cérium anhydre, et d'une solution de 2 g (5,5 mmol) de 2-benzyl-7-oxo-4-phényloctahydroisoindole-3a-carboxylate de méthyle-(3aRS, 4SR,7aRS), obtenu à l'étape B de l'exemple 1, dans 20 $cm^3$ d'oxyde de diéthyle, on obtient, après purification par flash-chromatographie sur gel de silice (230-400 mesh), en éluant avec du cyclohexane puis avec un mélange cyclohexane-acétate d'éthyle (60-40 en volumes), 507 g (20%) de 2-benzyl-7-(4-fluorophényl)-7-hydroxy-4-phényloctahydroisoindole-3a-carboxylate de méthyle-(3aRS,4SR,7RS,7aRS) sous forme de solide blanc dont la caractéristique est la suivante :

- point de fusion = 163°C

Etape B

En opérant comme à l'étape D de l'exemple 1, à partir de 433 mg (0,94 mmol) de 2-benzyl-7-(4-fluorophényl)-7-hydroxy-4-phényloctahydroisoindole-3a-carboxylate de méthyle-(3aRS,4SR,7RS,7aRS) et de 1,51 $cm^3$ d'acide trifluorométhanesulfonique dans 20 $cm^3$ de dichlorométhanc, pendant trente minutes à une température voisine de 0°C, puis 70 heures à température ambiante, on obtient, après purification par flash-chromatographie sur gel de silice (230-400 mesh), en éluant avec du cyclohexane puis avec un mélange cyclohexane-acétate d'éthyle (90-10 en volumes), 360 mg (86%) de 2-benzyl-4,9-éthano-9-(4-fluorophényl)-2,3,3a,4,9,9a-hexahydro-1H-benzo[f]isoindole-3a-carboxylate de méthyle-(3aRS,4SR,9SR,9aRS) sous forme de solide blanc dont la caractéristique est la suivante :

- point de fusion = 157°C

Etape C

En opérant comme à l'étape E de l'exemple 1, mais a partir de 358 mg (0.81 mmol) de 2-benzyl-4,9-éthano-9-(4-fluorophényl)-2,3,3a,4,9,9a-hexahydro-1H-benzo [f]isoindole-3a-carboxylate de méthyle-(3aRS,4SR,9SR,9aRS) et de 153 mg (2,43 mmol) de formiate d'ammonium en présence de 120 mg de palladium sur charbon à 10 % (p/p) dans 20 $cm^3$ de méthanol, en chauffant au reflux pendant 3 heures, on obtient, après agitation de la meringue obtenue dans 15 $cm^3$ de pentane, 236 mg (83 %) de 4,9-éthano-9-(4-fluorophényl)-2,3,3a,4,9,9a-hexahydro-1H-benzo [f]isoindole-3a-carboxylate de méthyle-(3aRS,4SR,9SR,9aRS) sous forme de solide blanc dont la caractéristique est la suivante :

- point de fusion = 163°C

Etape D

En opérant comme à l'étape A de l'exemple 5, à partir de 235 mg (0,67 mmol) de 4,9-éthano-9-(4-fluorophényl)-2,3,3a,4,9,9a-hexahydro-1H-benzo[f] isoindole-3a-carboxylate de méthyle-(3aRS,4SR,9SR,9aRS), de 112 mg (0,67 mmol) d'acide 2-méthoxyphénylacétique, de 142 mg (0,74 mmol) de chlorhydrate de 1-éthyl-3-[(3-diméthylamino) propyl]carbodiimide et de 19 mg (0,076 mmol) d'hydrate de N-hydroxybenzotriazole dans 10 $cm^3$ de dichlorométhane, on obtient, après purification par flash-chromatographie sur gel de silice (230-400 Mesh) en éluant par des mélanges cyclohexane-acétate d'éthyle (90-10 puis 80-20 en volumes), 230 mg (68%) de 4,9-éthano-9-(4-fluorophényl)-2-[(2-méthoxyphényl)acétyl]- 2,3,3a,4,9,9a-hexahydro-1H-benzo[f]isoindole-3a-carboxylate de méthyle-(3aRS, 4SR,9SR, 9aRS), sous forme d'une poudre blanche dont les caractéristiques sont les suivantes :

- point de fusion = 216°C
- spectre de masse (IE): M/Z= 499 ($M^+$)

Etape E

En opérant comme à l'exemple 2, mais à partir de 228 mg (0.45 mmol) de 4.9-éthano-9-(4-fluorophényl)-2-[(2-méthoxyphényl)acétyl]-2,3,3a,4,9,9a-hexahydro-1H-benzo[f]isoindole-3a-carboxylate de méthyle-(3aRS,4SR,9SR,9aRS), au reflux pendant dix huit heures dans 4,7 $cm^3$ d'une solution aqueuse normale d'hydroxyde de sodium et 5 $cm^3$ de méthanol, on obtient. après recristallisation dans 5 $cm^3$ d'oxyde de diisopropyle, 171 mg (77%) d'acide 4.9-éthano-9-(4-fluorophényl)-2-[(2-méthoxyphényl)acétyl]-2,3,3a,4,9,9a-hexahydro-1H-benzo[f]isoindole-3a-carboxylique-(3aRS,4SR,9SR,9aRS), sous forme d'une poudre blanche dont les caractéristiques sont les suivantes :

- point de fusion = 250°C
- spectre de R.M.N. $^1H$ (400 MHz, DMSO d6, à une température de 423 K, d en ppm) : 1,40 - 1,64 - 1,95 et 2,10 (4 mts, 1H chacun : $CH_2CH_2$) ; 2,41 (s, 3H : $ArCH_3$) ; de 3,30 à 3,65 (mt, 6H : $CH_2$ en 1 - $NCOCH_2Ar$ - 1H du $CH_2$ en 3 et H en 9a) ; 3,48 (mt, 1H : H en 4) ; 3,75 (s, 3H : $OCH_3$) ; 4,18 (d, J = 12,5 Hz, 1H: l'autre H du $CH_2$ en 3) ; 6.46 (d large, J = 7,5 Hz, 1H ; H en 8) ; 6,90 (t large, J = 7,5 Hz, 1H : H aromatique en para du $OCH_3$) ; 6,96 (d large, J = 7,5 Hz, 1H : H aromatique en ortho du $OCH_3$) ; de 7,00 à 7,35 (mt, 5H : H en 5 - H en 6 - H en 7 et H aromatiques en méta du $OCH_3$) ; 7,30 (AB limite, 4H : H aromatiques en ortho et méta de l'aromatique en 9).

**EXEMPLE 9**

Préparation de l'acide 4,9-éthano-2-[(2-méthoxyphényl)acétyl]-9-(3-méthylphényl)-2,3,3a,4,9,9a-hexahydro-1H-benzo[f]isoindole-3a-carboxylique-(3aRS,4SR,9SR,9aRS)

Etape A

En opérant comme à l'étape A de l'exemple 6, mais à partir de 2,42 $cm^3$ d'une solution 1M de bromure de méta-tolylmagnésium dans le tétrahydrofurane puis successivement de 543 mg (2,2 mmol) de chlorure de cérium anhydre, et d'une solution de 800 mg (2,2 mmol) de 2-benzyl-7-oxo-4-phényloctahydroisoindole-3a-carboxylate de méthyle-(3aRS,4SR,7aRS), obtenu à l'étape B de l'exemple 1, dans 10 $cm^3$ d'oxyde de diéthyle, on obtient, après purification par flash-chromatographie sur gel de silice (230-400 mesh), en éluant avec du cyclohexane puis avec un mélange cyclohexane-acétate d'éthyle (80-20 en volumes), 505 mg (50%) de 2-benzyl-7-hydroxy-7-(3-méthylphényl)-4-phényloctahydroisoindole-3a-carboxylate de méthyle-(3aRS,4SR,7RS,7aRS) sous forme de solide blanc dont la caractéristique est la suivante :

- point de fusion = 117-9°C

Etape B

En opérant comme à l'étape D de l'exemple 1, mais à partir de 669 mg (1,47 mmol) de 2-benzyl-7-hydroxy-7-(3-méthylphényl)-4-phényloctahydroisoindole-3a-carboxylate de méthyle-(3aRS,4SR,7RS,7aRS) et de 2,35 $cm^3$ d'acide trifluorométhanesulfonique dans 33 $cm^3$ de dichlorométhane, pendant trente minutes à une température voisine de 0°C, puis quarante huit heures à température ambiante, on obtient, après purification par flash-chromatographie sur gel de silice (230-400 mesh), en éluant avec du cyclohexane puis avec un mélange cyclohexane-acétate d'éthyle (90-10 en volumes), 582 mg (90%) de 2-benzyl-4,9-éthano-9-(3-méthylphényl)-2,3,3a,4,9,9a-hexahydro-1H-benzo[f] isoindole-3a-carboxylate de méthyle-(3aRS,4SR,9SR,9aRS) sous forme de solide blanc dont la caractéristique est la suivante :

- point de fusion = 113-5°C

Etape C

En opérant comme à l'étape E de l'exemple 1, mais à partir de 578 mg (1,27 mmol) de 2-benzyl-4,9-éthano-9-(3-méthylphényl)-2,3,3a,4,9,9a-hexahydro-1H-benzo[f]isoindole-3a-carboxylate de méthyle-(3aRS,4SR,9SR,9aRS) et de 241 mg (3,82 mmol) de formiate d'ammonium en présence de 185 mg de palladium sur charbon à 10 % (p/p) dans 20 $cm^3$ de méthanol, en chauffant au reflux pendant 3 heures, on obtient, après agitation de la meringue obtenue dans 10 $cm^3$ de pentane, 412 mg (93%) de 4,9-éthano-9-(3-methylphény])-2,3,3a,4,9,9a-hexahydro-1H-benzo[f]isoindole-3a-carboxylate de méthyle-(3aRS,4SR,9SR,9aRS) sous forme de solide blanc dont la caractéristique est la suivante :

- point de fusion = 103°C

Etape D

En opérant comme à l'étape A de l'exemple 6, mais à partir de 409 mg (1,18 mmol) de 4,9-éthano-9-(3-méthylphényl)-2,3,3a,4,9,9a-hexahydro-1H-benzo[f] isoindole-3a-carboxylate de méthyle-(3aRS,4SR,9SR,9aRS), de 196 mg (1,18 mmol) d'acide 2-méthoxyphénylacétique, de 248 mg (1,29 mmol) de chlorhydrate de 1-éthyl-3-[(3-diméthylamino)propyl]carbodiimide et de 32 mg (0,076 mmol) d'hydrate de N-hydroxybenzotriazole dans 12 $cm^3$ de dichlorométhane, on obtient, après purification par flash-chromatographie sur gel de silice (230-400 Mesh) en éluant par du cyclohexane puis par un mélange cyclohexane-acétate d'éthyle (90-10 en volumes), 509 mg (86%) de 4,9-éthano-2-[(2-méthoxyphényl)acétyl]-9-(3-méthylphényl)-2,3,3a,4,9,9a-hexahydro-1H-benzo[f]isoindole-3a-carboxylate de méthyle-(3aRS,4SR,9SR,9aRS), sous forme d'une poudre blanche dont les caractéristiques sont les suivantes :

- point de fusion = 132°C
- spectre de masse (IE) : M/Z= 495 ($M^+$)

Etape E

En opérant comme à l'exemple 2, mais à partir de 506 mg (1,02 mmol) de 4,9-éthano-2-[(2-méthoxyphényl) acétyl] -9-(3-méthylphényl)-2,3,3a,4,9,9a-hexahydro-1H-benzo[f]isoindole-3a-carboxylate de méthyle-(3aRS,4SR, 9SR,9aRS), au reflux pendant dix-huit heures dans 10,3 $cm^3$ d'une solution aqueuse normale d'hydroxyde de sodium et 7 $cm^3$ de méthanol, on obtient, après recristallisation dans 5 $cm^3$ d'oxyde de diisopropyle, 424 mg (87%) d'acide 4,9-éthano-2-[(2-méthoxyphényl)acétyl]-9-(3-méthylphényl)-2,3,3a,4,9,9a-hexahydro-1H-benzo[f] isoindole-3a-carboxylique-(3aRS,4SR,9SR,9aRS), sous forme d'une poudre blanche dont les caractéristiques sont les suivantes :

- point de fusion = 138°C
- spectre de R.M.N. $^1H$ (250 MHz, DMSO d6, à une température de 393 K, d en ppm) : 1,40 - 1,66 - 1,95 et 2,10 (4 mts, 1H chacun : $CH_2CH_2$) ; 2,43 (s, 3H : $ArCH_3$) ; de 3,30 à 3,65 (mt, 6H : $CH_2$ en 1 - $NCOCH_2Ar$ - 1H du $CH_2$ en 3 et H en 9a) ; 3,48 (mt, 1H : H en 4) ; 3,73 (s large, 3H : $OCH_3$) ; 4,18 (d, J = 12,5 Hz, 1H : l'autre H du $CH_2$ en 3) ; 6,45 (d large, J = 7,5 Hz, 1H : H en 8) ; 6,92 (t large, J = 7,5 Hz, 1H · H aromatique en para du $OCH_3$) ; 6,98 (d large, J = 7,5 Hz, 1H : H aromatique en ortho du $OCH_3$) ; de 7,00 à 7,35 (mt, 8H : H en 5 - H en 6 - H en 7 - H aromatiques en méta du $OCH_3$ et H aromatiques en ortho et en para du $CH_3$ pour l'aromatique en 9) ; 7,40 (t, J = 8 Hz, 1H : H aromatique en méta du $CH_3$ pour l'aromatique en 9).

## EXEMPLE 10

Préparation de l'acide 4,9-éthano9-(3-méthoxyphényl)-2-[(2-méthoxyphényl)acétyl]-2,3,3a,4,9,9a-hexahydro-1H-benzo[f]isoindole-3a-carboxylique-(3aRS,4SR,9SR,9aRS)

Etape A

En opérant comme à l'étape A de l'exemple 6, mais à partir de 1,56 $cm^3$ (12,4 mmol) de 3-bromoanisole et de 302 mg (12,4 mmol) de magnésium en tournures au reflux pendant une heure dans 5 $cm^3$ d'oxyde de diéthyle sec, puis successivement de 1,02 g (4,13 mmol) de chlorure de cérium anhydre et d'une solution de 1,5 g (4,13 mmol) de 2-benzyl-7-oxo-4-phényloctahydroisoindole-3a-carboxylate de méthyle-(3aRS,4SR,7aRS), obtenu à l'étape B de l'exemple 1, dans 8 $cm^3$ d'oxyde de diéthyle, on obtient, après purification par flash-chromatographie sur gel de silice (230-400 mesh), en éluant avec du cyclohexane puis avec un mélange cyclohexane-acétate d'éthyle (80-20 en volumes), 945 mg (48%) de 2-benzyl-7-hydroxy-7-(3-méthoxyphényl)-4-phényloctahydroisoindole-3a-carboxylate de méthyle-(3aRS,4SR,7RS,7aRS) sous forme de solide blanc dont la caractéristique est la suivante :

- point de fusion = 118°C

Etape B

En opérant comme à l'étape D de l'exemple 1, mais à partir de 941 mg (1,99 mmol) de 2-benzyl-7-hydroxy-7-(3-méthoxyphényl)-4-phényloctahydroisoindole-3a-carboxylate de méthyle-(3aRS,4SR,7RS,7aRS) et de 3,19 $cm^3$ d'acide trifluorométhanesulfonique dans 45 $cm^3$ de dichlorométhane, pendant 30 minutes à une température voisine de 0°C, puis quarante huit heures à température ambiante, on obtient, après purification par flash-chromatographie

sur gel de silice (230-400 mesh), en éluant avec du cyclohexane puis avec un mélange cyclohexane-acétate d'éthyle (90-10 en volumes), 755 mg (83%) de 2-benzyl-4,9-éthano-9-(3-méthoxyphényl)-2,3,3a,4,9,9a-hexahydro-1H-benzo [f]isoindole-3a-carboxylate de méthyle-(3aRS,4SR,9SR,9aRS) sous forme de solide blanc dont la caractéristique est la suivante :

- point de fusion = 117°C

Etape C

En opérant comme à l'étape E de l'exemple 1, mais à partir de 747 mg (1,65 mmol) de 2-benzyl-4,9-éthano-9-(3-méthoxyphényl)-2,3,3a,4,9,9a-hexahydro-1H-benzo[f]isoindole-3a-carboxylate de méthyle-(3aRS,4SR,9SR,9aRS) et de 311 mg (4,94 mmol) de formiate d'ammonium en présence de 240 mg de palladium sur charbon à 10 % (p/p) dans 18 $cm^3$ de méthanol, en chauffant au reflux pendant trois heures, on obtient, après agitation de la meringue obtenue dans 10 $cm^3$ de pentane, 501 mg (83 %) de 4,9-éthano-9-(3-méthoxyphényl)-2,3,3a,4,9,9a-hexahydro-1H-benzo[f]isoindole-3a-carboxylate de méthyle-(3aRS,4SR,9SR,9aRS) sous forme de solide blanc dont la caractéristique est la suivante :

- point de fusion = 96-8°C

Etape D

En opérant comme à l'étape A de l'exemple 5, mais à partir de 500 mg (1,38 mmol) de 4,9-éthano-9-(3-méthoxyphényl)-2,3,3a,4,9,9a-hexahydro-1H-benzo [f]isoindole-3a-carboxylate de méthyle-(3aRS,4SR,9SR,9aRS), de 229 mg (1,38 mmol) d'acide 2-méthoxyphénylacétique, de 291 mg (1,52 mmol) de chlorhydrate de 1-éthyl-3-[(3-diméthylamino)propyl]carbodiimide et de 37 mg (0,27 mmol) d'hydrate de N-hydroxybenzotriazole dans 14 $cm^3$ de dichlorométhane, on obtient, après purification par flash-chromatographie sur gel de silice (230-400 Mesh) en éluant par du cyclohexane puis par un mélange cyclohexane-acétate d'éthyle (80-20 en volumes), 451 mg (64%) de 4,9-éthano-9-(3-méthoxyphényl)-2-[(2-méthoxyphényl)acétyl]-2,3,3a,4,9,9a-hexahydro-1H-benzo[f]isoindole-3a-carboxylate de méthyle-(3aRS,4SR,9SR,9aRS), sous forme d'une poudre blanche dont les caractéristiques sont les suivantes :

- point de fusion = 128°C
- spectre de masse (IE) : M/Z= 511 ($M^+$)

Etape E

En opérant comme à l'exemple 2, à partir de 450 mg (0,88 mmol) de 4,9-éthano-9-(3-méthoxyphényl)-2-[(2-méthoxyphényl)acétyl]-2,3,3a,4,9,9a-hexahydro-1H-benzo[f]isoindole-3a-carboxylate de méthyle-(3aRS,4SR,9SR,9aRS), au reflux pendant 18 heures dans 4,7 $cm^3$ d'une solution aqueuse normale d'hydroxyde de sodium et 5 $cm^3$ de méthanol, on obtient, après recristallisation dans 10 $cm^3$ d'oxyde de diisopropyle, 391 mg (89%) d'acide 4,9-éthano-9-(3-méthoxyphényl)-2-[(2-méthoxyphényl)acétyl]-2,3,3a,4,9,9a-hexahydro-1H-benzo[f]isoindole-3a-carboxylique-(3aRS,4SR,9SR,9aRS), sous forme d'une poudre blanche dont les caractéristiques sont les suivantes :

- point de fusion = 135°C
- spectre de R.M.N. $^1H$ (250 MHz, DMSO d6, à une température de 393 K, d en ppm) : 1,40 - 1,63 - 1,94 et 2,10 (4 mts, 1H chacun : $CH_2CH_2$) ; de 3,35 à 3,70 (mt, 6H : $CH_2$ en 1 - $NCOCH_2Ar$ - 1H du $CH_2$ en 3 et H en 9a) ; 3,50 (mt, 1H : H en 4) ; 3,73 (s large, 3H : $OCH_3$) ; 3,87 (s, 3H : $OCH_3$ de l'aromatique en 9) ; 4,18 (d, J = 12,5 Hz, 1H : l'autre H du $CH_2$ en 3): 6,48 (d large, J = 7,5 Hz, 1H : H en 8) ; de 6,85 à 7,35 (mt, 10H : H en 5 - H en 6 - H en 7 - H aromatiques en ortho - méta et para du $OCH_3$ et H aromatiques en ortho et para du $OCH_3$ pour l'aromatique en 9) ; 7.43 (t, J = 8 Hz, 1H : H aromatique en méta du $OCH_3$ pour l'aromatique en 9).

**EXEMPLE 11**

Préparation de l'acide 9-(3,4-diméthylphényl)-4,9-éthano-2-[(2-méthoxyphényl) acétyl]-2,3,3a,4,9,9a-hexahydro-1H-benzo[f]isoindole-3a-carboxylate de méthyle-(3aRS,4SR,9SR,9aRS)

EtapeA

2,3 $cm^3$ de 4-bromo-ortho-xylene et 0,42 g de magnésium en tournures dans un mélange de 20 $cm^3$ d'oxyde

de diéthyle et 20 $cm^3$ de tétrahydrofurane sont chauffés au reflux pendant une heure. Après refroidissement à une température voisine de -7°C, une solution de 3,11 g de 2-benzyl-7-oxo-4-phényloctahydroisoindole-3a-carboxylate de méthyle-(3aRS,4SR,7aRS) dans 20 $cm^3$ d'oxyde de diéthyle est additionnée en 25 minutes. Le mélange réactionnel est agité pendant quatre heures à une température voisine de 25°C puis hydrolyse par 50 $cm^3$ d'une solution aqueuse saturée en chlorure d'ammonium. La phase aqueuse est séparée par décantation et extraite par 25 $cm^3$ d'acétate d'éthyle. Les phases organiques sont réunies, séchées sur sulfate de magnésium et concentrées sous pression réduite. Après purification par flash-chromatographie sur gel de silice (230-400 mesh), en éluant avec un mélange cyclohexane-acétate d'éthyle (80-20 en volumes), on obtient 1,995 g de 2-benzyl-7-hydroxy-7-(3,4-diméthylphényl)-4-phényloctahydroisoindole-3a-carboxylate de méthyle-(3aRS,4SR,7RS,7aRS), utilisé tel quel pour l'étape suivante dont la caractéristique est la suivante :

- spectre de masse (IE) : M/Z= 469 ($M^+$) et 410 ($M^+$-$CO_2$).

Etape B

En opérant comme à l'étape D de l'exemple 1, mais en partant de 1,99 g du mélange obtenu à l'étape précédente, en solution dans 100 $cm^3$ de dichlorométhane, maintenu à une température voisine de -5°C, et de 3,7 $cm^3$ d'acide trifluorométhanesulfonique, en agitant 72 heures à une température voisine de 25°C, on obtient 1,63 g de 2-benzyl-9-(3,4-diméthylphényl)-4,9-éthano-2,3,3a,4,9,9a-hexahydro-1H-benzo[f]isoindole-3a-carboxylate de méthyle-(3aRS,4SR,9SR,9aRS) sous forme d'huile brune, utilisée telle qu'elle à l'étape suivante, dont la caractéristique est la suivante

- spectre de masse (IE) : M/Z= 451 ($M^+$).

Etape C

On opère comme à l'étape E de l'exemple 1, mais en panant de 1,63 g de 2-benzyl-9-(3,4-diméthylphényl)-4,9-éthano-2,3,3a,4,9,9a-hexahydro-1H-benzo[f] isoindole-3a-carboxylate de méthyle-(3aRS,4SR,9SR,9aRS), dans 40 $cm^3$ de méthanol et de 0,68 g de formiate d'ammonium en présence de 0,41 g de palladium sur charbon à 10 % (p/p), en chauffant au reflux pendant deux heures, on obtient 0.79 g de 9-(3,4-diméthylphényl)-4,9-éthano-2,3,3a,4,9,9a-hexahydro-1H-benzo[f] isoindole-3a-carboxylate de méthyle-(3aRS,4SR,9SR,9aRS) sous forme de solide blanc, dont la caractéristique est la suivante :

- spectre de masse (IE) : M/Z = 361 ($M^+$).

Etape D

En opérant comme à l'étape A de l'exemple 5, mais à partir de 0,79 g de 9-(3,4-diméthylphényl)-4,9-éthane-2,3,3a,4,9,9a-hexahydro-1H-benzo[f]isoindole-3a-carboxylate de méthyle-(3aRS,4SR,9SR,9aRS), de 0,44 g d'acide 2-méthoxyphénylacétique, de 0,5 g de chlorhydrate de 1-éthyl-3-[(3-diméthylamino)propyl]carbodiimide et de 0,35 g d'hydrate de N-hydroxybenzotriazole dans 35 $cm^3$ de dichlorométhane, on obtient après purification par flash-chromatographie sur 37 g de gel de silice (230-400 Mesh) en éluant par du dichlorométhane pur, puis par un mélange dichlorométhane-éthanol (99-1 en volumes), 0,7 g de 9-(3,4-diméthylphényl)-4,9-éthano-2-[(2-méthoxyphényl)acétyl]-2,3,3a,4,9,9a-hexahydro-1H-benzo[f]isoindole-3a-carboxylate de méthyle-(3aRS, 4SR,9SR,9aRS), sous forme de meringue que l'on recristallise de la manière suivante: cette meringue est dissoute dans 5 $cm^3$ d'éthanol absolu, additionnée d'eau jusqu'à obtention d'un trouble, refroidie une heure à une température voisine de 0°C. On obtient ainsi 0,61 g de 9-(3,4-diméthylphényl)-4,9-éthano-2-[(2-méthoxyphényl)acétyl]-2,3,3a,4,9,9a-hexahydro-1H-benzo[f]isoindole-3a-carboxylate de méthyle-(3aRS,4SR,9SR,9aRS), sous forme de poudre jaune dont les caractéristiques sont les suivantes :

- point de fusion = 88°C
- spectre de masse (IE) : M/Z= 509 ($M^+$)

**EXEMPLE 12**

Préparation de l'acide 9-(3,4-diméthylphényl)-4,9-éthano-2-[(2-méthoxyphenyl) acétyl]-2,3,3a,4,9,9a-hexahydro-1H-benzo[f]isoindole-3a-carboxylique-(3aRS,4SR,9SR,9aRS)

En opérant comme à l'exemple 2, à partir de 637 mg de 9-(3,4-diméthylphényl)-4,9-éthano-2-[(2-méthoxyphényl)acétyl]-2,3,3a,4,9,9a-hexahydro-1H-benzo[f]isoindole-3a-carboxylate de méthyle-(3aRS,4SR,9SR,9aRS) au reflux pendant 18 heures dans 1,9 $cm^3$ d'une solution aqueuse normale d'hydroxyde de sodium et 60 $cm^3$ d'éthanol, on obtient, après recristallisation dans 5 $cm^3$ d'oxyde de diisopropyle, 176 mg d'acide 9-(3,4-diméthylphényl)-4,9-éthano-2-[(2-méthoxy-phényl)acétyl]-2,3,3a,4,9,9a-hexahydro-1H-benzo[f]isoindole-3a-carboxylique-(3aRS,4SR,9SR, 9aRS), sous forme d'une poudre blanche dont les caractéristiques sont les suivantes :

- point de fusion = 257°C
- spectre de masse (IE) : M/Z= 495 ($M^+$) et 451 ($M^+$ - $CO_2$)

**EXEMPLE 13**

Préparation du 3a-N-benzylcarbamoyl-4,9-ethano-2-[2-(2-méthoxyphényl)propènoyl]-9-(4-méthylphényl)-2,3,3a, 4,9,9a-hexahydro-1H-benzo[f]isoindole-(3aRS,4SR,9SR,9aRS)

A une solution de 1 g d'acide 4,9-éthano-2-[2-(2-méthoxyphényl) propènoyl]-9-(4-méthylphényl)-2,3,3a, 4,9,9a-hexahydro-1H-benzo[f]isoindole-3a-carboxylique-(3aRS,4SR,9SR,9aRS) dans 10 $cm^3$ de dichlorométhane contenant 1 goutte de N,N-diméthylformamide on ajoute goutte à goutte une solution de 0,19 $cm^3$ de chlorure d'oxalyle dans 5 $cm^3$ de dichlorométhane. Le mélange réactionnel est encore agité pendant 2 heures à une température voisine de 20°C puis refroidi à une température voisine de 0°C et coulé goutte à goutte dans une solution de 0,22 $cm^3$ de benzylamine dans 10 $cm^3$ de dichlorométhane et 0.57 $cm^3$ de triéthylarnine en maintenant la température au voisinage de 0°C. Le mélange réactionnel est encore agite pendant 15 minutes à une température voisine de 0°C puis pendant deux heures à une température voisine de 20°C et versé dans 50 $cm^3$ d'eau distillée. La phase organique est séparée par décantation, lavée par 50 $cm^3$ d'eau distillée puis 50 $cm^3$ d'une solution aqueuse saturée en chlorure de sodium, séchée sur sulfate de magnésium et concentrée sous pression réduite. Après purification par flash-chromatographie sur gel de silice (230-400 mesh), en éluant avec un mélange cyclohexane-acétate d'éthyle (50-50 en volumes), on obtient 0,8 g de 3a-N-benzylcarbamoyl-4,9-éthano-2-[2-(2-méthoxyphényl)propènoyl]-9-(4-méthylphényl)-2,3,3a, 4,9,9a-hexahydro-1H-benzo[f]isoindole-(3aRS,4SR,9SR,9aRS) sous forme de solide blanc dont les caractéristiques sont les suivantes :

- point de fusion = 227°C
- spectre de R.M.N. $^1H$ (300 MHz, DMSO d6, à une température de 413 K, d en ppm) : 1,40 - 1,64 - 2,02 et 2,15 (4 mts, 1H chacun : $CH_2CH_2$) ; 2,39 (s, 3H : $ArCH_3$) ; 3,31 et 3,40 (respectivement mt et d large, J = 13Hz, 1H chacun : $CH_2$ en 1); 3,50 et 4,20 (respectivement d et d large, J = 13 Hz, 1H chacun : $CH_2$ en 3) ; 3,59 (mt, 1H : H en 9a) ; 3,61 (s large, 1H : H en 4) ; 3,71 (s, 3H : $ArOCH_3$) ; 4,22 (AB limite, 2H : $CH_2NAr$) ; 5,52 et 5,67 (2s, 1H chacun ; $=CH_2$) ; 6,44 (d large, J = 7,5 Hz, 1H : H en 8) ; 6,95 (t large, J = 7,5 Hz, 1H : H aromatique en para du $OCH_3$) ; 7,01 (d large, J = 7.5 Hz, 1H : H aromatique en ortho du $OCH_3$) ; de 6,95 à 7,35 (mt, 14H : H en 5 - H en 6 - H en 7 - H aromatiques en méta du $OCH_3$ - H en ortho et méta de l'aromatique en 9 et H aromatiques du benzyle) ; 7,75 (mf, 1H : CONH).

**EXEMPLE 14**

Préparation du 4,9-éthano-2-[2-(2-méthoxyphényl)propènoyl]-9-(4-méthylphényl)-2,3,3a,4,9,9a-hexahydro-1H-benzo [f]isoindole-3a-hydroxamate de benzyle-(3aRS,4SR,9SR,9aRS)

En opérant comme à l'exemple 13, mais à partir de 2,49 g d'acide 4,9-éthano-2-[2-(2-méthoxyphényl)propènoyl]-9-(4-méthylphényl)-2,3,3a,4,9,9a-hexahydro-1H-benzo[f]isoindole-3a-carboxylique-(3aRS,4SR,9SR,9aRS) dans 20 $cm^3$ de dichlorométhane contenant 2 gouttes de N,N-diméthylformamide, d'une solution de 0,48 $cm^3$ de chlorure d'oxalyle dans 5 $cm^3$ de dichlorométhane et d'une solution de 0,8 g de chlorhydrate de O-benzylhydroxylamine dans un mélange de 2,1 $cm^3$ de triéthylamine et de 20 $cm^3$ de dichlorométhane, on obtient 2,4 g de 4,9-éthano-2-[2-(2-méthoxyphényl)propènoyl]-9-(4-méthylphényl)-2,3,3a,4,9,9a-hexahydro-1H-benzo[f]isoindole-3a-hydroxamate de benzyle-(3aRS,4SR,9SR,9aRS) sous forme de solide blanc dont les caractéristiques sont les suivantes :

- point de fusion = 227°C
- spectre de R.M.N. $^1H$ (300 MHz, DMSO d6, à une température de 413 K, d en ppm) 1,38 - 1,61 - 1,98 et 2,10 (4 mts, 1H chacun : $CH_2CH_2$) ; 2,39 (s, 3H : $ArCH_3$) ; 3,26 et 3,38 (respectivement dd et d très large, J = 13 et 9 Hz et J = 13 Hz, 1H chacun : $CH_2$ en 1) ; 3.46 et 4,14 (respectivement d et d large, J = 13 Hz, 1H chacun : $CH_2$ en 3) ; 3,50 (mt, 1H: H en 4); 3,52 (dt, J = 9 et 3 Hz, 1H : H en 9a) ; 3,72 (s, 3H: $ArOCH_3$) ; 4,63 et 4,70 (2 d, J = 11 Hz, 1H chacun : $OCH_2Ar$) ; 5,52 et 5,68 (respectivement s et s large, 1H chacun : $=CH_2$) ; 6,44 (d large, J = 7,5 Hz, 1H : H en 8) ; 6,95 (t large, J = 7,5 Hz, 1H : H aromatique en para du $OCH_3$) ; de 7,00 à 7,40 (mt, 15H : H en 5 - H en 6 - H en 7 - H aromatiques en méta et ortho du $OCH_3$ - H en ortho et méta de l'aromatique en 9 et H aromatiques du benzyle) ; 10,83 (mf, 1H : CONH).

**EXEMPLE 15**

Préparation du 3a-carbamoyl-4,9-éthano-2-(2-méthoxyphényl)propénoyl]-9-(4-méthylphényl)-2,3,3a,4,9,9a-hexahydro-1H-benzo[f]isoindole-(3aRS,4SR,9SR,9aRS)

A une solution de 1,24 g d'acide 4,9-éthano-2-[2-(2-méthoxyphényl) propènoyl]-9-(4-méthylphényl)-2,3,3a, 4,9,9a-hexahydro-1H-benzo[f]isoindole-3a-carboxylique-(3aRS,4SR,9SR,9aRS) dans 10 $cm^3$ de dichlorométhane contenant 1 goutte de N,N-diméthylformamide on ajoute goutte à goutte une solution de 0,24 $cm^3$ de chlorure d'oxalyle dans 5 $cm^3$ de dichlorométhane. Le mélange réactionnel est encore agité pendant deux heures à une température voisine de 20°C puis concentré sous pression réduite. Le résidu est dissout dans 10 $cm^3$ de dioxane et coulé goutte à goutte dans 10 $cm^3$ d'une solution aqueuse 2,5 N d'ammoniaque en maintenant la température au voisinage de 0°C. Le mélange réactionnel est encore agité pendant une heure à une température voisine de 0°C puis filtré. Le précipité est lavé successivement par trois fois 50 $cm^3$ d'eau distillée et 50 $cm^3$ d'oxyde de diisopropyle puis séché sous pression réduite à une température voisine de 40°C. On obtient ainsi, après recristallisation dans le méthanol, 0,85 g de 3a-carbamoyl-4,9-éthano-2-[2-(2-méthoxyphényl)propènoyl]-9-(4-méthylphényl)-2,3,3a,4,9,9a-hexahydro-1H-benzo[f]isoindole-(3aRS,4SR,9SR,9aRS) sous forme de solide blanc dont les caractéristiques sont les suivantes :

- point de fusion : > 260°C
- spectre de R.M.N. $^1H$ (300 MHz, DMSO d6, à une température de 413 K, d en ppm) 1,38 - 1,60 - 1,98 et 2,12 (4 mts, 1H chacun : $CH_2CH_2$) ; 2,39 (s, 3H : $ArCH_3$) ; 3,26 et 3,36 (respectivement dd et d très large, J = 13 et 9 Hz et J = 13 Hz, 1H chacun : $CH_2$ en 1) ; 3,48 et 4,16 (respectivement d et d large, J = 13 Hz, 1H chacun : $CH_2$ en 3) : 3,50 (dt, J = 9 et 3 Hz, 1H : H en 9a) ; 3,56 (mt, 1H : H en 4) ; 3,72 (s, 3H : $ArOCH_3$) ; 5;52 et 5;68 (respectivement s et s large, 1H chacun : $=CH_2$) ; 6,42 (d large, J = 7,5 Hz. 1H : H en 8) ; 6,70 (mf, 2H : $CONH_2$) ; 6,95 (t large, J = 7,5 Hz, 1H : H aromatique en para du $OCH_3$) ; 7,03 (d large, J = 7,5 Hz, 1H : H aromatique en ortho du $OCH_3$) ; de 7,00 à 7,40 (mt, 9H : H en 5 - H en 6 - H en 7 - H aromatiques en méta du $OCH_3$ et H en ortho et méta de l'aromatique en 9).

**EXEMPLE 16**

Préparation de l'acide 4,9-éthano-2-[2-(2-méthoxyphényl)propénoyl]-9-(4-méthylphényl)-2,3,3a,4,9,9a-hexahydro-1H-benzo[f]isoindole-3a-hydroxamique-(3aRS,4SR,9SR,9aRS)

A une solution de 1,3 g de 4,9-éthano-2-[2-(2-méthoxyphényl)propenoyl]-9-(4-méthylphényl)-2,3,3a,4,9,9a-hexahydro-1H-benzo[f]isoindole-3a-hydroxamate de benzyle-(3aRS,4SR,9SR,9aRS) dans un mélange de 10 $cm^3$ de nitrométhane et 10 $cm^3$ de dichlorométhane refroidie à une température voisine de -15°C, on ajoute 0,61 $cm^3$ d'anisole puis 0,72 g de chlorure d'aluminium. Le mélange réactionnel est agité pendant une heure à une température voisine de 20°C puis concentré sous pression réduite. Le résidu est repris par 50 $cm^3$ d'une solution aqueuse normale d'acide chlorhydrique et extrait par deux fois 50 $cm^3$ de dichlorométhane. Les extraits sont réunis, lavés successivement par 50 $cm^3$ d'eau distillée, 50 $cm^3$ d'une solution aqueuse saturée en chlorure de sodium, séchés sur sulfate de magnésium et concentrés sous pression réduite. Le résidu est purifié par flash-chromatographie sur gel de silice (230-400 mesh), en éluant avec un mélange dichlorométhane-méthanol (90-10 en volumes). On obtient ainsi, après recristallisation dans l'acétonitrile, 0,75 g d'acide 4,9-éthano-2-[2-(2-méthoxyphényl)propènoyl]-9-(4-méthylphényl)-2,3,3a, 4,9,9a-hexahydro-1H-benzo[f]isoindole-3a-hydroxamique-(3aRS,4SR,9SR,9aRS) sous forme de solide crème dont les caractéristiques sont les suivantes :

- point de fusion = 252°C

- spectre de R.M.N. $^1H$ (300 MHz, $(CD_3)_2SO$ d6, à une température de 413 K, d en ppm) : 1,36 - 1,60 - 1,98 et 2,08

(4 mts, 1H chacun : $CH_2CH_2$) ; 2,39 (s, 3H : $ArCH_3$) ; 3,26 et de 3,30 à 3,45 (respectivement dd et mt, J = 13 et 9 Hz, 1H chacun : $CH_2$ en 1) ; 3,43 et 4,12 (respectivement d et d large, J = 13 Hz, 1H chacun : $CH_2$ en 3) ; 3,50 (dt, J = 9 et 3 Hz, 1H : H en 9a) ; 3,56 (mt, 1H : H en 4) ; 3,72 (s, 3H : $ArOCH_3$) ; 5;52 et 5;68 (respectivement s et s large, 1H chacun : $=CH_2$) ; 6,42 (d large, J = 7,5 Hz, 1H : H en 8) ; 6,95 (t large, J = 7,5 Hz, 1H : H aromatique en para du $OCH_3$) ; 7,03 (d large, J = 7,5 Hz, 1H : H aromatique en ortho du $OCH_3$) ; de 7,00 à 7,40 (mt, 9H : H en 5 - H en 6 - H en 7 - H aromatiques en méta du $OCH_3$ et H en ortho et méta de l'aromatique en 9). A température ambiante, nous observons les signaux correspondant aux échangeables : 8.70 (mf, 1H : CONH) ; 10,65 (mf, 1H : OH).

**EXEMPLE 17**

Préparation du 4,9-éthano-2-[2-(2-méthoxyphényl)propènoyl]-9-(4-méthylphényl)-2,3,3a,4,9,9a-hexahydro-1H-benzo[f]isoindole-3a-N-(3-pyridylméthyl)-carboxamide-(3aRS,4SR,9SR,9aRS)

A une solution de 1,24 g d'acide 4,9-éthano-2-[2-(2-méthoxyphényl)propènoyl]-9-(4-méthylphényl)-2,3,3a,4,9,9a-hexahydro-1H-benzo[f]isoindole-3a-carboxylique-(3aRS,4SR,9SR,9aRS) dans 10 $cm^3$ de dichlorométhane et une goutte de N,N-diméthylformamide, on ajoute goutte à goutte une solution de 0,24 $cm^3$ de chlorure d'oxalyle dans 5 $cm^3$ de dichlorométhane. Le mélange réactionnel est agité pendant deux heures à une température voisine de 20°C puis refroidi à une température voisine de 0°C et coulé sur une solution de 0,26 $cm^3$ de 3-(aminométhyl)pyridine et de 0,7 $cm^3$ de triéthylamine dans 10 $cm^3$ de dichlorométhane refroidie à une température voisine de 0°C. Le mélange réactionnel est agité à une température voisine de 0°C pendant quinze minutes puis voisine de 20°C pendant deux heures puis coulé dans 100 $cm^3$ d'eau distillée. La phase aqueuse est séparée par décantation et extraite par deux fois 50 $cm^3$ de dichlorométhane. Les phases organiques sont réunies, lavées par 50 $cm^3$ d'eau distillée puis 50 $cm^3$ d'une solution aqueuse saturée en chlorure de sodium, séchées sur sulfate de magnésium et concentrées sous pression réduite. Après purification par flash-chromatographie sur gel de silice (230-400 mesh), en éluant avec un mélange dichlorométhane-méthanol (95-5 en volumes) et cristallisation dans l'isopropanol à reflux, on obtient 1,0 g de 4,9-éthano-2-[2-(2-méthoxyphényl)propènoyl]-9-(4-méthylphényl)-2,3,3a,4,9,9a-hexahydro-1H-benzo[f]isoindole-3a-N-(3-pyridylméthyl)-carboxamide-(3aRS,4SR,9SR,9aRS) dont les caractéristiques sont les suivantes :

- point de fusion : 234°C.
- spectre de R.M.N. $^1H$ (250 MHz, $(CD_3)_2SO$ d6, à une température de 393 K, d en ppm) : 1,40 - 1,62 et de 1,90 à 2,20 (3 mts, respectivement 1H - 1H et 2H : $CH_2CH_2$) ; 2,39 (s, 3H : $ArCH_3$) ; de 3,20 à 3,40 (AB limite, 2H : $CH_2$ en 1) ; 3.44 (d, J = 12,5 Hz, 1H : 1H du $CH_2$ en 3) ; de 3,55 à 3,65 (mt, 2H : CH en 4 et CH en 9a) ; 3,68 (s, 3H : $ArOCH_3$) ; de 4,10 à 4,35 (mt, 3H : l'autre H du $CH_2$ en 3 et $NCH_2Ar$) ; 5;51 et 5;68 (2 s, 1H chacun : $=CH_2$); 6,42 (d large, J = 7,5 Hz, 1H : H en 8) : de 6,90 à 7,50 (mt, 13H : H en 5 - H en 6 - H en 7 - H aromatiques du 2-méthoxyphényl - H aromatiques du 4- méthylphényl - H en 4 du pyridyle et H en 5 du pyridyle) ; 8,05 (mf, 1H : CONH) ; 8,39 (s large, 1H : H en 2 du pyridyle) ; 8.44 (d large, J = 5 Hz, 1H : H en 6 du pyridyle).

**EXEMPLE 18**

Isolement de l'énantiomère dextrogyre du 4,9-éthane-2-[2-(2-méthoxyphényl)propènoyl]-9-(4-méthylphényl)-2,3,3a,4,9,9a-hexahydro-1H-benzo[f]isoindole-3a-N-(3-pyridylméthyl)-carboxamide-(3aRS,4SR,9SR,9aRS)

12 g de 4,9-éthano-2-[2-(2-méthoxyphényl)propènoyl]-9-(4-méthylphényl)-2,3,3a,4,9,9a-hexahydro-1H-benzo[f]isoindole-3a-N-(3-pyridylméthyl)-carboxamide-(3aRS,4SR,9SR,9aRS), obtenus à l'exemple 17, sont dédoublés, en 12 injections de 1 g, sur une colonne de silice chirale Whelk 01 (SS), en éluant par un mélange n-heptane-dichlorométhane-propanol (50-50-2 en volumes). En recueillant la première fraction éluée (temps de rétention 42 mn), on obtient, après concentration du solvant sous pression réduite, 4,78 g de l'énantiomère dextrogyre du 4,9-éthano-2-[2-(2-méthoxyphényl)propènoyl]-9-(4-méthylphényl)-2,3,3a,4,9,9a-hexahydro-1H-benzo[f]isoindole-3a-N-(3-pyridylméthyl)-carboxamide-(3aRS,4SR,9SR,9aRS) dont les caractéristiques sont les suivantes :

- point de fusion = 210°C
- spectre de masse (IE) : M/Z= 583 ($M^+$)
- pouvoir rotatoire: $[a]_{365}^{20}$= + 104,6 +/- 1,6° (c = 0,5/ méthanol)

**EXEMPLE 19**

Isolement de l'énantiomère lévogyre du 4,9-éthano-2-[2-(2-méthoxyphényl)propènoyl]-9-(4-méthylphényl)-2,3,3a,4,9,9a-hexahydro-1H-benzo[f]isoindole-3a-N-(3-pyridylméthyl)-carboxamide-(3aRS,4SR,9SR,9aRS)

12 g de 4,9-éthano-2-[2-(2-méthoxyphényl)propènoyl]-9-(4-méthylphenyl)-2,3,3a,4,9,9a-hexahydro-1H-benzo[f]isoindole-3a-N-(3-pyridylméthyl)-carboxamide-(3aRS,4SR,9SR,9aRS), obtenus à l'exemple 17, sont dédoublés, en 12 injections de 1 g, sur une colonne de silice chirale Whelk 01 (SS), en éluant par un mélange n-heptane-dichlorométhane-propanol (50-50-2 en volumes). En recueuillant la deuxième fraction éluée (temps de rétention 65 mn), on obtient, après concentration du solvant sous pression réduite, 4,86 g de l'énantiomère lévogyre de 4,9-éthano-2-[2-(2-méthoxyphényl)propènoyl]-9-(4-méthylphényl)-2,3,3a,4,9,9a-hexahydro-1H-benzo[f]isoindole-3a-N-(3-pyridylméthyl)-carboxamide-(3aRS,4SR,9SR,9aRS) dont les caractéristiques sont les suivantes :

- point de fusion = 210°C
- spectre de masse (IE) : M/Z= 583 ($M^+$)
- pouvoir rotatoire: $[a]_{365}^{20}$ = - 102,2 +/- 1,5° (c = 0,5/ méthanol)

**EXEMPLE 20**

Préparation du 4,9-éthano-2-[2-(2-méthoxyphényl)propènoyl]-9-(4-méthylphényl)-2,3,3a,4,9,9a-hexahydro-1H-benzo[f]isoindole-3a-N-(4-pyridylméthyl)-carboxamide-(3aRS,4SR,9SR,9aRS)

En opérant comme à l'exemple 17, mais à partir de 1,24 g d'acide 4,9-éthano-2-[2-(2-méthoxyphényl)propènoyl]-9-(4-méthylphényl)-2,3,3a,4,9,9a-hexahydro-1H-benzo[f]isoindole-3a-carboxylique-(3aRS,4SR,9SR,9aRS), d'une goutte de N,N-diméthylformamide, de 0,24 $cm^3$ de chlorure d'oxalyle, 0,26 $cm^3$ de 4-(ammométhyl)pyridine et de 0,7 $cm^3$ de triéthylamine, on obtient, après cristallisation dans un mélange équivolume d'isopropanol et d'oxyde de diisopropyle, 0,7 g de 4.9-éthano-2-[2-(2-méthoxyphényl)propènoyl]-9-(4-méthylphényl)-2,3,3a,4,9,9a-hexahydro-1H-benzo[f]isoindole-3a-N-(4-pyridylméthyl)carboxamide-(3aRS,4SR,9SR,9aRS) dont la caractéristique est la suivante :

- point de fusion = 202°C.

**EXEMPLE 21**

Préparation du 4,9-éthano-2-[2-(2-méthoxyphényl)propènoyl]-9-(4-méthylphényl)-2,3,3a,4,9,9a-hexahydro-1H-benzo[f]isoindole-3a-hydroxamate de méthyle-(3aRS,4SR,9SR,9aRS)

A une solution de 1,24 g d'acide 4,9-éthano-2-[2-(2-méthoxyphényl)propènoyl]-9-(4-méthylphényl)-2,3,3a,4,9,9a-hexahydro-1H-benzo[f]isoindole-3a-carboxylique-(3aRS,4SR,9SR,9aRS) dans 10 $cm^3$ de dichlorométhane et une goutte de N,N-diméthylformamide, on ajoute goutte à goutte une solution de 0,24 $cm^3$ de chlorure d'oxalyle dans 5 $cm^3$ de dichlorométhane. Le mélange réactionnel est agité pendant deux heures à une température voisine de 20°C puis coulé sur une solution de 0,21 g de chlorhydrate de O-méthyl-hydroxylamine dans un mélange de 1,05 $cm^3$ de triéthylamine et de 10 $cm^3$ de dichlorométhane refroidi à une température voisine de 0°C. Le mélange réactionnel est agité à une température voisine de 0°C pendant trente minutes puis à une température voisine de 20°C pendant une heure, coulé dans 50 $cm^3$ d'eau distillée. La phase organique est séparée par décantation, lavée par deux fois 50 $cm^3$ d'eau distillée, deux fois 20 $cm^3$ d'une solution aqueuse normale d'hydroxyde de sodium, 50 $cm^3$ d'eau distillée, 50 $cm^3$ d'une solution aqueuse saturée en chlorure de sodium, séchée sur sulfate de magnésium et concentrée sous pression réduite. Après recristallisation dans 20 $cm^3$ d'isopropanol à reflux, on obtient 0,95 g de 4,9-éthano-2-[2-(2-méthoxyphényl)propenoyl]-9-(4-méthylphényl)-2,3,3a,4,9,9a-hexahydro-1H-benzo[f]isoindole-3a-hydroxamate de méthyle-(3aRS,4SR,9SR,9aRS) dont les caractéristiques sont les suivantes :

- point de fusion = 256°C.
- spectre de R.M.N. $^1H$ (250 MHz, $(CD_3)_2SO$ d6, à une température de 383K, d en ppm) : 1,38 - 1,62 et de 1,90 à 2,20 (3 mts, respectivement 1H - 1H et 2H : $CH_2CH_2$); 2,40 (s, 3H : $ArCH_3$) ; de 3,15 à 3,45 (repectivement dd et mt, J = 12 et 9 Hz, 2H : $CH_2$ en 1) ; de 3,30 à 3,60 (mt, 3H ; CH en 9a - CH en 4 et 1H du $CH_2$ en 3) ; 3,48 (s, 3H : $NOCH_3$) ; 3,71 (s, 3H : $ArOCH_3$) ; 4,14 (d large, J = 12,5 Hz, 1H : l'autre H du $CH_2$ en 3) ; 5,52 et 5,69 (2 s, 1H chacun : $=CH_2$) ; 6,43 (d large, J = 7,5 Hz, 1H : H en 8); de 6,90 à 7,40 (mt, 11H : H en 5 - H en 6 - H en 7 et H

aromatiques du 2-méthoxyphényl et H aromatiques du 4-méthylphényl).

**EXEMPLE 22**

Préparation du 4,9-éthano-2-[2-(2-méthoxyphényl)propènoyl]-9-(4-méthylphényl)-2,3,3a,4,9,9a-hexahydro-1H-benzo [f]isoindole-3a-N',N'-diméthylcarbohydrazide-(3aRS,4SR,9SR,9aRS)

A une solution de 1,24 g d'acide 4,9-éthano-2-[2-(2-méthoxyphényl)propènoyl]-9-(4-méthylphényl)-2,3,3a, 4,9,9a-hexahydro-1H-benzo[f]isoindole-3a-carboxylique-(3aRS,4SR,9SR,9aRS) dans 10 $cm^3$ de dichlorométhane et une goutte de N,N-diméthylformamide, on ajoute goutte à goutte une solution de 0,24 $cm^3$ de chlorure d'oxalyle dans 5 $cm^3$ de dichlorométhane. Le mélange réactionnel est agité pendant deux heures à une température voisine de 20°C puis coulé sur une solution de 0,2 $cm^3$ de N,N-diméthylhydrazine et de 0,7 $cm^3$ de triéthylamine dans 10 $cm^3$ de dichlorométhane refroidie à une température voisine de 0°C. Le mélange réactionnel est agité à une température voisine de 0°C pendant trente minutes puis à une température voisine de 20°C pendant une heure puis coulé dans 50 $cm^3$ d'eau distillée. La phase organique est séparée par décantation, lavée par 50 $cm^3$ d'eau distillée, deux fois 25 $cm^3$ d'une solution aqueuse normale d'hydroxyde de sodium, 50 $cm^3$ d'eau distillée, 50 $cm^3$ d'une solution aqueuse saturée en chlorure de sodium, séchée sur sulfate de magnésium et concentrée sous pression réduite. Apres purification par flash-chromatographie sur gel de silice (230-400 mesh), en éluant avec un mélange dichlorométhane-méthanol (97,5-2,5 en volumes) et recristallisation dans 50 $cm^3$ d'un mélange isopropanol-oxyde de diisopropyle (1-9 en volumes), on obtient 0,95 g de 4,9-éthano-2-[2-(2-méthoxyphényl)propènoyl]-9-(4-méthylphényl)-2,3,3a,4,9,9a-hexahydro-1H-bénzo[f]isoindole-3a-N',N'-dimethylcarbohydrazide-(3aRS,4SR,9SR,9aRS) dont les caractéristiques sont les suivantes :

- point de fusion : 230°C.
- spectre de R.M.N. $^1H$ (250 MHz, $(CD_3)_2SO$ d6, à une température de 383K, d en ppm) : 1,38 - 1,60 et de 1,90 à 2,20 (3 mts, respectivement 1H - 1H et 2H : $CH_2CH_2$) ; 2,40 (s, 3H : $ArCH_3$) ; 2,47 (s, 6H : $N(CH_3)_2$); 3,21 et 3,35 (respectivement dd et mt, J = 12 et 9 Hz, 1H chacun : $CH_2$ en 1) ; 3,40 et 4,19 (respectivement d et d large, J = 13 Hz, 1H chacun : $CH_2$ en 3); de 3,50 à 3,65 (mt, 2H : CH en 9a et CH en 4) ; 3,71 (s, 3H : $ArOCH_3$) ; 5,51 et 5,69 (2 s, 1H chacun ; $=CH_2$) ; 6,42 (d large, J = 7,5 Hz, 1H : H en 8) ; de 6,90 à 7,40 (mt, 11H : H en 5 - H en 6 - H en 7 - H aromatiques du 2-méthoxyphényl et H aromatiques 4-méthylphényl) ; 8,41 (mf, 1H : CONH).

**EXEMPLE 23**

Préparation du 4,9-éthano-2-[2-(2-méthoxyphenyl)propènoyl]-9-(4-méthylphènyl)-2,3,3a,4,9,9a-hexahydro-1H-benzo [f]isoindole-3a-N'-phénylcarbohydrazide-(3aRS,4SR,9SR,9aRS)

En opérant comme à l'exemple 22, mais à partir de 0.5 g d'acide 4,9-éthano-2-[2-(2-méthoxyphényl)propènoyl]-9-(4-méthylphényl)-2,3,3a,4,9,9a-hexahydro-1H-benzo[f]isoindole-3a-carboxylique-(3aRS,4SR,9SR,9aRS), d'une goutte de N,N-diméthylformamide, de 0,095 $cm^3$ de chlorure d'oxalyle, 0,098 $cm^3$ de N-phénylhydrazine et de 0,28 $cm^3$ de triéthylamine, on obtient, après cristallisation dans 10 $cm^3$ d'oxyde de diisopropyle, 0,22 g de 4,9-éthano-2-[2-(2-méthoxyphényl )propènoyl]-9-(4-méthylphényl)-2,3,3a,4,9,9a-hexahydro-1H-benzo[f]isoindole-3a-N'-phénylcarbohydrazide-(3aRS,4SR,9SR,9aRS) dont les caractéristiques sont les suivantes :

- point de fusion : 220°C.

**EXEMPLE 24**

Préparation du 4,9-éthano-2-[2-(2-méthoxyphényl)propènoyl]-9-(4-méthylphényl)-2,3,3a,4,9,9a-hexahydro-1H-benzo [f]isoindole-3a-N',N'-pentaméthylènecarbohydrazide-(3aRS,4SR,9SR,9aRS)

En opérant comme à l'exemple 22, mais à partir de 0,5 g d'acide 4,9-éthano-2-[2-(2-méthoxyphényl)propènoyl]-9-(4-méthylphényl)-2,3,3a,4,9,9a-hexahydro-1H-benzo[f]isoindole-3a-carboxylique-(3aRS,4SR,9SR,9aRS), d'une goutte de N,N-diméthylformamide, de 0,095 $cm^3$ de chlorure d'oxalyle, 0,095 $cm^3$ de 1-aminopipéridine et de 0,28 $cm^3$ de triéthylamine, on obtient, après purification par flash-chromatographie sur gel de silice (230-400 mesh), en éluant avec de l'acétate d'éthyle et après cristallisation dans l'oxyde de diisopropyle, 0,06 g de 4,9-éthano-2-[2-(2-méthoxyphényl)propènoyl]-9-(4-méthylphényl)-2,3,3a,4,9,9a-hexahydro-1H-benzo[f]isoindole-3a-N',N'-pentaméthylènecarbohydrazide-(3aRS,4SR,9SR,9aRS) dont les caractéristiques sont les suivantes

- point de fusion : 220°C.

**EXEMPLE 25**

4,9-éthano-2-[2-(méthoxyphényl)propénoyl]-9-(4-méthylphényl)-3a-phénylsulfonylaminocarbonyl-2,3,3a,4,9,9a-hexahydro-1H-benzo[f]isoindole-(3aRS,4SR,9SR,9aRS)

A une solution de 0,5 g d'acide 4,9-éthano-2-[2-(2-méthoxyphényl)propènoyl]-9-(4-méthylphényl)-2,3,3a,4,9,9a-hexahydro-1H-benzo[f]isoindole-3a-carboxylique-(3aRS,4SR,9SR,9aRS) dans 5 $cm^3$ de dichlorométhane et une goutte de N,N-diméthylformamide, on ajoute goutte à goutte une solution de 0,096 $cm^3$ de chlorure d'oxalyle dans 0,5 $cm^3$ de dichlorométhane. Le mélange réactionnel est agité pendant deux heures à une température voisine de 20°C puis coulé sur un mélange de 0,12 g d'une suspension à 60% d'hydrure de sodium dans la vaseline et 0,31 g de benzènesulfonamide dans 5 $cm^3$ de N,N-diméthylformamide. Le mélange réactionnel est agité à une température voisine de 20°C pendant trois jours, 10 $cm^3$ d'eau distillée sont ensuite ajoutés et le mélange est extrait par deux fois 20 $cm^3$ de dichlorométhane. Les extraits organiques sont réunis, lavés par 20 $cm^3$ d'eau distillée puis 20 $cm^3$ d'une solution aqueuse saturée en chlorure de sodium, séchés sur sulfate de magnésium et concentrés sous pression réduite. Apres purification par flash-chromatographie sur gel de silice (230-400 mesh), en éluant avec un mélange dichlorométhane-méthanol (98-2 en volumes) et cristallisation dans l'oxyde de diisopropyle, on obtient 0,005 g de 4,9-éthano-2-[2-(méthoxyphényl)propènoyl]-9-(4-méthylphényl)-3a-phénylsulfonylaminocarbonyl-2,3,3a,4,9,9a-hexahydro-1H-benzo[f]isoindole-(3aRS,4SR,9SR,9aRS) dont les caractéristiques sont les suivantes :

- point de fusion : vers 166°C.
- spectre de R.M.N. $^1H$ (400 MHz, $(CD_3)_2SO$ d6, à une température de 393 K, d en ppm) : 1,35 - 1,58 - 1,97 et 2,09 (4 mts, 1H chacun : $CH_2CH_2$) ; 2,39 (s, 3H : $ArCH_3$) ; 3,20 et 3,31 (respectivement dd et d large, J = 12,5 et 9 Hz et J = 12,5 Hz, 1H chacun : $CH_2$ en 1) ; de 3,40 à 3,50 (mt, 1H : CH en 9a) ; 3,45 et 4,14 (respectivement dd et d large, J = 13 Hz, 1H chacun : $CH_2$ en 3) ; 3,59 (mt, 1H : CH en 4) ; 3,66 (s, 3H : $ArOCH_3$) ; 5,45 et 5,65 (2 s, 1H chacun : $=CH_2$) ; 6,35 (mt, 1H : H en 8) ; de 6,85 à 7.60 (mt, 14H : H en 5 - H en 6 - H en 7 - H aromatiques du 2-méthoxyphényl - H aromatiques du 4-méthylphényl - H aromatiques en méta du phénylsulfonyle et H aromatique en para du phénylsulfonyle) ; 7,77 (d, J = 8 Hz, 2H : H aromatiques en ortho du phénylsulfonyle) ; 8,00 (s large, 1H : CONH).

**EXEMPLE 26**

Préparation du 4,9-éthano-2-[2-(2-méthoxyphényl)propènoyl]-9-(4-méthylphényl)-2,3,3a,4,9,9a-hexahydro-1H-benzo[f]isoindole-3a-N'-(N-oxo-3-pyridyl)méthyl-carboxamide-(3aRS,4SR,9SR,9aRS)

A une solution de 0,29 g de 4,9-ethano-2-[2-(2-méthoxyphényl)propenoyl]-9-(4-méthylphényl)-2,3,3a,4,9,9a-hexahydro-1H-benzo[f]isoindole-3a-N-(3-pyridylméthyl)-carboxamide-(3aRS,4SR,9SR,9aRS) dans 8 $cm^3$ de chloroforme, on ajoute 0,20 g de carbonate de sodium et 6 $cm^3$ d'eau distillée. Le mélange est refroidi à une température voisine de 0°C et 0,11 g d'acide méta chloro perbenzoïque est ajouté. Le milieu réactionnel est agité pendant une heure à 2°C puis pendant vingt deux heures à une température voisine de 20°C, 0,22 g d'acide méta chloro perbenzoïque est encore ajouté et le mélange agité pendant quatre heures à une température voisine de 20°C puis dilué par 10 $cm^3$ de dichlorométhane. La phase organique est séparée par décantation, lavée par trois fois 5 $cm^3$ d'une solution aqueuse à 10% de sulfite de sodium, trois fois 5 $cm^3$ d'une solution aqueuse saturée en hydrogénocarbonate de sodium et 5 $cm^3$ d'eau distillée, séchée sur sulfate de magnésium, et concentrée sous pression réduite. Le résidu est purifié par recristallisation dans 8 $cm^3$ d'acétate d'isopropyle pour donner 0,25 g de 4,9-éthano-2-[2-(2-méthoxyphényl)propènoyl]-9-(4-méthylphényl)-2,3,3a,4,9,9a-hexahydro-1H-benzo[f]isoindole-3a-N-(N-oxo-3-pyridyl)méthyl-carboxamide-(3aRS,4SR,9SR,9aRS) dont les caractéristiques sont les suivantes :

- point de fusion = 250°C.
- spectre de R.M.N. $^1H$ (250 MHz, $(CD_3)_2SO$ d6, à une température de 383 K, d en ppm) : 1,40 - 1,62 et de 1,90 à 2,25 (3 mts, respectivement 1H - 1H et 2H : $CH_2CH_2$) ; 2,40 (s, 3H : $ArCH_3$) ; de 3,20 à 3,50 (AB limite, 2H : $CH_2$ en 1) ; 3,44 (d, J = 12,5 Hz, 1H : 1H du $CH_2$ en 3); de 3,50 à 3,65 (mt, 1H : CH en 9a) ; 3.57 (mt, 1H : CH en 4) ; 3,70 (s, 3H : $ArOCH_3$) ; de 4,00 à 4,30 (mt, 3H : l'autre H du $CH_2$ en 3 et $NCH_2Ar$) ; 5,51 et 5,68 (2 s, 1H chacun : $=CH_2$) ; 6,41 (d large, J = 7.5 Hz, 1H : H en 8) ; de 6,90 à 7,40 (mt, 13H : H en 5 - H en 6 - H en 7 - H aromatiques du 2-méthoxyphényl - H en 4 du N-oxopyridyle - H en 5 du N-oxopyridyle et H aromatiques du 4-méthylphényl) ; 8,00 (s large, 1H : H en 2 du N-oxopyridyle) : 8,05 (d large, J = 5 Hz, 1H : H en 6 du N-oxopyridyle) ; 8,11 (t large. J = 5 Hz, 1H ; CONH).

**EXEMPLE 27**

Préparation du 4,9-éthano-2-[2-(2-méthoxyphényl)propènoyl]-9-(4-méthylphényl)-2,3,3a,4,9,9a-hexahydro-1H-benzo [f]isoindole-3a-N'-(4-méthoxyphényl)carbohydrazide-(3aRS,4SR,9SR,9aRS)

A une solution de 0,49 g d'acide 4,9-éthano-2-[2-(2-méthoxyphényl)propénoyl]-9-(4-méthylphényl)-2,3,3a,4,9,9a-hexahydro-1H-benzo[f]isoindole-3a-carboxylique-(3aRS,4SR,9SR,9aRS) dans 10 $cm^3$ de dichlorométhane, on ajoute successivement 0,21 g de chlorhydrate de 4-méthoxyphénylhydrazine, 0,23 g de chlorhydrate de 1-éthyl-3-[(diméthylamino)propyl]carbodiimide, 0,08g d'hydrate de N-1-hydroxybenzotriazole et 0,17 $cm^3$ de triéthylamine. Le mélange réactionnel est agité pendant vingt heures à une température voisine de 20°C puis dilué par 15 $cm^3$ de dichlorométhane. La phase organique est lavée par trois fois 10 $cm^3$ d'eau distillée, séchée sur sulfate de magnésium en présence de noir 3S et concentrée sous pression réduite. Par cristallisation dans 10 $cm^3$ d'acétate d'isopropyle, on obtient 0,36 g de 4,9-éthano-2-[2-(2-méthoxyphényl)propènoyl]-9-(4-méthylphényl)-2,3,3a,4,9,9a-hexahydro-1H-benzo[f]isoindole-3a-N'-(4-méthoxyphényl)carbohydrazide-(3aRS,4SR,9SR,9aRS) dont les caractéristiques sont les suivantes

- point de fusion = 180°C.

- spectre de R.M.N. $^1H$ (250 MHz, $(CD_3)_2SO$ d6, à une température de 383 K, d en ppm) : 1,40 - 1,62 et de 1,90 à 2,25(3 mts, respectivement 1H - 1H et 2H : $CH_2CH_2$); 2,38 (s, 3H : $ArCH_3$) ; 3,25 (dd, J = 12 et 9 Hz, 1H : 1H du $CH_2$ en 1); 3,39 (d large, J = 12 Hz, 1H : l'autre H du $CH_2$ en 1); 3,48 et 4,22 (respectivement d et d large, J = 12,5 Hz, 1H chacun : $CH_2$ en 3) ; 3,56 (mt, 1H : CH en 9a) ; de 3,60 à 3,75 (mt, 1H : CH en 4) ; 3,68 (s, 6H : les 2 $ArOCH_3$) : 5,52 et 5,69 (2 s, 1H chacun : $=CH_2$) : 6,42 (d large, J = 7,5 Hz, 1H : H en 8) ; 6,56 et 6,69 (2 d, J = 8 Hz, 2H chacun : H aromatiques en ortho et meta du $OCH_3$) : 6,80 (s large, 1H : ArNH) : de 6,90 à 7,40 (mt. 11H : H en 5 - H en 6 - H en 7 - H aromatiques du 2-méthoxyphényl et H aromatiques du 4-méthylphényle) ; 9,46 (s large, 1H : -CONH).

**EXEMPLE 28**

Préparation du 4,9-éthano-2-[2-(2-méthoxyphényl)propènoyl]-9-(4-méthylphényl)-2,3,3a,4,9,9a-hexahydro-1H-benzo [f]isoindole-3a-N'-méthyl,N'-phénylcarbohydrazide-(3aRS,4SR,9SR,9aRS)

En opérant comme à l'exemple 27, mais à partir de 0,49 g d'acide 4,9-éthano-2-[2-(2-méthoxyphényl)propènoyl]-9-(4-méthylphényl)-2,3,3a,4,9,9a-hexahydro-1H-benzo[f]isoindole-3a-carboxylique-(3aRS,4SR,9SR,9aRS), 0,14 $cm^3$ de N-méthyl,N-phénylhydrazine, 0,23 g de chlorhydrate de 1-éthyl-3-[(diméthylamino)propyl]carbodiimide, 80 mg d'hydrate de N-1-hydroxybenzotriazole et 0,17 $cm^3$ de triéthylamine, on obtient, après cristallisation dans 5 $cm^3$ d'isopropanot, 0,22 g de 4,9-éthano-2-[2-(2-méthoxyphényl)propènoyl]-9-(4-méthylphényl)-2,3,3a,4,9,9a-hexahydro-1H-benzo[f]isoindole-3a-N'-méthyl,N'-phénylcarbohydrazide-(3aRS,4SR,9SR,9aRS) dont la caractéristique est la suivante :

- point de fusion = 240°C.

**EXEMPLE 29**

Préparation du 4,9-éthano-2-[2-(2-méthoxyphényl)propènoyl]-9-(4-méthylphényl)-2,3,3a,4,9,9a-hexahydro-1H-benzo [f]isoindole-3a-N'-(2-méthylphényl)carbohydrazide-(3aRS,4SR,9SR,9aRS)

En opérant comme à l'exemple 27, mais à partir de 0,4 g d'acide 4,9-éthano-2-[2-(2-méthoxyphényl)propènoyl)-9-(4-méthylphényl)-2,3,3a,4,9,9a-hexahydro-1H-benzo[f]isoindole-3a-carboxylique-(3aRS,4SR,9SR,9aRS), 0,16 g de chlorhydrate de N-(2-méthylphényl)hydrazine, 0,19 g de chlorhydrate de 1-ethyl-3-[(diméthylamino)propyl] carbodiimide, 60 mg de d'hydrate de N-1-hydroxybenzotriazole et 0,14 $cm^3$ de triéthylamine, on obtient, après cristallisation dans 7,5 $cm^3$ d'acétate d'isopropyle, 0,28 g de 4,9-éthano-2-[2-(2-méthoxyphényl)propènoyl]-9-(4-mèthylphényl)-2,3,3a,4,9,9a-hexahydro-1H-benzo[f]isoindole-3a-N'-(2-methylphényl)carbohydrazide-(3aRS,4SR,9SR,9aRS) dont la caractéristique est la suivante :

- point de fusion = 235°C.

**EXEMPLE 30**

Isolement de l'énantiomère dextrogyre de l'acide 4,9-éthano-2-[2-(2-méthoxyphényl) acétyl]-9-(4-méthylphényl)-2,3,3a,4,9,9a-hexahydro-1H-benzo[f]isoindole-3a-carboxylique-(3aRS,4SR,9SR,9aRS)

L'acide 4,9-éthano-2-[2-(2-méthoxyphényl)acétyl]-9-(4-méthylphényl)-2,3,3a,4,9,9a-hexahydro-1H-benzo[f]isoindole-3a-carboxylique-(3aRS,4SR,9SR,9aRS), obtenu à l'exemple 5, peut être dédoublé, sur une colonne de silice chirale porteuse de greffons N-(3,5-dinitrobenzoyl)-phénylalanine-(S), en opérant comme à l'exemple 3. En recueuillant la deuxième fraction éluée par un mélange de dichlorométhane, d'isopropanol et de n-heptane (85-10-5 en volumes), on obtient, après concentration sous pression réduite, 70 mg de l'énantiomère dextrogyre de l'acide 4,9-éthano-2-[2-(2-méthoxyphényl)acétyl]-9-(4-méthylphényl)-2,3,3a,4,9,9a-hexahydro-1H-benzo[f]isoindole-3a-carboxylique-(3aRS,4SR,9SR,9aRS) dont les caractéristiques sont les suivantes :

- point de fusion = 205°C
- spectre de masse (IE) : M/Z= 481 ($M^-$)
- pouvoir rotatoire: $[a]_{365}^{20}$= + 59,5 +/- 0,9° (c = 0,5/ méthanol)

**EXEMPLE 31**

Préparation du 9-(2,3-dihydro-1,4-benzodioxin-6-yl)-4,9-ethano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS)

Etape A

A une solution de 6,3 g de 2-benzyl-7-oxo-4-phényloctahydroisoindole-3a-carboxylate de méthyle-(3aRS, 4SR,7aRS) dans 60 $cm^3$ de dichlorométhane stabilisé sur amylène, on additionne 7,1 $cm^3$ de 1,4-benzodioxane, sous atmosphère d'argon; cette solution est refroidie par un bain d'eau glacée, et on coule en 5 minutes 10,7 $cm^3$ d'acide trifluorométhanesulfonique à 99% Après agitation dans le bain froid, on laisse remonter à une température voisine de 20°C, et agiter à cette température pendant vingt et une heures, sous atmosphère d'argon. On coule alors 200 $cm^3$ d'eau distillée, puis 300 $cm^3$ d'acétate d'éthyle. La phase organique est décantée et lavée successivement trois fois avec 180 $cm^3$ d'une solution aqueuse 1N d'hydroxyde de sodium, puis 240 $cm^3$ d'une solution aqueuse saturée en chlorure de sodium, puis séchée sur sulfate de magnésium. Après filtration, la phase organique est évaporée sous pression réduite pour donner 10,8 g d'une huile. Cette huile est purifiée par flash-chromatographie sur une colonne de 8 cm de diamètre contenant 430 g de gel de silice (230-400 mesh), en éluant sucessivement avec 1 $dm^3$ du mélange cyclohexane-acétate d'éthyle (98-2 en volumes), puis 2 $dm^3$ du mélange cyclohexane-acétate d'éthyle (95-5 en volumes), puis du mélange cyclohexane et d'acétate d'éthyle (90-10 en volumes), en recueillant des fractions de 50 $cm^3$. Après concentration du solvant sous pression réduite, on obtient 5,41 g de 2-benzyl-9-(2,3-dihydro-1,4-benzodioxin-6-yl)-4,9-éthano-2,3,3a,4,9,9a-hexahydro-1H-benzo[f]isoindole-3a-carboxylate de méthyle-(3aRS,4SR,9SR,9aRS) impur, sous forme d'une meringue blanche, utilisable telle qu'elle pour l'étape suivante, dont les caractéristiques sont les suivantes :

- spectre de masse (IE) : M/Z = 481 ($M^+$)
- spectre de R.M.N. $^1H$ (300 MHz, $CDCl_3$, d en ppm) : 1,45 - 1,65 et de 2,40 à 2,55 (3 mts respectivement 1H -1H et 2H : $CH_2CH_2$) ; 2,31 et 3,20 (2 d. J = 10 Hz, 1H chacun : $CH_2$ en 3) : de 2,35 à 2,45 (mt, 1H : 1H du $CH_2$ en 1) ; 2,81 (d, J = 10 Hz, 1H : l'autre H du $CH_2$ en 1) ; de 3,00 a 3,20 (mt, 1H : CH en 9a) : de 3,30 à 3,45 et 3,71 (respectivement mt et d large, J = 13 Hz, iH chacun : $NCH_2Ar$) ; 3,39 (s large, 1H : CH en 4) ; 3,55 (s. 3H : $COOCH_3$) ; 4,31 (s, 4H : $OCH_2CH_2O$) ; 6,63 (d large, J = 7,5 Hz, 1H : H en 8) ; de 6,85 à 7,40 (mt, 11H : H en 5 - H en 6 - H en 7 - H aromatiques du benzyle et H aromatiques du 1-4-benzodroxanyl).

Etape B

En opérant comme à l'exemple 1, étape E, en partant de 5,4 g de 2-benzyl-9-(2,3-dihydro-1,4-benzodioxin-6-yl)-4,9-éthano-2,3,3a,4,9,9a-hexahydro-1H-benzo[f] isoindole-carboxylate de méthyle-(3aRS,4SR,9SR,9aRS), de 2,16 g de formiate d'ammonium, de 1,35 g d'hydroxyde de palladium sur charbon à 10% (p/p) dans 80 $cm^3$ de méthanol au reflux pendant cinq heures, on obtient 4 g de-9-(2,3-dihydro-1,4-benzodioxin-6-yl)-4,9-éthano-2,3,3a,4,9,9a-hexahydro-1H-benzo[f]isoindole-3a-carboxylate de méthyle-(3aRS,4SR,9SR,9aRS) impurs sous forme d'une meringue

blanchâtre utilisable telle quelle pour l'étape suivante, dont la caractéristique est la suivante :

- Spectre de masse (DCI) : M/Z = 392 ($M+H^+$)

Etape C

En opérant comme à l'exemple 1, étape G, en panant d'une solution de 0,275 g d'acide 2-(2-méthoxyphényl) propènoïque dans 5 $cm^3$ de dichlorométhane contenant 4 gouttes de N,N-diméthyl-formamide on ajoute goutte à goutte une solution de 0,2 $cm^3$ de chlorure d'oxalyle dans 7 $cm^3$ de dichlorométhane, Le mélange réactionnel est encore agité pendant deux heures à une température voisine de 20°C puis refroidi à une température voisine de 0°C; on introduit alors en vingt minutes une solution de 0,73 g de-9-(2,3-dihydro-1,4-benzodioxin-6-yl)-4,9-éthano-2,3,3a, 4,9,9a-hexahydro-1H-benzo[f]isoindole-3a-carboxylate de méthyle-(3aRS,4SR,9SR,9aRS) impur, dans15 $cm^3$ de dichlorométhane puis 0.4 $cm^3$ de triéthylamine. Le mélange réactionnel est agité à la même température pendant une heure et on coule 100 $cm^3$ d'eau distillée; on dilue au dichlorométhane de façon à porter la phase organique à environ 200 $cm^3$. Le pH est porté à 11 par coulée de 10 $cm^3$ d'une solution aqueuse à 32 % d'ammoniaque, et la phase aqueuse est decantée, puis la phase organique est lavée avec successivement 100 $cm^3$ d'une solution aqueuse IN d'acide chlorhydrique, puis 100 $cm^3$ d'eau distillée, puis 100 $cm^3$ d'une solution aqueuse saturée en chlorure de sodium. Après séchage sur sulfate de magnésium, la phase organique est évaporée pour donner 0,75 g d'une huile qui est purifiée par flash-chromatographie sur gel de silice (230-400 mesh), en éluant avec un mélange de cyclohexane et d'acétate d'éthyle (60-40 en volumes), en recueillant des fractions de 10 $cm^3$. Les fractions contenant le bon produit (140 à 650 $cm^3$) sont jointes et évaporées sans aller à sec pour donner une huile qui est reprise dans de l'éther diisopropylique; on obtient ainsi 465 mg de 9-(2,3-dihydro-1,4-benzodioxin-6-yl)-4,9-éthano-2-[2-(2-méthoxyphényl)propènoyl]-2,3,3a, 4,9,9a-hexahydro-1H-benzo[f]isoindole-3a-carboxylate de méthyle-(3aRS,4SR,9SR,9aRS) sous forme d'une poudre blanche, dont les caractéristiques sont les suivantes :

- point de fusion = 184°C.
- spectre de masse (IE) : M/Z = 551 ($M^+$)
- spectre de R.M.N. $^1H$ (400 MHz, $(CD_3)_2SO$ d6, à une température de 403 K, d en ppm) : 1,40 - 1,58 - 1,96 et 2,12 (4 mts, 1H chacun : $CH_2CH_2$) : de 3,25 à 3,45 (mt, 3H : $CH_2$ en 1 et CH en 9a) : 3,42 (mt, 1H : CH en 4), 3,52 (s, 3H : $COOCH_3$) ; 3,60 et 4,06 (respectivement d et d large, J = 13 Hz, 1H chacun : $CH_2$ en 3) : 3,73 (s, 3H : $ArOCH_3$) ; 4,31 (s, 4H : $OCH_2CH_2O$) ; 5,54 et 5,68(2 s, 1H chacun: $=CH_2$) : 6,51 (d large, J = 7,5 Hz. 1H, H en 8) ; de 6,75 à 7,35 (mt, 10H : H en 5 - H en 6 - H en 7 - H aromatiques du 2-méthoxyphényl et H aromatiques du 1-4-benzodioxanyl).

## EXEMPLE 32

Préparation de l'acide 9-(2,3-dihydro-1,4-benzodioxin-6-yl)-4,9-éthano-2-[2-(2-méthoxyphényl)propénoyl]-2,3,3a, 4,9,9a-hexahydro-1H-benzo[f]isoindole-3a-carboxylique-(3aRS,4SR,9SR,9aRS)

En opérant comme à l'exemple 2, mais à partir de 5,43 g de 9-(2,3-dihydro-1,4-benzodioxin-6-yl)-4,9-éthano-2-[2-(2-méthoxyphényl)propènoyl]-2,3,3a,4,9,9a-hexahydro-1H-benzo[f]isoindole-3a-carboxylate de méthyle-(3aRS, 4SR,9SR,9aRS) obtenus à l'exemple précédent et de 40 $cm^3$ d'une solution aqueuse normale d'hydroxyde de sodium, dans 150 $cm^3$ de dioxane au reflux pendant seize heures, puis addition de 50 $cm^3$ d'une solution aqueuse normale d''hydroxyde de sodium au reflux pendant quatre heures, puis 3,5 $cm^3$ d'une solution aqueuse 10N d'hydroxyde de sodium, au reflux pendant seize heures, on obtient après reprise dans de l'oxyde de diisopropyle 3,76 g d'un solide de couleur beige. Par dissolution dans 50 $cm^3$ d'éthanol absolu, puis dilution avec 12,5 $cm^3$ d'eau distillée, on obtient 2,64 g d'acide 9-(2,3-dihydro-1,4-benzodioxin-6-yl)-4,9-éthano-2-[2-(2-méthoxyphényl)propénoyl]-2,3,3a,4,9,9a-hexahydro-1H-benzo[f]isoindole-3a-carboxylique-(3aRS,4SR,9SR,9aRS), sous forme d'une poudre blanche, dont les caractéristiques sont les suivantes :

- point de fusion = 78°C.
- spectre de masse (IE) : M/Z = 537 ($M^+$)
- spectre de R.M.N. $^1H$ (250 MHz, $(CD_3)_2SO$ d6, à une température de 383 K, d en ppm) : 1,38 - 1,57 - 1,94 et 2,11 (4 mts, 1H chacun : $CH_2CH_2$) ; de 3,20 à 3,45 (mt, 3H : $CH_2$ en 1 et CH en 9a) ; 3,42 (mt, 1H : CH en 4); 3,59 et 4,06 (respectivement d et d large, J = 13 Hz, 1H chacun : $CH_2$ en 3) ; 3,73 (s, 3H : $ArOCH_3$) ; 4,31 (s, 4H : $OCH_2CH_2O$) ; 5,54 et 5,69 (2 s, 1H chacun : $=CH_2$) ; 6,50 (d large, J = 7,5 Hz, 1H : H en 8); de 6,75 à 7,40 (mt, 10H : H en 5 - H en 6 - H en 7 - H aromatiques du 2-méthoxyphényl et H aromatiques du 1-4-benzodioxanyl).

**EXEMPLE 33**

Préparation du 9-(2,3-dihydro-1,4-benzodioxin-6-yl)-4,9-éthano-2-[2-(2-méthoxyphényl)propènoyl)]-2,3,3a,4,9,9a-hexahydro-1H-benzo[f]isoindole-3a-N-(3-pyridylméthyl)carboxamide-(3aRS,4SR,9SR,9aRS)

En opérant comme à l'exemple 13, ci en partant de 0,5 g d'acide 9-(2,3-dihydro 1,4-benzodioxin-6-yl)-4,9-éthano-2-[2-(2-méthoxyphényl)propènoyl]-2,3,3a,4,9,9a-hexahydro-1H-benzo[f]isoindole-3a-carboxylique-(3aRS,4SR,9SR,9aRS), de 0,24 $cm^3$ de chlorure d'oxalyle, de 0,5 $cm^3$ de N,N-diméthylformamide, dans 20 $cm^3$ de dichlorométhane, pendant 2 heures, puis évaporation à sec, reprise dans 20 $cm^3$ de dichlorométhane, et addition de 0,09 $cm^3$ de 3-aminomèthylpyridine, et de 0,26 $cm^3$ de triéthylamine dans 15 $cm^3$ de dichlorométhane; puis après agitation dix huit heures à une température voisine de 20°C, coulees successives de 50 $cm^3$ d'eau distillée et de 40 $cm^3$ d'une solution aqueuse normale d'hydroxyde de sodium; dilution avec 200 $cm^3$ de dichloromethane; décantation et lavage de la phase organique avec deux fois 100 $cm^3$ d'une solution aqueuse normale d'acide chlorhydrique, puis 200 $cm^3$ d'une solution aqueuse saturée en chlorure de sodium et enfin séchage sur sulfate de magnésium, on obtient 0,38 g d'une meringue jaune-vert que l'on recristallise dans de l'éther diisopropylique pour obtenir 249 mg d'un solide jaune qui est ensuite purifié sur une plaque préparative de silice MERCK 60F254 , en éluant avec le mélange dichlorométhane-méthanol (90-10 en volumes). On obtient ainsi une huile orangée, qui reprise dans de l'éther diisopropylique donne 165 mg de 9-(2,3-dihydro-1,4-benzodioxin-6-yl)-4,9-éthano-2-[2-(2-méthoxyphényl)propènoyl)]-2,3,3a,4,9,9a-hexahydro-1H-benzo[f]isoindole-3a-N-(3-pyridylméthyl)carboxamide-(3aRS,4SR,9SR,9aRS), sous forme d'une poudre blanche dont les caractéristiques sont les suivantes :

- point de fusion = 228°C.
- spectre de masse (IE) : M/Z = 627 ($M^+$)
- spectre de R.M.N. $^1H$ (250 MHz, $(CD_3)_2SO$ d6, à une température de 373 K, d en ppm) : 1,38 - 1,56 - 1,93 et 2,10 (4 mts, 1H chacun : $CH_2CH_2$) ; de 3,20 à 3,55 (mt, 3H : $CH_2$ en 1 et CH en 9a) ; 3,43 (d, J = 12,5 Hz, 1H : 1H du $CH_2$ en 3) ; 3,57 (s large, 1H : CH en 4) ; 3,71 (s, 3H : $ArOCH_3$) ; de 4,05 à 4,30 (mt, 3H : l'autre H du $CH_2$ en 3 et $NCH_2Ar$) ; 4,31 (s, 4H : $OCH_2CH_2O$) ; 5,52 et 5,70 (2s, 1H chacun : $=CH_2$) ; 6,48 (mt, 1H : H en 8) ; de 6,75 à 7,45 (mt, 12H : H en 5 - H en 6 - H en 7-H aromatiques du 2-méthoxyphényl - H aromatiques du 1-4-benzodioxanyl - H en 4 du pyridyle et H en 5 du pyridyle) ; 8,10 (mt, 1H : CONH) ; 8,38 (s large, 1H : H en 2 du pyridyle); 8,45 (d large, J = 5 Hz, 1H : H en 6 du pyridyle),

**EXEMPLE 34**

Isolement de l'énantiomère dextrogyre du 9-(2,3-dihydro-1,4-benzodioxin-6-yl)-4,9-éthano-2-[2-(2-méthoxyphényl) propénoyl)]-2,3,3a,4,9,9a-hexahydro-1H-benzo[f]isoindole-3a-N-(3-pyridylméthyl)carboxamide-(3aRS,4SR,9SR, 9aRS)

En opérant comme à l'exemple 18, mais à partir de 17 g de 9-(2,3-dihydro-1,4-benzodioxin-6-yl)-4,9-éthane-2-[2-(2-méthoxyphenyl)propenoyl)]-2,3,3a,4,9,9a-hexahydro-1H-benzo[f]isoindole-3a-N-(3-pyridylméthyl)carboxamide-(3aRS,4SR,9SR,9aRS), en 10 injections successives, sur une colonne de silice chirale Whelk 01 (SS), en éluant par un mélange n-heptane-éthanol-triéthylamine (70-30-0,05 en volumes) et en isolant le premier produit élué (temps de rétention = 45 mn), on obtient, après concentration du solvant sous pression réduite, 8,4 g de l'énantiomère dextrogyre du 9-(2,3-dihydro-1,4-benzodioxin-6-yl)-4,9-éthano-2-[2-(2-méthoxyphényl)propènoyl)]-2,3,3a,4,9,9a-hexahydro-1H-benzo[f]isoindole-3a-N-(3-pyridylméthyl)carboxamide-(3aRS,4SR,9SR,9aRS), sous forme d'une poudre blanche dont les caractéristiques sont les suivantes :

- spectre de masse (IE) : M/Z= 627 ($M^+$)

- pouvoir rotatoire: $[a]_{365}^{20}$= + 103,2 +/- 1,5° (c = 0,5/ méthanol)

**EXEMPLE 35**

Isolement de l'énantiomère lévogyre du 9-(2,3-dihydro-1,4-benzodioxin-6-yl)-4,9-éthano-2-[2-(2-méthoxyphényl)propénoyl)]-2,3,3a,4,9,9a-hexahydro-1H-benzo[f]isoindole-3a-N-(3-pyridylméthyl)carboxamide-(3aRS,4SR,9SR,9aRS)

En opérant comme à l'exemple 19, mais à partir de 17 g de 9-(2,3-dihydro-1,4-benzodioxin-6-yl)-4,9-éthano-2-[2-(2-méthoxyphényl)propènoyl)]-2,3,3a,4,9,9a-hexahydro-1H-benzo[f]isoindole-3a-N-(3-pyridylméthyl)carboxamide-(3aRS,4SR,9SR,9aRS), en 10 injections successives, sur une colonne de silice chirale Whelk 01 (SS), en éluant

par un mélange de n-heptane, d'éthanol et de triéthylamine (70-30-0,05 en volumes) et en isolant le deuxième produit élué (temps de rétention = 67 mn), on obtient, après concentration du solvant sous pression réduite, 6,6 g de l'énantiomère lévogyre du 9-(2,3-dihydro- 1,4-benzodioxin-6-yl)-4,9-éthano-2-[2-(2-méthoxyphényl)propènoyl)]-2,3,3a, 4,9,9a-hexahydro-1H-benzo[f]isoindole-3a-N-(3-pyridylméthyl)carboxamide-(3aRS,4SR,9SR,9aRS), sous forme d'une poudre blanche dont les caractéristiques sont les suivantes :

- spectre de masse (IE) : M/Z = 627 ($M^+$)
- pouvoir rotatoire: $[a]_{365}^{20}$= - 102 +/- 1,5° (c = 0,5/ méthanol)

**EXEMPLE 36**

Preparation du 9-(2,3-dihydro-1,4-benzodioxin-6-yl)-4,9-éthano-2-[2-(2-méthoxyphényl)propènoyl)]-2,3,3a,4,9,9a-hexahydro-1H-benzo[f]isoindole-3a-N-(2-thiénylméthyl)carboxamide-(3aRS,4SR,9SR,9aRS)

En opérant comme à l'exemple 33, et en partant de 0,5 g d'acide 9-(2,3-dihydro-1,4-benzodioxin-6-yl)-4,9-éthano-2-[2-(2-méthoxyphényl)propènoyl]-2,3,3a, 4,9,9a-hexahydro-1H-benzo[f]isoindole-3a-carboxylique-(3aRS,4SR,9SR,9aRS), de 0,24 $cm^3$ de chlorure d'oxalyle, de 0,1 $cm^3$ de N,N-diméthylformamide, dans 20 $cm^3$ de dichlorométhane, puis de 0,1 $cm^3$ de 2-(aminométhyl)thiophène, de 0,26 $cm^3$ de triéthylamine dans 15 $cm^3$ de dichlorométhane, on obtient, après lyophilisation, 29 mg de 9-(2,3-dihydro-1,4-benzodioxin-6-yl)-4,9-éthano-2-[2-(2-méthoxyphényl)propènoyl)]-2,3,3a,4,9,9a-hexahydro-1H-benzo[f]isoindole-3a-N-(2-thiénylmethyl)carboxamide-(3aRS, 4SR,9SR,9aRS), sous la forme d'une poudre dont les caractéristiques sont les suivantes :

- point de fusion = 134°C
- spectre de masse (IE) : M/Z= 632 ($M^+$)

**EXEMPLE 37**

Préparation de l'acide 4,9-éthano-2-[(2-méthoxyphényl)acétyl]-9-(3,4,5-triméthylphényl)-2,3,3a,4,9,9a-hexahydro-1H-benzo[f]isoindole-3a-carboxylique-(3aRS,4SR,9SR,9aRS)

Etape A

On opérant comme à l'exemple 31, étape A, en partant de 3,49 g de 2-[(2-méthoxyphényl)acétyl]-7-oxo-4-phényloctahydroisoindole-3a-carboxylate de méthyle-(3aRS,4SR,7aRS) dans 40 $cm^3$ de dichlorométhane stabilisé sur amylène, de 3,34 $cm^3$ de-1,2,3-triméthylbenzène et de 5,1 $cm^3$ d'acide trifluorométhane sulfonique; après dix huit heures d'agitation à une température voisine de 20°C, on additionne 2,8 $cm^3$ d'anhydride trifluorométhane sulfonique, et agite encore 18 heures. On obtient ainsi 263 mg de 4,9-éthano-2-[(2-méthoxyphényl)acétyl]-9-(3,4,5-triméthylphényl)-2,3,3a,4,9,9a-hexahydro-1H-benzo[f]isoindole-3a-carboxylate de méthyle-(3aRS,4SR, 9SR,9aRS), sous forme d'une poudre blanche, dont les caractéristiques sont les suivantes:

- point de fusion = 159°C.
- spectre de masse (IE) : M/Z= 523 ($M^+$)

Etape B

En opérant comme à l'exemple 2, à partir de 192 mg de 4,9-éthano-2-[(2-méthoxyphényl)acétyl]-9-(3,4,5-triméthylphényl)-2,3,3a,4,9,9a-hexahydro-1H-benzo[f]isoindole-3a-carboxylate de méthyle-(3aRS,4SR,9SR,9aRS), au reflux pendant dix-huit heures dans 0,55 $cm^3$ de solution aqueuse IN d'hydroxyde de sodium et 20 $cm^3$ d'éthanol, on obtient, après recristallisation dans 5 $cm^3$ d'oxyde de diisopropyle, 69,3 mg d'acide 4,9-éthano-2-[(2-méthoxyphényl) acétyl]-9-(3,4,5-triméthylphényl)-2,3,3a,4,9,9a-hexahydro-1H-benzo[f]isoindole-3a-carboxylique-(3aRS,4SR,9SR, 9aRS), sous forme d'une poudre blanche, dont les caractéristiques sont les suivantes :

- point de fusion = >260°C.
- spectre de masse (IE) : M/Z= 509 ($M^+$)

**EXEMPLE 38**

Préparation de l'acide 4,9-éthano-2-[2-(2-méthoxyphényl)propenoyl]-9-(2-méthylphényl)-2,3,3a,4,9,9a-hexahydro-1H-benzo[f]isoindole-3a-carboxylique-(3aRS,4SR,9SR,9aRS)

Etape A

En opérant comme à l'étape C de l'exemple 1, mais à partir de 1,33 $cm^3$ de 2-bromotoluène, de 0,27 g de magnésium en tournures et de 2 g de 2-benzyl-7-oxo-4-phényloctahydroisoindole-3a-carboxylate de méthyle-(3aRS, 4SR,7aRS), on obtient, après purification par flash-chromatographie sur gel de silice (230-400 mesh), en éluant avec un mélange cyclohexane-acétate d'éthyle (90-10 en volumes), 2,2 g de 2-benzyl-7-hydroxy-7-(2-méthylphényl)-4-phényloctahydroisoindole-3a-carboxylate de méthyle-(3aRS,4SR,7RS,7aRS) sous forme d'une meringue jaune pâle utilisée telle quelle dans les synthèses ultérieures.

Etape B

A une solution de 2,2 g de 2-benzyl-7-hydroxy-7-(2-méthylphényl)-4-phényloctahydroisoindole-3a-carboxylate de méthyle-(3aRS,4SR,7RS,7aRS) dans 20 $cm^3$ de dichlorométhane, maintenue à une température voisine de 0°C, on ajoute goutte à goutte un mélange de 3,2 $cm^3$ d'acide trifluorométhanesulfonique et de 3 $cm^3$ de dichlorométhane. Le mélange réactionnel est agité pendant trente minutes à une température voisine de 0°C, vingt heures à une température voisine de 20°C puis refroidi à une température voisine de 0°C. Le pH est alors amené à 8 par addition d'une solution aqueuse normale d'hydroxyde de sodium. La phase aqueuse est séparée par décantation et extraite par trois fois 50 $cm^3$ de dichlorométhane. Les phases organiques sont réunies, lavées par trois fois 100 $cm^3$ d'eau distillée, 100 $cm^3$ d'une solution aqueuse saturée en chlorure de sodium, séchées sur sulfate de magnésium et concentrées sous pression réduite. On obtient ainsi 1,9 g de 2-benzyl-7-(2-methylphényl)-4-phényl-1,3,3a,4,5,6-hexahydroisoindole-3a-carboxylate de méthyle-(3aRS,4SR) sous forme d'une huile utilisée telle quelle dans les synthèses ultérieures, dont les caractéristiques sont les suivantes :

- spectre de R.M.N. $^1H$ (250 MHz, $CDCl_3$ avec ajout de quelques gouttes de $CD_3COOD$ d4, d en ppm) ; 1.90 (mt, 1H : 1H du $CH_2$ en 5) ; 2,17 (s, 3H : $ArCH_3$) ; de 2,40 à 2,55 (mt, 2H : l'autre H du $CH_2$ en 5 et 1H du $CH_2$ en 6) ; 2.66 (mt, 1H : l'autre H du $CH_2$ en 6) ; 3,08 (dd, J = 11 et 2,5 Hz, 1H : CH en 4) ; 3,32 (mt, 1H: 1H du $CH_2$ en 1) ; 3,63 (s, 3H : $COOCH_3$) : de 3,60 à 3,70 (mt, 1H : 1H du $CH_3$ en 3) ; 3.75(d, J = 11,5 Hz, l'autre H du $CH_2$ en 1) ; 3,92 (d, J = 10 Hz, l'autre H du $CH_2$ en 3) ; 3,98 et 4,32 (2 d, J = 11 Hz, 1H chacun : $NCH_2Ar$) ; de 6,90 à 7,40 (mt, 14H : H aromatiques du phényle - H aromatiques du benzyle et H aromatiques du 2-méthylphényl).

Etape C

A une sclution de 1,9 g de 2-benzyl-7-(2-méthylphényl)-4-phényl-1,3,3a,4,5,6-hexahydroisoindole -3a-carboxylate de méthyle-(3aRS,4SR,9SR,9aRS) dans 50 $cm^3$ de dichlorométhane, maintenue à une température voisine de 0°C, on ajoute goutte à goutte un mélange de 5,8 $cm^3$ d'acide trifluorométhanesulfonique et de 5 $cm^3$ de dichlorométhane. Le mélange réactionnel est agité pendant une heure à une température voisine de 0°C, quatre-vingt seize heures à une température voisine de 20°C puis refroidi à une température voisine de 0°C. Le pH est alors amené à 8 par addition d'une solution aqueuse normale d'hydroxyde de sodium. La phase organique est séparée par décantation, lavée par 100 $cm^3$ d'eau distillée, 100 $cm^3$ d'une solution aqueuse saturée en chlorure de sodium, séchée sur sulfate de magnésium et concentrée sous pression réduite. Le résidu est purifié par flash-chromatographie sur gel de silice (230-400 mesh), en éluant avec un mélange cyclohexane-acétate d'éthyle (95-5 en volumes), on obtient 0,85 g de 2-benzyl-4,9-éthano-9-(2-méthylphényl)-2,3,3a,4,9,9a-hexahydro-1H-benzo[f]isoindole-3a-carboxylate de méthyle-(3aRS,4SR,9SR,9aRS) sous forme d'une huile utilisée telle quelle dans les synthèses ultérieures.

Etape D

En opérant comme à l'étape E de l'exemple 1, mais à partir de 0,85 g de 2-benzyl-4,9-éthano-9-(2-méthylphényl)-2,3,3a,4,9,9a-hexahydro-1H-benzo[f]isoindole-3a-carboxylate de méthyle-(3aRS,4SR,9SR,9aRS), de 0,37 g de formiate d'ammonium et de 0,15 g de palladium sur charbon à 10 % (p/p). on obtient après flash-chromatographie sur gel de silice (230-400 mesh), en éluant avec un mélange cyclohexane-acétate d'éthyle (70-30 en volumes), 0,6 g de 4,9-éthano-9-(2-méthylphényl)-2,3,3a,4,9,9a-hexahydro-1H-benzo[f]isoindole-3a-carboxylate de méthyle-(3aRS, 4SR,9SR,9aRS) sous forme d'une huile incolore utilisée telle quelle dans les synthèses ultérieures.

Etape E

En opérant comme a l'étape G de l'exemple 1, mais a partir de 0.6 g de 4,9-éthano-9-(2-méthylphényl)-2,3,3a,4,9,9a-hexahydro-1H-benzo[f]isoindole-3a-carboxylate de méthyle-(3aRS,4SR,9SR,9aRS), de 0,31 g d'acide 2-(2-méthoxyphényl)propènoïque et de 0,15 $cm^3$ de chlorure d'oxalyle, on obtient, après purification par flash-chromatographie sur gel de silice (230-400 mesh), en éluant avec un mélange cyclohexane-acétate d'éthyle (70-30 en volumes), 0,51 g de 4,9-éthano-2-[2-(2-méthoxyphényl)propènoyl]-9-(2-méthylphényl)-2,3,3a,4,9,9a-hexahydro-1H-benzo[f]isoindole-3a-carboxylate de méthyle-(3aRS,4SR,9SR,9aRS) sous forme d'une meringue blanchâtre utilisée telle quelle dans les synthèses ultérieures.

Etape F

En opérant comme à l'exemple 2, mais à partir de 0,51 g de 4,9-éthano-2-[2-(2-méthoxyphényl)propènoyl]-9-(2-méthylphényl)-2,3,3a,4,9,9a-hexahydro-1H-benzo[f]isoindole-3a-carboxylate de méthyle-(3aRS,4SR,9SR,9aRS), dans 1,5 $cm^3$ d'une solution aqueuse normale d'hydroxyde de sodium et 15 $cm^3$ d'éthanol, on obtient, après recristallisation dans l'isopropanol, 0,22 g d'acide 4,9-éthano-2-[2-(2-méthoxyphényl)propénoyl]-9-(2-méthylphényl)-2,3,3a,4,9,9a-hexahydro-1H-benzo[f]isoindole-3a-carboxylique-(3aRS,4SR,9SR,9aRS) dont la caractéristique est la suivante :

- point de fusion : au dessus de 260°C

- spectre de R.M.N. $^1H$ (250 MHz, $(CD_3)_2SO$ d6, à une température de 393 K, d en ppm) : de 1,15 à 1,50 et de 2,05 à 2,35 (2 mts, 2H chacun : $CH_2CH_2$) : 2,25 (s, 3H : $ArCH_3$) ; 3,13 (dd, J = 12 et 9 Hz, 1H : 1H du $CH_2$ en 1) ; 3,31 (d large, J = 12 Hz, 1H : l'autre H du $CH_2$ en 1) ; 3,44 (mt, 1H : CH en 4) ; 3,60 et 4,05 (respectivement d et d large, J = 12,5 Hz, 1H chacun : $CH_2$ en 3) ; 3,75 (s, 3H : $ArOCH_3$) : 5,54 et 5,69 (2 s, 1H chacun : = $CH_2$) ; 6,62 (d large, J = 7,5 Hz, 1H : H en 8) ; de 6,90 à 7,45 (mt, 11H : H en 5 - H en 6 - H en 7 - H aromatiques du 2-méthoxyphényl et H aromatiques du 2-méthylphényl).

**EXEMPLE 39**

Préparation du 4,9-éthano-2-[2-(2-méthoxyphényl)propènoyl]-9-[(4-trifluorométhyl)phényl]-2,3,3a,4,9,9a-hexahydro-1H-benzo[f]isoindole-3a-carboxylate de méthyle-(3aRS,4SR,9SR,9aRS)

Etape A

A une solution de 7,2 g de 4-trifluorométhyl-bromobenzène dans 40 $cm^3$ d'oxyde de diéthyle sec, on joute 0,8 g de magnésium en tournures. Le mélange réactionnel se porte spontanément au reflux pendant dix minutes puis est chauffé au reflux pendant vingt minutes. Le milieu réactionnel est ensuite refroidi et on additionne en quinze minutes, à une température voisine de 10°C, une solution de 5,6 g de 2-benzyl-7-oxo-4-phényloctahydroisoindole-3a-carboxylate de méthyle-(3aRS,4SR,7aRS) dans 40 $cm^3$ d'oxyde de diéthyle sec. Le mélange réactionnel est agité pendant dix minutes à une température voisine de 10°C puis versé dans 150 $cm^3$ d'une solution aqueuse saturée en chlorure d'ammonium. La phase aqueuse est séparée par décantation et extraite par trois fois 75 $cm^3$ d'oxyde de diéthyle. Les phases organiques sont réunies, lavées successivement par trois fois 75 $cm^3$ d'eau distillée, séchées sur sulfate de magnésium en présence de noir 3S et concentrées sous pression réduite. Après cristallisation dans 25 $cm^3$ de pentane, on obtient 5,2 g de 2-benzyl-7-hydroxy-7-[(4-trifluorométhyl)phényl]-4-phényloctahydroisoindole-3a-carboxylate de méthyle-(3aRS,4SR,7RS,7aRS) sous forme de solide beige dont la caractéristique est la suivante :

- point de fusion = 164°C.

Etape B

A 20 $cm^3$ d'acide trifluoromethanesulfonique refroidis à une température voisine de 0°C, on ajoute 4 g de 2-benzyl-7-hydroxy-7-[(4-trifluorométhyl)phènyl]-4-phényloctahydroisoindole-3a-carboxylate de méthyle-(3aRS,4SR,7RS,7aRS). Le mélange réactionnel est agité pendant 3 heures à une température voisine de 20°C puis refroidi à une température voisine de 10°C. On ajoute alors 50 $cm^3$ d'eau distillée puis le pH de la phase aqueuse est amené entre 8 et 9 par addition d'une solution aqueuse à 30% d'hydroxyde de sodium en maintenant toujours la température au voisinage de 10 à 15°C par un bain contenant un mélange éthanol-carboglace. La phase aqueuse est séparée par décantation et extraite par trois fois 75 $cm^3$ d'acétate d'éthyle. Les extraits organiques sont réunis, lavés par trois fois

75 $cm^3$ d'eau distillée, séchés sur sulfate de magnésium en présence de noir 3S et concentrés sous pression réduite. On obtient ainsi en deux jets, après cristallisation dans l'éther de pétrole, 3,4 g de 2-benzyl-4,9-éthano-9-[(4-trifluorométhyl)phényl]-2,3,3a,4,9,9a-hexahydro-1H-benzo[f]isoindole-3a-carboxylate de méthyle-(3aRS,4SR,9SR,9aRS) sous forme de solide blanc dont la caractéristique est la suivante :

- point de fusion : 132°C.

Etape C

En opérant comme à l'étape E de l'exemple 1, mais à partir de 3,4 g de 2-benzyl-4,9-éthano-9-[(4-trifluorométhyl)phényl]-2,3,3a,4,9,9a-hexahydro-1H-benzo[f]isoindole-3a-carboxylate de méthyle-(3aRS,4SR,9SR,9aRS), de 1,3 g de formiate d'ammonium et de 0,4 g de palladium sur charbon à 10 % (p/p), on obtient 2,2 g de 4,9-éthano-9-(4-trifluorométhylphényl)-2,3,3a,4,9,9a-hexahydro-1H-benzo[f]isoindole-3a-carboxylate de méthyle-(3aRS,4SR,9SR,9aRS) sous forme de solide blanc dont la caractéristique est la suivante :

- point de fusion = 172°C.

Etape D

En opérant comme à l'étape G de l'exemple 1, mais à partir de 1,4 g de 4,9-éthano-9-[(4-trifluorométhyl)phényl]-2,3,3a,4,9,9a-hexahydro-1H-benzo[f]isoindole-3a-carboxylate de méthyle-(3aRS,4SR,9SR,9aRS), de 0,62 g d'acide 2-(2-méthoxyphényl)propénoïque, de 0,94 $cm^3$ de triéthylamine et de 0,3 $cm^3$ de chlorure d'oxalyle, on obtient, après purification par flash-chromatographie sur gel de silice (230-400 mesh) en éluant avec un mélange cyclohexane-acétate d'éthyle (50-50 en volumes) et cristallisation dans l'oxyde de diisopropyle, 1,3 g de 4,9-éthano-2-[2-(2-méthoxyphényl)propenoyl]-9-[(4-trifluorométhyl)phényl]-2,3,3a,4,9,9a-hexahydro-1H-benzo[f]isoindole-3a-carboxylate de méthyle-(3aRS,4SR,9SR,9aRS) dont les caractéristiques sont les suivantes :

- point de fusion = 163°C.
- spectre de R.M.N. $^1H$ (250 MHz, $(CD_3)_2SO$ d6, à une température de 373 K, d en ppm) : 1,45 - 1,72 et de 1,95 à 2,25 (3 mts, respectivement 1H - 1H et 2H : $CH_2CH_2$) ; de 3,20 à 3,45 (mt, 3H : $CH_2$ en 1 et CH en 9a) ; 3,48 (mt, 1H: CH en 4) : 3,55 (s, 3H : $COOCH_3$) ; 3,61 et 4,11 (respectivement d et d large, J = 12,5 Hz, 1H chacun : $CH_2$ en 3) ; 3,70 (s, 3H : $ArOCH_3$) ; 5,55 et 5,68 (2 s, 1H chacun : $=CH_2$) ; 6,36 (d large, J = 7,5 Hz, 1H : H en 8) ; de 6,90 à 7,40 (mt, 7H : H en 5 - H en 6 - H en 7 et H aromatiques du 2-méthoxyphényl) ; 7,59 et 7,83 (2d larges, J = 8 Hz, 2H chacun : H aromatiques du 4-trifluorométhylphényl).

**EXEMPLE 40**

Préparation de l'acide 4,9-éthano-2-[2-(2-méthoxyphényl)propènoyl]-9-[(4-trifluorométhyl)phényl]-2,3,3a,4,9,9a-hexahydro-1H-benzo[f]isoindole-3a-carboxylique-(3aRS,4SR,9SR,9aRS)

En opérant comme à l'exemple 2, mais à partir de 1,1 g de 4,9-éthano-2-[2-(2-méthoxyphényl)propènoyl]-9-[(4-trifluorométhyl)phényl]-2,3,3a,4,9,9a-hexahydro-1H-benzo[f]isoindole-3a-carboxylate de méthyle-(3aRS,4SR,9SR,9aRS), de 9,8 $cm^3$ d'une solution aqueuse normale d'hydroxyde de sodium et 20 $cm^3$ de méthanol, on obtient, après cristallisation dans l'oxyde de diisopropyle, passage base acide et recristallisation dans 8 $cm^3$ d'acétate d'isopropyle, 0.65 g d'acide 4,9-éthano-2-[2-(2-méthoxyphényl)propènoyl]-9-[(4-trifluorométhyl)phényl]-2,3,3a,4,9,9a-hexahydro-1H-benzo[f]isoindole-3a-carboxylique-(3aRS,4SR,9SR,9aRS) dont la caractéristique est la suivante :

- point de fusion = 153°C.

**EXEMPLE 41**

Isolement de l'énantiomère dextrogyre de l'acide 4,9-éthano-2-[2-(2-méthoxyphényl)propènoyl]-9-[(4-trifluorométhyl)phényl]-2,3,3a,4,9,9a-hexahydro-1H-benzo[f]isoindole-3a-carboxylique-(3aRS,4SR,9SR,9aRS)

11 g d'acide 4,9-éthano-2-[2-(2-méthoxyphényl)propènoyl]-9-[(4-trifluorométhyl)phényl]-2,3,3a,4,9,9a-hexahydro-1H-benzo[f]isoindole-3a-carboxylique-(3aRS,4SR,9SR,9aRS), obtenus à l'exemple 40, sont dédoublés, sur une colonne de silice chirale porteuse de greffons N-(3,5-dinitrobenzoyl)-phénylalanine-(R), en 4 injections successives et en éluant par un mélange de n-heptane, de dichlorométhane et d'éthanol (50/50/1 en volumes). En recueillant les

premières fractions éluées (temps de rétention 31 mn), on obtient après concentration sous pression réduite 5 g de l'énantiomère dextrogyre de l'acide 4,9-éthano-2-[2-(2-méthoxyphényl)propènoyl]-9-[(4-trifluorométhyl)phényl]-2,3,3a,4,9,9a-hexahydro-1H-benzo[f]isoindole-3a-carboxylique-(3aRS,4SR,9SR,9aRS), sous forme d'une poudre blanche dont les caractéristiques sont les suivantes :

- point de fusion = 228°C
- spectre de masse (IE) : M/Z= 547 ($M^+$)
- pouvoir rotatoire: $[a]_{365}^{20}$= + 63,1 +/- 1.2° (c = 0,5/ méthanol)

La phase stationnaire chirale porteuse de greffons N-(3,5-dinitrobenzoyl)-phénylalanine-(R) peut être préparée selon la synthèse décrite à l'exemple 3, en remplaçant la N-(3,5-dinitrobenzoyl)-phénylalanine-(S) par de la N-(3,5-dinitrobenzoyl)-phénylalanine-(R).

**EXEMPLE 42**

<u>Préparation du 4,9-éthano-2-[2-(2-méthoxyphényl)propenoyl]-9-[(4-trifluorométhyl)phényl]-2,3,3a,4,9,9a-hexahydro-1H-benzo[f]isoindole-3a-N-(3-pyridylméthyl)-carboxamide-(3aRS,4SR,9SR,9aRS)</u>

A une solution de 1,1 g d'acide 4,9-éthano-2-[2-(2-méthoxyphényl)propènoyl]-9-[(4-trifluorométhyl)phényl]-2,3,3a,4,9,9a-hexahydro-1H-benzo[f]isoindole-3a-carboxylique-(3aRS,4SR,9SR,9aRS) dans 20 $cm^3$ de dichlorométhane, on ajoute successivement 0,24 $cm^3$ de 3-(aminométhyl)pyridine, 0,46 g de chlorhydrate 1-éthyl-3-[(3-diméthylamino)propyl)carbodiimide et 0,16 g d'hydrate de N-1-hydroxybenzotriazole. Le mélange réactionnel est agité pendant quarante huit heures à une température voisine de 20°C. La phase organique est lavée par trois fois 10 $cm^3$ d'eau distillée, séchée sur sulfate de magnésium et concentrée sous pression réduite. Après recristallisation dans 15 $cm^3$ d'isopropanol, on obtient 0,95 g de 4,9-éthano-2-[2-(2-méthoxyphényl)propènoyl]-9-[(4-trifluorométhyl)phényl]-2,3,3a,4,9,9a-hexahydro-1H-benzo[f]isoindole-3a-N-(3-pyridylméthyl)-carboxamide-(3aRS,4SR,9SR,9aRS) dont la caractéristique est la suivante :

- point de fusion = 214°C.

**EXEMPLE 43**

<u>Préparation du 4,9-éthano-2-[2-(2-méthoxyphényl)propènoyl]-9-[(4-trifluorométhyl)phényl]-2,3,3a,4,9,9a-hexahydro-1H-benzo[f]isoindole-3a-N'-benzoyl-carbohydrazide-(3aRS,4SR,9SR,9aRS)</u>

A une solution de 0,55 g d'acide 4,9-éthano-2-[2-(2-méthoxyphényl)propènoyl]-9-[(4-trifluorométhyl)phényl]-2,3,3a,4,9,9a-hexahydro-1H-benzo[f]isoindole-3a-carboxylique-(3aRS,4SR,9SR,9aRS) dans 10 $cm^3$ de dichlorométhane, on ajoute successivement 0,16 g de benzoylhydrazine, 0,23 g de chlorhydrate de 1-éthyl-3-[(3-diméthylamino)propyl]carbodiimide et 81 mg de d'hydrate de N-1-hydroxybenzotriazole. Le mélange réactionnel est agité pendant vingt heures à une température voisine de 20°C puis filtré sur verre fritté. Le précipité est lavé successivement par trois fois 1,5 $cm^3$ de dichlorométhane puis trois fois 2,5 $cm^3$ d'eau distillée. On obtient ainsi 0,34 g de 4,9-éthano-2-[2-(2-méthoxyphényl)propènoyl]-9-[(4-trifluorométhyl)phényl]-2,3,3a,4,9,9a-hexahydro-1H-benzo[f]isoindole-3a-N'-benzoyl-carbohydrazide-(3aRS,4SR,9SR,9aRS) dont les caractéristiques sont les suivantes :

- point de fusion = 275°C.
- spectre de R.M.N. $^1H$ (250 MHz, $(CD_3)_2SO$ d6, à une température de 383 K, d en ppm) : 1,44 - 1,70 - 2,01 et 2,18 (4 mts, 1H chacun : $CH_2CH_2$) ; de 3,20 à 3,40 (AB limite, 2H : $CH_2$ en 1) ; de 3,55 à 3,85 (mt, 3H : 1H du $CH_2$ en 3 - CH en 9a et CH en 4) ; 3,72 (s, 3H : $ArOCH_3$) ; 4,36 (d large, J = 12,5 Hz, 1H : l'autre H du $CH_2$ en 3) ; 5,57 et 5,70 (2 s, 1H chacun : $=CH_2$) ; 6,34 (d large, J = 7,5 Hz, 1H : H en 8) ; de 6,85 à 7,60 (mt, 10H : H en 5 - H en 6 - H en 7 - H aromatiques en méta du benzoylcarbohydrazide- H aromatique en para du benzoylcarbohydrazide et H aromatiques du 2-méthoxyphényl) ; 7,60 et 7,85 (2d, J = 8 Hz, 2H chacun : H aromatiques du 4-trifluorométhylphényl) ; 7,81 (d, J = 8 Hz, 2H : H aromatiques en ortho du benzoylcarbohydrazide) ; 9,66 et 9,91 (2 s larges, 1H chacun CONHNHCO).

## EXEMPLE 44

Préparation du 4,9-éthano-2-[2-(2-méthoxyphényl)propènoyl]-9-(4-trifluorométhylphényl)-2,3,3a,4,9,9a-hexahydro-1H-benzo[f]isoindole-3a-N'-phényl-carbohydrazide-(3aRS,4SR,9SR,9aRS)

A une solution de 0,55 g d'acide 4,9-ethano-2-[2-(2-méthoxyphényl)propènoyl]-9-(4-trifluorométhylphényl)-2,3,3a,4,9,9a-hexahydro-1H-benzo[f]isoindole-3a-carboxylique-(3aRS,4SR,9SR,9aRS) dans 10 $cm^3$ de dichlorométhane, on ajoute successivement 0,12 $cm^3$ de phénylhydrazine, 0,23 g de chlorhydrate de 1-éthyl-3-[(3-diméthylamino) propyl]carbodiimide et 81 mg de d'hydrate de N-1-hydroxybenzotriazole. Le mélange réactionnel est agité pendant vingt heures à une température voisine de 20°C. Le milieu réactionnel est dilué par 15 $cm^3$ de dichlorométhane, lavé par trois fois 10 $cm^3$ d'eau distillée, séché sur sulfate de magnésium en présence de noir 3S et concentré sous pression réduite. Après recrisrallisation dans 10 $cm^3$ d'isopropanol, on obtient 0.21 g de 4,9-éthano-2-[2-(2-méthoxyphényl) propènoyl]-9-(4-trifluorométhylphènyl)-2,3,3a,4,9,9a-hexahydro-1H-benzo[f]isoindole-3a-N'-phényl-carbohydrazide-(3aRS,4SR,9SR,9aRS) dont les caractéristiques sont les suivantes :

- point de fusion : 226°C.
- spectre de R.M.N. $^1H$ (250 MHz, $(CD_3)_2SO$ d6, à une température de 383 K, d en ppm) : 1,44 - 1,69 et de 1,95 à 2,25 (3 mts, respectivement 1H - 1H et 2H : $CH_2CH_2$); de 3,20 à 3,45 (AB limite, 2H : $CH_2$ en 1) ; 3,52 et 4,26 (respectivement d et d large, J = 12,5 Hz, 1H chacun : $CH_2$ en 3); 3,63 (mt, 1H : CH en 9a); 3,69 (s, 3H : $ArOCH_3$) ; 3,75 (mt, 1H : CH en 4); 5,55 et 5,68 (2 s, 1H chacun : $=CH_2$); 6,35 (d large, J = 7,5 Hz, 1H : H en 8); 6,60 (d, J = 7,5 Hz, 2H : H aromatiques en ortho du phénylcarbohydrazide) ; 6,70 (t, J = 7,5 Hz, 1H : H aromatique en para du phénylcarbohydrazide) ; de 6,90 à 7,45 (mt, 10H : H en 5 - H en 6 - H en 7 - H aromatiques en méta du phényl-carbohydrazide - H aromatiques du 2-méthoxyphényl et ArNH) ; 7,61 et 7,82 (2d. J = 8 Hz, 2H chacun : H aromatiques du 4-trifluorométhylphényl) ; 9,52 (s large, 1H : CONH).

## EXEMPLE 45

Préparation du 4,9-éthano-2-[2-(2-méthoxyphényl)propénoyl]-9-(2-naphthyl)-2,3,3a,4,9,9,9a-hexahydro-1H-benzo[f]isoindole-3a-carboxylate de méthyle-(3aRS,4SR,9SR,9aRS)

Etape A

A une solution de 40 g de 2-benzyl-7-oxo-4-phényloctahydroisoindole-3a-carboxylate de méthyle, obtenu à l'étape B de l'exemple 1, dans 300 $cm^3$ d'éthanol, on ajoute 21,4 g d'hydrate d'hydrazine puis on porte au reflux pendant deux heures trente minutes. Après concentration du solvant sous pression réduite, on reprend le résidu à l'eau et on extrait au dichlorométhane. La phase organique est décantée, lavée a l'eau et séchée sur sulfate de magnésium. Apres concentration sous pression réduite, on obtient 38,73 g (93%) de 2-benzyl-7-hydrazono-4-phenyloctahydroisoindole-3a-carboxylate de méthyle-(3aRS,4SR,7aRS), sous forme d'une huile brune dont les caractéristiques sont les suivantes :

- spectre de masse (IE) : M/Z= 377 ($M^+$)
- spectre IR (en solution dans le dichlorométhane) :

| | |
|---|---|
| 3400 $cm^{-1}$ | n NH |
| 3080, 3060, 3045, 3030 $cm^{-1}$ | n CH aromatiques |
| 2950, 2800 $cm^{-1}$ | n CH aliphatiques |
| 2730 $cm^{-1}$ | n CH $N(CH_2)_3$ |
| 1720 $cm^{-1}$ | n C=O ester méthylique |
| 1605, 1495, 1455, 1435 $cm^{-1}$ | respiration des noyaux aromatiques |
| 1435 $cm^{-1}$ | $d_s$ $CH_3$ ester méthylique |
| 1220 $cm^{-1}$ | n O-C=O ester méthylique |

Etape B

A une solution de 37,75 g de 2-benzyl-7-hydrazono-4-phényloctahydroisoindole-3a-carboxylate de méthyle-(3aRS,4SR,7aRS) dans 200 $cm^3$ de tétrahydrofurane et 43 $cm^3$ de triéthylamine, on ajoute goutte à goutte 52,07

g d'iode en solution dans 250 $cm^3$ de tétrahydrofurane. L'agitation est maintenue à température ambiante une heure après la fin de l'addition. On ajoute alors 1 $dm^3$ d'eau et 1 $dm^3$ d'acétate d'éthyle. La phase organique est décantée, lavée successivement par une solution saturée d'hydrosulfite de sodium puis par une solution saturée de chlorure de sodium et séchée sur sulfate de magnésium. Après concentration du solvant sous pression réduite, le résidu est purifié par flash-chromatographie sur gel de silice (230-400 Mesh) en éluant par un mélange de cyclohexane et d'acétate d'éthyle (95-5 en volumes). On obtient ainsi 22,25 g (47%) de 2-benzyl-7-iodo-2,3,3a,4,5,7a-hexahydro-1H-isoindole-3a-carboxylate de méthyle-(3aRS,4SR,7aRS), sous forme d'un solide pâteux jaune dont les caractéristiques sont les suivantes :

- spectre de masse (IE) : M/Z= 473 ($M^+$)
- spectre IR (en solution dans le tétrachlorure de carbone):

| | |
|---|---|
| 3080, 3060, 3030 $cm^{-1}$ | n CH aromatiques |
| 2925, 2800 $cm^{-1}$ | n CH aliphatiques |
| 2730 $cm^{-1}$ | n CH $N(CH_2)_3$ |
| 1730 $cm^{-1}$ | n C=O ester méthylique |
| 1600, 1495, 1455, 1435 $cm^{-1}$ | respiration des noyaux aromatiques |
| 1435 $cm^{-1}$ | $d_s$ $CH_3$ ester méthylique |
| 1210 $cm^{-1}$ | n O-C=O ester méthylique |
| 700 $cm^{-1}$ | g CH aromatiques |

Etape C

A une solution de 2 g de 2-benzyl-7-iodo-4-phényl-2,3,a,4,5,7a-hexahydroisoindole-3a-carboxylate de méthyle-(3aRS,4SR,7aRS) dans 40 $cm^3$ de toluène, on ajoute 240 mg de tétrakis(triphénylphosphine)palladium puis une solution de 0,69 g d'acide 2-naphtylboronique, isolé sous forme d'anhydride trimère, dans 20 $cm^3$ de méthanol. On ajoute goutte à goutte 45 $cm^3$ d'une solution aqueuse 2N de carbonate de sodium et l'on porte au reflux pendant quatre heures. On ajoute, après refroidissement, 80 $cm^3$ d'eau et 60 $cm^3$ d'acétate d'éthyle. La phase organique est décantée, lavée à l'eau jusqu'à neutralité et séchée sur sulfate de magnésium. Après concentration du solvant sous pression réduite, le résidu est purifié par flash-chromatographie sur gel de silice (230-400 Mesh) en éluant par un mélange de cyclohexane et d'acétate d'éthyle (90-10 en volumes). On obtient ainsi 1,33 g (67%) de 2-benzyl-7-(2-naphtyl)-4-phényl-2,3,3a,4,5,7a-hexahydro-1H-isoindole-3a-carboxylate de méthyle-(3aRS,4SR,7aRS), sous forme d'une meringue blanche dont la caractéristique est la suivante :

- spectre de masse (IE) : M/Z = 473 ($M^+$).

Etape D

En opérant comme à l' étape C de l'exemple 38, mais à partir de 1,66 g de 2-benzyl-7-(2-naphtyl )-4-phényl-2,3,3a,4,5,7a-hexahydro-1H-isoindole-3a-carboxylate de méthyle-(3aRS,4SR,7aRS) et de 1,9 $cm^3$ d'acide trifluorométhanesulfonique en solution dans 20 $cm^3$ de dichlorométhane sec pendant quatre heures à température ambiante, après purification par flash-chromatographie sur gel de silice (230-400 Mesh) en éluant par un mélange de cyclohexane et d'acétate d'éthyle (95-5 en volumes), on isole 1.22 g (51%) d'une meringue blanche contenant majoritairement (environ 90% par dosage RMN) du 2-benzyl-4,9-éthano-9-(2-naphtyl)-2,3,3a,4,9,9a-hexahydro-1H-benzo[f]isoindole-3a-carboxylate de methyle-(3aRS,4SR,9SR,9aRS), utilisée telle quelle dans la suite de la synthese, dont les caractéristiques sont les suivantes :

- spectre de R.M.N. $^1H$ (300 MHz. $CDCl_3$, d en ppm) : de 1,40 à 2,00 et 2,52 (2 mts, 2H chacun : $CH_2CH_2$) ; de 2,20 à 2,40 (mt, 2H : 1H du $CH_2$ en 1 et 1H $CH_2$ en 3) ; 2,68 (d large, J = 10 Hz, 1H ; l'autre H du $CH_2$ en 1) ; 3,21 (d large, J = 10 Hz, l'autre H du $CH_2$ en 3) ; 3,31 et 3,67 (d, J = 12,5 Hz, 2H : $NCH_2Ar$) ; 3,36 (mt, 1H : CH en 9a) ; 3,45 (s large, 1H : CH en 4) ; 3,58 (s, 3H : $COOCH_3$) ; 6,61 (d large, J = 7,5 Hz, 1H : H en 8) ; de 6,91 à 8,00 (mt, 15H : H en 5 - H en 6 - H en 7 - H aromatiques du naphtyle et H aromatiques du benzyle).

Etape E

1,2 g de 2-benzyl-4,9-éthano-9-(2-naphtyl)-2,3,3a,4,9,9a-hexahydro-1H-benzo[f]isoindole-3a-carboxylate de

méthyle-(3aRS,4SR,9SR,9aRS), en solution dans 50 $cm^3$ de méthanol et 2 $cm^3$ d'une solution 2M d'acide chlorhydrique dans le méthanol, sont agités, en présence de 115 mg de palladium sur charbon à 10% (p/p), pendant cinq heures à 50°C sous hydrogène à pression atmosphérique. Apres filtration du catalyseur, le solvant est concentré sous pression réduite. On obtient ainsi 0,96 g (91%) de chlorhydrate de 4,9-éthano-9-(2-naphtyl)-2,3,3a,4,9,9a-hexahydro-1H-benzo[f]isoindole-3a-carboxylate de méthyle-(3aRS,4SR,9SR,9aRS), sous forme d'une poudre beige dont les caractéristiques sont les suivantes:

- point de fusion > 260°C
- spectre de masse (IE) : M/Z = 383 ($M^+$).

Etape F

En opérant comme à l'étape G de l'exemple 1, mais à partir de 0,96 g de chlorhydrate de 4,9-éthano-9-(2-naphtyl)-2,3,3a,4,9,9a-hexahydro-1H-benzo[f] isoindole-3a-carboxylate de méthyle-(3aRS,4SR,9SR,9aRS), de 0,41 g d'acide 2-(2-méthoxyphényl)propénoique, de 0,2 $cm^3$ de chlorure d'oxalyle et de 0,64 $cm^3$ de triéthylamine en solution dans 60 $cm^3$ de dichlorométhane pendant vingt heures a température ambiante, on obtient, après purification sur gel de silice (230-400 Mesh) en éluant par un mélange de cyclohexane et d'acétate d'éthyle (70-30 en volumes), 1,15 g (78%) de 4,9-éthano-2-[2-(2-méthoxyphényl)propènoyl]-9-(2-naphtyl)-2,3,3a,4,9,9a-hexahydro-1H-benzo[f]isoindole-3a-carboxylate de méthyle-(3aRS,4SR, 9SR,9aRS), sous forme d'une poudre blanche dont les caractéristiques sont les suivantes :

- point de fusion = 140-2°C
- spectre de masse (IE) : M/Z = 473 ($M^+$).

**EXEMPLE 46**

Préparation du sel de sodium de l'acide 4,9-éthano-2-[2-(2-méthoxyphényl)propènoyl]-9-(2-naphtyl)-2,3,3a,4,9,9a-hexahydro-1H-benzo[f]isoindole-3a-carboxylique-(3aRS,4SR,9SR,9aRS)

En opérant comme à l'exemple 2, mais à partir de 1,15 g de 4,9-éthano-2-[2-(2-méthoxyphényl)propènoyl]-9-(2-naphtyl)-2,3,3a,4,9,9a-hexahydro-1H-benzo[f]isoindole-3a-carboxylate de méthyle-(3aRS,4SR,9SR,9aRS) au reflux pendant cinq heures dans 6,3 $cm^3$ d'une solution aqueuse normale d'hydroxyde de sodium et de 40 $cm^3$ d'éthanol, on obtient, après purification par flash-chromatographie sur gel de silice (230-400 Mesh) en éluant par un mélange de dichlorométhane et de méthanol (95-5 en volumes) puis par chromatographie liquide haute performance sur silice greffée C18 Waters en éluant par un mélange d'eau et d'acétonitrile (70-30 en volumes), 50 mg (4,8%) de sel de sodium de l'acide 4,9-éthano-2-[2-(2-méthoxyphényl)propènoyl]-9-(2-naphtyl)-2,3,3a,4,9,9a-hexahydro-1H-benzo[f]isoindole-3a-carboxylique-(3aRS,4SR,9SR,9aRS), sous forme d'un solide blanc dont les caractéristiques sont les suivantes :

- point de fusion > 260°C
- spectre de masse (IE) : M/Z = 529 ($M^+$).

**EXEMPLE 47**

Préparation du 4,9-éthano-2-[2-(2-méthoxyphényl)propènoyl]-9-(5-méthyl-2-thiényl)-2,3,3a,4,9,9a-hexahydro-1H-benzo[f]isoindole-3a-carboxylate de méthyle-(3aRS,4SR,9SR,9aRS)

Etape A

En opérant comme à l'étape A de l'exemple 31, mais à partir de 2 g de 2-benzyl-7-oxo-4-phényloctahydroisoindole-3a-carboxylate de méthyle-(3aRS,4SR,9SR,9aRS), de 0,8 $cm^3$ de 2-méthylthiophène et de 3 $cm^3$ d'acide trifluorométhanesulfonique dans 30 $cm^3$ de dichlorométhane pendant cinq heures à température ambiante, on obtient après purification sur gel de silice (230-400 Mesh) en éluant par des mélanges de cyclohexane et d'acétate d'éthyle (95-5 puis 90-10 en volumes), 0,49 g de 2-benzyl-4,9-éthano-9-(5-méthyl-2-thiényl)-2,3,3a,4,9,9a-hexahydro-1H-benzo[f]isoindole-3a-carboxylate de méthyle-(3aRS,4SR,9SR,9aRS), sous forme d'une poudre blanche dont les caractéristiques sont les suivantes :

- spectre de R.M.N. $^1$H (300 MHz, $CDCl_3$, d en ppm) : 1,14 - 1,66 et de 2,35 à 2,55 (3 mts, respectivement 1H - 1H et 2H : $CH_2CH_2$) ; 2,29 et 3,18 (d, J = 10 Hz, 1H chacun : $CH_2$ en 3) ; de 2,35 à 2,55 et 3,00 (respectivement mt et d, J = 10 Hz, 1H chacun : $CH_2$ en 1) ; 2,52 (s, 3H : $ArCH_3$) ; 3,10 (d large, J = 8 Hz, 1H : CH en 9a) ; de 3,30 à 3,40 (mt, 2H : H en 4 et 1H du $NCH_2Ar$) ; 3,54 (s, 3H : $COOCH_3$) ; 3,74 (d, J = 12,5 Hz, 1H : l'autre H du $NCH_2Ar$) ; 6,71 (mt, 1H : H en 8) ; 6,71 et 6,74 (respectivement mt et d, J = 4Hz, 1H chacun : H aromatiques du thiényle) ; de 6,95 à 7,45 (mt, 8H : H en 5 - H en 6 - H en 7 et H aromatiques du benzyle).

Etape B

Une solution de 0,48 g de 2-benzyl-4,9-éthano-9-(5-méthyl-2-thiényl)-2,3,3a,4,9,9a-hexahydro-1H-benzo[f] isoindole-3a-carboxylate de méthyle-(3aRS,4SR,9SR,9aRS) et de 0,17 $cm^3$ de choroformiate de vinyle dans 5 $cm^3$ de dichlorométhane est agitée à température ambiante pendant dix-huit heures. Après concentration du solvant sous pression réduite le résidu est purifié par flash-chromatographie sur gel de silice en éluant par un mélange de cyclohexane et d'acétate d'éthyle (85-15 en volumes) pour donner 0,37 g d'une meringue qui est redissoute dans 7 $cm^3$ de méthanol. On ajoute alors 1,65 $cm^3$ d'une solution aqueuse normale d'acide chlorhydrique et on porte au reflux pendant trois heures. Après concentration du solvant, le résidu est recristallisé dans un mélange d'isopropanol et d'éther isopropylique (50-50 en volumes). On obtient alors 0,3 g (70%) de chlorhydrate de 4,9-éthano-9-(5-méthyl-2-thiényl)-2,3,3a,4,9,9a-hexahydro-1H-benzo[f]isoindole-3a-carboxylate de méthyle-(3aRS,4SR,9SR,9aRS), sous forme d'un solide vert pâle dont les caractéristiques sont les suivantes :

- point de fusion = 226-30°C
- spectre de masse (IE) : M/Z = 353 ($M^+$).

Etape C

En opérant comme à l'étape G de l'exemple 1, mais à partir de 0,58 g de chlorhydrate de 4,9-éthano-9-(5-méthyl-2-thiényl)-2,3,3a,4,9,9a-hexahydro-1H-benzo[f] isoindole-3a-carboxylate de méthyle-(3aRS,4SR,9SR,9aRS), de 0,27 g d'acide 2-(2-méthoxyphényl)propènoique, de 0,13 $cm^3$ de chlorure d'oxalyle et de 0,42 $cm^3$ de triéthylamine en solution dans 35 $cm^3$ de dichlorométhane pendant vingt heures à température ambiante, on obtient, après purification sur gel de silice (230-400 Mesh) en éluant par des mélanges de cyclohexane et d'acétate d'éthyle (80-20 puis 60-40 en volumes), 0,53 g (69%) de 4,9-éthano-2-[2-(2-méthoxyphényl)propènoyl]-9-(5-méthyl-2-thiényl)-2,3,3a,4,9,9a-hexahydro-1H-benzo[f]isoindole-3a-carboxylate de méthyle-(3aRS,4SR,9SR,9aRS), sous forme d'une poudre beige-clair dont les caractéristiques sont les suivantes:

- point de fusion = 1 12-5°C
- spectre de masse (IE): M/Z = 513 ($M^+$).

**EXEMPLE 48**

Préparation de l'acide 4,9-éthano-2-[2-(2-méthoxyphényl)propènoyl]-9-(5-méthyl-2-thiényl)-2,3,3a,4,9,9a-hexahydro-1H-benzo[f]isoindole-3a-carboxylique-(3aRS,4SR, 9SR,9aRS)

En opérant comme à l'exemple 2, mais à partir de 0,45 g de 4.9-éthano-2-[2-(2-méthoxyphényl)propènoyl]-9-(5-méthyl-2-thiényl)-2,3,3a,4,9,9a-hexahydro-1H-benzo[f]isoindole-3a-carboxylate de méthyle-(3aRS,4SR, 9SR, 9aRS) au reflux pendant cinq heures dans 2,6 $cm^3$ d'une solution aqueuse normale d'hydroxyde de sodium et de 15 $cm^3$ d'éthanol, on obtient, après purification par recristallisation dans l'oxyde de diisopropyle, 285 mg d'acide 4,9-éthano-2-[2-(2-méthoxyphényl)propènoyl]-9-(5-méthyl-2-thiényl)-2,3,3a,4,9,9a-hexahydro-1H-benzo[f]isoindole-3a-carboxylique-(3aRS,4SR,9SR,9aRS), sous forme d'un solide beige clair dont les caractéristiques sont les suivantes :

- point de fusion = 164-6°C
- spectre de masse (IE) : M/Z = 499 ($M^+$).

**EXEMPLE 49**

Préparation du 9-(4-bromophényl)-4,9-éthano-2-[2-(2-méthoxyphényl)propènoyl]-2,3,3a,4,9,9a-hexahydro-1H-benzo [f]isoindole-3a-carboxylate de méthyle-(3aRS, 4SR,9SR,9aRS)

Etape A

En opérant comme à l'étape C de l'exemple 45, mais à partir de 1 g de 2-benzyl-7-iodo-4-phényl-2,3,a,4,5,7a-hexahydroisoindole-3a-carboxylate de méthyle-(3aRS,4SR,7aRS) dans 20 $cm^3$ de toluène, de 120 mg de tétrakis (triphénylphosphine)palladium, de 0,46 g d'acide 4-bromophénylboronique dans 10 $cm^3$ de méthanol et de 15 $cm^3$ d'une solution aqueuse 2N de carbonate de sodium au reflux pendant dix huit heures, on obtient, après purification par flash-chromatographie sur gel de silice (230-400 Mesh) en éluant par un mélange de cyclohexane et d'acétate d'éthyle (90-10 en volumes), 0,77 g (73%) de 2-benzyl-7-(4-bromophényl)-4-phényl-2,3,3a,4,5,7a-hexahydro-1H-isoindole-3a-carboxylate de méthyle-(3aRS,4SR,7aRS), sous forme d'une meringue blanche dont la caractéristique est la suivante:

- spectre de masse (IE) : M/Z = 502 ($M^+$).

Etape B

En opérant comme à l'étape C de l'exemple 38, mais à partir de 0,77 g de 2-benzyl-7-(4-bromophényl)-4-phényl-2,3,3a,4,5,7a-hexahydro-1H-isoindole-3a-carboxylate de méthyle-(3aRS,4SR,7aRS) et de 1,18 $cm^3$ d'acide trifluorométhanesulfonique dans 20 $cm^3$ de dichlorométhane pendant dix huit heures à température ambiante, on obtient 0,71 g (92%) de 2-benzyl-9-(4-bromophényl)-4,9-éthano-2,3,3a,4,9,9a-hexahydro-1H-benzo[f]isoindole-3a-carboxylate de méthyle-(3aRS,4SR,9SR,9aRS), sous forme d'une poudre beige, utilisée telle quelle dans la suite de la synthèse, dont la caractéristique est la suivante :

- spectre de masse (IE) : M/Z = 502 ($M^+$).

Etape C

En opérant comme à l'étape B de l'exemple 47, mais à partir de 0,71 g de 2-benzyl-9-(4-bromophényl)-4,9-éthano-2,3,3a,4,9,9a-hexahydro-1H-isoindole-3a-carboxylate de méthyle-(3aRS,4SR,9SR,9aRS) et de 0,4 $cm^3$ de chloroformiate de vinyle pendant soixante douze heures à température ambiante dans 20 $cm^3$ de dichlorométhane, puis en reprenant le concentrat dans 40 $cm^3$ de méthanol et 5 $cm^3$ d'une solution 6M d'acide chlorhydrique dans le dioxane au reflux pendant trois heures, on obtient, après cristallisation dans l'oxyde de diisopropyle, 0,45 g (77%) de chlorhydrate de 9-(4-bromophényl)-4,9-éthano-2,3,3a,4,9,9a-hexahydro-1H-isoindole-3a-carboxylate de méthyle-(3aRS,4SR,9SR,9aRS), sous forme de cristaux blancs dont la caractéristique est la suivante :

- spectre de masse (IE) : M/Z = 412 ($M^+$).

Etape D

En opérant comme à l'étape G de l'exemple 1, mais à partir de 0,45 g de chlorhydrate de 9-(4-bromophényl)-4,9-éthano-2,3,3a,4,9,9a-hexahydro-1H-benzo[f] isoindole-3a-carboxylate de méthyle-(3aRS,4SR,9SR,9aRS), de 0,21 g d'acide 2-(2-méthoxyphényl)propènoique, de 0,10 $cm^3$ de chlorure d'oxalyle et de 0,33 $cm^3$ de triéthylamine en solulion dans 30 $cm^3$ de dichlorométhane pendant vingt heures à température ambiante, on obtient, après purification sur gel de silice (230-400 Mesh) en éluant par un mélange de cyclohexane et d'acétate d'éthyle (70-30 en volumes), 0,25 g (40%) de 9-(4-bromophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS), sous forme d'une poudre beige-clair dont les caractéristiques sont les suivantes :

- point de fusion = 190-2°C

- spectre de masse (IE) : M/Z = 572 ($M^+$)

**EXEMPLE 50**

Préparation de l'acide 9-(4-bromophényl)-4,9-éthano-2-[2-(2-méthoxyphényl) propènoyl]-2,3,3a,4,9,9a-hexahydro-1H-benzo[f]isoindole-3a-carboxylique-(3aRS,4SR,9SR,9aRS)

En opérant comme à l'exemple 2, mais à partir de 0,19 g de 9-(4-bromophényl)-4,9-éthano-2-[2-(2-méthoxyphényl)propènoyl]-2,3,3a,4,9,9a-hexahydro -1H-benzo[f]isoindole-3a-carboxylate de méthyle-(3aRS,4SR,9SR,9aRS) au reflux pendant trois heures dans 0,44 $cm^3$ d'une solution aqueuse normale d'hydroxyde de sodium et de 10 $cm^3$ d'éthanol, on obtient, après purification par recristallisation dans l'oxyde de diisopropyle, 120 mg (66%) d'acide 9-(4-bromophényl)-4,9-éthano-2-[2-(2-méthoxyphényl)propènoyl]-2,3,3a,4,9,9a-hexahydro-1H-benzo[f]isoindole-3a-carboxylique-(3aRS,4SR,9SR,9aRS), sous forme d'un solide blanc dont les caractéristiques sont les suivantes :

- point de fusion = 169°C
- spectre de masse (IE) : M/Z = 558 ($M^+$).

**EXEMPLE 51**

Préparation de l'acide 9-(3,4-dichlorophényl)-4,9-éthano-2-[2-(2-méthoxyphényl) propènoyl]-2,3,3a,4,9,9a-hexahydro-1H-benzo[f]isoindole-3a-carboxylique-(3aRS,4SR,9SR,9aRS)

Etape A

En opérant comme à l'étape A de l'exemple 39, mais à partir de 3,6 g de 3,4-dichloro-bromobenzène, de 0,4 g de magnésium en toumures dans 20 $cm^3$ d'oxyde de diéthyle de 2,9 g de 2-benzyl-7-oxo-4-phényloctahydroisoindole-3a-carboxylate de méthyle-(3aRS,4SR,7aRS) dans 20 $cm^3$ d'oxyde de diéthyle, on obtient, après recristallisation dans 35 $cm^3$ d'isopropanol, 1,8 g de 2-benzyl-7-(3,4-dichlorophényl)-7-hydroxy-4-phényloctahydroisoindole-3a-carboxylate de méthyle-(3aRS,4SR,7RS,7aRS) sous forme d'un solide blanc dont la caractéristique est la suivante :

- point de fusion = 144°C.

La cristallisation par l'oxyde de diisopropyle du filtrat amené à sec permet d'obtenir 0.6 g supplémentaires. La purification de ce second filtrat par flash-chromatographie sur gel de silice (230-400 mesh), en éluant avec un mélange cyclohexane-acétate d'éthyle (80-20 en volumes) permet d'obtenir, après cristallisation dans l'oxyde de diisopropyle, 0,25 g additionnels.

Etape B

En opérant comme à l'étape D de l'exemple 1, mais a partir de 1,2 g de 2-benzyl-7-(3,4-dichlorophényl)-7-hydroxy-4-phényl-1,3,3a,4,5,6-hexahydroisoindole-3a-carboxylate de méthyle-(3aRS,4SR,7RS,7aRS), de 3 $cm^3$ d'acide trifluorométhanesulfonique et de 45 $cm^3$ de dichlorométhane, on obtient 1,1 g de 2-benzyl-9-(3,4-dichlorophényl)-4,9-éthano-2,3,3a,4,9,9a-hexahydro-1H-benzo[f]isoindole-3a-carboxylate de méthyle-(3aRS,4SR,9SR,9aRS) sous forme d'une meringue blanche utilisée telle quelle dans les synthèses ultérieures.

Etape C

A une solution de 0,7 g de 2-benzyl-9-(3,4-dichlorophényl)-4,9-éthano-2,3,3a,4,9,9a-hexahydro-1H-benzo[f] isoindole-3a-carboxylate de méthyle-(3aRS,4SR,9SR,9aRS) dans 7,5 $cm^3$ de dichlorométhane, on ajoute 0,18 $cm^3$ de chloroformiate de vinyle. Le mélange réactionnel est agité pendant vingt heures à une température voisine de 20°C puis concentré sous pression réduite. Le résidu est dilué avec 7,5 $cm^3$ de méthanol. A cette solution, on ajoute 14 $cm^3$ d'une solution normale de gaz chlorhydrique dans l'oxyde de dièthyle. Le mélange réactionnel est agité pendant vingt heures à une température voisine de 20°C puis concentré sous pression réduite. Le résidu est repris dans un mélange de 25 $cm^3$ de dichlorométhane et 20 $cm^3$ d'une solution aqueuse saturée en hydrogénocarbonate de sodium. La phase aqueuse est séparée par décantation et extraite par deux fois 15 $cm^3$ d'oxyde de diéthyle. Les phases organiques sont réunies, lavées par trois fois 15 $cm^3$ d'eau distillée, séchées sur sulfate de magnésium et concentrées sous pression réduite. On obtient ainsi, après flash-chromatographie sur gel de silice (230-400 mesh), en éluant avec un mélange cyclohexane-acétate d'éthyle (80-20 en volumes) et cristallisation dans 5 $cm^3$ d'oxyde de diisopropyle, 0,21 g de 9-(3,4-dichlorophényl)-4,9-éthano-2,3,3a,4,9,9a-bexahydro-1H-benzo[f]isoindole-3a-carboxylate de méthyle-(3aRS,

4SR,9SR,9aRS) sous forme d'un solide blanc dont la caractéristique est la suivante :

- point de fusion = 185°C.

Etape D

En opérant comme à l'étape G de l'exemple 1, mais a partir de 0,2 g de 9-(3,4-dichlorophényl)-4,9-éthano-2,3,3a,4,9,9a-hexahydro-1H-benzo[f]isoindole-3a-carboxylate de méthyle-(3aRS,4SR,9SR,9aRS), de 0,1 g d'acide 2-(2-méthoxyphényl)propènoïque, de 0,04 $cm^3$ de chlorure d'oxalyle et de 0,14 $cm^3$ de triéthylamine, on obtient, après cristallisation dans l'oxyde de diisopropyle, 0,19 g de 9-(3,4-dichlorophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS) sous forme d'un solide blanc dont la caractéristique est la suivante :

- point de fusion = 150°C.

Etape E

En opérant comme à l'exemple 2, mais à partir de 0,16 g de 9-(3,4-dichlorophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR, 9aRS), de 1,4 $cm^3$ d'une solution aqueuse normale d'hydroxyde de sodium et 3 $cm^3$ de méthanol, on obtient, après recristallisation dans 2,5 $cm^3$ d'acétate d'isopropyle, 0,1 g d'acide 9-(3,4-dichlorophényl)-4,9-éthano-2-[2-(2-méthoxyphényl)propènoyl]-2,3,3a,4,9,9a-hexahydro-1H-benzo[f]isoindole-3a-carboxylique-(3aRS,4SR,9SR, 9aRS) dont la caractéristique est la suivante :

- point de fusion = 168°C.

**EXEMPLE 52**

Préparation du 4,9-éthano-2-[2-(2-méthoxyphényl)propénoyl]-9-(4-chlorophényl)-2,3,3a,4,9,9a-hexahydro-1H-benzo[f]isoindole-3a-carboxylate de méthyle-(3aRS,4SR,9SR,9aRS)

Etape A

En opérant comme à l'étape C de l'exemple 1, mais à partir de 9,58 g de 4-chloro-bromobenzène, de 1,2 g de magnésium en tournures et de 9,1 g de 2-benzyl-7-oxo-4-phényloctahydroisoindole-3a-carboxylate de méthyle-(3aRS,4SR,7aRS), on obtient, après recristallisation dans 25 $cm^3$ d'isopropanol, 7,9 g de 2-benzyl-7-(4-chlorophényl)-7-hydroxy-4-phényloctahydroisoindole-3a-carboxylate de méthyle-(3aRS,4SR,7RS,7aRS) sous forme d'un solide blanc dont la caractéristique est la suivante :

- point de fusion = 189°C.

Etape B

En opérant comme à l'étape D de l'exemple 1, mais à partir de 7,5 g de 2-benzyl-7-(4-chlorophényl)-7-hydroxy-4-phényl-2,3,3a,4,5,6-hexahydroisoindole-3a-carboxylate de méthyle-(3aRS,4SR,7RS,7aSR), de 25 $cm^3$ d'acide trifluorométhanesulfonique et de 80 $cm^3$ de dichlorométhane, on obtient, après cristallisation dans 100 $cm^3$ d'isopropanol, 6,35 g de 2-benzyl-9-(4-chlorophényl)-4,9-éthano-2,3,3a,4,9,9a-hexahydro-1H-benzo[f]isoindole-3a-carboxylate de méthyle-(3aRS,4SR,9SR,9aRS) sous forme d'un solide blanc dont la caractéristique est la suivante :

- point de fusion = 142°C.

Etape C

En opérant comme à l'étape C de l'exemple 51, mais à partir de 2,29 g de 2-benzyl-9-(4-chlorophényl)-4,9-éthano-2,3,3a,4,9,9a-hexahydro-1H-benzo[f]isoindole-3a-carboxylate de méthyle-(3aRS,4SR,9SR,9aRS) et de 0,98 $cm^3$ de chloroformiate de vinyle dans 7,5 $cm^3$ de dichlorométhane puis en traitant l'intermédiaire dilué dans 25 $cm^3$ de méthanol par 8,5 $cm^3$ d'une solution hexanormale de gaz chlorhydrique dans le dioxane on obtient, après flash-chromatographie sur gel de silice (230-400 mesh), en éluant avec un mélange dichlorométhane-méthanol (98-2 en

volumes), 0,8 g de 9-(4-chlorophényl)-4,9-éthano-2,3,3a,4,9,9a-hexahydro-1H-benzo[f]isoindole-3a-carboxylate de méthyle-(3aRS,4SR,9SR,9aRS) sous forme d'une meringue claire utilisée telle quelle dans les synthèses ultérieures.

Etape D

En opérant comme à l'étape G de l'exemple 1, mais à partir de 0,75 g de 9-(4-chlorophényl)-4,9-éthano-2,3,3a,4,9,9a-hexahydro-1H-benzo[f]isoindole-3a-carboxylate de méthyle-(3aRS,4SR,9SR,9aRS), de 0,36 g d'acide 2-(2-méthoxyphényl)propènoique, de 0,17 $cm^3$ de chlorure d'oxalyle et de 0,57 $cm^3$ de triéthylamine, on obtient 0,8 g de 9-(4-chlorophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS) dont la caractéristique est la suivante :

- point de fusion = 90°C.

**EXEMPLE 53**

Préparation de l'acide 9-(4-chlorophényl)-4,9-éthano-2-[2-(2-méthoxyphényl) propènoyl]-2,3,3a,4,9,9a-hexahydro-1H-benzo[f]isoindole-3a-carboxylique-(3aRS,4SR,9SR,9aRS)

En opérant comme à l'exemple 2, mais à partir de 0,7 g de 9-(4-chlorophényl)-4,9-éthano-2-[2-(2-méthoxyphenyl)propènoyl]-2,3,3a,4,9,9a-hexahydro-1H-benzo[f]isoindole-3a-carboxylate de méthyle-(3aRS,4SR,9SR,9aRS), de 2 $cm^3$ d'une solution aqueuse normale d'hydroxyde de sodium et 30 $cm^3$ d'éthanol, on obtient, après flash-chromatographie sur gel de silice (230-400 mesh), en éluant avec un mélange dichlorométhane-méthanol (98-2 en volumes), 0,45 g d'acide 9-(4-chlorophényl)-4,9-éthano-2-[2-(2-méthoxyphényl)propènoyl]-2,3,3a,4,9,9a-hexahydro-1H-benzo[f]isoindole-3a-carboxylique-(3aRS,4SR,9SR,9aRS) dont la caractéristique est la suivante :

- point de fusion : vers 160°C.

**EXEMPLE 54**

Préparation de l'acide 4,9-éthano-2-[2-(2-méthoxyphényl)propènoyl]-9-(4-méthoxy-3-méthylphényl)-2,3,3a,4,9,9a-hexahydro-1H-benzo[f]isoindole-3a-carboxylique-(3aRS, 4SR,9SR,9aRS)

Etape A

En opérant comme à l'étape A de l'exemple 31, mais à partir de 2 g de 2-benzyl-7-oxo-4-phényloctahydroisoindole-3a-carboxylate de méthyle-(3aRS,4SR,7aRS), de 2 g de 2-méthylanisole et de 2,9 $cm^3$ d'acide trifluorométhanesulfonique dans 30 $cm^3$ de dichlorométhane, on obtient, après purification par flash-chromatographie sur gel de silice (230-400 mesh), en éluant avec un mélange cyclohexane-acétate d'éthyle (90-10 en volumes), 2,3 g de 2-benzyl-4,9-éthano-9-(4-méthoxy-3-méthylphényl)-2,3,3a,4,9,9a-hexahydro-1H-benzo[f]isoindole-3a-carboxylate de méthyle-(3aRS,4SR,9SR,9aRS) sous forme d'une meringue blanchâtre utilisée telle quelle dans les synthèses ultérieures.

Etape B

En opérant comme à l'étape E de l'exemple 1, mais à partir de 2,3 g de 2-benzyl-4,9-éthano-9-(4-méthoxy-3-méthylphényl)-2,3,3a,4,9,9a-hexahydro-1H-benzo[f]isoindole-3a-carboxylate de méthyle-(3aRS,4SR,9SR,9aRS), de 0,93 g de formiate d'ammonium et de 0,5 g de palladium sur charbon à 10 % (p/p), on obtient, après par flash-chromatographie sur gel de silice (230-400 mesh), en éluant avec un mélange dichlorométhane-méthanol (99-1 en volumes) et recristallisation dans 20 $cm^3$ d'isopropanol, 0,5 g de 4,9-éthano-9-(4-méthoxy-3-méthylphényl)-2,3,3a,4,9,9a-hexahydro-1H-benzo[f]isoindole-3a-carboxylate de méthyle-(3aRS,4SR,9SR,9aRS) sous forme de solide blanc dont la caractéristique est la suivante :

- point de fusion = 123°C.

Etape C

En opérant comme à l'étape G de l'exemple 1, mais à partir de 1,1 g de 4,9-éthano-9-(4-méthoxy-3-méthylphényl)-2,3,3a,4,9,9a-hexahydro-1H-benzo[f]isoindole-3a-carboxylate de méthyle-(3aRS,4SR,9SR,9aRS), de 0,52 g d'acide 2-(2-méthoxyphényl)propènoïque, de 0,25 $cm^3$ de chlorure d'oxalyle et de 0,82 $cm^3$ de triéthylamine, on obtient,

après purification par flash-chromatographie sur gel de silice (230-400 mesh), en éluant avec un mélange dichlorométhane-méthanol (99-1 en volumes) et recristallsation dans 5 $cm^3$ d'isopropanol, 0,85 g de 4,9-éthano-2-[2-(2-méthoxyphényl)propenoyl]-9-(4-méthoxy-3-méthylphényl)-2,3,3a,4,9,9a-hexahydro-1H-benzo[f]isoindole-3a-carboxylate de méthyle-(3aRS,4SR,9SR,9aRS) dont la caractéristique est la suivante :

- point de fusion = 125°C.

Etape D

En opérant comme à l'exemple 2, mais à partir de 0,8 g de 4,9-éthano-2-[2-(2-méthoxyphényl)propènoyl]-9-(4-méthoxy-3-méthylphényl)-2,3,3a,4,9,9a-hexahydro-1H-benzo[f]isoindole-3a-carboxylate de méthyle-(3aRS, 4SR,9SR,9aRS), de 2,25 $cm^3$ d'une solution aqueuse normale d'hydroxyde de sodium et 40 $cm^3$ d'éthanol, on obtient, après flash-chromatographie sur gel de silice (230-400 mesh), en éluant avec un mélange dichlorométhane-méthanol (97,5-2,5 en volumes), 0,45 g d'acide 4,9-éthano-2-[2-(2-méthoxyphényl)propènoyl]-9-(4-méthoxy-3-méthylphényl)-2,3,3a,4,9,9a-hexahydro-1H-benzo[f]isoindole-3a-carboxylique-(3aRS,4SR,9SR,9aRS) sous forme de solide blanc amorphe.

- spectre de masse (IE) : M/Z = 523 ($M^+$)

**EXEMPLE 55**

<u>Préparation du 4,9-éthano-2-[2-(2-méthoxyphényl)propènoyl]-9-(3-indolyl)-2,3,3a,4,9,9a-hexahydro-1H-benzo[f] isoindote-3a-carboxylate de méthyle-(3aRS, 4SR,9SR,9aRS)</u>

Etape A

En opérant comme à l'étape A de l'exemple 31, mais à partir de 3,63 g de 2-benzyl-7-oxo-4-phényloctahydroisoindole-3a-carboxylate de méthyle-(3aRS,4SR,7aRS), de 3,85 g de 1-phénylsulfonylindole et de 5,3 $cm^3$ d'acide trifluorométhanesulfonique dans 30 $cm^3$ de dichlorométhane, on obtient, après purification par flash-chromatographie sur gel de silice (230-400 mesh), en éluant avec un mélange cyclohexane-acétate d'éthyle (80-20 en volumes), 2,1 g d'un produit brut qui est joint à un deuxième lot provenant d'une expérience réalisée sur les mêmes quantités. La purification de ce mélange par flash-chromatographie sur gel de silice (230-400 mesh), en éluant avec un mélange cyclohexane-acétate d'éthyle (95-5 en volumes) fournit 1 g de 2-benzyl-4,9-éthano-9-(3-indolyl)-2,3,3a,4,9,9a-hexahydro-1H-benzo[f]isoindole-3a-carboxylate de méthyle-(3aRS,4SR,9SR,9aRS) sous forme d'une meringue blanchâtre utilisée telle quelle dans les synthèses ultérieures, dont les caractéristiques sont les suivantes :

- spectre de R.M.N. $^1H$ (300 MHz, $CDCl_3$, d en ppm) : de 1,30 à 1,60 et 2,55 (2 mts, 2H chacun : $CH_2CH_2$) ; 2,11 et 2,65 (respectivement dd et d, J = 10 et 9 Hz et J = 10 Hz, 1H chacun : $CH_2$ en 1) ; 2,31 et 3,21 (2d, J = 10 Hz, 1H chacun : $CH_2$ en 3) ; 3,37 et 3,51 (2 d, J = 12,5 Hz, 1H chacun : $NCH_2Ar$)) ; 3,42 (s large, 1H : CH en 4) ; de 3,50 à 3,70 (mt, 1H : CH en 9a) ; 3,59 (s, 3H : $COOCH_3$) ; 6,81 (d large, J = 7,5 Hz, 1H : H en 8) ; de 6,90 à 7,60 (mt, 13H : H en 5 - H en 6 - H en 7 - H aromatiques de l'indole et H aromatiques du benzyle) ; 8,11 (s large, 1H : NH).

Etape B

En opérant comme à l'étape C de l'exemple 51, mais à partir de 1 g de 2-benzyl-4,9-éthano-9-(3-indolyl)-2,3,3a,4,9,9a-hexahydro-1H-benzo[f]isoindole-3a-carboxylate de méthyle-(3aRS,4SR,9SR,9aRS) et de 0,285 $cm^3$ de chloroformiate de vinyle dans 20 $cm^3$ de dichlorométhane puis en traitant l'intermédiaire dissous dans 20 $cm^3$ de méthanol par 21,4 $cm^3$ d'une solution normale de gaz chlorhydrique dans l'oxyde de diéthyle, on obtient, après flash-chromatographie sur gel de silice (230-400 mesh), en éluant avec un mélange dichlorométhane-méthanol (97,5-2,5 en volumes), 0,3 g de 4,9-éthano-9-(3-indolyl)-2,3,3a,4,9,9a-hexahydro-1H-benzo[f]isoindole-3a-carboxylate de méthyle-(3aRS,4SR,9SR,9aRS) sous forme d'une meringue orangée utilisée telle quelle dans les synthèses ultérieures.

Etape C

En opérant comme à l'étape G de l'exemple 1 mais a partir de 0,3 g de 4,9-éthano-9-(3-indolyl)-2,3,3a,4,9,9a-hexahydro-1H-benzo[f]isoindole-3a-carboxylate de méthyle-(3aRS,4SR,9SR,9aRS), de 0,145 g d'acide 2-(2-méthoxyphényl)propenoïque et de 0,07 $cm^3$ de chlorure d'oxalyle, on obtient, après purification par flash-chromatographie sur gel de silice (230-400 mesh), en éluant avec un mélange cyclohexane-acétate d'éthyle (50-50 en volumes) et cristallisation dans l'oxyde de diisopropyle, 0,16 g de 4,9-éthano-2-[2-(2-méthoxyphényl)propènoyl]-9-(3-indolyl)-2,3,3a,4,9,9a-hexahydro-1H-benzo[f]isoindole-3a-carboxylate de méthyle-(3aRS, 4SR,9SR,9aRS) dont les caractéristiques sont les suivantes :

- spectre de masse (IE) : M/Z = 532 ($M^+$)
- spectre de R.M.N. $^1H$ (250 MHz, $(CD_3)_2SO$ d6, à une température de 383 K, d en ppm) : 1,46 et 2,16 (2 mts, 2H chacun : $CH_2CH_2$) ; de 3,30 à 3,80 (mt, 4H : $CH_2$ en 1 - CH en 9a et 1H du $CH_2$ en 3) ; 3,47 (mt, 1H : CH en 4) ; 3,57 (s, 3H : $COOCH_3$) ; 3,71 (s, 3H : $ArOCH_3$) ; 4,10 (d large, J = 12,5 Hz, 1H : l'autre H du $CH_2$ en 3) ; 5,52 et 5,67 (2 s, 1H chacun : $=CH_2$) ; 6,65 (d large, J = 7,5 Hz, 1H : H en 8) ; de 6,90 à 7,88 (mt, 12H : H en 5 - H en 6 - H en 7 - H aromatiques de l'indole et H aromatiques du 2-méthoxyphényl).

**EXEMPLE 56**

Préparation de l'acide 4,9-éthano-9-(4-isopropylphényl)-2-[(2-méthoxyphènyl)acétyl]-2,3,3a,4,9,9a-hexahydro-1H-benzo[f]isoindole-3a-carboxylique-(3aRS,4SR,9SR,9aRS)

Etape A

En opérant comme à l'étape A de l'exemple 1, mais à partir de 6 g de 2-benzyl-7-oxo-4-phényl-octahydroisoindole-3a-carboxylate de méthyle-(3aRS,4SR,7aRS) dans 60 $cm^3$ d' oxyde de diéthyle sec et d'une solution de bromure de 4-isopropylphénylmagnésium, préparée extemporanément à partir de 4 g de 4-bromo-isopropylbenzène et 0,52 g de magnésium en tournures dans 7 $cm^3$ d'oxyde de diéthyle sec, pendant dix huit heures à température ambiante, on obtient, après purification par flash-chromatogragraphie sur gel de silice (230-400 Mesh) en éluant par un gradient de mélanges de cyclohexane et d'acétate d'éthyle (de 98-2 à 80-20 en volumes), 3,3 g (41%) de 2-benzyl-7-(4-isopropylphényl)-7-hydroxy-4-phényl-octahydroisoindole-3a-carboxylate de méthyle-(3aRS,4SR,7RS,7aRS), sous forme d'une huile jaune, dont la caractéristique est la suivante :

- spectre de masse (IE) : M/Z = 483 ($M^+$).

Etape B

En opérant comme à l'étape D de l'exemple 1, mais à partir de 1,7 g de 2-benzyl-7-(4-isopropylphényl)-7-hydroxy-4-phényl-octahydro-isoindole-3a-carboxylate de méthyle-(3aRS,4SR,7RS,7aRS) et de 2,3 $cm^3$ d'acide trifluormèthanesulfonique dans 22 $cm^3$ de dichlorométhane pendant trois heures à température ambiante, on obtient 1,44 g (90%) de 2-benzyl-4,9-éthano-9-(4-isopropylphényl)-2,3,3a,4,9,9a-hexahydro-1H-benzo[f]isoindole-3a-carboxylate de méthyle-(3aRS,4SR,9SR,9aRS), sous forme d'une huile visqueuse brune, utilisée telle quelle dans la suite de la synthèse, dont la caractéristique est la suivante :

- spectre de masse (IE) : M/Z = 465 ($M^+$).

Etape C

En opérant comme à l'étape C de l'exemple 45, mais à partir de 1,4 g (3 mmol) de 2-benzyl-4,9-éthano-9-(4-isopropylphényl)-2,3,3a,4,9,9a-hexahydro-1H-benzo[f] isoindole-3a-carboxylate de méthyle-(3aRS,4SR,9SR,9aRS) et de 140 mg de palladium sur charbon à 10% (p/p) dans 125 $cm^3$ de méthanol et 3,3 $cm^3$ d'une solution aqueuse normale d'acide chlorhydrique pendant quatre heures à température ambiante sous hydrogène à pression atmosphérique, on obtient, après cristallisation dans l'oxyde de diisopropyle, 1,03 g (86%) de chlorhydrate de 4,9-éthano-9-(4-isopropylphényl)-2,3,3a,4,9,9a-hexahydro-1H-benzo[f]isoindole-3a-carboxylate de méthyle-(3aRS,4SR,9SR,9aRS), sous forme d'une poudre beige dont la caractéristique est la suivante :

- spectre de masse (IE) : M/Z = 375 ($M^+$).

Etape D

En opérant comme à l'étape A de l'exemple 5, mais à partir de 500 mg de chlorhydrate de 4,9-éthano-9-(4-isopropylphényl)-2,3,3a,4,9,9a-hexahydro-1H-benzo[f]isoindole-3a-carboxylate de méthyle-(3aRS, 4SR,9SR,9aRS), de 240 mg d'acide 2-méthoxyphénylacétique, de 280 mg de chlorhydrate de 1-éthyl-3-[(3-diméthylamino)propyl]carbodiimide, de 16 mg d'hydrate de N-1-hydroxybenzotriazole et de 0,2 $cm^3$ de triéthylamine dans 55 $cm^3$ de dichlorométhane,

on obtient, après purification par flash-chromatographie sur gel de silice (240-400 Mesh) en éluant par un mélange cyclohexane-acétate d'éthyle (70-30 en volumes), 460 mg (76%) de 4,9-éthano-9-(4-isopropylphényl)-2-[(2-méthoxyphényl)acétyl]-2,3,3a,4,9,9a-hexahydro-1H-benzo[f]isoindole-3a-carboxylate de méthyle-(3aRS,4SR,9SR,9aRS), sous forme d'une poudre blanche dont les caractéristiques sont les suivantes :

- point de fusion = 85-7°
- spectre de masse (IE) : M/Z= 523 ($M^+$)

Etape E

En opérant comme à l'exemple 2, mais à partir de 0,379 g de 4,9-éthano-9-(4-isopropylphényl)-2-[(2-méthoxyphényl)acétyl]-2,3,3a,4,9,9a-hexahydro-1H-benzo[f]isoindole-3a-carboxylate de methyle-(3aRS,4SR, 9SR,9aRS) au reflux pendant trois heures dans 0,85 $cm^3$ d'une solution aqueuse normale d'hydroxyde de sodium et 15 $cm^3$ d'éthanol, on obtient, après purification par agitation dans l'acétate d'éthyle en présence de gel de silice (230-400 Mesh) puis recristallisation dans le pentane, 170 mg (47%) d'acide 4,9-éthano-9-(4-isopropylphényl)-2-[(2-méthoxyphényl)acétyl]-2,3,3a,4,9,9,9a-hexahydro-1H-benzo[f]isoindole-3a-carboxylique-(3aRS,4SR,9SR,9aRS), sous forme d'un solide blanc dont les caractéristiques sont les suivantes :

- point de fusion = 135-6°C
- spectre de masse (IE) : M/Z = 509 ($M^+$).

**EXEMPLE 57**

<u>Préparation de l'acide 4,9-éthano-9-(4-isopropylphényl)-2-[2-(2-méthoxyphényl) propènoyl]-2,3,3a,4,9,9a-hexahydro-1H-benzo[f]isoindole-3a-carboxylique-(3aRS,4SR,9SR,9aRS)</u>

En opérant comme à l'étape G de l'exemple 1, mais à partir de 0,53 g chlorhydrate de 4,9-éthano-9-(4-isopropylphényl)-2,3,3a,4,9,9a-hexahydro-1H-benzo[f]isoindole-3a-carboxylate de méthyle-(3aRS,4SR,9SR,9aRS), obtenu à l'étape C de l'exemple 56, de 0,25 g d'acide 2-(2-méthoxyphényl)propènoique, de 0,12 $cm^3$ de chlorure d'oxalyle et de 0,4 $cm^3$ de triéthylamine en solution dans 30 $cm^3$ de dichlorométhane pendant vingt heures à température ambiante, on obtient, après purification sur gel de silice (230-400 Mesh) en éluant par un gradient de mélanges de cyclohexane et d'acétate d'éthyle ( de 98-2 à 70-30 en volumes), 0,375 g (54%) de 4,9-éthano-9-(4-isopropylphényl)-2-[2-(2-méthoxyphényl)propènoyl]-2,3,3a,4,9,9a-hexahydro-1H-benzo[f]isoindole-3a-carboxylate de méthyle-(3aRS, 4SR,9SR,9aRS), sous forme d'une poudre blanche, dont les caractéristiques sont les suivantes :

- point de fusion = 98-100°C
- spectre de masse (IE): M/Z = 535 ($M^+$).

En opérant comme à l'exemple 2, mais à partir de 0,32 g de 4,9-éthano-9-(4-isopropylphényl)-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS) au reflux pendant deux heures dans 0,71 $cm^3$ d'une solution aqueuse normale d'hydroxyde de sodium et 15 cm3 d'éthanol, on obtient, après purification par agitation dans l'acétate d'éthyle en présence de gel de silice (230-400 Mesh) puis recristallisation dans le pentane, 190 mg (59%) d'acide 4.9-éthano-9-(4-isopropylphényl)-2-[2-(2-méthoxyphényl)propènoyl]-2,3,3a,4,9,9a-hexahydro-1H-benzo[f]isoindole-3a-carboxylique-(3aRS,4SR,9SR,9aRS), sous forme d'un solide blanc dont les caractéristiques sont les suivantes :

- point de fusion = 168-70°C
- spectre de masse (IE) : M/Z = 521 ($M^+$).

**EXEMPLE 58**

Préparation du 4,9-éthano-2-[2-(2-méthoxyphényl)propènoyl]-9-(3-thiényl)-2,3,3a,4,9,9a-hexahydro-1H-benzo[f]isoindole-3a-carboxylate de méthyle-(3aRS,4SR,9SR,9aRS)

Etape A

En opérant comme à l'étape C de l'exemple 45, mais à partir de 1,2 g de 2-benzyl-7-iodo-4-phényl-2,3,a,4,5,7a-hexahydroisoindole-3a-carboxylate de méthyle-(3aRS,4SR,7aRS) dans 30 $cm^3$ de toluène, de 150 mg de tétrakis(triphénylphosphine)palladium, de 0,35 g d'acide 3-thiénylboronique dans 15 $cm^3$ de méthanol et de 28 $cm^3$ d'une solution aqueuse 2N de carbonate de sodium au reflux pendant trois heures, on obtient, après purification par flash-chromatographie sur gel de silice (230-400 Mesh) en éluant par un mélange de cyclohexane et d'acétate d'éthyle (85-15 en volumes), 0,99 g (93%) de 2-benzyl-4-phényl-7-(3-thiényl)-2,3,3a,4,5,7a-hexahydro-1H-isoindole-3a-carboxylate de méthyle-(3aRS,4SR,7aRS), sous forme d'une huile jaune, dont la caractéristique est la suivante :

- spectre de masse (IE) : M/Z = 429 ($M^+$).

Etape B

En opérant comme à l'étape C de l'exemple 38, mais à partir de 1,1 g de 2-benzyl-4-phényl-7-(3-thiényl)-2,3,3a,4,5,7a-hexahydro-1H-isoindole-3a-carboxylate de méthyle-(3aRS,4SR,7aRS) et de 1,4 $cm^3$ d'acide trifluorométhanesulfonique dans 15 $cm^3$ de dichlorométhane pendant dix huit heures à température ambiante, on obtient 0,62 g (56%) de 2-benzyl-4,9-éthano-9-(3-thienyl)-2,3,3a,4,9,9a-hexahydro-1H-benzo[f]isoindole-3a-carboxylate de méthyle-(3aRS,4SR,9SR,9aRS), sous forme d'une poudre beige, utilisée telle quelle dans la suite de la synthèse, dont la caractéristique est la suivante :

- spectre de masse (IE) : M/Z = 429 ($M^+$).

Etape C

En opérant comme à l'étape B de l'exemple 47, mais à partir de 0,62 g de 2-benzyl-4,9-éthano-9-(3-thiényl)-2,3,3a,4,9,9a-hexahydro-1H-benzo[f]isoindole-3a-carboxylate de méthyle-(3aRS,4SR,9SR,9aRS) et de 0,19 $cm^3$ de chloroformiate de vinyle pendant soixante douze heures à température ambiante dans 10 $cm^3$ de dichlorométhane, puis en reprenant le concentrat dans 20 $cm^3$ de méthanol et 2,7 $cm^3$ d'une solution 5M d'acide chlorhydrique dans l'isopropanol au reflux pendant deux heures, on obtient, après cristallisation dans l'oxyde de diisopropyle, 0,48 g (89%) de chlorhydrate de 4,9-éthano-9-(3-thiényl)-2,3,3a,4,9,9a-hexahydro-1H-benzo[f]isoindole-3a-carboxylate de méthyle-(3aRS,4SR,9SR,9aRS), sous forme d'un solide blanc, dont la caractéristique est la suivante :

- spectre de masse (IE) : M/Z = 339 (($M^+$).

Etape D

En opérant comme à l'étape G de l'exemple 1, mais à partir de 0,48 g de chlorhydrate de 4,9-éthano-9-(3-thiényl)-2,3,3a,4,9,9a-hexahydro-1H-benzo[f] isoindole-3a-carboxylate de méthyle-(3aRS,4SR,9SR,9aRS), de 0,27 g d'acide 2-(2-méthoxyphényl)propènoique, de 0,12 $cm^3$ de chlorure d'oxalyle et de 0,36 $cm^3$ de triéthylamine en solution dans 35 $cm^3$ de dichlorométhane pendant vingt heures à température ambiante, on obtient, après purification par flash-chromatographie sur gel de silice (230-400 Mesh) en éluant par un mélange de cyclohexane et d'acétate d'éthyle (60-40 en volumes) suivie d'une recristallisation dans l'oxyde de diisopropyle, 0.40 g (63%) de 4,9-éthano-2-[2-(2-méthoxyphényl)propenoyl]-9-(3-thiényl)-2,3,3a,4,9,9a-hexahydro-1H-benzo[f]isoindole-3a-carboxylate de méthyle-(3aRS,4SR,9SR,9aRS), sous forme d'un solide blanc, dont les caractéristiques sont les suivantes :

- point de fusion = 174°C
- spectre de masse (IE) : M/Z = 499 ($M^+$).

Etape E

En opérant comme à l'exemple 2, mais à partir de 0,35 g de 4,9-éthano-2-[2-(2-méthoxyphényl)propènoyl]-9-(3-thiényl)-2,3,3a,4,9,9a-hexahydro-1H-benzo[f]isoindole-3a-carboxylate de méthyle-(3aRS,4SR,9SR,9aRS) au reflux pendant trois heures dans 2,1 $cm^3$ d'une solution aqueuse normale d'hydroxyde de sodium et 12 $cm^3$ d'éthanol, on obtient, après purification par recristallisation dans l'éther de pétrole (40-65°C), 340 mg (88%) d'acide 4,9-éthano-2-[2-(2-méthoxyphényl)propènoyl]-9-(3-thiényl)-2,3,3a,4,9,9a-hexahydro-1H-benzo[f]isoindole-3a-carboxylique-(3aRS,4SR,9SR, 9aRS), sous forme d'un solide blanc dont les caractéristiques sont les suivantes :

- point de fusion = 170°C
- spectre de masse (IE) : M/Z = 485 ($M^+$).

**EXEMPLE 59**

Préparation du 4,9-éthano-9-(4-éthylphényl)-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS)

Etape A

En opérant comme à l'étape C de l'exemple 45, mais à partir de 1,35 g de 2-benzyl-7-iodo-4-phényl-2,3,a,4,5,7a-hexahydroisoindole-3a-carboxylate de méthyle-(3aRS,4SR,7aRS) dans 20 $cm^3$ de toluène, de 340 mg de tétrakis(triphénylphosphine)palladium, de 0,38 g d'anhydride trimère de l'acide 4-éthylphénylboronique dans 10 $cm^3$ de méthanol et de 15 $cm^3$ d'une solution aqueuse 2N de carbonate de sodium au reflux pendant dix huit heures, on obtient, après purification par flash-chromatographie sur gel de silice (230-400 Mesh) en éluant par un mélange de cyclohexane et d'acétate d'éthyle (90-10 en volumes), 0,92 g (72%) de 2-benzyl-7-(4-éthylphényl)-4-phényl-2,3,3a,4,5,7a-hexahydro-1H-isoindole-3a-carboxylate de méthyle-(3aRS,4SR,7aRS), sous forme d'une huile jaune visqueuse, dont la caractéristique est la suivante :

- spectre de masse (IE) : M/Z = 451 ($M^+$).

Etape B

En opérant comme à l'étape C de l'exemple 38, mais à partir de 0,92 g de 2-benzyl-7-(4-éthylphényl)-4-phényl-2,3,3a,4,5,7a-hexahydro-1H-isoindole-3a-carboxylate de méthyle-(3aRS,4SR,7aRS) et de 1,18 $cm^3$ d'acide trifluorométhanesulfonique dans 18 $cm^3$ de dichlorométhane pendant six heures à température ambiante, on obtient, après purification par flash-chromatographie sur gel de silice (230-400 Mesh) en éluant avec un mélange de cyclohexane et d'acétate d'éthyle (95-5 en volumes), 0.68 g (74%) de 2-benzyl-4,9-éthano-9-(4-éthylphényl)-2,3,3a.4,9,9a-hexahydro-1H-benzo[f]isoindole-3a-carboxylate de méthyle-(3aRS,4SR,9SR,9aRS), sous forme d'une huile visqueuse jaune, dont la caractéristique est la suivante :

- spectre de masse (IE ) : M/Z = 451 ($M^+$).

Etape C

En opérant comme à l'étape E de l'exemple 1, mais à partir de 0,68 g de 2-benzyl-4,9-éthano-9-(4-éthylphényl)-2,3,3a,4,9,9a-hexahydro-1H-benzo[f]isoindole-3a-carboxylate de méthyle-(3aRS,4SR,9SR,9aRS) et de 0,27 g de formiate d'ammonium dans 40 $cm^3$ de méthanol en présence de 200 mg de palladium sur charbon à 3% (p/p) au reflux pendant quatre heures, on obtient 0,47 g (87%) de 4,9-éthano-9-(4-éthylphényl)-2,3,3a,4,9,9a-hexahydro-1H-benzo[f]isoindole-3a-carboxylate de méthyle-(3aRS,4SR,9SR,9aRS), sous forme d'un solide pâteux jaune, dont la caractéristique est la suivante :

- spectre de masse (IE) : M/Z = 461 ($M^+$).

Etape D

En opérant comme à l'étape A de l'exemple 5, mais à partir de 0,47 g de 4,9-èthano-9-(4-éthylphényl)-2,3,3a,4,9,9a-hexahydro-1H-benzo[f]isoindole-3a-carboxylate de méthyle-(3aRS,4SR,9SR,9aRS), de 0,23 g d'acide 2-(2-méthoxyphényl)propènoique, de 0,3 g de chlorhydrate de 1-éthyl-3-[(3-diméthylamino)propyl]carbodiimide, de 20 mg d'hydrate de N-1-hydroxybenzotriazole dans 30 $cm^3$ de dichlorométhane pendant six heures à température ambiante, on obtient, après purification par flash-chromatographie sur gel de silice (230-400 Mesh) en éluant par un mélange de

cyclohexane et d'acétate d'éthyle (70-30 en volumes), 0,26 g (38%) de 4,9-éthano-9-(4-éthylphényl)-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS), sous forme d'une poudre blanche, dont les caractéristiques sont les suivantes :

- point de fusion = 90-2°C
- spectre de masse (IE) : M/Z = 521 ($M^+$).

**EXEMPLE 60**

Préparation de l'acide 4,9-éthano-9-(4-éthylphényl)-2-[2-(2-méthoxyphényl)propènoyl]-2,3,3a,4,9,9a-hexahydro-1H-benzo[f]isoindole-3a-carboxylique-(3aRS,4SR,9SR,9aRS)

En opérant comme à l'exemple 2, mais en partant de 0,18 g de 4,9-éthano-9-(4-éthylphényl)-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS) au reflux pendant trois heures dans 0,43 $cm^3$ d'une solution aqueuse normale d'hydroxyde de sodium et de 10 $cm^3$ de méthanol, on obtient, après purification par recristallisation dans l'éther de pétrole, 59 mg (34%) d'acide 4,9-éthano-9-(4-éthylphényl)-2-[2-(2-méthoxyphényl)propènoyl]-2,3,3a,4,9,9a-hexahydro-1H-benzo[f]isoindole-3a-carboxylique-(3aRS,4SR,9SR, 9aRS), sous forme d'un solide blanc dont les caractéristiques sont les suivantes :

- point de fusion = 141°C
- spectre de masse (IE) : M/Z = 507 ($M^+$).

**EXEMPLE 61**

Préparation du 9-(2,3-dihydrobenzofuran-5-yl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS)

Etape A

En opérant comme à l'exemple 31, étape A, mais en partant de 2,5 g de 2-benzyl-7-oxo-4-phényloctahydroisoindole-3a-carboxylate de méthyle-(3aRS,4SR,7aRS) dans 250 $cm^3$ de dichlorométhane, de 2,4 $cm^3$ de -2,3-dihydrobenzofurane, et de 4,25 $cm^3$ d'acide trifluorométhanesulfonique; apres dix huit heures d'agitation à une température voisine de 20°C, on obtient 1,56 g de 2-benzyl-9-(2,3-dihydrobenzofuran-5-yl)-4,9-éthano-2,3,3a,4,9,9a-hexahydro-1H-benzo[f]isoindole-3a-carboxylate de méthyle-(3aRS,4SR,9SR,9aRS), sous forme d'une huile jaune-orangée, utilisée telle quelle pour l'étape suivante, dont la caractéristique est la suivante :

- spectre de masse (IE) : M/Z = 465 ($M^+$)

Etape B

En opérant comme à l'étape E de l'exemple 1, mais à partir de 1,56 g de 2-benzyl-9-(2,3-dihydrobenzofuran-5-yl)-4,9-éthano-2,3,3a,4,9,9a-hexahydro-1H-benzo[f]isoindole-3a-carboxylate de méthyle-(3aRS,4SR,9SR,9aRS), de 0,63 g de formiate d'ammonium et de 0,2 g de palladium sur charbon à 10 % (p/p), dans 30 $cm^3$ de méthanol, au reflux pendant cinq heures, on obtient 0,995 g de 9-(2,3-dihydrobenzofuran-5-yl)-4,9-éthano-2,3,3a,4,9,9a-hexahydro-1H-benzo[f]isoindole-3a-carboxylate de méthyle-(3aRS,4SR, 9SR,9aRS), impur, sous forme d'une laque orangée, utilisable telle qu'elle pour l'étape suivante, dont la caractéristique est la suivante :

- spectre de masse (DCI) : M/Z = 376 ($M+H^+$)

Etape C

En opérant comme à l'étape G de l'exemple 1, mais à partir d'une solution de 0,45 g d'acide 2-(2-méthoxyphényl)propènoïque dans 20 $cm^3$ de dichlorométhane contenant 4 gouttes de N,N-diméthylformamide, de 0,54 $cm^3$ de chlorure d'oxalyle, après une nuit à une température voisine de 20°C, en évaporant à sec et en reprenant dans 20 $cm^3$ de dichlorométhane; puis en versant cette solution sur la solution de 0,99 g de 9-(2,3-dihydrobenzofuran-5-yl)-4,9-éthano-2,3,3a,4,9,9a-hexahydro-1H-benzo[f]isoindole-3a-carboxylate de méthyle-(3aRS,4SR,9SR,9aRS), obtenu à l'étape précédente, additionnée de 0,31 $cm^3$ de triéthylamine, dans 20 $cm^3$ de dichlorométhane, on obtient 39 mg de 9-(2,3-dihydrobenzofuran-5-yl)-4,9-éthano-2-[2-(2-méthoxyphényl)propénoyl]-2,3,3a,4,9,9a-hexahydro-1H-benzo[f] isoindoie-3a-carboxylate de méthyle-(3aRS,4SR, 9SR, 9aRS), sous forme d'un solide beige, dont les caractéristiques sont les suivantes :

- point de fusion = 200°C (dec)
- spectre de masse (IE) : M/Z = 535 ($M^+$)

**EXEMPLE 62**

Préparation de l'acide 9-(2,3-dihydrobenzofuran-5-yl)-4,9-éthano-2-[2-(2-méthoxyphényl)propènoyl]-2,3,3a,4,9,9a-hexahydro-1H-benzo[f]isoindole-3a-carboxylique-(3aRS,4SR,9SR,9aRS)

En opérant comme à l'exemple 2, mais à partir de 7,21 g de 9-(2,3-dihydrobenzofuran-5-yl)-4,9-éthano-2-[2-(2-méthoxyphényl)propénoyl]-2,3,3a,4,9,9a-hexahydro-1H-benzo[f]isoindole-3a-carboxylate de méthyle-(3aRS, 4SR,9SR,9aRS), préparés à l'exemple précédent, de 50 $cm^3$ d'une solution aqueuse normale d'hydroxyde de sodium et de 100 $cm^3$ de dioxane, pendant trois heures à reflux, on obtient après cristallisation dans 80 $cm^3$ d'un mélange éthanol-eau (75-25 en volumes), 3,44 g d'acide 9-(2,3-dihydrobenzofuran-5-yl)-4,9-éthano-2-[2-(2-méthoxy phényl) propènoyl]-2,3,3a,4,9,9a-hexahydro- 1H-benzo[f]isoindole-3a-carboxylique-(3aRS,4SR,9SR,9aRS), sous forme d'une poudre cristalline blanche, dont les caractéristiques sont les suivantes :

- point de fusion = 241°C
- spectre de masse (IE) : M/Z = 521 ($M^+$)

**EXEMPLE 63**

Préparation du 9-(2,3-dihydrobenzofuran-5-yl)-4,9-éthano-2-[2-(2-méthoxyphényl)propènoyl)]-2,3,3a,4,9,9a-hexahydro-1H-benzo[f]isoindole-3a-N-(3-pyridylméthyl)carboxamide-(3aRS,4SR,9SR,9aRS)

En opérant comme à l'exemple 42, mais à partir de 522 mg d'acide 9-(2,3-dihydrobenzofuran-5-yl)-4,9-éthano-2-[2-(2-méthoxyphényl)propènoyl]-2,3,3a,4,9,9a-hexahydro-1H-benzo[f]isoindole-3a-carboxylique-(3aRS,4SR, 9SR,9aRS), de 0,122 $cm^3$ de 3-(aminométhyl)pyridine, de 0,23 g de chlorhydrate de 1-ethyl-3-[3-diméthylamino)propyl]-carbodiimide et de 80 mg d'hydrate de N-1-hydroxybenzotriazole dans 15 $cm^3$ de dichlorométhane, on obtient 462 mg de 9-(2,3-dihydrobenzofuran-5-yl)-4,9-éthano-2-[2-(2-méthoxyphényl)propenoyl)]-2,3,3a,4,9,9a-hexahydro-1H-benzo[f]isoindole-3a-N-(3-pyridylméthyl)carboxamide-(3aRS,4SR,9SR,9aRS), sous forme d'une poudre blanche, dont les caractéristiques sont les suivantes :

- point de fusion = 238°C (dec)
- spectre de masse (IE) : M/Z = 611 ($M^+$)
- spectre de R.M.N. $^1H$ (400 MHz, $(CD_3)_2SO$ d6, à une température de 383 K, d en ppm) : 1,40 - 1,60 - 1,99 et 2,14 (4 mts, 1H chacun : $CH_2CH_2$) ; 3,29 et 3,41(respectivement dd et d large, J = 12 et 9 Hz et J = 12 Hz, 1H chacun : $CH_2$ en 1) ; 3,26 (t large, J = 8,5 Hz, 2H : $ArCH_2$ du 2-3-dihydrobenzofurane) ; 3,47 et 4,17 (2 d, J = 12,5 Hz, 1H chacun : $CH_2$ en 3) ; 3.53 (mt, 1H : CH en 9a) ; 3,56 (s large, 1H : CH en 4) : 3,70 (s, 3H : $ArOCH_3$) ; 4.22 (AB, J = 15 et 6 Hz, 2H : $NCH_2Ar$) ; 4,60 (t, J = 8,5 Hz. 2H : $CH_2O$ du 2-3-dihydrobenzofurane) ; 5,52 et 5,68 (2 s, 1H chacun : $=CH_2$) ; 6,48 (mt, 1H : H en 8) ; de 6,75 à 7,50 (mt, 12H : H en 5 - H en 6 - H en 7-H aromatiques du 2-méthoxyphényl - H aromatiques du 2-3-dihydrobenzofurane - H en 4 du pyridyle et H en 3 du pyridyle) ; 7,96 (mt, 1H : CONH) ; 8,38 (s large, 1H : H en 2 du pyridyle) ; 8,44 (d large, J = 5 et 1,5 Hz, 1H : H en 6 du pyridyle).

**EXEMPLE 64**

Préparation du 4,9-éthano-9-(4-fluorophényl)-2-[2-(2-méthoxyphényl)propènoyl)-2,3,3a,4,9,9a-hexahydro-1H-benzo [f]isoindole-3a-carboxylate de méthyle-(3aRS,4SR,9SR,9aRS)

Etape A

En opérant comme à l'étape A de l'exemple 39, mais à partir de 2,8 g de 4-fluoro-bromobenzène, de 0,4 g de magnésium en tournures dans 20 $cm^3$ d'oxyde de diéthyle et de 2,9 g de 2-benzyl-7-oxo-4-phényl-octahydroisoindole-3a-carboxylate de méthyle-(3aRS,4SR,9RS,9aRS) dans 20 $cm^3$ d'oxyde de diéthyle, on obtient, après cristalli-

sation dans 40 $cm^3$ d'un mélange isopropanol-oxyde de diisopropyle (50-50 en volumes), 1,2 g de 2-benzyl-7-(4-fluorophényl)-7-hydroxy-4-phényloctahydroisoindole-3a-carboxylate de méthyle-(3aRS,4SR,7RS,7aRS) sous forme d'un solide blanc dont la caractéristique est la suivante :

- point de fusion = 130°C.

La purification de ce filtrat par flash-chromatographie sur gel de silice (230-400 mesh), en éluant avec un mélange cyclohexane-acétate d'éthyle (80-20 en volumes) permet d'obtenir 1,2 g additionnels.

Etape B

En opérant comme à l'étape C de l'exemple 38, mais à partir de 2,2 g de 2-benzyl-7-(4-fluorophényl)-7-hydroxy-4-phényl-2,3,3a,4,5,6-hexahydroisoindole-3a-carboxylate de méthyle-(3aRS,4SR), de 6,3 $cm^3$ d'acide trifluorométhanesulfonique et de 21 $cm^3$ de dichlorométhane, on obtient, après recristallisation dans 24 $cm^3$ d'oxyde de diisopropyle, 1,0 g de 2-benzyl-4,9-éthano-9-(4-fluorophényl)-2,3,3a,4,9,9a-hexahydro-1H-benzo[f]isoindole-3a-carboxylate de méthyle-(3aRS,4SR,9SR,9aRS) sous forme d'un solide blanc dont la caractéristique est la suivante :

- point de fusion = 135°C.

Par concentration du filtrat et cristallisation dans 25 $cm^3$ d'éther de pétrole, on obtient 0,75 g supplémentaires

Etape C

En opérant comme à l'étape E de l'exemple 1, mais à partir de 1,5 g de 2-benzyl-4,9-éthano-9-(4-fluorophényl)-2,3,3a,4,9,9a-hexahydro-1H-benzo[f]isoindole-3a-carboxylate de méthyle-(3aRS,4SR,9SR,9aRS), de 0,64 g de formiate d'ammonium et de 0,2 g de palladium sur charbon à 10 % (p/p), on obtient 1,0 g de 4,9-éthano-9-(4-fluorophényl)-2,3,3a,4,9,9a-hexahydro-1H-benzo[f]isoindole-3a-carboxylate de méthyle-(3aRS,4SR,9SR,9aRS) sous forme de solide blanc dont la caractéristique est la suivante :

- point de fusion = 145°C.

Etape D

En opérant comme à l'étape G de l'exemple 1, mais à partir de 1,0 g de 4,9-éthano-9-(4-fluorophényl)-2,3,3a,4,9,9a-hexahydro-1H-benzo[f]isoindole-3a-carboxylate de méthyle-(3aRS,4SR,9SR,9aRS), de 0,51 g d'acide 2-(2-méthoxyphényl)propènoïque, de 0,24 $cm^3$ de chlorure d'oxalyle et de 0,79 $cm^3$ de triéthylamine, on obtient, après cristallisation dans 16 $cm^3$ d'isopropanol, 0,7 g de 4,9-éthano-9-(4-fluorophényl)-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS) sous forme d'un solide blanc dont la caractéristique est la suivante :

- point de fusion = 165°C.

**EXEMPLE 65**

<u>Préparation de l'acide 4,9-éthano-9-(4-fluorophényl)-2-[2-(2-méthoxyphényl)propènoyl]-2,3,3a,4,9,9a-hexahydro-1H-benzo[f]isoindole-3a-carboxylique-(3aRS,4SR,9SR,9aRS)</u>

En opérant comme à l'exemple 2, mais à partir de 0,85 g de 4,9-éthano-9-(4-fluorophényl)-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS), de 8,5 $cm^3$ d'une solution aqueuse normale d'hydroxyde de sodium et de 15 $cm^3$ de méthanol, on obtient, après cristallisation en milieu acide aqueux, 0,65 g d'acide 4,9-éthano-9-(4-fluorophényl)-2-[2-(2-méthoxyphényl)propènoyl]-2,3,3a,4,9,9a-hexahydro-1H-benzo[f]isoindole-3a-carboxylique-(3aRS,4SR,9SR,9aRS) dont la caractéristique est la suivante :

- point de fusion = 170°C.

**EXEMPLE 66**

Préparation du 9-(4-chloro-3-fluorophényl)-4,9-éthano-2-[2-(2-méthoxyphényl)propénoyl]-2,3,3a,4,9,9a-hexahydro-1H-benzo[f]isoindole-3a-carboxylate de méthyle-(3aRS,4SR,9SR,9aRS)

Etape A

En opérant comme à l'étape C de l'exemple 1, mais à partir de 6,3 g de 4-chloro-3-fluoro-bromobenzène, de 0,73 g de magnésium en tournures et de 5,45 g de 2-benzyl-7-oxo-4-phényloctahydroisoindole-3a-carboxylate de méthyle-(3aRS,4SR,7aRS), on obtient, après flash-chromatographie sur gel de silice (230-400 mesh), en éluant avec un mélange cyclohexane-acétate d'éthyle (95-5 en volumes) et recristallisation dans 75 $cm^3$ d'isopropanol, 4.8 g de 2-benzyl-7-(4-chloro-3-fluorophényl)-7-hydroxy-4-phényloctahydroisoindole-3a-carboxylate de méthyle-(3aRS,4SR,7RS,7aRS) sous forme d'un solide blanc dont la caractéristique est la suivante :

- point de fusion = 175°C.

Etape B

En opérant comme à l'étape D de l'exemple 1, mais à partir de 4,6 g de 2-benzyl-7-(4-chloro-3-fluorophényl)-7-hydroxy-4-phényl-octahydroisoindole-3a-carboxylate de méthyle-(3aRS,4SR,7RS,7aSR), de 12,4 $cm^3$ d'acide trifluorométhanesulfonique et de 50 $cm^3$ de dichlorométhane, on obtient, après cristallisation dans 25 $cm^3$ d'isopropanol, 3 g de 2-benzyl-9-(4-chloro-3-fluorophényl)-4,9-éthano-2,3,3a,4,9,9a-hexahydro-1H-benzo[f]isoindole-3a-carboxylate de méthyle-(3aRS,4SR,9SR,9aRS) sous forme d'un solide blanc dont la caractéristique est la suivante :

- point de fusion = 136°C.

Etape C

En opérant comme à l'étape C de l'exemple 51, mais à partir de 2,8 g de 2-benzyl-9-(4-chloro-3-fluorophényl)-4,9-éthano-2,3,3a,4,9,9a-hexahydro-1H-benzo[f]isoindole-3a-carboxylate de méthyle-(3aRS,4SR,9SR,9aRS) et de 1,95 $cm^3$ de chloroformiate de vinyle dans 40 $cm^3$ de dichlorométhane puis en traitant l'intermédiaire dissous dans 40 $cm^3$ de méthanol par 59 $cm^3$ d'une solution normale de gaz chlorhydrique dans l'oxyde de diéthyle on obtient, après flash-chromatographie sur gel de silice (230-400 mesh), en éluant avec un mélange dichlorométhane-méthanol (97,5-2,5 en volumes), 1 g de 9-(4-chloro-3-fluorophényl)-4,9-éthano-2,3,3a,4,9,9a-hexahydro-1H-benzo[f]isoindole-3a-carboxylate de méthyle-(3aRS,4SR,9SR,9aRS) sous forme d'une meringue jaune utilisée telle quelle dans les synthèses ultérieures,

Etape D

En opérant comme à l'étape G de l'exemple 1, mais à partir de 1,4 g de 9-(4-chloro-3-fluorophényl)-4,9-éthano)-2,3,3a,4,9,9a-hexahydro-1H-benzo[f]isoindole-3a-carboxylate de méthyle-(3aRS,4SR,9SR,9aRS), de 0,67 g d'acide 2-(2-méthoxyphényl)propénoïque, de 0,33 $cm^3$ de chlorure d'oxalyle et de 1,06 $cm^3$ de triéthylamine, on obtient, après flash-chromatographie sur gel de silice (230-400 mesh), en éluant avec un mélange cyclohexane-acétate d'éthyle (70-30 en volumes) et recristallisation dans un mélange isopropanol-oxyde de diisopropyle (1-1 en volumes), 1,5 g de 9-(4-chloro-3-fluorophényl)-4,9-éthano-2-[2-(2-méthoxyphényl)propènoyl]- 2,3,3a,4,9,9a-hexahydro-1H-benzo[f]isoindole-3a-carboxylate de méthyle-(3aRS,4SR,9SR,9aRS) dont la caractéristique est la suivante :

- point de fusion = 173°C.

**EXEMPLE 67**

Préparation de l'acide 9-(4-chloro-3-fluorophényl)-4,9-éthano-2-[2-(2-méthoxyphényl)propènoyl]-2,3,3a,4,9,9a-hexahydro-1H-benzo[f]isoindole-3a-carboxylique-(3aRS,4SR,9SR,9aRS)

En opérant comme à l'exemple 2, mais à partir de 1,2 g de 9-(4-chloro-3-fluorophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR, 9aRS), de 3,3 $cm^3$ d'une solution aqueuse normale d'hydroxyde de sodium et 20 $cm^3$ d'éthanol, on obtient, après recristallisation dans 5 $cm^3$ d'isopropanol, 1,15 g d'acide 9-(4-chloro-3-fluorophényl)-4,9-éthano-2-[2-(2-méthoxyphényl)propènoyl]-2,3,3a,4,9,9a-hexahydro-1H-benzo[f]isoindole-3a-carboxylique-(3aRS,4SR,9SR,9aRS) dont la caractéristique est la suivante

- point de fusion = 170°C.

**EXEMPLE 68**

Préparation du 4,9-éthano-2-[2-(2-méthoxyphényl)propénoyl]-9-(3-méthylphényl)-2,3,3a,4,9,9a-hexahydro-1H-benzo [f]isoindole-3a-carboxylate de méthyle-(3aRS,4SR,9SR,9aRS)

Etape A

En opérant comme à l'étape C de l'exemple 1, mais à partir de 3,68 $cm^3$ de 3-bromotoluène, 0,73 g de magnésium en tournures et de 5,45 g de 2-benzyl-7-oxo-4-phényloctahydroisoindole-3a-carboxylate de méthyle-(3aRS,4SR,7aRS), on obtient, après flash-chromatographie sur gel de silice (230-400 mesh), en éluant avec un mélange cyclohexane-acétate d'éthyle (95-5 en volumes) et recristallisation dans 75 $cm^3$ d'isopropanol, 4,2 g de 2-benzyl-7-hydroxy-7-(3-méthylphényl)-4-phényl-octahydroisoindole-3a-carboxylate de méthyle-(3aRS,4SR,7RS,7aRS) sous forme d'une meringue jaune-orangée utilisée telle quelle dans les synthèses ultérieures.

Etape B

En opérant comme à l'étape C de l'exemple 38, mais à partir de 4,2 g de 2-benzy]-7-hydroxy-7-(3-méthylphényl)-4-phényl-octahydroisoindole-3a-carboxylate de méthyle-(3aRS,4SR,7RS,7aRS), de 12,4 $cm^3$ d'acide trifluorométhanesulfonique et de 42 $cm^3$ de dichlorométhane, on obtient 4 g de 2-benzyl-4,9-éthano-9-(3-méthylphényl)-2,3,3a,4,9,9a-hexahydro-1H-benzo[f]isoindole-3a-carboxylate de méthyle-(3aRS,4SR,9SR,9aRS) sous forme d'une meringue orangée utilisée telle quelle dans les synthèses ultérieures.

Etape C

En opérant comme à l'étape E de l'exemple 1, mais à partir de 4 g de 2-benzyl-4,9-éthano-9-(3-mèthylphényl)-2,3,3a,4,9,9a-hexahydro-1H-benzo[f]isoindole-3a-carboxylate de méthyle-(3aRS,4SR,9SR,9aRS), de 1,73 g de formiate d'ammonium et de 0,75 g de palladium sur charbon à 10 % (p/p), on obtient 3 g de 4,9-éthano-9-(3-méthylphényl)-2,3,3a,4,9,9a-hexahydro-1H-benzo[f]isoindole-3a-carboxylate de méthyle -(3aRS,48R,9SR,9aRS) sous forme d'une huile jaune utilisée telle quelle dans les synthèses ultérieures.

Etape D

En opérant comme à l'étape G de l'exemple 1, mais à partir de 3 g de 4,9-éthano-9-(3-méthylphényl)-2,3,3a,4,9,9a-hexahydro-1H-benzo[f]isoindole-3a-carboxylate de méthyle-(3aRS,4SR,9SR,9aRS), de 1,54 g d'acide 2-(2-méthoxyphényl)propenoïque, de 0,74 $cm^3$ de chlorure d'oxalyle et de 2,45 $cm^3$ de triéthylamine, on obtient, après flash-chromatographie sur gel de silice (230-400 mesh), en éluant avec un mélange cyclohexane-acétate d'éthyle (50-50 en volumes) et recristallisation dans 10 $cm^3$ d'isopropanol, 2,5 g de 4,9-éthano-2-[2-(2-méthoxyphényl)propènoyl]-9-(3-méthylphényl)-2,3,3a,4,9,9a-hexahydro-1H-benzo[f]isoindole-3a-carboxylate de méthyle-(3aRS,4SR,9SR, 9aRS) dont la caractéristique est la suivante :

- point de fusion = 142°C.

**EXEMPLE 69**

Préparation de l'acide 4,9-éthano-2-[2-(2-méthoxyphényl)propénoyl]-9-(3-méthylphényl)-2,3,3a,4,9,9a-hexahydro-1H-benzo[f]isoindole-3a-carboxylique-(3aRS,4SR,9SR,9aRS)

En opérant comme à l'exemple 2, mais à partir de 2,2 g de 4,9-éthano-2-[2-(2-méthoxyphényl)propènoyl]-9-(3-méthylphényl)-2,3,3a,4,9,9a-hexahydro-1H-benzo[f]isoindole-3a-carboxylate de méthyle-(3aRS,4SR,9SR, 9aRS), de 8,7 $cm^3$ d'une solution aqueuse normale d'hydroxyde de sodium et 22 $cm^3$ d'éthanol, on obtient, après recristallisation dans 50 $cm^3$ d'isopropanol, 1,75 g d'acide 4,9-éthano-2-[2-(2-méthoxyphényl)propènoyl]-9-(3-méthylphényl)-2,3,3a,4,9,9a-hexahydro-1H-benzo[f]isoindole-3a-carboxylique-(3aRS,4SR,9SR,9aRS) dont la caractéristique est la suivante :

- point de fusion = 252°C.

**EXEMPLE 70**

Préparation du 9-(1,3-benzodioxol-5-yl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS)

Etape A

En opérant comme à l'étape A de l'exemple 31, mais à partir de 5,9 g de 2-benzyl-7-oxo-4-phényloctahydroisoindole-3a-carboxylate de méthyle-(3aRS,4SR,7aRS), de 2,5 $cm^3$ de vératrole et de 6,3 $cm^3$ d'acide trifluorométhanesulfonique dans 26 $cm^3$ de dichlorométhane, on obtient, après purification par flash-chromatographie sur gel de silice (230-400 mesh), en éluant avec un mélange cyclohexane-acétate d'éthyle (90-10 en volumes), 1,8 g de 2-benzyl-9-(3,4-diméthoxyphényl)-4,9-éthano-2,3,3a,4,9,9a-hexahydro-1H-benzo[f]isoindole-3a-carboxylate de méthyle-(3aRS,4SR,9SR,9aRS) sous forme d'une huile incolore utilisée telle quelle dans les synthèses ultérieures.

Etape B

A 22,3 $cm^3$ de tribromure de bore refroidis à une température voisine de-50°C, on ajoute une solution de 1,8 g de 2-benzyl-9-(3,4-diméthoxyphényl)-4,9-éthano-2,3,3a,4,9,9a-hexahydro-1H-benzo[f]isoindole-3a-carboxylate de méthyle-(3aRS,4SR,9SR,9aRS) dans 20 $cm^3$ de dichlorométhane. Le mélange réactionnel est agité pendant une heure à une température voisine de -50°C puis 75 $cm^3$ d'une solution aqueuse saturée en hydrogénocarbonate de sodium sont ajoutés. La phase aqueuse est séparée par décantation et extraite par deux fois 25 $cm^3$ de dichlorométhane. Les phases organiques sont réunies, lavées par trois fois 15 $cm^3$ d'eau distillée, séchées sur sulfate de magnésium et concentrées sous pression réduite. Le résidu est purifié par flash-chromatographie sur gel de silice (230-400 mesh), en éluant avec un mélange dichlorométhane-méthanol (97,5-2,5 en volumes). On obtient ainsi 0,8 g de 2-benzyl-9-(3,4-dihydroxyphényl)-4,9-éthano-2,3,3a,4,9,9a-hexahydro-1H-benzo[f]isoindole-3a-carboxylate de méthyle-(3aRS,4SR,9SR,9aRS), sous forme d'une huile orange utilisée telle quelle dans les synthèses ultérieures, et 1,0 g d'un mélange de 2-benzyl-4,9-éthano-9-(3-hydroxy-4-méthoxyphényl)-2,3,3a,4,9,9a-hexahydro-1H-benzo[f]isoindole-3a-carboxylale de méthyle-(3aRS,4SR,9SR,9aRS) et de 2-benzyl-4,9-éthano-9-(4-hydroxy-3-méthoxyphényl)-2,3,3a,4,9,9a-hexahydro-1H-benzo[f]isoindole-3a-carboxylate de méthyle-(3aRS,4SR,9SR,9aRS), sous forme d'une huile orange utilisée telle quelle dans les synthèses ultérieures.

Etape C

A une solution de 0,58 g de 2-benzyl-9-(3,4-dihydroxyphényl)-4,9-éthano-2,3,3a,4,9,9a-hexahydro-1H-benzo [f]isoindole-3a-carboxylate de méthyle-(3aRS,4SR,9SR,9aRS) dans 4 $cm^3$ de dibromométhane, on ajoute une solution de 1,8 g de carbonate de potassium dans 4 $cm^3$ d'eau distillée puis 60 mg d'Adogen 464. Le mélange réactionnel est chauffé au reflux sous agitation pendant quatre vingt dix minutes puis refroidis à une température voisine de 20°C, dilué par 25 $cm^3$ de dichlorométhane. La phase aqueuse est séparée par décantation et extraite par deux fois 15 $cm^3$ de dichlorométhane. Les phases organiques sont réunies, lavées par trois fois 15 $cm^3$ d'eau distillée, séchées sur sulfate de magnésium et concentrées sous pression réduite. On obtient ainsi 0,8 g d'un produit brut qui est joint à un deuxième lot de 0,4 g provenant d'une autre expérience. La purification de ce mélange par flash-chromatographie sur gel de silice (230-400 mesh), en éluant avec un mélange cyclohexane-acétate d'éthyle (90-10 en volumes) fournit 0,45 g de 9-(1,3-benzodioxol-5-yl)-2-benzyl-4,9-éthano-2,3,3a,4,9,9a-hexahydro-1H-benzo[f]isoindole-3a-carboxylate de méthyle-(3aRS,4SR,9SR,9aRS) sous forme d'une meringue blanche utilisée telle quelle dans les synthèses ultérieures, dont les caractéristiques sont les suivantes :

- spectre de R.M.N. $^1H$ (400 MHz, $CDCl_3$, à une température de 333K, d en ppm) : 1,43 - 1,65 et 2,47 (3 mts. respectivement 1H - 1H et 2H : $CH_2CH_2$) ; de 2,25 à 2,40 (mt, 1H : 1H du $CH_2$ en 1) ; 2,36 (d, J = 10 Hz, 1H : 1H du $CH_2$ en 3) ; 2,77 (d large, J = 9,5 Hz, 1H : l'autre H du $CH_2$ en 1) ; de 3,15 à 3,25 (mt, 1H : CH en 9a) ; 3,21 (d. J = 10 Hz, 1H : l'autre H du $CH_2$ en 3) ; 3,39 (s large, 1H : CH en 4) ; 3,43 et 3,67 (2 d, J = 13 Hz, 1H chacun : $NCH_2Ar$) ; 3.55 (s, 3H : $COOCH_3$) ; 5,98 (AB limite, 2H $OCH_2O$) ; 6,63 (d large, J = 7,5 Hz, 1H : H en 8) ; 6,85 et 6,91 (2 d larges, J = 8,5 Hz, 1H chacun : H en 6 et H en 7 du 1-3-benzodioxol) ; 6,96 (s large, 1H : H en 4 du 1-3-benzodioxol) ; de 7,00 à 7,35 (mt, 8H : H en 5 - H en 6 - H en 7 et H aromatiques du benzyle).

Etape D

En opérant comme a l'étape E de l'exemple 1, mais à partir de 0,5 g de 9-(1,3-benzodioxol-5-yl)-2-benzyl-4,9-éthano-2,3,3a,4,9,9a-hexahydro-1H-benzo[f]isoindole-3a-carboxylate de méthyle-(3aRS,4SR,9SR,9aRS), de 0,21 g de formiate d'ammonium et de 70 mg de palladium sur charbon à 10 % (p/p), on obtient 0,25 g de 9-(1,3-benzodioxol-5-yl)-4,9-éthano-2,3,3a,4,9,9a-hexahydro-1H-benzo[f]isoindole-3a-carboxylate de méthyle-(3aRS,4SR,9SR,9aRS) sous forme d'un solide blanc utilisé tel quel dans les synthèses ultérieures.

Etape E

En opérant comme à l'étape G de l'exemple 1, mais à partir de 0,25 g de 9-(1,3-benzodioxol-5-yl)-4,9-éthano-2,3,3a,4,9,9a-hexahydro-1H-benzo[f]isoindole-3a-carboxylate de méthyle-(3aRS,4SR,9SR,9aRS), de 0,12 g d'acide 2-(2-méthoxyphényl)propènoïque, de 0,06 $cm^3$ de chlorure d'oxalyle et de 0,19 $cm^3$ de triéthylamine, on obtient, après flash-chromatographie sur gel de silice (230-400 mesh), en éluant avec un mélange dichlorométhane-méthanol (97,5-2,5 en volumes), 0,26 g de 9-(1,3-benzodioxol-5-yl)-4,9-éthano-2-[2-(2-méthoxyphényl)propènoyl]-2,3,3a,4,9,9a-hexahydro-1H-benzo[f]isoindole-3a-carboxylate de methyle-(3aRS,4SR,9SR,9aRS) sous forme d'une meringue blanche utilisée telle quelle dans les synthèses ultérieures.

Etape F

En opérant comme à l'exemple 2, mais à partir de 0,26 g de 9-(1,3-benzodioxol-5-yl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS), de 2,4 $cm^3$ d'une solution aqueuse normale d'hydroxyde de sodium et 5 $cm^3$ de méthanol, on obtient, après purification par flash-chromatographie sur gel de silice (230-400 mesh), en eluant avec un mélange dichlorométhane-méthanol (97,5-2,5 en volumes) et cristallisation dans 2,5 cm3 d'oxyde de diisopropyle, 80 mg d'acide 9-(1,3-benzodioxol-5-yl)-4,9-éthano-2-[2-(2-méthaxyphényl)propènoyl]-2,3,3a,4,9,9a-hexahydro-1H-benzo[f]isoindole-3a-carboxylique-(3aRS,4SR,9SR,9aRS) dont la caractéristique est la suivante :

- point de fusion = 204°C.

**EXEMPLE 71**

<u>Préparation du 9-(3,4-diméthylphényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS)</u>

Etape A

En opérant comme à l'étape A de l'exemple 31, mais à partir de 10,34 g de 2-benzyl-7-oxo-4-phènyl-octahydroisoindole-3a-carboxylate de méthyle-(3aRS,4SR,7aRS) dans 40 $cm^3$ de dichlorométhane, de 50 $cm^3$ d'orthoxylène, de 24 $cm^3$ d'acide trifluorométhanesulfonique à 99% et de 6,5 $cm^3$ d'anhydride trifluorométhanesulfonique, sous atmosphère d'argon, pendant vingt trois heures à une température voisine de 20°C, on obtient 7,27 g de-2-benzyl-9-(3.4-diméthylphényl)-4,9-éthano-2,3,3a,4,9,9a-hexahydro-1H-benzo[f]isoindole-3a-carboxylate de méthyle-(3aRS, 4SR, 9SR,9aRS) impur, sous forme d'une huile incolore, utilisable telle quelle pour l'étape suivante, dont la caractéristique est la suivante :

- spectre de masse (DCI) : M/Z = 452 ($M+H^+$).

Etape B

En opérant comme à l'étape E de l'exemple 1, mais à partir de 9,51 g de-2-benzyl-9-(3,4-diméthylphényl)-4,9-éthano-2,3,3a,4,9,9a-hexahydro-1H-benzo[f]isoindole-3a-carboxylate de méthyle-(3aRS,4SR,9SR,9aRS), obtenus comme à l'étape précédente, de 4 g de formiate d'ammonium. de 0,5 g de palladium sur charbon à 10 % (p/p), dans 150 $cm^3$ de méthanol, et de 100 $cm^3$ de dioxane, à une température voisine de 50°C, pendant six heures, après flash-chromatographie sur 500 g de gel de silice, en éluant successivement avec un $dm^3$ de dichlorométhane pur, puis un $dm^3$ du mélange dichlorométhane-éthanol (98-2 en volumes) puis un $dm^3$ du mélange dichlorométhane-éthanol (95-5 en volumes) puis $dm^3$ litres du mélange dichlorométhane-éthanol (90-10 en volumes), puis du mélange dichlorométhane-éthanol (80-20 en volumes), on obtient 5,79 g de-9-(3,4-diméthylphenyl)-4,9-éthano-2,3,3a,4,9,9a-hexahydro-1H-benzo[f]isomdole-3a-carboxylate de méthyle-(3aRS,4SR,9SR,9aRS), sous forme d'une laque incolore légèrement meringuée utilisée telle quelle dans l'étape suivante, dont la caractéristique est la suivante ;

- spectre de masse (DCI) : M/Z = 361 ($M^+$).

Etape C

En opérant comme à l'étape G de l'exemple 1, mais à partir d'une solution de 2,67 g d'acide 2-(2-méthoxyphényl)propènoïque dans 50 $cm^3$ de dichlorométhane contenant 2 gouttes de N,N-diméthylformamide de 1,31 $cm^3$ de chlorure d'oxalyle, pendant deux heures, et d'une solution de 5,075 g de 9-(3,4-diméthylphényl)-4,9-éthano-2,3,3a, 4,9,9a-hexahydro-1H-benzo[f]isoindole-3a-carboxylate de méthyle-(3aRS, 4SR,9SR,9aRS), obtenu à l'étape précédente, et de 4,4 $cm^3$ de triéthylamine dans 100 $cm^3$ de dichlorométhane, puis coulée après 1 heure de 100 $cm^3$ d'eau, on obtient 3,4 g de 9-(3,4-diméthylphényl)-4,9-éthano-2[2-(2-mèthoxyphènyl)propènoyl]-2,3,3a,4,9,9a-hexahydro-1H-benzo[f]isoindole-3a-carboxylate de méthyle-(3aRS,4SR,9SR,9aRS), sous forme d'une poudre blanche, dont les caractéristiques sont les suivantes :

- point de fusion = 163°C (dec)
- spectre de masse (IE) : M/Z = 521 ($M^+$).

**EXEMPLE 72**

Préparation de l'acide 4,9-éthano-9-(3,4-diméthylphényl)-2[2-(2-méthoxyphényl)propènoyl]-2,3,3a,4,9,9a-hexahydro-1H-benzo[f]isoindole-3a-carboxylique-(3aRS,4SR,9SR,9aRS)

En opérant comme à l'exemple 2, mais à partir de 4,736 g de 9-(3,4-diméthyl phényl)-4,9-éthano-2 [2-(2-méthoxyphényl)propènoyl]-2,3,3a,4,9,9a-hexahydro-1H-benzo[f]isoindole-3a-carboxylate de méthyle-(3aRS,4SR,9SR, 9aRS), obtenus à l'étape précédente et de 36 $cm^3$ d'une solution aqueuse normale d'hydroxyde de sodium dans 50 $cm^3$ d'éthanol et 100 $cm^3$ de dioxane, pendant seize heures à une température voisine de 70°C, on obtient 3,375 g d'acide 9-(3,4-diméthylphényl)-4,9-éthano-2[2-(2-méthoxyphényl)propénoyl]-2,3,3a,4,9,9a-hexahydro-1H-benzo[f] isoindole-3a-carboxylique-(3aRS,4SR,9SR,9aRS), sous forme d'une poudre blanche, dont les caractéristiques sont les suivantes :

- point de fusion = 200°C, puis 245°C.
- Spectre de R.M.N n°127748
- spectre de masse (IE) : M/Z = 507 ($M^+$).

**EXEMPLE 73**

Préparation du 4,9-éthano-9-(4-méthoxyphényl)-2-[2-(2-méthoxyphényl)propènoyl]-2,3,3a,4,9,9a-hexahydro-1H-benzo[f]isoindole-3a-carboxylate de méthyle-(3aRS, 4SR,9SR,9aRS)

Etape A

En opérant comme à l'étape A de l'exemple 31, mais à partir de 11,5 g de 2-benzyl-7-oxo-4-phényloctahydroisoindole-3a-carboxylate de méthyle-(3aRS,4SR,7aRS), de 13,1 $cm^3$ d'anisole et de 21,2 $cm^3$ d'acide trifluorométhanesulfonique dans 120 $cm^3$ de dichlorométhane, on obtient, après purification par flash-chromatographie sur gel de silice (230-400 mesh), en éluant avec un mélange cyclohexane-acétate d'éthyle (70-30 en volumes), 12 g de 2-benzyl-4,9-éthano-9-(4-méthoxyphényl)-2,3,3a,4,9,9a-hexahydro-1H-benzo[f]isoindole-3a-carboxylate de méthyle-(3aRS,4SR,9SR,9aRS) dont la caractéristique est la suivante :

- point de fusion = 150°C.

Etape B

En opérant comme à l'étape E de l'exemple 1, mais à partir de 11,4 g de 2-benzyl-4,9-éthano-9-(4-méthoxyphényl)-2,3,3a,4,9,9a-hexahydro-1H-benzo[f]isoindole-3a-carboxylate de méthyle-(3aRS,4SR,9SR,9aRS), de 4,76 g de formiate d'ammonium et de 1 g de palladium sur charbon à 10 % (p/p), on obtient 9 g de 4,9-éthano-9-(4-méthoxyphényl)-2,3,3a,4,9,9a-hexahydro-1H-benzo[f]isoindole-3a-carboxylate de méthyle-(3aRS,4SR,9SR,9aRS) utilisé tel quel dans les synthèses ultérieures.

Etape C

En opérant comme à l'etape G de l'exemple 1, mais à partir de 8,9 g de 4,9-éthano-9-(4-méthoxyphényl)-2,3,3a,4,9,9a-hexahydro-1H-benzo[f]isoindole-3a-carboxylate de méthyle-(3aRS,4SR,9SR,9aRS), de 4,36 g d'acide 2-(2-méthoxyphényl)propènoïque, de 2,1 $cm^3$ de chlorure d'oxalyle et de 6,9 $cm^3$ de triéthylamine, on obtient, après purification par flash-chromatographie sur gel de silice (230-400 mesh), en éluant avec un mélange cyclohexane-acétate d'éthyle (50-50 en volumes), 0,47 g de 4,9-éthano-9-(4-méthoxyphényl)-2-[2-(2-méthoxyphényl) propènoyl]-2,3,3a,4,9,9a-hexahydro-1H-benzo[f]isoindole-3a-carboxylate de méthyle-(3aRS,4SR,9SR,9aRS) sous forme d'une meringue blanche dont la caractéristique est la suivante :

- point de fusion = 90°C.

**EXEMPLE 74**

Préparation de l'acide 4,9-éthano-9-(4-méthoxyphényl)-2-[2-(2-méthoxyphényl) propènoyl]-2,3,3a,4,9,9a-hexahydro-1H-benzo[f]isoindole-3a-carboxylique-(3aRS, 4SR,9SR,9aRS)

En opérant comme à l'exemple 2, mais à partir de 11 g de 4,9-éthano-9-(4-méthoxyphényl)-2-[2-(2-méthoxyphényl)propènoyl]-2,3,3a,4,9,9a-hexahydro-1H-benzo[f]isoindole-3a-carboxylate de méthyle-(3aRS,4SR,9SR, 9aRS), de 42 $cm^3$ d'une solution aqueuse normale d'hydroxyde de sodium et 100 $cm^3$ de méthanol, on obtient, après purification par flash-chromatographie sur gel de silice (230-400 mesh), en éluant avec des mélanges dichlorométhane-méthanol (100-0 à 98-2 en volumes), 2,15 g d'acide 4,9-éthano-9-(4-méthoxyphényl)-2-[2-(2-méthoxyphényl)propènoyl]-2,3,3a,4,9,9a-hexahydro-1H-benzo[f]isoindole-3a-carboxylique-(3aRS,4SR,9SR, 9aRS) dont la caractéristique est la suivante :

- point de fusion = 165°C.

**EXEMPLE 75**

Isolement de l'énantiomère dextrogyre de l'acide 4,9-éthano-9-(4-méthoxyphényl)-2-[2-(2-méthoxyphényl)propènoyl]-2,3,3a,4,9,9a-hexahydro-1H-benzo[f]isoindole-3a-carboxylique-(3aRS,4SR,9SR,9aRS)

9,2 g d'acide 4,9-éthano-9-(4-méthoxyphényl)-2-[2-(2-méthoxyphényl)propènoyl]-2,3,3a,4,9,9a-hexahydro-1H-benzo[f]isoindole-3a-carboxylique-(3aRS,4SR,9SR, 9aRS), obtenus à l'exemple 74, sont dédoublés, sur une colonne de silice chirale porteuse de greffons N-(3,5-dinitrobenzoyl)-phénylalanine-(R), en 5 injections successives et en éluant par un mélange n-heptane-dichlorométhane-méthanol (50-50-3 en volumes). En recueuillant les premières fractions éluées (temps de rétention 42 mn), on obtient, après concentration du solvant sous pression réduite, 3,51 g de l'énantiomère dextrogyre de l'acide 4,9-éthano-9-(4-méthoxyphényl)-2-[2-(2-méthoxyphényl)propènoyl]-2,3,3a,4,9,9a-hexahydro-1H-benzo[f]isoindole-3a-carboxylique-(3aRS,4SR,9SR,9aRS), sous forme d'une poudre blanche, dont les caractéristiques sont les suivantes :

- point de fusion = 203°C
- spectre de masse (IE) : M/Z= 509 ($M^+$)
- pouvoir rotatoire : $[a]_{365}^{20}$= + 71,7 +/- 1,2° (c = 0,5/ méthanol).

**EXEMPLE 76**

Préparation du 4,9-éthano-9-(4-méthoxyphényl)-2-[2-(2-méthoxyphènyl)propènoyl)-2,3,3a,4,9,9a-hexahydro-1H-benzo[f]isoindole-3a-N-(3-pyridylméthyl)-carboxamide-(3aRS,4SR,9SR,9aRS)

En opérant comme à l'exemple 42, mais à partir de 1 g d'acide 4,9-éthano-9-(4-méthoxyphènyl)-2-[2-(2-méthoxyphényl)propénoyl]-2,3,3a,4,9,9a-hexahydro-1H-benzo[f]isoindole-3a-carboxylique-(3aRS,4SR,9SR,9aRS), 0,24 $cm^3$ de 3-(aminomethyl)pyridine, 0,46 g de chlorhydrate de 1-ethyl-3-[(3-diméthylamino)propyl]carbodrimide et 0,16 g d'hydrate de N-1-hydroxybenzotriazole, on obtient, après cristallisation dans 11$cm^3$ d'un mélange isopropanol-oxyde de diisopropyle (10-90 en volumes), 0,72 g de 4,9-éthano-9-(4-méthoxyphényl)-2-[2-(2-méthoxyphényl) propènoyl]-2,3,3a,4,9,9a-hexahydro-1H-benzo[f]isoindole-3a-N-(3-pyridylméthyl)-carboxamide-(3aRS,4SR, 9SR,9aRS)

dont la caractéristique est la suivante :

- point de fusion = 121°C.

**EXEMPLE 77**

Préparation du 4,9-éthano-9-(4-méthoxyphényl)-2-[2-(2-méthoxyphényl)propènoyl]-2,3,3a,4,9,9a-hexahydro-1H-benzo[f]isoindole-3a-N'-phénylcarbohydrazide-(3aRS,4SR,9SR,9aRS)

En opérant comme à l'exemple 27, mais à partir de 1 g d'acide 4,9-éthano-9-(4-méthoxyphényl)-2-[2-(2-méthoxyphényl)propènoyl]-2,3,3a,4,9,9a-hexahydro-1H-benzo[f]isoindole-3a-carboxylique-(3aRS,4SR,9SR,9aRS), 0,25 $cm^3$ de N-phénylhydrazine, 0,45 g de chlorhydrate de 1-éthyl-3-[(3-diméthylamino)propyl]carbodiimide et 0,16 g d'hydrate de N-1-hydroxybenzotriazole, on obtient, après cristallisation dans 11$cm^3$ d'un mélange isopropanol-oxyde de diisopropyle (10-90 en volumes), 0,9 g de 4,9-éthano-9-(4-méthoxyphényl)-2-[2-(2-méthoxyphényl)propènoyl]-2,3,3a,4,9,9a-hexahydro-1H-benzo[f]isoindole-3a-N'-phénylcarbohydrazide-(3aRS,4SR,9SR,9aRS) dont la caractéristique est la suivante :

- point de fusion = 180°C.

**EXEMPLE 78**

Préparation de l'acide 4,9-éthano-]-9-(4-méthoxyphényl)-2-[2-(2-méthoxyphényl)propènoyl]-2,3,3a,4,9,9a-hexahydro-1H-benzo[f]isoindole-3a-hydroxamique-(3aRS,4SR,9SR,9aRS)

Etape A

A une solution de 2,2 g d'acide 4,9-éthano-9-(4-méthoxyphényl)-2-[2-(2-méthoxyphényl)propènoyl]-2,3,3a,4,9,9a-hexahydro-1H-benzo[f]isoindole-3a-carboxylique-(3aRS,4SR,9SR,9aRS), de 1 g 1-éthyl-3-[(3-diméthylamino)propyl]carbodiimide et de 0,35 g d'hydrate de N-1-hydroxybenzotriazole dans 10 $cm^3$ de dichlorométhane, on ajoute une solution de 0.83 g de O-benzylhydroxylamine et de 0,73 $cm^3$ de triéthylamine dans 10 $cm^3$ de dichlorométhane. Le mélange réactionnel est agité pendant vingt quatre heures à une température voisine de 20°C puis on ajoute 15$cm^3$ d'eau distillée. La phase organique est séparée par décantation, lavée par 20 $cm^3$ d'eau distillée, séchée sur sulfate de magnésium et concentrée sous pression réduite. Le résidu est cristallisé dans 25 $cm^3$ d'un mélange isopropanol-oxyde de diisopropyle (20-80 en volumes). On obtient ainsi 2,03 g de 4,9-éthano-9-(4-méthoxyphény])-2-[2-(2-méthoxyphényl)propènoyl]-2,3,3a,4,9,9a-hexahydro-1H-benzo[f]isoindole-3a-hydroxamate de benzyle-(3aRS,4SR,9SR,9aRS) sous d'un solide blanc dont la caractéristique est la suivante :

- point de fusion = 201°C.

Etape B

En opérant comme à l'exemple 16, mais à partir de 1,84 g de 4,9-éthano-9-(4-méthoxyphényl)-2-[2-(2-méthoxyphényl)propénoyl]-2,3,3a,4,9,9a-hexahydro-1H-benzo[f]isoindole-3a-hydroxamate de benzyle-(3aRS,4SR,9SR,9aRS), de 1 g de chlorure d'aluminium et de 0,98 $cm^3$ d'anisole, on obtient, après flash-chromatographie sur gel de silice (230-400 mesh), en éluant avec un mélange dichlorométhane-méthanol (90-10 en volumes), 0,5 g d'acide 4,9-éthano-9-(4-méthoxyphényl)-2-[2-(2-méthoxyphényl)propènoyl]-2,3,3a,4,9,9a-hexahydro-1H-benzo[f]isoindole-3a-hydroxamique-(3aRS,4SR,9SR,9aRS) sous forme d'un solide écru dont la caractéristique est la suivante :

- point de fusion = 165°C.

**EXEMPLE 79**

Préparation du 4,9-éthano-9-(4-méthoxyphényl)-2-[2-(2-méthoxyphényl)propènoyl]-2,3,3a,4,9,9a-hexahydro-1H-benzo[f]isoindole-3a-N'-(3-pyridyl)carbohydrazide-(3aRS,4SR,9SR,9aRS)

En opérant comme à l'exemple 27, mais à partir de 1 g d'acide 4,9-éthano-9-(4-méthoxyphényl)-2-[2-(2-méthoxyphényl)propènoyl]-2,3,3a,4,9,9a-hexahydro-1H-benzo[f]isoindole-3a-carboxylique-(3aRS,4SR,9SR,9aRS), 0,26 g de N-(3-pyridyl)hydrazine, 0,45 g de chlorhydrate de 1-éthyl-3-[(3-diméthylamino)propyl]carbodiimide et de 0,16

g d'hydrate de N-1-hydroxybenzotriazole, on obtient, après purification par flash-chromatographie sur gel de silice (230-400 mesh), en éluant avec des mélanges dichlorométhane-méthanol (95-5 puis 90-10 en volumes) et recristallisation dans l'isopropanol, 0,6 g de 4,9-éthano-9-(4-méthoxyphényl)-2-[2-(2-méthoxyphényl)propènoyl]-2,3,3a,4,9,9a-hexahydro-1H-benzo[f]isoindole-3a-N'-(3-pyridyl)carbohydrazide-(3aRS,4SR,9SR,9aRS) dont la caractéristique est la suivante :

- point de fusion = 180°C.

**EXEMPLE 80**

Préparation du 4,9-ethano-9-(4-méthoxyphényl)-2-[2-(2-méthoxyphényl)propènoyl]-2,3,3a,4,9,9a-hexahydro-1H-benzo[f]isoindole-3a-N-(3-thiénylméthyl)-carboxamide-(3aRS,4SR,9SR,9aRS)

En opérant comme à l'exemple 42, mais à partir de 1 g d'acide 4,9-éthano-9-(4-méthoxyphényl)-2-[2-(2-méthoxyphényl)propènoyl]-2,3,3a,4,9,9a-hexahydro-1H-benzo[f]isoindole-3a-carboxylique-(3aRS,4SR,9SR,9aRS), 0,2 g de 3-aminométhylthiophène, 0,45 g de chlorhydrate de 1-éthyl-3-[(3-diméthylamino)propyl]carbodiimide et 0,16 g d'hydrate de N-1-hydroxybenzotriazole, on obtient, après recristallisation dans 11cm$^3$ d'un mélange isopropanol-oxyde de diisopropyle (10-90 en volumes), 1 g de 4,9-éthano-9-(4-méthoxyphényl)-2-[2-(2-méthoxyphényl) propènoyl]-2,3,3a,4,9,9a-hexahydro-1H-benzo[f]isoindole-3a-N-(3-thiénylméthyl)-carboxamide-(3aRS,4SR,9SR,9aRS) dont la caractéristique est la suivante :

- point de fusion = 130°C.

**EXEMPLE 81**

Préparation des acides 2-{4,9-éthano-9-(4-méthoxyphényl)-2-[2-(2-méthoxyphényl)propènoyl]-2,3,3a,4,9,9a-hexahydro-1H-benzo[f]isoindole-3a-carbonylamino-(3aRS,4SR,9SR,9aRS)}phénylacétiques-(RS) et (SR)

Etape A

En opérant comme a l'exemple 42, mais à partir de 1.27 g d'acide 4,9-éthano-9-(4-méthoxyphényl)-2-[2-(2-méthoxyphényl)propènoyl]-2,3,3a,4,9,9a-hexahydro-1H-benzo[f]isoindole-3a-carboxylique-(3aRS,4SR,9SR,9aRS), 0,61 g de chlorhydrate de phénylglycinate de méthyle, 0,59 g de chlorhydrate de 1-éthyl-3-[(3-diméthylamino)propyl] carbodiimide, 0,42 cm$^3$ de triéthylamine et 0,2 g d'hydrate de N-1-hydroxybenzotriazole, on obtient, après flash-chromatographie sur gel de silice (230-400 mesh), en éluant avec un mélange dichlorométhane-méthanol (99-1 en volumes), 1 g d'un mélange de 2-{4,9-éthano-9-(4-méthoxyphényl)-2-[2-(2-méthoxyphényl)propènoyl]-2,3,3a,4,9,9a-hexahydro-1H-benzo[f]isoindole-3a-carbonylamino-(3aRS,4SR,9SR,9aRS)}phénylacétates de méthyle-(RS) et (SR) sous forme d'une meringue utilisée telle quelle dans les synthèses ultérieures.

Etape B

En opérant comme à l'exemple 2, mais à partir de 0,6 g d'un mélange de 2-{4,9-éthano-9-(4-méthoxyphényl)-2-[2-(2-méthoxyphényl)propènoyl]-2,3,3a,4,9,9a-hexahydro-1H-benzo[f]isoindole-3a-carbonylamino-(3aRS,4SR, 9SR,9aRS); phénylacètates de méthyle-(RS) et (SR), dans 1,83 cm$^3$ d'une solution aqueuse normale d'hydroxyde de sodium et 20 cm$^3$ d'éthanol, on obtient 0,5 g de produit brut qui est joint à un deuxième lot de 0,65 g provenant d'une autre expérience. La purification de ce mélange par recristallisation dans 50 cm$^3$ d'un mélange isopropanol-oxyde de diisopropyle (50-50 en volumes) fournit 1,05 g d'un mélange équimolaire des acides 2-{4,9-éthano-9-(4-méthoxyphényl)-2-[2-(2-méthoxyphényl)propènoyl]-2,3,3a,4,9,9a-hexahydro-1H-benzo[f]isoindole-3a-carbonylamino-(3aRS, 4SR,9SR,9aRS)}phénylacétiques-(RS) et (SR) dont les caractéristiques sont les suivantes

- point de fusion = 196°C
- spectre de R.M.N. $^1$H (250 MHz, $(CD_3)_2SO$ d6, à une température de 383 K, d en ppm). Nous observons le mélange des deux diastéréoisomères dans les proportions 50/50.
  * 1,38 - 1,60 - 1,98 et 2,14 (4 mts, 1H chacun : $CH_2CH_2$) : de 3,15 à 3,80 (mt, 5H : $CH_2$ en 1 - 1H du $CH_2$ en 3 - CH en 9a et CH en 4) ; 3,64 - 3,71 et 3,84 (3 s, 6H en totalité : les 2 $ArOCH_3$) ; de 4,20 à 4,45 (mt, 1H : l'autre H du $CH_2$ en 3) ; 5,19 (mt, 1H : NCHAr) ; 5,47 - 5,51 - 5,66 et 5,69 (4s larges, 2H en totalité : $=CH_2$) ; 6,42 (mt, 1H : H en 8) ; de 6,80 à 7,50 (mt, 16H : H en 5 - H en 6 - H en 7 - H aromatiques du 4-méthoxyphényl - H aromatiques du 2-méthoxyphényl et H aromatiques du phényl); 7,98 et 8,02 (2 s larges, 1H en totalité : CONH).

**EXEMPLE 82**

Préparation de l'acide 4,9-éthano-2-[2-(2-méthoxyphényl)propénoyl]-9-(4-trifluorométhoxyphényl)-2,3,3a,4,9,9a-hexahydro-1H-benzo[f]isoindole-3a-carboxylique-(3aRS,4SR,9SR,9aRS)

Etape A

En opérant comme à l'étape C de l'exemple 45, mais à partir de 1,51 g de 2-benzyl-7-iodo-4-phényl-2,3,a,4,5,7a-hexahydroisoindole-3a-carboxylate de méthyle-(3aRS,4SR,7aRS), de 0,17 g de tétrakis(triphénylphosphine) palladium, de 0,73 g d'acide 4-trifluorométhoxyphénylboronique, obtenu sous forme d'anhydride trimérique en opérant comme dans le brevet D.E. 4.218.614, et 1,5 g de carbonate de sodium au reflux pendant deux heures dans 15 $cm^3$ de toluène et 13 $cm^3$ de méthanol, on obtient, après purification par flash-chromatographie sur gel de silice (230-400 Mesh) en éluant par un mélange de cyclohexane et d'acétate d'éthyle (80-20 en volumes), 1,2 g (79%) de 2-benzyl-4-phényl-7-(4-trifluorométhoxyphényl)-2,3,3a,4,5,7a-hexahydro-1H-isoindole-3a-carboxylate de méthyle-(3aRS,4SR,7aRS), sous forme d'une huile jaune pâle, dont la caractéristique est la suivante :

- spectre de masse (IE) : M/Z = 507 ($M^+$).

Etape B

En opérant comme à l'étape C de l'exemple 38, mais à partir de 0,48 g de 2-benzyl-4-phényl-7-(4-trifluorométhoxyphényl)-2,3,3a,4,5,7a-hexahydro-1H-isoindole-3a-carboxylate de méthyle-(3aRS,4SR,7aRS) et de 1,18 $cm^3$ d'acide trifluorométhanesulfonique dans 2 $cm^3$ de dichlorométhane pendant deux heures à température ambiante, on obtient, après purification par flash-chromatographie sur gel de silice (230-40 Mesh) en éluant par un mélange de cyclohexane et d'acétate d'éthyle (90-10 en volumes), 0,43 g (89%) de 2-benzyl-4,9-éthano-9-(4trifluorométhoxyphényl)-2,3,3a,4,9,9a-hexahydro-1H-benzo[f]isoindole-3a-carboxylate de méthyle-(3aRS,4SR,9SR,9aRS), sous forme d'une pâte beige utilisée telle quelle à l'étape suivante, dont la caractéristique est la suivante :

- spectre de masse (IE) : M/Z = 507($M^+$).

Etape C

En opérant comme à l'étape B de l'exemple 47, mais à partir de 0,83 g de 2-benzyl-4,9-éthano-9-(4-trifluorométhoxyphényl)-2,3,3a,4,9,9a-hexahydro-1H-isoindole-3a-carboxylate de méthyle-(3aRS,4SR,9RS,9aRS) et de 0,29 $cm^3$ de chloroformiate de vinyle pendant dix huit heures à température ambiante dans 10 $cm^3$ de dichlorométhane, puis en reprenant le concentrât dans 20 $cm^3$ d'une solution normale de gaz chlorhydrique dans l'isopropanol au reflux pendant une heure, on obtient, apres neutralisation avec une solution aqueuse normale d'hydroxyde de sodium, puis extraction avec du dichlorométhane et concentration du solvant sous pression réduite, 0,59 g (75%) de 4,9-éthano-9-(4-trifluorométhoxyphényl)-2,3,3a,4,9,9a-hexahydro-1H-benzo[f]isoindole-3a-carboxylate de méthyle-(3aRS,4SR,9RS,9aRS), sous forme d'une meringue blanche utilisée telle quelle à l'étape suivante, dont la caractéristique est la suivante :

- spectre de masse (IE) : M/Z = 417 ($M^+$).

Etape D

En opérant comme à l'étape E de l'exemple 5, mais à partir de 0,59 g de 4,9-éthano-9-(4-trifluorométhoxyphényl)-2,3,3a,4,9,9a-hexahydro-1H-benzo[f]isoindole-3a-carboxylate de méthyle-(3aRS,4SR,9SR,9aRS), de 0,25 g d'acide 2-(2-méthoxyphényl)propènoique, de 0,2 g de chlorhydrate de 1-éthyl-3-[(3-diméthylamino)propyl]carbodiimide, de 27 mg d'hydrate de N-1-hydroxybenzotriazole dans 15 $cm^3$ de dichlorométhane pendant dix huit heures à température ambiante, on obtient, après purification par flash-chromatographie sur gel de silice (230-400 Mesh) en éluant par un mélange de cyclohexane et d'acétate d'éthyle (60-40 en volumes), 0,41 g (53%) de 4,9-éthano-2-[2-(2-méthoxyphényl)propènoyl]-9-(4-trifluorométhoxyphényl)-2,3,3a,4,9,9a-hexahydro-1H-benzo[f]isoindole-3a-carboxylate de méthyle-(3aRS,4SR,9SR,9aRS), sous forme d'une poudre beige-clair dont les caractéristiques sont les suivantes :

- point de fusion = 86°C
- spectre de masse (IE) : M/Z = 577 ($M^+$).

Etape E

En opérant comme à l'exemple 2, mais à partir de 0.38 g de 4,9-éthano-2-[2-(2-methoxyphényl)propénoyl]-9-(4-trifluorométhoxyphényl)-2,3,3a,4,9,9a-hexahydro-1H-benzo[f]isoindole-3a-carboxylate de méthyle-(3aRS,4SR,9SR,9aRS) au reflux pendant quatre heures dans 13 $cm^3$ d'une solution aqueuse décinormale d'hydroxyde de sodium et 13 $cm^3$ de méthanol, on obtient, après purification par recristallisation dans le pentane, 200 mg (54%) d'acide 4,9-éthano-2-[2-(2-méthoxyphényl)propènoyl]-9-(4-trifluorométhoxyphényl)-2,3,3a,4,9,9a-hexahydro-1H-benzo[f]isoindole-3a-carboxylique-(3aRS,4SR,9SR,9aRS), sous forme d'un solide blanc dont les caractéristiques sont les suivantes :

- point de fusion = 160°C

- spectre de masse (IE) : M/Z = 563 ($M^+$).

**EXEMPLE 83**

<u>Préparation du 9-(3-bromophényl)-4,9-éthano-2-[2-(2-méthoxyphenyl)propènoyl]-2,3,3a,4,9,9a-hexahydro-1H-benzo[f]isoindole-3a-carboxylate de méthyle-(3aRS,4SR,9SR,9aRS)</u>

Etape A

En opérant comme à l'étape C de l'exemple 45, mais à partir de 11,61 g de 2-benzyl-7-iodo-4-phényl-2,3,a,4,5,7a-hexahydroisoindole-3a-carboxylate de méthyle-(3aRS,4SR,7aRS), de 1,33 g de tétrakis(triphénylphosphine) palladium, de 5,46 g d'acide 3-bromophénylboronique. obtenu sous forme d'anhydride trimérique, et de 11,5 g de carbonate de sodium au reflux pendant deux heures dans 70 $cm^3$ de toluène et 50 $cm^3$ de méthanol, on obtient, après purification par flash-chromatographie sur gel de silice (230-400 Mesh) en éluant par un mélange de cyclohexane et d'acétate d'éthyle (80-20 en volumes), 8,5 g (75%) de 2-benzyl-7-(3-bromophényl)-4-phényl-2,3,3a,4,5,7a-hexahydro-1H-isoindole-3a-carboxylate de méthyle-(3aRS,4SR,7aRS), sous forme d'une huile jaune pâle, dont la caractéristique est la suivante :

- spectre de masse (IE) : M/Z = 502 ($M^+$).

Etape B

En opérant comme à l'étape C de l'exemple 38, mais a partir de 8,6 g de 2-benzyl-7-(3-bromophényl)-4-phényl-2,3,3a,4,5,7a-hexahydro-1H-isoindole-3a-carboxylate de méthyle-(3aRS,4SR,7aRS) et de 25 $cm^3$ d'acide trifluorométhanesulfonique dans 25 $cm^3$ de dichlorométhane pendant deux heures à température ambiante, on obtient, après purification par flash-chromatographie sur gel de silice (230-400 Mesh) en éluant par un mélange de cyclohexane et d'acétate d'éthyle (90-10 en volumes), 4,58 g (64%) de 2-benzyl-9-(3-bromophényl)-4,9-éthano-2,3,3a,4,9,9a-hexahydro-1H-benzo[f]isoindole-3a-carboxylate de méthyle-(3aRS,4SR,9SR,9aRS), sous forme d'une poudre beige, dont les caractéristiques sont les suivantes :

- point de fusion = 74°C
- spectre de masse (IE) : M/Z = 502 ($M^+$).

Etape C

En opérant comme à l'étape B de l'exemple 47, mais à partir de 1,88 g de 2-benzyl-9-(3-bromophényl)-4,9-éthano-2,3,3a,4,9,9a-hexahydro-1H-benzo[f]isoindole-3a-carboxylate de méthyle-(3aRS,4SR,9SR,9aRS) et de 0,6 $cm^3$ de chloroformiate de vinyle pendant deux heures à température ambiante dans 15 $cm^3$ de dichlorométhane, puis en reprenant le concentrat dans 30 $cm^3$ d'une solution 2M de gaz chlorhydrique dans le méthanol au reflux pendant trois heures, on obtient, après cristallisation dans l'oxyde de diisopropyle, 1,72 g (76%) de chlorhydrate de 9-(3-bromophényl)-4,9-éthano-2,3,3a,4,9,9a-hexahydro-1H-benzo[f]isoindole-3a-carboxylate de méthyle-(3aRS,4SR,9SR,9aRS), sous forme de cristaux blancs dont la caractéristique est la suivante :

- spectre de masse (IE) : M/Z = 412 ($M^+$).

Etape D

En opérant comme à l'étape E de l'exemple 5, mais à partir de 1,72 g de chlorhydrate de 9-(3-bromophényl)-4,9-éthano-2,3,3a,4,9,9a-hexahydro-1H-benzo[f]isoindole-3a-carboxylate de méthyle-(3aRS,4SR,9SR,9aRS), de 0,68 g d'acide 2-(2-méthoxyphényl)propènoique, de 0,73 g de chlorhydrate de 1-éthyl-3-[(3-diméthylamino)propyl] carbodiimide, de 50 mg d'hydrate de N-1-hydroxybenzotriazole et de 0,54 $cm^3$ de triéthylamine dans 15 $cm^3$ de dichlorométhane pendant dix huit heures à température ambiante, on obtient, après purification sur gel de silice (230-400 Mesh) en éluant par un mélange de cyclohexane et d'acétate d'éthyle (70-30 en volumes), 0,58 g (27%) de 9-(3-bromophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS), sous forme d'une poudre beige-clair dont les caractéristiques sont les suivantes :

- point de fusion = 184-5°C
- spectre de masse (IE) : M/Z = 572 ($M^+$).

**EXEMPLE 84**

Préparation de l'acide 9-(3-bromophényl)-4,9-éthano-2-[2-(2-méthoxyphényl)propènoyl]-2,3,3a,4,9,9,9a-hexahydro-1H-benzo[f]isoindole-3a-carboxylique-(3aRS,4SR,9SR,9aRS)

En opérant comme à l'exemple 2, mais à partir de 0,50 g de 9-(3-bromophényl)-4,9-éthano-2-[2-(2-méthoxyphényl)propènoyl]-2,3,3a,4,9,9a-hexahydro -1H-benzo[f]isoindole-3a-carboxylate de méthyle-(3aRS,4SR,9SR,9aRS) au reflux pendant quatre heures dans 70 $cm^3$ d'une solution aqueuse décinormale d'hydroxyde de sodium et 70 $cm^3$ de méthanol, on obtient, après purification par recristallisation dans un mélange d'eau et d'éthanol (90-10 en volumes), 170 mg (35%) d'acide 9-(3-bromophényl)-4,9-éthano-2-[2-(2-méthoxyphényl)propènoyl]-2,3,3a,4,9,9a-hexahydro-1H-benzo[f]isoindole-3a-carboxylique-(3aRS,4SR,9SR,9aRS), sous forme d'un solide blanc dont les caractéristiques sont les suivantes :

- point de fusion = 164°C
- spectre de masse (IE) : M/Z = 558 ($M^+$).

**EXEMPLE 85**

Préparation du 4,9-éthano-9-(3-fluorophényl)-2-[2-(2-méthoxyphényl)propènoyl]-2,3,3a,4,9,9a-hexahydro-1H-benzo [f]isoindole-3a-carboxylate de méthyle-(3aRS,4SR,9SR,9aRS)

Etape A

En opérant comme à l'étape C de l'exemple 1, mais à partir de 2 g de magnésium en tournures, de 9,1 $cm^3$ de 3-fluoro-bromobenzène, dans 60 $cm^3$ d'oxyde de diéthyle, puis dans 20 $cm^3$ de toluène, puis de 15 g de 2-benzyl-7-oxo-4-phényloctahydroisoindole-3a-carboxylate de méthyle-(3aRS,4SR,7aRS) dans 60 $cm^3$ de toluène, on obtient 21,29 g de 2-benzyl-7-(3-fluorophényl)-7-hydroxy-4-phényloctahydroisoindole-3a-carboxylate de méthyle-(3aRS, 4SR,9RS,9aRS), impurs, sous forme d'une huile brune utilisée telle quelle à l'étape suivante, dont la caractéristique est la suivante :

- spectre de masse (DCI) : M/Z = 460 ($M+H^+$).

Etape B

En opérant comme à l'étape D de l'exemple 1, mais à partir de 21,29 g de 2-benzyl-7-(3-fluorophényl)-7-hydroxy-4-phényloctahydroisoindole-3a-carboxylate de méthyle-(3aRS,4SR,9RS,9aRS) et de 40 $cm^3$ d'acide trifluorométhanesulfonique a 99% dans 300 $cm^3$ de dichlorométhane, sous atmosphère d'argon pendant quarante trois heures à une température voisine de 20°C, on obtient 14,38 g de 2-benzyl-4,9-éthano-9-(3-fluorophényl)-2,3,3a,4,9,9a-hexahydro-1H-benzo[f]isoindole-3a-carboxylate de méthyle-(3aRS,4SR,9SR,9aRS), impurs, sous forme d'une huile brune utilisée telle quelle pour l'étape suivante, dont la caractéristique est la suivante :

- spectre de masse (IE) : M/Z = 441 ($M^+$).

Etape C

En opérant comme à l'étape E de l'exemple 1, mais à partir de 14,38 g de 2-benzyl-4,9-éthano-9-(3-fluorophényl)-2,3,3a,4,9,9a-hexahydro-1H-benzo[f]isoindole-3a-carboxylate de methyle-(3aRS,4SR,9SR,9aRS), de 5,53 g de formiate d'ammonium et de 1,8 g de palladium sur charbon à 10 % (p/p) dans 200 $cm^3$ de méthanol, pendant seize heures au reflux, on obtient 10,36 g de 4,9-éthano-9-(3-fluorophényl)-2,3,3a,4,9,9a-hexahydro-1H-benzo[f]isoindole-3a-carboxylate de méthyle-(3aRS,4SR,9SR,9aRS) impurs, sous forme d'une huile brune utilisée telle quelle pour l'étape suivante, dont la caractéristique est la suivante :

- spectre de masse (IE) : M/Z = 351 ($M^+$).

Etape D

En opérant comme à l'étape G de l'exemple 1, mais à partir d'une solution de 5,66 g d'acide 2-(2-méthoxyphényl)propènoïque dans 50 $cm^3$ de dichlorométhane contenant 2 gouttes de N,N-diméthylformamide de 2,75 $cm^3$ de chlorure d'oxalyle, pendant deux heures, et d'une solution de 10,36 g de 4,9-éthano-9-(3-fluorophènyl)-2,3,3a,4,9,9a-hexahydro-1H-benzo[f]isoindole-3a-carboxylate de méthyle-(3aRS,4SR,9SR,9aRS) obtenus à l'étape précédente, et de 7,3 $cm^3$ de triéthylamine dans 100 $cm^3$ de dichlorométhane, puis, on obtient, après cristallisation dans l'éther de pétrole (40-60°C), 3,59 g de 4,9-éthano-9-(3-fluorophényl)-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS), sous forme d'une poudre beige, dont les caractéristiques sont les suivantes

- point de fusion = 151°C.
- Spectre de R.M.N. $^1H$ (250MHz, $(CD_3)_2SO$, d en ppm) : 1,43-1,67-2,00 et 2,14(4mts, 1H : $CH_2CH_2$): de 3,30 à 3,45 (mt, 3H : $CH_2$ en 1 et CH en 9a) ; 3,46 (mt, 1H : CH en 4); 3,54 (s,3H:$COOCH_3$): 3,61 et 4,09 (respectivement d et d large, J=12,5Hz, 1H chacun : $CH_2$ en 3); 3,71 (s,3H:$ArOCH_3$): 5,5 et 5,68 (2s,1H chacun : $CH_2$; 6,41 (d large, J=7,5Hz, 1H : H en 8); de 6,90 à 7,40 (mt, 10H : H en 6, en 7 et H aromatiques du 3-fluorophényl); 7,54 (q large,J =7,5Hz, 1H : H en 5 du 3-fluorphényl)
- spectre de masse (IE) : M/Z = 511 ($M^+$).

**EXEMPLE 86**

Préparation de l'acide 4,9-éthano-9-(3-fluorophényl)-2-[2-(2-méthoxyphényl)propènoyl])-2,3,3a,4,9,9a-hexahydro-1H-benzo[f]-isoindole-3a-carboxylique-(3aRS, 4SR, 9SR, 9aRS)

En opérant comme à l'exemple 2, mais à partir de 1,02 g de-4,9-éthano-9-(3-fluorophényl)-2-[2-(2-méthoxyphényl)propènoyl]-2,3,3a,4,9,9a-hexahydro-1H-benzo[f]isoindole-3a-carboxylate de méthyle-(3aRS,4SR,9SR, 9aRS), obtenu comme à l'étape précédente et de 24 $cm^3$ d'une solution aqueuse normale d'hydroxyde de sodium dans 30 $cm^3$ de dioxane, pendant dix huit heures au reflux, on obtient 0,71 g d'acide 4,9-éthano-9-(3-fluorophényl)-2-[2-(2-méthoxyphényl)propènoyl])-2,3,3a,4,9,9a-hexahydro-1H-benzo[f]isoindole-3a-carboxylique-(3aRS,4SR,9SR,9aRS), sous forme d'une poudre blanche, dont les caractéristiques sont les suivantes :

- point de fusion = 161°C.
- spectre de masse (IE) : M/Z = 497 ($M^+$).

**EXEMPLE 87**

Préparation du 4,9-éthano-9-(3-fluorophényl)-2-[2-(2-méthoxyphényl)propènoyl)]-2,3,3a,4,9,9a-hexahydro-1H-benzo[f]isoindole-3a-N-(3-pyridylméthyl)carboxamide-(3aRS,4SR,9SR,9aRS)

En opérant comme à l'exemple 42, mais à partir de 0,52 g d'acide 4,9-éthano-9-(3-fluorophényl)-2[2-(2-méthoxyphényl)propénoyl])-2,3,3a,4,9,9a-hexahydro-1H-benzo[f]isoindole-3a-carboxylique-(3aRS, 4SR, 9SR, 9aRS), de 0,128 $cm^3$ de 3-(aminométhyl)pyridine, de 0,24 g de chlorhydrate de l-éthyl-3-[3-diméthylamino)propyl]-carbodiimide et de 85 mg d'hydrate de N-1-hydroxybenzotriazole dans 30 $cm^3$ de dichlorométhane, on obtient, après seize heures à une température voisine de 20°C, 183,6 mg de 4,9-éthano-9-(3-fluorophényl)-2-[2-(2-méthoxyphényl)propènoyl)]-2,3,3a,4,9,9a-hexahydro-1H-benzo[f]isoindole-3a-N-(3-pyridylméthyl)carboxamide-(3aRS,4SR,9SR,9aRS),

sous forme d'une poudre écrue, dont les caractéristiques sont les suivantes :

- point de fusion = 223°C.
- spectre de masse (IE) : M/Z = 587 ($M^+$).

**EXEMPLE 88**

Préparation du 9-(3-chlorophényl)-4,9-éthano-2-[2-(2-méthoxyphényl)propenoyl]-2,3,3a,4,9,9a-hexahydro-1H-benzo[f]isoindole-3a-carboxylate de méthyle-(3aRS,4SR,9SR,9aRS)

Etape A

En opérant comme à l'étape C de l'exemple 1, mais à partir de 2 g de magnésium en tournures, de 9,75 $cm^3$ de 3-chloro-bromobenzène, dans 60 $cm^3$ d'oxyde de diéthyle, puis dans 20 $cm^3$ de toluène, puis de 15 g de 2-benzyl-7-oxo-4-phényloctahydroisoindole-3a-carboxylate de méthyle-(3aRS,4SR,7aRS) dans 60 $cm^3$ de toluène, on obtient 31,2 g de 2-benzyl-7-(3-chlorophényl)-7-hydroxy-4-phényloctahydroisoindole-3a-carboxylate de méthyle-(3aRS,4SR,9RS,9aRS), impurs utilisés tels quels pour l'étape suivante, sous forme d'une huile orangée, dont la caractéristique est la suivante :

- spectre de masse (DCl) : M/Z = 476 ($M+H^+$).

Etape B

En opérant comme à l'étape D de l'exemple 1, mais à partir de 31,2 g de 2-benzyl-7-(3-chlorophényl)-7-hydroxy-4-phényloctahydroisoindole-3a-carboxylate de méthyle-(3aRS,4SR,7aRS), obtenus à l'étape précédente et de 40 $cm^3$ d'acide trifluorométhanesulfonique à 99% dans 300 $cm^3$ de dichlorométhane sous atmosphère d'argon, pendant quatre jours à une température voisine de 20°C, on obtient 23,88 g de 2-benzyl-9-(3-chlorophényl)-4,9-éthano-2,3,3a,4,9,9a-hexahydro-1H-benzo[f]isoindole-3a-carboxylate de méthyle-(3aRS,4SR,9SR,9aRS) impurs, sous forme d'une huile utilisée telle quelle pour l'étape suivante, dont la caractéristique est la suivante :

- spectre de masse (IE) : M/Z = 457 ($M^+$).

Etape C

En opérant comme à l'étape B de l'exemple 47, mais à partir de 23,88 g de 2-benzyl-9-(3-chlorophényl)-4,9-éthano-2,3,3a,4,9,9a-hexahydro-1H-benzo[f]isoindole-3a-carboxylate de méthyle-(3aRS,4SR,9SR,9aRS), impurs, de 5,4 $cm^3$ de chloroformiate de vinyle et de 11,5 g de carbonate de potassium, dans 200 $cm^3$ de dichlorométhane, pendant seize heures a une température voisine de 20°C, on obtient 24,4 g de-4,9-éthano-9-3-chlorophényl)-2-vinyloxycarbonyl-2,3,3a,4,9,9a-hexahydro-1H-benzo[f]isoindole-3a-carboxylate de méthyle-(3aRS,4SR,9SR,9aRS), impurs, sous forme d'une huile brune utilisée telle quelle pour l'étape suivante, dont la caractéristique est la suivante :

- spectre de masse (IE) : M/Z = 437 ($M^+$).

Après reprise de cette huile pendant dix huit heures dans une solution 6N de gaz chlorhydrique dans le dioxane, on obtient 18,28 g de chlorhydrate de-7-(3-chlorophényl)-4,9-éthano-2,3,3a,4,9,9a-hexahydro-1H-benzo[f]isoindole-3a-carboxylate de méthyle-(3aRS,4SR,9SR,9aRS) impurs, sous forme d'une huile brune épaisse, utilisable telle quelle pour l'étape suivante, dont la caractéristique est la suivante :

- spectre de masse (1E) : M/Z = 368 ($M^+$).

Etape D

En opérant comme à l'étape G de l'exemple 1, mais à partir d'une solution de 8,76 g d'acide 2-(2-méthoxyphényl)propènoïque dans 50 $cm^3$ de dichlorométhane contenant 2 gouttes de N,N-diméthylformamide et de 4,21 $cm^3$ de chlorure d'oxalyle, pendant deux heures trente minutes, puis d'une solution de 18,5 g de chlorhydrate de-7-(3 -chlorophényl)-4,9-éthano-2,3,3a,4,9,9a-hexahydro-1H-benzo[f]isoindole-3a-carboxylate de méthyle-(3aRS, 4SR, 9SR,9aRS), obtenus à l'étape précédente, et de 11,5 $cm^3$ de triéthylamine dans 100 $cm^3$ de dichlorométhane, puis coulée après quarante deux heures de 100 $cm^3$ d'eau, on obtient 0,95 g de 9-(3-chlorophényl)-4,9-éthano-2-[2-(2-méthoxyphényl)propènoyl]-2,3,3a,4,9,9a-hexahydro-1H-benzo[f]isoindole-3a-carboxylate de méthyle, (3aRS,4SR,9SR, 9aRS), sous forme d'une poudre beige, dont les caractéristiques sont les suivantes, dont les caractéristiques sont les suivantes :

- point de fusion = 159°C.
- spectre de masse (1E) : M/Z = 527 ($M^+$).

**EXEMPLE 89**

Préparation de l'acide 9-(3-chlorophényl)-4,9-éthano-2-[2-(2-méthoxyphényl) propènoyl]-2,3,3a,4,9,9a-hexahydro-1H-benzo[f]isoindole-3a-carboxylique-(3aRS,4SR,9SR,9aRS)

En opérant comme à l'exemple 2, mais à partir de 795 mg de-9-(3-chlorophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR, 9aRS), obtenus comme à l'étape précédente et de 30 $cm^3$ d'une solution aqueuse normale d'hydroxyde de sodium dans 30 $cm^3$ de dioxane, pendant dix huit heures au reflux, on obtient 0,77 g d'acide 9-(3-chlorophényl)-4,9-éthano-2-[2-(2-méthoxyphényl)propènoyl]-2,3,3a,4,9,9a-hexahydro-1H-benzo[f]isoindole-3a-carboxylique-(3aRS,4SR,9SR, 9aRS), sous forme d'une poudre blanche, dont les caractéristiques sont les suivantes :

- point de fusion = 170°C
- spectre de masse (IE) : M/Z = 513 ($M^+$)

**EXEMPLE 90**

Préparation du 9-(3-chlorophényl)-4,9-éthano-2-[2-(2-méthoxyphényl)propènoyl)]-2,3,3a,4,9,9a-hexahydro-1H-benzo [f]isoindole-3a-N-(3-pyridylméthyl)carboxamide-(3aRS,4SR,9SR,9aRS)

En opérant comme à l'exemple 42, mais à partir de 0,46 g d'acide 9-(3-chlorophényl)-4,9-éthano-2-[2-(2-méthoxyphènyl)propènoyl]-2,3,3a,4,9,9a-hexahydro-1H-benzo[f]isoindole-3a-carboxylique-(3aRS,4SR,9SR,9aRS), de 0,11 $cm^3$ de 3-(aminométhyl)pyridine, de 0,21 g de chlorhydrate de 1-éthyl-3-[3-diméthylamino)propyl]carbodiimide et de 70 mg d'hydrate de N-1-hydroxybenzotriazole dans 30 $cm^3$ de dichlorométhane, on obtient, après seize heures à une température voisine de 20°C, 269,8 mg de 9-(3-chlorophényl)-4,9-éthano-2-[2-(2-méthoxyphényl)propenoyl)]-2,3,3a,4,9,9a-hexahydro-1H-benzo[f]isoindole-3a-N-(3-pyridylméthyl)carboxamide-(3aRS,4SR,9SR,9aRS), sous forme d'une poudre blanc cassé, dont les caractéristiques sont les suivantes :

- point de fusion = 244°C.
- spectre de masse (IE) : M/Z = 603 ($M^-$).

**EXEMPLE 91**

Préparation du 9-(3-N,N-diméthylaminophényl)-4,9-éthano-2-[2-(2-méthoxyphényl)propènoyl]-2,3,3a,4,9,9,9a-hexahydro-1H-benzo[f]isoindole-3a-carboxylate de méthyle-(3aRS, 4SR,9SR,9aRS)

Etape A

En opérant comme à l'étape C de l'exemple 45, mais à partir de 2,5 g de 2-benzyl-7-iodo-4-phényl-2,3,3a, 4,5,7a-hexahydroisoindole-3a-carboxylate de méthyle-(3aRS,4SR,7aRS), de 0,30 g de tétrakis(triphénylphosphine) palladium, de 0,77 g d'acide 3-N,N-diméthylaminophénylboronique, obtenu sous forme d'anhydride trimérique, et 20 $cm^3$ d'une solution aqueuse 2M de carbonate de sodium au reflux pendant sept heures dans 20 $cm^3$ de toluène et 10 $cm^3$ de méthanol, on obtient, après purification par flash-chromatographie sur gel de silice (230-400 Mesh) en éluant par un mélange de cyclohexane et d'acétate d'éthyle (90-10 en volumes), 2,09 g (85%) de 2-benzyl-7-(3-N,N-diméthylaminophényl)-4-phényl-2,3,3a,4,5,7a-hexahydro-1H-isoindole-3a-carboxylate de méthyle-(3aRS,4SR,7aRS), sous forme d'un solide orangé pâteux utilisé tel quel à l'étape suivante, dont la caractéristique est la suivante :

- spectre de masse (IE) : M/Z = 466 ($M^+$).

Etape B

On opère comme à l'étape B de l'exemple 39, mais à partir de 2,09 g de 2-benzyl-7-(3-N,N-diméthylaminophényl)-4-phényl-2,3,3a,4,5,7a-hexahydro-1H-isoindole-3a-carboxylate de méthyle-(3aRS,4SR,7aRS) et de 20 $cm^3$ d'acide trifluorométhanesulfonique pur pendant quatre jours à température ambiante. Après neutralisation avec une solution aqueuse d'hydroxyde de sodium à 30% en présence de dichlorométhane, la phase organique est décantée puis lavée à l'eau et séchée sur sulfate de magnésium puis agitée pendant une heure avec 20 g de gel de silice (230-400 Mesh). On obtient alors, après concentration du solvant sous pression réduite, 1,74 g (83%) de 2-benzyl-9-(3-N,N-diméthylaminophényl)-4,9-éthano-2,3,3a,4,9,9a-hexahydro-1H-benzo[f]isoindole-3a-carboxylate de méthyle-(3aRS,4SR,9SR,9aRS), sous forme d'un solide jaune pâteux utilisé tel quel à l'étape suivante, dont la caractéristique est la suivante :

- spectre de masse (IE) : M/Z = 466 ($M^+$).

Etape C

On opère comme à l'étape B de l'exemple 47, mais à partir de 2,21 g de 2-benzyl-9-(3-N,N-diméthylaminophényl)-4,9-éthano-2,3,3a,4,9,9a-hexahydro-1H-isoindole-3a-carboxylate de méthyle-(3aRS,4SR,9SR,9aRS), de 3 $cm^3$ de chloroformiate de vinyle et de 4,6 $cm^3$ de triéthylamine pendant sept heures à température ambiante dans 20 $cm^3$ de dichlorométhane. Après hydrolyse, la phase organique est décantée puis concentrée sous pression réduite. On obtient alors, après purification par flash-chromatographie sur gel de silice (230-400 Mesh) en éluant par un mélange de cyclohexane et d'acétate d'éthyle (85-15 en volumes), 0,27 g (27%) de 9-(3-N,N-diméthylaminophényl)-4,9-éthano-2-vinyloxycarbonyl-2,3,3a,4,9,9a-hexahydro-1H-isoindole-3a-carboxylate de méthyle-(3aRS,4SR,7aRS), qui est mis en solution dans $cm^3$ de méthanol et chauffé au reflux pendant cinq heures en présence de 5 $cm^3$ d'une solution 5M de gaz chlorhydrique dans le dioxane. On obtient ainsi, après cristallisation dans l'oxyde de diisopropyle, 0,68 g (98%) de dichlorhydrate de 9-(3-N,N-diméthylaminophényl)-4,9-éthano-2,3,3a,4,9,9a-hexahydro-1H-isoindole-3a-carboxylate de méthyle-(3aRS,4SR,7aRS), sous forme d'un solide blanc, dont la caractéristique est la suivante :

- spectre de masse (IE) : M/Z = 376 ($M^+$).

Etape D

En opérant comme à l'étape E de l'exemple 5, mais à partir de 0,68 g de dichlorhydrate de 9-(3-N,N-diméthylaminophényl)-4,9-éthano-2,3,3a,4,9,9a-hexahydro-1H-benzo[f]isoindole-3a-carboxylate de méthyle-(3aRS,4SR,9SR,9aRS), de 0,29 g d'acide 2-(2-méthoxyphényl)propènoique, de 0,38 g de chlorhydrate de 1-éthyl-3-[(3-diméthylamino)propyl]carbodiimide, de 20 mg d'hydrate de N-1-hydroxybenzotriazole et de 0,42 $cm^3$ de triéthylamine dans 15 $cm^3$ de dichlorométhane pendant vingt quatre heures à température ambiante, on obtient, après purification par flash-chromatographie sur gel de silice (230-400 Mesh) en éluant par un mélange de cyclohexane et d'acétate d'éthyle (70-30 en volumes) puis recristallisation au pentane, 0,27 g (33%) de 9-(3-N,N-diméthylaminophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS), sous forme d'une poudre blanche, dont les caractéristiques sont les suivantes :

- point de fusion = 95°C
- spectre de masse (IE) : M/Z = 536 ($M^+$).

**EXEMPLE 92**

Préparation de l'acide 9-(3-N,N-diméthylaminophényl)-4,9-éthano-2-[2-(2-méthoxyphényl)propènoyl]-2,3,3a,4,9,9a-hexahydro-1H-benzo[f]isoindole-3a-carboxylique-(3aRS,4SR,9SR,9aRS)

En opérant comme à l'exemple 2, mais à partir de 0,23 g de 9-(3-N,N-diméthylaminophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS) au reflux pendant cinq heures dans 2 $cm^3$ d'une solution aqueuse normale d'hydroxyde de sodium et 5 $cm^3$ d'éthanol, on obtient, après purification par recristallisation dans un mélange de dichlorométhane et de pentane (50-50 en volumes), 120 mg (55%) d'acide 9-(3-N,N-diméthylaminophényl)-4,9-éthano-2-[2-(2-méthoxyphényl)propènoyl]-2,3,3a,4,9,9a-hexahydro-1H-benzo[f]isoindole-3a-carboxylique-(3aRS,4SR,9SR,9aRS), sous forme d'un solide blanc dont les caractéristiques sont les suivantes

- point de fusion = 194°C
- spectre de masse (IE) : MZ = 522 ($M^+$).

**EXEMPLE 93**

Préparation du chlorhydrate de 9-(3-aminophényl)-4,9-éthano-2-[2-(2-méthoxyphényl)propènoyl]-2,3,3a,4,9,9,9a-hexahydro-1H-benzo[f]isoindole-3a-carboxylate de méthyle-(3aRS,4SR,9SR,9aRS)

Etape A

En opérant comme à l'étape C de l'exemple 45, mais à partir de 2,5 g de 2-benzyl-7-iodo-4-phényl-2,3,3a,4,5,7a-hexahydroisoindole-3a-carboxylate de méthyle-(3aRS,4SR,7aRS), de 0,30 g de tétrakis(triphénylphosphine) palladium, de 0,72 g d'acide 3-aminophénylboronique et de 20 $cm^3$ d'une solution aqueuse 2M de carbonate de sodium au reflux pendant sept heures dans 20 $cm^3$ de toluène et 10 $cm^3$ de méthanol, on obtient, après purification par flash-chromatographie sur gel de silice (230-400 Mesh) en éluant par un mélange de cyclohexane et d'acétate d'éthyle (80-20 en volumes), 1,60 g (70%) de 7-(3-aminophényl)-2-benzyl-4-phényl-2,3,3a,4,5,7a-hexahydro-1H-isoindole-3a-carboxylate de méthyle-(3aRS,4SR,7aRS), sous forme d'un solide orangé utilisé tel quel à l'étape suivante, dont la caractéristique est la suivante :

- spectre de masse (IE) : M/Z = 438 ($M^+$).

Etape B

On opère comme à l'étape B de l'exemple 39, mais à partir de 0,20 g (0,4 mmol) de 7-(3-aminophényl)-2-benzyl-4-phényl-2,3,3a,4,5,7a-hexahydro-1H-isoindole-3a-carboxylate de méthyle-(3aRS,4SR,7aRS) et de 5 $cm^3$ d'acide trifluorométhanesulfonique pur pendant trois jours à température ambiante. Après neutralisation avec une solution aqueuse d'hydroxyde de sodium à 30% en présence de dichlorométhane, la phase organique est décantée puis lavée à l'eau et séchée sur sulfate de magnésium puis agitée pendant une heure avec 20 g de gel de silice (230-400 Mesh). On obtient alors, après concentration du solvant sous pression réduite, 0,16 g (80%) de 2-benzyl-9-(3-aminophényl)-4,9-éthano-2,3,3a,4,9,9a-hexahydro-1H-benzo[f]isoindole-3a-carboxylate de méthyle-(3aRS,4SR,9SR,9aRS), sous forme d'une huile brune utilisée telle quelle à l'étape suivante, dont la caractéristique est la suivante :

- spectre de masse (IE) : M/Z = 438 ($M^+$).

Etape C

En opérant comme à l'étape E de l'exemple 45, mais à partir de 1,5 g de 9-(3-aminophényl)-2-benzyl-4,9-éthano-2,3,3a,4,9,9a-hexahydro-1H-isoindole-3a-carboxylate de méthyle-(3aRS,4SR,9RS,9aRS), de 0,6 g de palladium sur charbon à 10% (p/p) dans 100 $cm^3$ de méthanol et 3,5 $cm^3$ de gaz chlorhydrique en solution 1M dans le méthanol pendant huit heures à 50°C sous atmosphère d'hydrogéne, on obtient, après cristailisation dans l'oxyde de diisopropyle, 1,27 g (79%) de dichlorhydrate de 9-(3-aminophényl)-4,9-éthano-2,3,3a,4,9,9a-hexahydro-1H-isoindole-3a-carboxylate de méthyle-(3aRS,4SR,9RS,9aRS), sous forme d'un solide blanc, dont les caractéristiques sont les suivantes :

- spectre de masse (IE) : M/Z = 348 ($M^+$).

Etape D

En opérant comme à l'étape G de l'exemple 1, mais à partir de 0,74 g de dichlorhydrate de 9-(3-aminophényl)-4,9-éthano-2,3,3a,4,9,9a-hexahydro-1H-benzo[f] isoindole-3a-carboxylate de méthyle-(3aRS,4SR,9SR,9aRS), de 0,41 g d'acide 2-(2-methoxyphenyl)propènoique, de 0,22 $cm^3$ de chlorure d'oxalyle et de 0,76 $cm^3$ de triéthylamine dans 10 $cm^3$ de dichlorométhane pendant vingt heures à température ambiante, on obtient, après purification par flash-chromatographie sur gel de silice (230-400 Mesh) en éluant par un mélange de cyclohexane et d'acétate d'éthyle (50-50 en volumes) puis recristallisation à l'oxyde diisopropyle, 0,20 g (21%) de chlorhydrate de 9-(3-aminophényl)-4,9-éthano-2-[2-(2-méthoxyphényl)propènoyl]-2,3,3a,4,9,9a-hexahydro-1H-benzo[f]isoindole-3a-carbaxylate de méthyle-(3aRS,4SR,9SR,9aRS), sous forme d'une poudre blanche, dont les caractéristiques sont les suivantes :

- point de fusion = 186°C

- spectre de masse (IE) : M/Z = 508 ($M^+$).

**EXEMPLE 94**

Préparation du 9-(4-N,N-diméthylaminophényl)-4,9-éthano-2-[2-(2-méthoxyphényl) propènoyl]-2,3,3a,4,9,9,9a-hexahydro-1H-benzo[f]isoindole-3a-carboxylate de méthyle-(3aRS,4SR,9SR,9aRS)

Etape A

En opérant comme à l'étape C de l'exemple 45, mais à partir de 1,15 g de 2-benzyl-7-iodo-4-phényl-2,3,a,4,5,7a-hexahydroisoindole-3a-carboxylate de méthyle-(3aRS,4SR,7aRS), de 0,12 g de tétrakis(triphénylphosphine) palladium, de 0,44 g d'acide 4-N,N-diméthylaminophénylboronique et de 15 $cm^3$ d'une solution aqueuse 2M de carbonate de sodium au reflux pendant quatre heures dans 20 $cm^3$ de toluène et 10 $cm^3$ de méthanol, on obtient, après purification par flash-chromatographie sur gel de silice (230-400 Mesh) en éluant par un mélange de cyclohexane et d'acétate d'éthyle (80-20 en volumes), 0,93 g (82%) de 2-benzyl-7-(4-N,N-diméthylaminophényl)-4-phényl-2,3,3a,4,5,7a-hexahydro-1H-isoindole-3a-carboxylate de méthyle-(3aRS,4SR,7aRS), sous forme d'une huile orangée utilisée telle quelle à l'étape suivante, dont la caractéristique est la suivante :

- spectre de masse (IE) : M/Z = 466 ($M^+$).

Etape B

On opère comme à l'étape B de l'exemple 39, mais à partir de 0,53 g de 2-benzyl-7-(4-N,N'-diméthylaminophényl)-4-phényl-2,3,3a,4,5,7a-hexahydro-1H-isoindofe-3a-carboxylate de methyle-(3aRS,4SR,7aRS) et de 10 $cm^3$ d'acide trifluorométhanesulfonique pur pendant deux jours à température ambiante. Après neutralisation avec une solution aqueuse d'hydroxyde de sodium a 30% en présence de dichlorométhane, la phase organique est décantée puis lavée à l'eau et séchée sur sulfate de magnésium. Après concentration du solvant sous pression réduite, le résidu est purifie par flash-chromatographie sur gel de silice (230-400 Mesh) en éluant avec un mélange de cyclohexane et d'acétate d'éthyle (90-10 en volumes), on obtient alors 0,34 g (64%) de 2-benzyl-9-(4-N,N-diméthylaminophényl)-4,9-éthano-2,3,3a,4,9,9a-hexahydro-1H-benzo[f]isoindole-3a-carboxylate de méthyle-(3aRS,4SR,9SR,9aRS), sous forme d'une huile incolore, dont la caractéristique est la suivante :

- spectre de masse (IE) : M/Z = 466 ($M^+$).

Etape C

En opérant comme à l'étape E de l'exemple 45, mais à partir de 0,34 g de 2-benzyl-9-(4-N,N-diméthylaminophényl)-4,9-éthano-2,3,3a,4,9,9a-hexahydro-1H-isoindole-3a-carboxylate de méthyle-(3aRS,4SR,9RS,9aRS), de 0,15 g de palladium sur charbon à 10% (p/p) dans 20 $cm^3$ de méthanol, 10 $cm^3$ de chloroforme et 1,5 $cm^3$ de gaz chlorhydrique en solution 1M dans le méthanol pendant cinq heures à 40°C sous atmosphère d'hydrogène, on obtient, après cristallisation dans l'oxyde de diisopropyle, 0,31 g (96%) de dichlorhydrate de 9-(4-N,N-diméthylaminophényl)-4,9-éthano-2,3,3a,4,9,9a-hexahydro-1H-isoindole-3a-carboxylate de méthyle-(3aRS,4SR,9RS,9aRS), sous forme d'un solide vert clair, dont la caractéristique est la suivante :

- spectre de masse (IE) : M/Z = 376 ($M^+$).

Etape D

En opérant comme à l'étape G de l'exemple 1, mais à partir de 0,30 g de dichlorhydrate de 9-(4-N,N-diméthylaminophényl)-4.9-éthano-2,3,3a,4,9,9a-hexahydro-1H-benzo[f] isoindole-3a-carboxylate de méthyle-(3aRS,4SR,9SR,9aRS), de 0,13 g d'acide 2-(2-méthoxyphényl)propènoique, de 0,07 $cm^3$ de chlorure d'oxalyle et de 0,3 $cm^3$ de triéthylamine dans 5 $cm^3$ de dichlorométhane pendant dix huit heures à température ambiante, on obtient, après purification sur gel de silice (230-400 Mesh) en éluant par un mélange de cyclohexane et d'acétate d'éthyle et d'une solution d'ammoniaque à 28% (60-40-0.2 en volumes), 0,18 g (50%) de 9-(4-N,N-diméthylaminophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS), sous forme d'une poudre blanche, dont les caractéristiques sont les suivantes :

- point de fusion = 86-90°C

- spectre de masse (IE) : M/Z = 536 ($M^+$).

**EXEMPLE 95**

Préparation de l'acide 9-(4-cyanophényl)-4,9-éthano-2-[2-(2-méthoxyphényl)propènoyl]-2,3,3a,4,9,9a-hexahydro-1H-benzo[f]isoindole-3a-carboxylique-(3aRS,4SR,9SR,9aRS)

Etape A

A une solution, maintenue sous atmosphère d'argon, de 5,02 g de 2-benzyl-9-(4-bromophényl)-4,9-éthano-2,3,3a,4,9,9a-hexahydro-1H-benzo[f]isoindole-3a-carboxylate de méthyle-(3aRS,4SR,9SR,9aRS), obtenus à l'étape B de l'exemple 49, dans 50 $cm^3$ de diméthylformamide, on ajoute 1,15 g de tétrakis(triphénylphosphine)palladium et 5,84 g de cyanure de zinc et on porte au reflux pendant cinq heures, puis on agite pendant dix huit heures supplémentaires à température ambiante toujours sous atmosphère d'argon. Apres addition d'eau et d'acétate d'éthyle, on décante la phase organique qui est lavée à l'eau, séchée sur sulfate de magnésium et concentrée sous pression réduite. Le résidu est purifié par flash-chromatographie sur gel de silice (230-400 Mesh) en éluant par un mélange de cyclohexane et d'acétate d'éthyle (95-5 en volumes), on obtient ainsi 3,09 g (69%) de 2-benzyl-9-(4-cyanophényl)-4,9-éthano-2,3,3a,4,9,9a-hexahydro-1H-benzo[f]isoindole-3a-carboxylate de méthyle-(3aRS,4SR,9SR,9aRS), sous forme d'une poudre blanche, dont les caractéristiques sont les suivantes :

- point de fusion = 158°C
- spectre de R.M.N. $^1H$ (300 MHz, $CDCl_3$, d en ppm) ; 1,46 - 1,70 et 2,49 (3 mts, respectivement 1H - 1H et 2H : $CH_2CH_2$) ; de 2,25 à 2,40 et 2,63 (respectivement mt et d, J = 10 Hz, 1H chacun : $CH_2$ en 1) ; 2,31 et 3,21 (2 d, J = 10 Hz, 1H chacun : $CH_2$ en 3) ; 3,23 (mt, 1H : CH en 9a) ; 3,41 et 3,63 (2 d, J = 12,5 Hz, 1H chacun : $NCH_2Ar$) ; 3,45 (s large, 1H : CH en 4) ; 3,55 (s, 3H : $COOCH_3$) ; 6,43 (d, J = 7,5 Hz, 1H: H en 8); de 6,95 à 7,80 (mts, 12H : H en 5 - H en 6 - H en 7 - H aromatiques du 4-cyanophényl et H aromatiques du benzyle).
- spectre de masse (IE) : M/Z = 448 ($M^+$).

Etape B

On opère comme à l'étape B de l'exemple 47, mais à partir de 0,80 g de 2-benzyl-9-(4-cyanophényl)-4,9-éthano-2,3,3a,4,9,9a-hexahydro-1H-isoindole-3a-carboxylate de méthyle-(3aRS,4SR,9RS,9aRS) et de 1 $cm^3$ de chloroformiate de vinyle pendant vingt quatre heures à température ambiante dans 20 $cm^3$ de dichlorométhane. Après hydrolyse, la phase organique est décantée puis concentrée sous pression réduite. On obtient alors, après purification par flash-chromatographie sur gel de silice (230-400 Mesh) en éluant par un mélange de cyclohexane et d'acétate d'éthyle (90-10 en volumes), 0,69 g (91%) de 9-(4-cyanophényl)-4,9-éthano-2-vinyloxycarbonyl-2,3,3a,4,9,9a-hexahydro-1H-isoindole-3a-carboxylate de méthyle-(3aRS,4SR,7aRS), qui est mis en solution dans 20 $cm^3$ de méthanol et chauffé au reflux pendant cinq heures en présence de 5 $cm^3$ d'une solution 5M de gaz chlorhydrique dans le dioxane. On obtient ainsi après cristallisation dans l'oxyde de diisopropyle, 0,62 g (98%) de chlorhydrate de 9-(4-cyanophényl)-4,9-éthano-2,3,3a,4,9,9a-hexahydro-1H-isoindole-3a-carboxylate de méthyle-(3aRS,4SR,9RS,9aRS), sous forme d'un solide blanc, dont la caractéristique est la suivante :

- spectre de masse (IE) : M/Z = 358 ($M^+$).

Etape C

En opérant comme à l'étape G de l'exemple 1, mais à partir de 0,72 g de chlorhydrate de 9-(4-cyanophényl)-4,9-éthano-2,3,3a,4,9,9a-hexahydro-1H-benzo[f]isoindole-3a-carboxylate de méthyle-(3aRS,4SR,9SR,9aRS), de 0,32 g d'acide 2-(2-méthoxyphényl)propénoique, de 0,175 $cm^3$ de chlorure d'oxalyle et de 0.55 $cm^3$ de triéthylamine dans 20 $cm^3$ de dichlorométhane pendant dix huit heures à température ambiante, on obtient, après purification sur gel de silice (230-400 Mesh) en éluant par un mélange de cyclohexane et d'acétate d'éthyle (70-30 en volumes), 0,52 g (55%) de 9-(4-cyanophényl)-4,9-éthano-2-[2-(2-méthoxyphényl)propènoyl]-2,3.3a,4,9,9a-hexahydro-1H-benzo[f]isoindole-3a-carboxylate de méthyle-(3aRS,4SR, 9SR,9aRS), sous forme d'une poudre blanche, dont les caractéristiques sont les suivantes :

- point de fusion = 90-2°C

- spectre de masse (IE) : M/Z = 518 ($M^+$).

Etape D

En opérant comme à l'exemple 2, mais à partir de 0,50 g de 9-(4-cyanophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR, 9SR,9aRS) au reflux pendant cinq heures dans 1,1 $cm^3$ d'une solution aqueuse normale d'hydroxyde de sodium et 20 $cm^3$ d'éthanol, on obtient, après purification par flash-chromatographie sur gel de silice en éluant par un gradient de mélanges de cyclohexane et d'acétate d'éthyle (de 95-5 à 50-50 en volumes), 220 mg (45%) d'acide 9-(4-cyanophényl)-4,9-éthano-2-[2-(2-méthoxyphényl)propènoyl]-2,3,3a,4,9,9a-hexahydro-1H-benzo[f]isoindole-3a-carboxylique-(3aRS,4SR,9SR, 9aRS), sous forme d'un solide blanc dont les caractéristiques sont les suivantes :

- point de fusion = 182°C
- spectre de masse (IE) : M/Z = 504 ($M^+$).

**EXEMPLE 96**

Préparation du 9-(3-cyanophényl)-4,9-éthano-2-[2-(2-méthoxyphényl)propénoyl]-2,3,3a,4,9,9a-hexahydro-1H-benzo [f]isoindole-3a-carboxylate de méthyle-(3aRS,4SR,9SR,9aRS)

Etape A

En opérant comme à l'étape A de l'exemple 95, mais à partir de 2,69 g de 2-benzyl-9-(3-bromophényl)-4,9-éthano-2,3,3a,4,9,9a-hexahydro-1H-benzo[f]isoindole-3a-carboxylate de méthyle-(3aRS,4SR,9SR,9aRS), obtenus à l'étape B de l'exemple 83, de 0,62 g de tétrakis(triphénylphosphine)palladium et 3,14 g de cyanure de zinc dans 50 $cm^3$ de diméthylformamide au reflux pendant cinq heures, puis pendant dix huit heures supplémentaires à température ambiante sous atmosphère d'argon, on obtient, après purification par flash-chromatographie sur gel de silice (230-400 Mesh en éluant par un mélange de cyclohexane et d'acétate d'éthyle (90-10 en volumes), 2,0 g (83%) de 2-benzyl-9-(3-cyanophényl)-4,9-éthano-2,3,3a,4,9,9a-hexahydro-1H-benzo[f]isoindole-3a-carboxylate de méthyle-(3aRS,4SR,9SR,9aRS), sous forme d'une poudre blanche, dont les caractéristiques sont les suivantes :

- point de fusion = 88°C
- spectre de masse (IE) : M/Z = 448 ($M^+$).

Etape B

En opérant comme à l'étape B de l'exemple 47, mais à partir de 2,0 g de 2-benzyl-9-(3-cyanophényl)-4,9-éthane-2,3,3a,4,9,9a-hexahydro-1H-isoindole-3a-carboxylate de méthyle-(3aRS,4SR,9RS,9aRS) et de 0,6 $cm^3$ de chloroformiate de vinyle pendant quatre heures à température ambiante dans 20 $cm^3$ de dichlorométhane, puis en reprenant le concentrat au reflux pendant deux heures dans 20 $cm^3$ de méthanol et 10 $cm^3$ d'une solution normale de gaz chlorhydrique dans l'isopropanol, on obtient, après neutralisation avec une solution aqueuse 5N d'hydroxyde de sodium et extraction à l'oxyde de diéthyle, 1,22 g (77%) de 9-(3-cyanophényl)-4,9-éthano-2-3,3a,4,9,9a-hexahydro- 1H-isoindole-3a-carboxylate de méthyle-(3aRS,4SR,9RS,9aRS), sous forme d'une meringue blanche utilisée telle quelle à l'étape suivante, dont la caractéristique est la suivante :

- spectre de masse (IE) : M/Z = 358 ($M^+$).

Etape C

En opérant comme à l'étape G de l'exemple 1, mais à partir de 1,22 g de chlorhydrate de 9-(3-cyanophényl)-4,9-éthano-2,3,3a,4,9,9a-hexahydro-1H-benzo[f]isoindole-3a-carboxylate de méthyle-(3aRS,4SR,9SR,9aRS), de 0,61 g d'acide 2-(2-méthoxyphényl)propènoique, de 0,3 $cm^3$ de chlorure d'oxalyle et de 0,96 $cm^3$ de triéthylamine dans 25 $cm^3$ de dichlorométhane pendant deux heures à température ambiante, on obtient, après purification sur gel de silice (230-400 Mesh) en éluant par des mélanges de cyclohexane et d'acétate d'éthyle (95-5 puis 80-20 en volumes), 0,75 g (48%) de 9-(3-cyanophényl)-4,9-éthano-2-[2-(2-méthoxyphényl)propènoyl]-2,3,3a,4,9,9a-hexahydro-1H-benzo [f]isoindole-3a-carboxylate de méthyle-(3aRS,4SR,9SR,9aRS), sous forme d'une poudre blanche, dont les caractéristiques sont les suivantes :

- point de fusion = 176-8°C

- spectre de masse (IE) : M/Z = 518 ($M^+$).

**EXEMPLE 97**

Préparation de l'acide 4,9-éthano-9-(3-hydroxy-4-méthoxyphényl)-2-[2-(2-méthoxyphényl)propènoyl]-2,3,3a,4,9,9a-hexahydro-1H-benzo[f]isoindole-3a-carboxylique-(3aRS,4SR,9SR,9aRS)

Etape A

En opérant comme à l'étape E de l'exemple 1, mais à partir de 3,1 g d'un mélange de 2-benzyl-4,9-éthano-9-(3-hydroxy-4-méthoxyphényl)-2,3,3a,4,9,9a-hexahydro-1H-benzo[f]isoindole-3a-carboxylate de méthyle-(3aRS, 4SR,9SR,9aRS) et de 2-benzyl-4,9-éthano-9-(4-hydroxy-3-méthoxyphényl)-2,3,3a,4,9,9a-hexahydro-1H-benzo[f] isoindole-3a-carboxylate de méthyle-(3aRS,4SR,9SR,9aRS), obtenus à l'étape B de l'exemple 70, de 1,25 g de formiate d'ammonium et de 0,4 g de palladium sur charbon à 10 % (p/p), on obtient 2,1 g d'un mélange de 4,9-éthano-9-(3-hydroxy-4-méthoxyphényl)-2,3,3a,4,9,9a-hexahydro-1H-benzo[f]isoindole-3a-carboxylate de méthyle-(3aRS, 4SR,9SR,9aRS) et de 4,9-éthano-9-(4-hydroxy-3-méthoxyphényl)-2,3,3a,4,9,9a-hexahydro-1H-benzo[f]isoindole-3a-carboxylate de méthyle-(3aRS,4SR,9SR,9aRS) sous forme d'une huile incolore utilisée telle quelle dans les synthèses ultérieures.

Etape B

En opérant comme à l'étape G de l'exemple 1, mais à partir de 2,1 g d'un mélange de 4,9-étbano-2-9-(3-hydroxy-4-méthoxyphényl)-2,3,3a,4,9,9a-hexahydro-1H-benzo[f]isoindole-3a-carboxylate de méthyle-(3aRS,4SR,9SR, 9aRS) et de 4,9-éthano-9-(4-hydroxy-3-méthoxyphényl)-2,3,3a,4,9,9a-hexahydro-1H-benzo[f]isoindole-3a-carboxylate de méthyle-(3aRS,4SR,9SR,9aRS), de 1,0 g d'acide 2-(2-méthoxyphényl)propènoïque, de 0,47 $cm^3$ de chlorure d'oxalyle et de 1,5 $cm^3$ de triéthylamine, on obtient, après trois flash-chromatographie sur gel de silice (230-400 mesh), en éluant avec un mélange dichlorométhane-méthanol (96,5-3,5 en volumes) la première, dichlorométhane-méthanol (99-1 en volumes) la seconde et dichlorométhane-méthanol (99,5-0,5 en volumes) la troisième, et après recristallisation dans 25 $cm^3$ d'acétonitrile, 0,4 g de 4,9-éthano-9-(3-hydroxy-4-méthoxyphényl)-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS) contenant 5% de 4,9-éthano-9-(4-hydroxy-3-méthoxyphényl)-2-[2-(2-méthoxyphényl)propènoyl]-2,3,3a,4,9,9a-hexahydro-1H-benzo[f] isoindole-3a-carboxylate de méthyle-(3aRS,4SR,9SR,9aRS) dont la caractéristique est la suivante :

- point de fusion = 242°C.

Etape C

En opérant comme à l'exemple 2, mais à partir de 0,35 g de 4,9-éthano-9-(3-hydroxy-4-méthoxyphényl)-2-[2-(2-méthoxyphényl)propènoyl]-2,3,3a,4,9,9a-hexahydro-1H-benzo[f]isoindole-3a-carboxylate de méthyle-(3aRS, 4SR,9SR,9aRS), de 3,25 $cm^3$ d'une solution aqueuse normale d'hydroxyde de sodium et 5 $cm^3$ de méthanol, on obtient, après recristallisation dans 6 $cm^3$ d'isopropanol, 0,24 g d'acide 4,9-éthano-9-(3-hydroxy-4-méthoxyphènyl)-2-[2-(2-méthoxyphényl)propènoyl]-2,3,3a,4,9,9a-hexahydro-1H-benzo[f]isoindole-3a-carboxylique-(3aRS,4SR,9SR,9aRS) dont les caractéristiques sont les suivantes :

- point de fusion = 278°C.
- spectre de R.M.N. $^1H$ (250 MHz, $(CD_3)_2SO$ d6, à une température de 383 K, d en ppm) : 1,38 - 1,56 - 1,96 et 2,12 (4 mts, 1H chacun : $CH_2CH_2$); de 3,20 à 3,50 (mt, 3H : $CH_2$ en 1 et CH en 9a) : 3,42 (mt, 1H : CH en 4); 3,59 et 4,06 (respectivement d et d large, J = 12,5 Hz, 1H chacun : $CH_2$ en 3), 3,73 et 3,86 (2 s, 3H chacun : les 2 $ArOCH_3$) : 5,54 et 5,69 (respectivement d et s large, J = 1 Hz, 1H chacun : $=CH_2$) ; 6,52 (d large, J = 7,5 Hz, 1H : H en 8) ; 6,73 (dd, J = 8 et 2 Hz, 1H : H en 6 du 3-hydroxy-4-méthoxyphényl) ; 6,86 (d, J = 2 Hz, 1H : H en 2 du 3-hydroxy-4méthoxyphényl) ; de 6,90 à 7,40 (mt, 8H : H en 5 - H en 6 - H en 7 - H en 5 du 3-hydroxy-4méthoxyphényl et H aromatiques du 2-méthoxyphenyl) : de 8,20 à 8,90 (mf etale, 1H : ArOH).

## EXEMPLE 98

Préparation du 4,9-éthano-5-méthoxy-2-[2-(2-méthoxyphényl)-2-propènoyl]-9-(4-méthylphényl)-2,3,3a,4,9,9a-hexahydro-1H-benzo[f]isoindole-3a-carboxylate de méthyle-(3aRS,4SR,9SR,9aRS)

Etape A

A une solution refroidie à 0°C de 178 g (0,917 mol) d'acide (2-méthoxyphényl)pyruvique, qui peut être obtenu selon Org. Synth., 1939, 19, 1-3, dans 970 $cm^3$ de méthanol on ajoute successivement 77,5 g (1,1 mol) de méthylvinylcétone et 1,1 $dm^3$ d'une solution aqueuse normale d'hydroxyde de sodium, puis on agite à une température voisine de 20°C pendant deux heures. Après neutralisation avec 1,1 $dm^3$ d'une solution aqueuse normale d'acide chlorhydrique et concentration du méthanol, le précipité formé est essoré, lavé à l'eau, puis séché à 20°C. On obtient ainsi 189,7 g (78%) d'acide 6-(2-méthoxyphényl)-3-oxo-cyclohexan-1-ol-1-carboxylique, sous forme d'une poudre jaune dont les caractéristiques sont les suivantes :

- point de fusion = 204°C
- spectre de R.M.N. $^1H$ (250 MHz, $CDCl_3$, d en ppm) ; 1.20 (t, J = 7 Hz, 3H : $CH_3$ de l'éthyle) ; 1,47 (d, J = 7 Hz, 3H : $CH_3$) ; 4,17 (q, J = 7 Hz, 2H : $OCH_2$ de l'éthyle) ; 4,49 (q, J = 7 Hz, 1H : ArCH) ; de 7,15 à 7,45 (mt, 5H : H aromatiques).

Etape B

396,7 g (1,5 mol) d'acide 6-(2-méthoxyphényl)-3-oxo-cyclohexan-1-ol-1-carboxylique sont chauffés à reflux, pendant deux heures, dans 13,3 $dm^3$ de toluène en présence de 39,7 g d'acide para toluène sulfonique. Le mélange réactionnel est ensuite concentré sous pression réduite et le précipité formé est filtré, lavé à l'éther isopropylique puis séché à 50°C. On obtient ainsi 288 g (78%) d'acide 6-(2-méthoxyphényl)-3-oxo-cyclohexène-1-carboxylique-(RS) sous forme d'un solide marron dont les caractéristiques sont les suivantes :

- point de fusion = 177°C
- spectre de masse (IE) : M/Z = 246 ($M^+$)
- spectre de R.M.N. $^1H$ (300 MHz, $CDCl_3$, d en ppm) : de 2,10 à 2,50 (mt, 4H : $CH_2CH_2$) ; 3,70 (s, 3H : $COOCH_3$) ; 3,90 (s, 3H : $ArOCH_3$) ; 4,61 (mt, 1H : ArCH) ; de 6,80 à 7,00 et 7,25 (2 mts, 5H en totalité : =CH et H aromatiques).

Etape C

A une solution de 288 g (1,17 mol) d'acide 6-(2-méthoxyphényl)-3-oxo-cyclohexène-1-carboxylique-(RS), dans 2.5 $dm^3$ d'acétone, on ajoute successivement 235 g (1,66 mol) d'iodure de méthyle et 212 g (1.39 mol) de 1,8-diazabicyclo[5.4.0]undéc-7-ene goutte a goutte, puis on porte au reflux pendant une heure. L'acétone est ensuite concentrée, puis le résidu est agité avec 1 $dm^3$ d'eau. Après refroidissement à 10°C, le précipité formé est essoré, lavé à l'éther de pétrole, puis séché à 50°C. On obtient ainsi 289 g (95 %) de 6-(2-méthoxyphényl)-3-oxo-cyclohexène-1-carboxylate de méthyle-(RS), sous forme d'une poudre moutarde dont les caractéristiques sont les suivantes :

- point de fusion = 76°C
- spectre de masse (IE) : M/Z = 260 ($M^+$)

Etape D

Une solution de 6 g (0,023 mol) de 6-(2-méthoxyphényl)-3-oxo-cyclohexène-1-carboxylate de méthyle-(RS), et de 0,3 $cm^3$ d'acide trifluoroacétique dans 185 $cm^3$ de dichlorométhane est portée au reflux. On ajoute alors, goutte à goutte, 7,7 g (0,0275 mol) de N-n,butoxyméthyl-N-triméthylsilylméthyl-benzylamine, qui peut être obtenue selon Chem. Pharm. Bull., 1985, 276 et porte au reflux pendant trois heures. Le milieu réactionnel est alors refroidi à 20°C. Après agitation pendant une heure avec environ 5 g de carbonate de potassium, la phase organique est concentrée et l'huile jaune obtenue est purifiée par chromatographie sur gel de silice (230-400 Mesh), en éluant avec un mélange cyclohexane-acétate d'éthyle (95-05 en volumes). On obtient alors 6,1 g (68%) de 2-benzyl-4-(2-méthoxyphényl)-7-oxo-octahydroisoindole-3a-carboxylate de méthyle-(3aRS,4SR,7aRS), sous forme d'une huile jaune, dont les caractéristiques sont les suivantes :

- spectre de masse (IE) : M/Z = 393 ($M^+$)

Etape E

Une solution de 166,6 g (0,424 mol) de 2-benzyl-4-(2-méthoxyphényl)-7-oxo-octahydroisoindole-3a-carboxylate de méthyle-(3aRS,4SR,7aRS) et de 84,5 g (1,69 mol) d'hydrate d'hydrazine dans 2,8 $dm^3$ de méthanol est portée au reflux pendant deux heures. Après concentration du méthanol sous pression réduite, le résidu est repris avec 2 $dm^3$ de dichlorométhane, lavé avec quatre fois 1 $dm^3$ d'eau distillée, séché sur sulfate de magnésium et concentré sous pression réduite. On obtient ainsi 175,2 g de 2-benzyl-7-hydrazono-4-(2-méthoxyphényl)-octahydro-isoindole-3a-carboxylate de méthyle-(3aRS,4SR,7aRS) sous forme d'une huile jaune dont les caractéristiques sont les suivantes :

- spectre de masse (IE) : M/Z = 407 ($M^+$)

Etape F

A une solution de 85 g (0.209 mol) de 2-benzyl-7-hydrazono-4-(2-méthoxyphényl)-octahydro-isoindole-3a-carboxylate de méthyle-(3aRS,4SR,7aRS) dans 2 $dm^3$ de tétrahydrofurane on ajoute goutte à goutte une solution de 106,1 g (0,418 mol) d'iode dans 1,1 $dm^3$ de tétrahydrofurane. Après addition de 2 $dm^3$ d'acétate d'éthyle, les phases organiques sont lavées avec deux fois 1 $dm^3$ d'une solution aqueuse saturée en hydrogénocarbonate de sodium, lavées avec deux fois 1 $dm^3$ d'une solution aqueuse saturée en thiosulfate de sodium puis séchées sur sulfate de magnésium et concentrées sous pression réduite. Apres purification par chromatographie sur gel de silice (230-400 Mesh), en éluant avec un mélange cyclohexane-acétate d'éthyle (95-05 en volumes), on obtient 51 g (48%) de 2-benzyl-7-iodo-4-(2-méthoxyphényl)-1,2,3,4,5,7a-hexahydro-isoindole-3a-carboxylate de méthyle-(3aRS,4SR,7aRS) sous forme d'une huile marron dont les caractéristiques sont les suivantes :

- spectre de masse (IE) : M/Z = 503 ($M^+$)

Etape G

A une solution de 1,37 g (2,7 mmol) de 2-benzyl-7-iodo-4-(2-méthoxyphényl)-1,2,3,4,5,7a-hexahydro-isoindole-3a-carboxylate de méthyle-(3aRS,4SR,7aRS) et de 0,15 g (0,13 mmol) de tétrakis(triphénylphosphine)palladium dans 30 $cm^3$ de toluène, on ajoute successivement une solution de 0,41 g (2,99 mmol) d'acide 4-méthylphénylboronique dans 13 $cm^3$ de méthanol et 30 $cm^3$ d'une solution aqueuse 2 N de carbonate de sodium, puis on porte au reflux pendant deux heures. Après retour à température voisine de 20°C, le mélange réactionnel est extrait avec 100 $cm^3$ d'acétate d'éthyle, lavé avec deux fois 50 $cm^3$ d'eau distillée, séché sur sulfate de magnésium puis concentré sous pression réduite. Le résidu marron obtenu est purifié par flash-chromatographie sur gel de silice (230-400 mesh), en éluant avec un mélange cyclohexane-acétate d'éthyle (95-05 en volumes), on obtient ainsi, 1,2 g (93%) de 2-benzyl-4-(2-méthoxyphényl)-7-(4-méthylphényl)-1,2,3,4,5,7a-hexahydro-isoindole-3a-carboxylate de méthyle-(3aRS,4SR,7aRS) sous forme dune huile jaune dont les caractéristiques sont les suivantes :

- spectre de masse (IE) : M/Z = 467 ($M^+$)

Etape H

A une solution de 66,4 g (0,142 mol) de 2-benzyl-4-(2-méthoxyphényl)-7-(4-méthylphényl)-1,2,3,4,5,7a-hexahydro-isoindole-3a-carboxylate de méthyle-(3aRS,4SR,7aRS) dans 750 $cm^3$ de dichlorométhane, maintenue à une température voisine de 0°C, on ajoute goutte à goutte 94 $cm^3$ d'acide trifluorométhanesulfonique. Le mélange réactionnel est agité pendant trois heures à une température voisine de 20°C puis refroidi à une température voisine de 0°C. On ajoute alors 700 $cm^3$ d'une solution aqueuse saturée en carbonate de potassium. La phase organique est séparée par décantation, lavée successivement par trois fois 150 $cm^3$ d'eau distillée et par deux fois 100 $cm^3$ d'une solution aqueuse saturée en chlorure de sodium, séchée sur sulfate de magnésium et concentrée sous pression réduite. On obtient ainsi, après lavage dans l'oxyde de diisopropyle, 54,9 g (82%) de 2-benzyl-4,9-éthano-5-méthoxy-9-(4-méthylphényl)-2,3,3a,4,9,9a-hexahydro-1H-benzo[f]isoindole-3a-carboxylate de méthyle-(3aRS,4SR,9SR,9aRS) sous forme de solide blanc-cassé dont les caractéristiques sont les suivantes :

- point de fusion = 129°C
- spectre de R.M.N. $^1H$ (250 MHz, $CDCl_3$, d en ppm) ; 1,41 - 1,73 et de 2,40 à 2,60 (3 mts, respectivement 1H - 1H et 2H : $CH_2CH_2$) ; de 2,30 à 2,40 (mt, 2H : 1H du $CH_2$ en 1 et 1H du $CH_2$ en 3) ; 2,39 (s, 3H : $ArCH_3$) ; 2,75 (d large, J = 10 Hz, 1H : l'autre H du $CH_2$ en 1) ; 3,23 (d, J = 9,5 Hz, 1H : l'autre H du $CH_2$ en 3) ; 3,26 (mt, 1H : CH

en 9a) ; 3,41 et 3,65 (2 d, J = 12,5 Hz, 1H chacun : $NCH_2Ar$) ; 3,55 (s, 3H : $COOCH_3$) ; 3,81 (s, 3H : $ArOCH_3$) ; 3,96 (mt, 1H : CH en 4) ; 6,21 (d, J = 7,5 Hz, 1H : H en 8) ; 6,71 (d, J = 7,5 Hz, 1H : H en 6) ; 6,99 (t, J = 7,5 Hz, 1H : H en 7) ; de 7,15 à 7,40 (mt, 9H : H aromatiques du 4-méthylphényle et H aromatiques du benzyle).

Etape I

A partir de 0,54 g (1,1 mmol) de 2-benzyl-4,9-éthano-5-méthoxy-9-(4-méthylphényl)-2,3,3a,4,9,9a-hexahydro-1H-benzo[f]isoindole-3a-carboxylate de méthyle-(3aRS,4SR,9SR,9aRS) en solution dans 100 $cm^3$ de méthanol, et en présence de 54 mg de palladium sur charbon à 10 % (p/p), on obtient, après une heure d'agitation sous hydrogène à pression atmosphérique et à 40°C, filtration du catalyseur et concentration à sec sous pression réduite, 0,5 g de chlorhydrate de 4,9-éthano-5-méthoxy-9-(4-méthylphényl)-2,3,3a,4,9,9a-hexahydro-1H-benzo[f]isoindole-3a-carboxylate de méthyle-(3aRS,4SR,9SR,9aRS) sous forme de solide beige dont les caractéristiques sont les suivantes :

- point de fusion > 260°C
- spectre IR (KBr) :

| | |
|---|---|
| 3050-2250 $cm^{-1}$ | n $N^+$ H + n CH aromatiques et aliphatiques |
| 2833 $cm^{-1}$ | n CH $OCH_3$ |
| 1738 $cm^{-1}$ | n C=O ester méthylique |
| 1603, 1584, 1514, 1477 $cm^{-1}$ | respiration des noyaux aromatiques |
| 1258 $cm^{-1}$ | n O-C=O ester méthylique + $n_a$ C-O ether |
| 824 $cm^{-1}$ | g CH aromatiques phényl para disubstitué |
| 776, 755 $cm^{-1}$ | g CH aromatiques phényl trisubstitué |

Etape J

A une solution de 0,12 g d'acide 2-(2-méthoxyphenyl)propènoique dans 5 $cm^3$ de dichlorométhane contenant 3 gouttes de N,N-diméthylformamide on ajoute goutte à goutte une solution de 0,058 $cm^3$ de chlorure d'oxalyle dans 5 $cm^3$ de dichlorométhane. Le mélange reactionnel est encore agité pendant deux heures à une température voisine de 20°C puis refroidi à une température voisine de 0°C et coulé goutte à goutte dans une solution de 0,25 g de chlorhydrate de 4,9-éthano-5-méthoxy-9-(4-méthylphényl)-2,3,3a,4,9,9a-hexahydro-1H-benzo[f]isoindole-3a-carboxylate de méthyle-(3aRS,4SR,9SR,9aRS) dans 7 $cm^3$ de dichlorométhane et 0,19 $cm^3$ de triéthylamine en maintenant la température au voisinage de 0°C. Le mélange reactionnel est encore agité pendant une heure à température voisine de 0°C puis à 20°C pendant une nuit et versé dans 15 $cm^3$ d'eau distillée. La phase organique est séparée par décantation. lavée par deux fois 15 $cm^3$ d'eau distillée puis 15 $cm^3$ d'une solution aqueuse saturée en chlorure de sodium, séchée sur sulfate de magnésium et concentrée sous pression réduite. Après purification par flash-chromatographie sur gel de silice (230-400 mesh), en éluant avec un mélange cyclohexane-acétate d'éthyle (70-30 en volumes), on obtient 0,17 g de 4,9-éthano-5-méthoxy-2-[2-(2-méthoxyphényl)propènoyl]-9-(4-méthylphényl)-2,3,3a,4,9,9a-hexahydro-1H-benzo[f]isoindole-3a-carboxylate de méthyle-(3aRS,4SR,9SR,9aRS) sous forme de solide blanc dont les caractéristiques sont les suivantes :

- point de fusion = 185°C
- spectre de masse (IE) : M/Z = 537 ($M^+$)

**EXEMPLE 99**

<u>Préparation de l'acide 4,9-éthano-5-méthoxy-2-[2-(2-méthoxyphényl)propènoyl]-9-(4-méthylphényl)-2,3,3a,4,9,9a-hexahydro-1H-benzo[f]isoindole-3a-carboxylique-(3aRS,4SR,9SR,9aRS)</u>

0,365 g de 4,9-éthano-5-méthoxy-2-[2-(2-méthoxyphényl)propénoyl]-9-(4-méthylphényl)-2,3,3a,4,9,9a-hexahydro-1H-benzo[f]isoindole-3a-carboxylate de méthyle-(3aRS,4SR,9SR,9aRS) sont chauffés à reflux, pendant trois heures, dans 30 $cm^3$ d'éthanol en présence de 0,82 $cm^3$ d'une solution aqueuse normale d'hydroxyde de sodium. Le mélange réactionnel est ensuite concentré sous pression réduite et le résidu dissout dans 10 $cm^3$ d'eau distillée. La phase aqueuse est lavée par deux fois 10 $cm^3$ de dichlorométhane, acidifiée par une solution aqueuse normale d'acide chlorhydrique jusqu'à un pH voisin de 2, extraite avec 30 $cm^3$ de dichlorométhane, lavée avec de l'eau distillée,

séchée sur sulfate de magnésium puis concentrée sous pression réduite. Le solide orange obtenu est purifié par flash-chromatographie sur gel de silice (230-400 mesh), en éluant avec un mélange dichlorométhane-méthanol-acide acétique (98-1,5-0,5 en volumes). On obtient ainsi, 0,09 g (26%) d'acide 4,9-éthano-5-méthoxy-2-[2-(2-méthoxyphényl) propènoyl]-9-(4-méthylphényl)-2,3,3a,4,9,9a-hexahydro-1H-benzo[f]isoindole-3a-carboxylique-(3aRS,4SR,9SR, 9aRS) sous forme d'un solide blanc dont les caractéristiques sont les suivantes :

- point de fusion > 260°C
- spectre de R.M.N. $^1H$ (250 MHz, $(CD_3)_2SO$ d6, à une température de 383 K, d en ppm) : 1,32 - 1,60 et de 1,85 à 2,20 (3 mts, respectivement 1H - 1H et 2H : $CH_2CH_2$) ; 2,38 (s, 3H : $ArCH_3$) ; 3,34 (mt, 3H : $CH_2$ en 1 et CH en 9a) : 3,62 et 4,04 (2 d, J = 12,5 Hz, 1H chacun : $CH_2$ en 3) ; 3,72 et 3,81 (2 s, 3H chacun : $ArOCH_3$) ; 3,93 (mt, 1H : CH en 4) ; 5,52 et 5,66 (2 s larges, 1H chacun : = $CH_2$) ; 6,06 (d, J = 7,5 Hz, 1H : H en 8) ; 6,82 (d, J = 7,5 Hz, 1H : H en 6) ; de 6,90 à 7,10 (mt, 3H : H en 7 et 2H aromatiques du 2-méthoxyphényl) ; de 7,20 à 7,40 (mt, 6H : 2H aromatiques du 2-méthoxyphényl et H aromatiques du 4-méthoxyphényl).

**EXEMPLE 100**

Isolement de l'énantiomère dextrogyre de l'acide 4,9-éthano-5-méthoxy-2-[2-(2-méthoxyphényl)-2-propènoyl]-9-(4-méthylphényl)-2,3,3a,4,9,9a-hexahydro-1H-benzo[f]isoindole-3a-carboxylique-(3aRS,4SR,9SR,9aRS)

6,5 g d'acide 4,9-éthano-5-méthoxy-2-[2-(2-méthoxyphényl)propènoyl]-9-(4-méthyl-phényl)-2,3,3a,4,9,9a-hexahydro-1H-benzo[f]isoindole-3a-carboxylique-(3aRS,4SR,9SR,9aRS), obtenu à l'exemple 99, sont dédoublés, sur une colonne de silice chirale porteuse de greffons N-(3,5-dinitrobenzoyl)phénylalamine-(R), en 4 injections successives et en éluant par un mélange de n-heptane-dichlorométhaneéthanol (50-50-1 en volumes). En recueillant les premières fractions éluées (temps de rétention 30 mn), on obtient, après concentration du solvant sous pression réduite, 2,81 g de l'énantiomère dextrogyre de l'acide 4,9-éthano-5-méthoxy-2-(2-(2-méthoxyphényl)propenoyl]-9-(4-méthylphényl)-2,3,3a,4,9,9a-hexahydro-1H-benzo[f]isoindole-3a-carboxylique-(3aRS,4SR,9SR,9aRS), sous forme d'une poudre blanche dont les caractéristiques sont les suivantes :

- spectre de masse (IE) : M/Z= 523 ($M^+$)

- pouvoir rotatoire: $[a]_{365}^{20}$= + 36.5 - 1° (c = 0.5/ dichlorométhane)

**EXEMPLE 101**

Préparation de l'acide 4,9-éthano-2[2-(2-méthoxyphényl)-2-propènoyl]-4-méthyl-9-(4-méthoxyphényl)-2,3,3a,4,9,9a-hexahydro-1H-benzo[f]isoindole-3a-carboxylique-(3aRS,4SR,9SR,9aRS)

Etape A

A une solution refroidie au voisinage de -70°C de 33 g (0,220 mol) d'acide 2-phényl-propionique-(RS) dans 220 $cm^3$ de tétrahydrofurane et de 44 $cm^3$ de N-N'-tetraméthyléthylènediamine, on coule goutte à goutte une solution 1,6 N de n-butyllithium dans l'hexane. Après avoir agité pendant trois heures à - 70°C, on revient à une température voisine de 20°C et on coule la solution obtenue sur une solution de 75 $cm^3$ (0,55 mol) d'oxalate d'éthyle dans 110 $cm^3$ de tétrahydrofurane en maintenant la température à -75°C pendant 1 heure. Le mélange réactionnel est hydrolysé par 800 $cm^3$ d'une solution aqueuse saturée en hydrogénocarbonate de sodium, extrait avec trois fois 100 $cm^3$ d'acétate d'éthyle, lavé avec trois fois 50 $cm^3$ d'une solution aqueuse normale d'acide chlorhydrique, séché sur sulfate de sodium et concentré sous pression réduite. On obtient alors 44 g (97%) de 2-oxo-3-phénylbutyrate d'éthyle-(RS) sous forme d'une huile orange dont les caractéristiques sont les suivantes :

- spectre de R.M.N. $^1H$ (250 MHz, $CDCl_3$, d en ppm) : 1,20 (t, J = 7 Hz, 3H : $CH_3$ de l'éthyle) ; 1,47 (d, J = 7 Hz, 3H : $CH_3$) ; 4,17 (q, J = 7 Hz, 2H : $OCH_2$ de l'éthyle) ; 4,49 (q, J = 7 Hz, 1H : ArCH) ; de 7,15 à 7,45 (mt, 5H : H aromatiques).

Etape B

A une solution refroidie au voisinage de 7°C de 6,7 g (32,5 mmol) de 2-oxo-3-phényl-butyrate d'éthyle-(RS), dans 68 $cm^3$ d'éthanol on ajoute successivement 3,5 $cm^3$ (39 mmol) de méthylvinylcétone et 72 $cm^3$ (71,5 mmol) d'une solution aqueuse normale d'hydroxyde de sodium. puis on agite à une température voisine de 20°C pendant deux

heures. Le mélange réactionnel est refroidi à 15°C et 72 $cm^3$ d'une solution aqueuse normale d'acide chlorhydrique sont ajoutés. Apres concentration de l'éthanol, le précipité formé est essoré, lave à l'eau, puis séché à 50°C. On obtient ainsi 4,4 g (50%) d'acide 2-méthyl-5-oxo-2-phényl-cyclohexan-1-ol-1-carboxylique-(1RS,2RS), sous forme d'une poudre beige dont les caractéristiques sont les suivantes:

- spectre de R.M.N. $^1H$ (300 MHz, $CDCl_3$, d en ppm) : 1,79 (s, 3H : $CH_3$) ; de 2,00 à 2,25 et de 2,25 à 2,55 (2 mts, 2H chacun : $CH_2CH_2$) ; 3,59 (s, 3H : $COOCH_3$) ; 6,72 (s, 1H : =CH) ; de 7,15 à 7,35 (mt, 5H : H aromatiques).

Etape C

4,4 g (17,6 mmol) d'acide 2-méthyl-5-oxo-2-phényl-cyclohexan-1-ol-1-carboxylique-(1RS,2RS) sont chauffés à reflux, pendant une heure, dans 100 $cm^3$ de toluène en présence de 0,34 g d'acide para-toluène-sulfonique. Le mélange réactionnel est refroidi à 0°C et le précipité formé est filtré, lavé à l'éther éthylique puis séché à 50°C. On obtient ainsi 2,8 g (69%) d'acide 2-méthyl-5-oxo-2-phénylcyclohexène-1-carboxylique-(RS) sous forme d'un solide rose dont les caractéristiques sont les suivantes :

- point de fusion = 206°C
- spectre de masse (IE) : M/Z = 230 ($M^+$)

Etape D

A une solution de 2,3 g (10 mmol) d'acide 2-méthyl-5-oxo-2-phénylcyclohexène-1-carboxylique-(RS), dans 20 $cm^3$ d'acétone, on ajoute successivement 0,88 $cm^3$ (14 mmol) d'iodure de méthyle et 1.8 $cm^3$ (12 mmol) de 1,8-diazabicyclo[5.4.0]undéc-7-ène goutte à goutte. Le mélange réactionnel est ensuite chauffé à reflux pendant deux heures. L'acétone est ensuite concentrée, puis le résidu est agité avec 100 $cm^3$ d'eau puis extrait avec trois fois 30 $cm^3$ d'acétate d'éthyle. On obtient après séchage sur sulfate de magnésium et concentration sous pression réduite 2,3 g (94%) de 2-méthyl-5-oxo-2-phényl-cyclohexène-1-carboxylate de méthyle-(RS) sous forme d'une huile orange dont la caractéristique est la suivante :

- spectre de masse (IE) : M/Z = 244 ($M^+$)

Etape E

Une solution de 18,3 g (0,075 mol) de 2-méthyl-5-oxo-2-phényl-cyclohexène-1-carboxylate de méthyle-(RS) et de 0,57 $cm^3$ d'acide trifluoroacétique dans 145 $cm^3$ de dichlorométhane est portée au reflux. On ajoute alors, goutte à goutte, 31,4 g (0.112 mol) de N-n.butoxyméthyl-N-triméthylsilylméthyl-benzylamine, qui peut être obtenue selon Chem. Pharm. Bull., 1985, 276 et porte au reflux pendant quatre heures. Le milieu réactionnel est alors refroidi à 20°C. Après agitation pendant une heure avec environ 15 g de carbonate de potassium, la phase organique est concentrée et l'huile obtenue est cristallisée dans du pentane. Après filtration on obtient 19 g (67%) de 2-benzyl-4-méthyl-7-oxo-4-phényl-octahydroisoindole-3a-carboxylate de méthyle-(3aRS,4SR,7aRS), sous forme d'un solide jaune, dont les caractéristiques sont les suivantes :

- point de fusion = 115°C
- spectre de masse (IE) : M/Z = 377 ($M^+$)

Etape F

Une solution de 3 g (7,95 mmol) de 2-benzyl-4-méthyl-7-oxo-4-phényloctahydroisoindole-3a-carboxylate de méthyle-(3aRS,4SR,7aRS), et de 1,55 $cm^3$ (31,8 mmol) d'hydrate d'hydrazine dans 30 $cm^3$ de méthanol est portée au reflux pendant deux heures. Après concentration du méthanol sous pression réduite, le résidu est repris avec du dichlorométhane, lavé avec trois fois 30 $cm^3$ d'eau distillée, séché sur sulfate de magnésium et concentré sous pression réduite. On obtient ainsi 2,8 g (89%) de 2-benzyl-7-hydrazono-4-méthyl-4-phényl-octahydroisoindole-3a-carboxylate de méthyle-(3aRS,4SR,7aRS) sous forme d'une huile marron dont les caractéristiques sont les suivantes :

- spectre de R.M.N. $^1H$ (300 MHz, $CDCl_3$, d en ppm) : 1,33 (s, 3H : $CH_3$) ; 1,86 - 2,24 - 2,48 et 2,84 (4 mts, 1H chacun : $CH_2$ en 5 et $CH_2$ en 6) ; 2,43 et 3,47 (2 d, J = 9 Hz, 1H chacun : $CH_2$ en 3) ; 2,67 et 3,19 (2 t, J = 8,5 Hz,

1H chacun : $CH_2$ en 1) ; 3,10 (s, 3H : $COOCH_3$) ; 3,53 et 3,71 (2 d, J = 13 Hz, 1H chacun : $NCH_2Ar$) ; 3,78 (t, J = 8,5 Hz, 1H : H en 7a) ; 4,98 (s large, 2H : $NH_2$) ; de 7,10 à 7,35 (mt, 10H: H aromatiques du phényle et H aromatiques du benzyle).

Etape G

A une solution de 2,8 g (7 mmol) de 2-benzyl-7-hydrazono-4-méthyl-4-phényl-octahydroisoindole-3a-carboxylate de méthyle-(3aRS,4SR,7aRS) dans 80 $cm^3$ de tétrahydrofurane on ajoute 2,95 $cm^3$ de triéthylamine et on coule goutte à goutte une solution de 3,6 g (14,1 mmol) d'iode dans 40 $cm^3$ de tétrahydrofurane. Après addition d'acétate d'éthyle, les phases organiques sont lavées avec deux fois 50 $cm^3$ d'une solution aqueuse saturée avec de l'hydrogénocarbonate de sodium, lavées avec deux fois 50 $cm^3$ d'une solution aqueuse saturée avec du thiosulfate de sodium puis séchées sur sulfate de magnésium et concentrées sous pression réduite. Après purification par chromatographie sur gel de silice (230-400 Mesh), en éluant avec un mélange cyclohexane-acétate d'éthyle (85-15 en volumes), on obtient 2,4 g (69%) de 2-benzyl-7-iodo-4-(2-méthoxyphényl)-1,2,3,4,5,7a-hexahydro-isoindole-3a-carboxylate de méthyle-(3aRS,4SR,7aRS) sous forme d'une huile jaune dont la caractéristique est la suivante :

- spectre de masse (IE) : M/Z = 487 ($M^+$)

Etape H

A une solution de 58,6 g (0,12 mol) de 2-benzyl-7-iodo-4-(2-méthoxyphényl)-1,2,3,4,5,7a-hexahydro-isoindole-3a-carboxylate de méthyle-(3aRS,4SR,7aRS) et de 7 g de tétrakis(triphénylphosphine)palladium dans 30 $cm^3$ de toluène, on ajoute successivement une solution de 20,1 g (0,132 mol) d'acide 4-méthoxyphénylboronique dans 580 $cm^3$ de méthanol et 1170 $cm^3$ d'une solution aqueuse 2 N de carbonate de sodium, puis on porte au reflux pendant vingt quatre heures. Après retour à température voisine de 20°C, le mélange réactionnel est extrait avec de l'acétate d'éthyle, lavé avec de l'eau distillée, séché sur sulfate de magnésium puis concentré sous pression réduite. Le résidu obtenu est purifié par flash-chromatographie sur gel de silice (230-400 mesh), en éluant avec un mélange cyclohexane-acétate d'éthyle (98-02 en volumes), on obtient ainsi, 29 g (52%) de 2-benzyl-7-(4-méthoxyphényl)-4-méthyl-4-phényl-1,2,3,4,5,7a-hexahydro-isoindole-3a-carboxylate de méthyle-(3aRS,4SR,7aRS) sous forme d'une poudre marron dont les caractéristiques sont les suivantes :

- point de fusion = 132°C
- spectre de R.M.N. $^1H$ (250 MHz, $CDCl_3$, d en ppm) : 1,60 (s, 3H : $CH_3$ en 4) ; 2,03 (dd, J = 9 et 5 Hz, 1H : 1H du $CH_2$ en 5) ; de 2,40 à 2,90 (mt, 1H : l'autre H du $CH_2$ en 5) ; 3,56 (s, 3H : $COOCH_3$) ; de 3,40 à 3,95 (mt, 2H : $NCH_2Ar$) ; 3,79 (mt, 1H : CH en 7a) ; 3,83 (s, 3H : $ArOCH_3$) ; 6,08 (mt, 1H : =CH en 6) ; de 6,75 à 7,45 (mt, 14H : H aromatiques du 4-méthoxyphényl- H aromatiques du benzyle et H aromatiques du phényle).

Etape I

A une solution de 54,7 g (0,117 mol) de 2-benzyl-7-(4-méthoxyphényl)-4-méthyl-4-phényl-1,2,3,4,5,7a-hexahydro-isoindole-3a-carboxylate de méthyle-(3aRS,4SR,7aRS) dans 700 $cm^3$ de dichlorométhane, maintenue à une temperature voisine de 5°C, on ajoute goutte à goutte 105 $cm^3$ d'acide trifluorométhanesulfonique. Le mélange réactionnel est agité pendant trois heures à une température voisine de 20°C puis refroidi à une température voisine de 0°C. On ajoute alors 100 $cm^3$ d'une solution aqueuse 10N d'hydroxyde de sodium La phase organique est séparée, lavée successivement par trois fois 100 $cm^3$ d'eau distillée, séchée sur sulfate de magnésium et concentrée sous pression réduite. On obtient ainsi, après lavage dans l'éther de pétrole, 50 g (92%) de 2-benzyl-4,9-éthano-9-(4-méthoxyphényl)-4-méthyl-2,3,3a,4,9,9a-hexahydro-1H-benzo[f]isoindole-3a-carboxylate de méthyle-(3aRS,4SR,9SR, 9aRS) sous forme de solide gris dont les caractéristiques sont les suivantes :

- point de fusion = 143°C
- spectre de R.M.N. $^1H$ (400 MHz, $CDCl_3$, d en ppm) : 1,21 - 1,79 - 2,36 et 2,53 (4 mts, 1H chacun : $CH_2CH_2$) ; 1,41 (s, 3H : $CH_3$) ; 2,37 et 2,55 (2 mts, 1H chacun : $CH_2$ en 1); 2,74 et 3,14 (2 d, J = 10 Hz, 1H chacun : $CH_2$ en 3) ; 3,19 (mt, 1H : CH en 9a) ; 3,44 (s, 3H : $COOCH_3$) ; 3,47 et 3,61 (2 d, J = 13 Hz, 1H chacun : $NCH_2Ar$); 3,86 (s, 3H : $ArOCH_3$) ; 6,59 (d, J = 7,5 Hz, 1H : H en 8) ; 6,96 (d large, J = 7,5 Hz, 2H : H aromatiques en ortho du $OCH_3$) ; de 7,05 à 7,25 (mt, 3H : H en 5 - H en 6 et H en 7) ; de 7,25 à 7,45 (mt, 7H : H aromatiques en méta du $OCH_3$ et H aromatiques du benzyle).

Etape J

A partir de 13 g (0,028 mol) de 2-benzyl-4,9-éthano-9-(4-méthoxyphényl)-4-méthyl-2,3,3a,4,9,9a-hexahydro-1H-benzo[f]isoindole-3a-carboxylate de méthyle-(3aRS,4SR,9SR,9aRS) en solution dans 260 $cm^3$ de méthanol, et en présence de 1,7 g de palladium sur charbon à 10 % (p/p), on obtient, après quatre heures d'agitation sous hydrogène à pression atmosphérique et à 40°C, filtration du catalyseur et concentration à sec sous pression réduite, 10,2 g (97%) de chlorhydrate de 4,9-éthano-9-(4-méthoxyphényl)-4-méthyl-2,3,3a,4,9,9a-hexahydro-1H-benzo[f]isoindole-3a-carboxylate de méthyle-(3aRS,4SR,9SR,9aRS) sous forme de solide jaune dont les caractéristiques sont les suivantes :

- spectre IR (KBr)

| | |
|---|---|
| 3050-2250 $cm^{-1}$ | n $N^-$ H + n CH aromatiques et aliphatiques |
| 2842 $cm^{-1}$ | n CH $OCH_3$ |
| 1737 $cm^{-1}$ | n C=O ester méthylique |
| 1611, 1571, 1516, 1460 $cm^{-1}$ | respiration des noyaux aromatiques |
| 1253 $cm^{-1}$ | n O-C=O ester méthylique + $n_a$ C-O ether |
| 825 $cm^{-1}$ | g CH aromatiques phényl para disubstitué |
| 768 $cm^{-1}$ | g CH aromatiques phényl ortho disubstitué |

Etape K

A une solution de 4,62 g (0.026 mol) d'acide 2-(2-méthoxyphényl)propènoïque dans 100 cm3 de dichlorométhane contenant 2 gouttes de N,N-diméthylformamide on ajoute goutte à goutte une solution de 3,1 $cm^3$ de chlorure d'oxalyle dans 50 $cm^3$ de dichlorométhane. Le mélange réactionnel est encore agité pendant deux heures à une température voisine de 20°C puis refroidi à une température voisine de 0°C et coulé goutte à goutte dans une solution de 8,3 g (0,024 mol) de chlorhydrate de 4,9-éthano-9-(4-méthoxyphényl)-4-méthyl-2,3,3a,4,9,9a-hexahydro-1H-benzo[f] isoindole-3a-carboxylate de méthyle-(3aRS,4SR,9SR,9aRS) dans 100 $cm^3$ de dichlorométhane et 6,1 $cm^3$ de triéthylamine en maintenant la température au voisinage de 0°C. Le mélange réactionnel est encore agité pendant une heure à température voisine de 0°C puis pendant une nuit à 20°C. Après lavage avec deux fois 100 $cm^3$ d'eau distillée, la phase organique est séparée par décantation, lavée successivement avec deux fois 100 $cm^3$ d'une solution aqueuse normale d'acide chlorydrique et avec deux fois 100 $cm^3$ d'une solution aqueuse normale d'hydroxyde de sodium, séchée sur sulfate de magnésium et concentrée sous pression réduite. Après purification par flash-chromatographie sur gel de silice (230-400 mesh), en éluant avec un mélange cyclohexane-acétate d'éthyle (70-30 en volumes), on obtient 9,1 g de 4,9-éthano-2-[2-(2-méthoxyphényl)propènoyl]-4-méthyl-9-(4-méthoxyphényl)-2,3,3a,4,9,9a-hexahydro-1H-benzo[f]isoindole-3a-carboxylate de méthyle-(3aRS,4SR,9SR,9aRS) sous forme de solide orangé dont les caractéristiques sont les suivantes :

- point de fusion = 141°C
- spectre de R.M.N. $^1H$ (300 MHz, $(CD_3)_2SO$ d6, d en ppm : de 0,95 à 1,25 et de 1,55 à 2,10(2 mts, 4H en totalité : $CH_2CH_2$) ; 1,38 et 1,46(2 s, 3H en totalité : $CH_3$) ; de 3,05 à 3,35 (mt, 3H : $CH_2$ en 1 et CH en 9a) ; de 3,30 à 3,40 et 3,41 (respectivement mt et s, 3H en totalité : $COOCH_3$) ; 3,62 - 3,72 - 3,80 et 3,83 (4 s, 6H en totalité : $ArOCH_3$) ; de 3,80 à 4,20 (mt, 2H : $CH_2$ en 3) ; 5,43 - 5,56 - 5,60 et 5,78 (4 s, 2H en totalité : = $CH_2$) ; 6,39 et 6,42 (2 d, J = 7,5 Hz, 1H en totalité : H en 8) ; de 6,85 à 7,45 (mts, 11H : H en 5 - H en 6 - H en 7 - H aromatiques du 4-méthoxyphényl et H aromatiques du 2-méthoxyphényl).

Etape L

13,4 g (0,025 mol) de 4,9-éthano-2-[2-(2-méthoxyphényl)propènoyl]-4-méthyl-9-(4-méthoxyphényl)-2,3,3a,4,9,9a-hexahydro-1H-benzo[f]isoindole-3a-carboxylate de méthyle-(3aRS,4SR,9SR,9aRS) sont chauffés à reflux, pendant trois heures, dans 250 $cm^3$ d'éthanol en présence de 52 $cm^3$ d'une solution aqueuse normale d'hydroxyde de sodium. Le mélange réactionnel est ensuite concentré sous pression réduite et le résidu dissout dans 250 $cm^3$ d'eau distillée. La phase aqueuse est lavée par deux fois 100 $cm^3$ d'éther éthylique, acidifiée par 60 $cm^3$ d'une solution aqueuse normale d'acide chlorhydrique, extraite avec deux fois 150 $cm^3$ de dichlorométhane, séchée sur sulfate de magnésium puis concentrée sous pression réduite. Le solide blanc obtenu est purifié par lavage dans 100 $cm^3$ de pentane. On obtient ainsi 10,8 g (83%) d'acide 4,9-éthano-2-[2-(2-méthoxyphényl)propènoyl]-9-(4-méthoxyphényl)-4-méthyl-2,3,3a,4,9,9a-hexahydro-1H-benzo[f]isoindole-3a-carboxylique-(3aRS,4SR,9SR,9aRS) sous forme d'un solide blanc dont les caractéristiques sont les suivantes :

- point de fusion = 157°C
- spectre de masse (IE) : M/Z = 523 ($M^+$)

Isolement de l'énantiomère dextrogyre de l'acide 4,9-éthano-2-[2-(2-méthoxyphényl)propènoyl]-9-(4-méthoxyphényl)-4-méthyl-2,3,3a,4,9,9a-hexahydro-1H-benzo[f]isoindole-3a-carboxylique-(3aRS,4SR,9SR,9aRS)

17,6 g d'acide 4,9-éthano-2-[2-(2-méthoxyphényl )propènoyl]-9-(4-méthoxyphényl)-4-méthyl-2,3,3a,4,9,9a-hexahydro-1H-benzo[f]isoindole-3a-carboxylique-(3aRS,4SR, 9SR,9aRS) sont dédoublés, en 4 injections , sur une colonne de silice chirale Whelk 01 (SS), en éluant avec un mélange de n-heptane-dichlorométhane- propanol (50-48-2 en volumes) contenant 0,05% d'acide trifluoroacétique. En recueuillant la première fraction éluée (temps de rétention 38 mn), on obtient après concentration sous pression réduite 7,01 g de l'énantiomère dextrogyre de l'acide 4,9-éthano-[2-(2-méthoxyphényl)propenoyl]-9-(4-méthoxyphényl)-4-méthyl-2,3,3a,4,9,9a-hexahydro-1H-benzo[f]isoindole-3a-carboxylique-(3aRS,4SR,9SR,9aRS), sous forme d'une poudre blanche, dont les caractéristiques sont les suivantes :

- point de fusion = 238 °C
- spectre de masse (IE) : M/Z = 523 ($M^+$)
- pouvoir rotatoire : $[a]_{365}^{20}$ = + 74,4 +/- 1,3 ° (c = 0,5 / méthanol )

## EXEMPLE 102

Evaluation de l'activité farnésyl transférase de composés selon l'invention

L'activité farnésyl transférase est mesurée par la quantité farnésylée de substrat K-ras ou de substrat dérivé du peptide correspondant à sa partie C-terminale, le groupement farnésyl étant apporté par le pyrophosphate de famésyle (FPP).

Précisément, le substrat biotinylé utilisé, représentatif de K-ras ; BIOT-$(\beta A)_3$ -S-K-D-G-$(K)_6$ -S-K-T-K-C-V-I-M, est [$^3$H] farnésylé sur sa cystéine C, par la farnésyl transférase en présence de [$^3$H]FPP. Il est ensuite mis en contact de billes PVT*-streptavidine (AMERSHAM)® , et est quantifié par scintillation de proximité (dosage SPA) entre le tritium et les billes PVT, grâce à l'interaction streptavidine/biotine.

Expérimentalement, la farnésyl transférase purifiée selon le protocole ci-joint, est diluée pour ce dosage à une concentration telle qu'on obtienne une consommation en substrats inférieure à 30 %. Les concentrations molaires finales de chaque substrat sont ajustées à leur Km respectif : 50 nM pour le peptide K ras biotinylé et 120 nM pour le FPP, amenés sous 20 µl dans un volume final de 100 µl du mélange réactionnel à base de tampon HEPES 50 mM pH 7,5, $MgCl_2$ 5 mM, KCl 40 mM, dithiothréitol 5 mM, Triton X100 0,01 %.

Les inhibiteurs à tester, dissous initialement à 1 mM dans un solvant adéquat (DMF ou DMSO) sont dilués dans le tampon de dosage et sont ajoutés sous forme de 10 µl, en triplicate, dans le mélange réactionnel à une concentration 10 fois supérieure à leur concentration finale.

La réaction, effectuée dans des plaques de microtitrations OPTIPLATES 96® , est initiée par l'enzyme et dure soixante minutes à 37°C. Elle est stoppée par addition de 150 µl de mélange d'un tampon d'arrêt à pH 4 constitué de $H_3PO_4$ 0,2 M, $MgCl_2$ 1.5 mM, BSA 0.5 % (p/v), azoture de sodium 0.05 % (p/v) contenant 200 µg de billes PVT-streptavidine.

Après agitation lente de trente minutes (pour éliminer de la chemiluminescence), les plaques sont lues en [$^3$H]CPM dans un compteur a scintillation pour microplaques TOP COUNT® (PACKARD) où elles sont transformées en [$^3$H]DPM à partir d'une gamme d'agent coloré atténuant la scintillation (« quenching »).

Les pourcentages d'inhibition sont calculés par rapport à un témoin sans inhibiteur après soustraction de toutes les valeurs de celle d'un blanc ne contenant que les substrats et le tampon.

Les $CI_{50}$ sont calculées ou mesurées à partir des inhibitions obtenues à neuf concentrations différentes avec les logiciels Enzfitter® ou Grafit® .Les $CI_{50}$ figurant dans les résultats, sont la moyenne de mesures et calculs de deux $CI_{50}$. Les résultats obtenus sont rassemblés dans le tableau I.

TABLEAU I

| Produit | Activité inhibitrice vitro (KiRas) $CI_{50}$(μM) |
|---|---|
| Exemple 1 | 0,305 |
| Exemple 2 | 0,045 |
| Exemple 3 | 0,028 |
| Exemple 4 | 1,80 |
| Exemple 5 | 0,050 |
| Exemple 6 | 0,0575 |
| Exemple 7 | 0,050 |
| Exemple 8 | 0,100 |
| Exemple 9 | 0,055 |
| Exemple 10 | 0,160 |
| Exemple 11 | 0,345 |
| Exemple 12 | 0,045 |
| Exemple 13 | 0,140 |
| Exemple 14 | 0,210 |
| Exemple 15 | 0,170 |
| Exemple 16 | 0,360 |
| Exemple 17 | 0,235 |
| Exemple 18 | 0,050 |
| Exemple 19 | 0,181 |
| Exemple 20 | 0,140 |
| Exemple 21 | 0,111 |
| Exemple 22 | 0,131 |
| Exemple 23 | 0,277 |
| Exemple 24 | 0,084 |
| Exemple 25 | 0,193 |
| Exemple 26 | 0,145 |
| Exemple 27 | 0,009 |
| Exemple 28 | 0,054 |
| Exemple 29 | 0,140 |
| Exemple 30 | 0,074 |
| Exemple 31 | 0,101 |
| Exemple 32 | 0,106 |
| Exemple 33 | 0,242 |
| Exemple 34 | <0,02 |
| Exemple 35 | 0,084 |
| Exemple 36 | 0,120 |
| Exemple 37 | 0,088 |

TABLEAU I (suite)

| Produit | Activité inhibitrice vitro (KiRas) $CI_{50}$(μM) |
|---|---|
| Exemple 38 | 0,099 |
| Exemple 39 | 0,273 |
| Exemple 40 | 0,083 |
| Exemple 41 | 0,009 |
| Exemple 42 | 0,083 |
| Exemple 43 | 0,590 |
| Exemple 44 | 0,369 |
| Exemple 45 | 0,181 |
| Exemple 46 | 0,132 |
| Exemple 47 | 0,207 |
| Exemple 48 | 0,095 |
| Exemple 49 | 0,229 |
| Exemple 50 | 0,059 |
| Exemple 51 | 0,068 |
| Exemple 52 | 0,181 |
| Exemple 53 | 0,020 |
| Exemple 54 | 0,078 |
| Exemple 55 | 0,558 |
| Exemple 56 | 0,589 |
| Exemple 57 | 0,280 |
| Exemple 58 | 0,138 |
| Exemple 59 | 0,408 |
| Exemple 60 | 0,124 |
| Exemple 61 | 0,178 |
| Exemple 62 | 0,036 |
| Exemple 63 | <0,020 |
| Exemple 64 | 0,325 |
| Exemple 65 | 0,040 |
| Exemple 66 | 0,379 |
| Exemple 67 | 0,101 |
| Exemple 68 | 0,431 |
| Exemple 69 | 0,053 |
| Exemple 70 | 0,067 |
| Exemple 71 | 0,335 |
| Exemple 72 | 0,098 |
| Exemple 73 | 0,460 |
| Exemple 74 | 0,048 |
| Exemple 75 | 0,008 |

TABLEAU I (suite)

| Produit | Activité inhibitrice vitro (KiRas) $CI_{50}$(μM) |
|---|---|
| Exemple 76 | 0,244 |
| Exemple 77 | 0,204 |
| Exemple 78 | 0,048 |
| Exemple 79 | 0,045 |
| Exemple 80 | 0,067 |
| Exemple 81 | 0,018 |
| Exemple 82 | 0,097 |
| Exemple 83 | 0,319 |
| Exemple 84 | 0,008 |
| Exemple 85 | 0,508 |
| Exemple 86 | 0,005 |
| Exemple 87 | 0,064 |
| Exemple 88 | 0,404 |
| Exemple 89 | 0,005 |
| Exemple 90 | 0,068 |
| Exemple 91 | 0,183 |
| Exemple 92 | 0,028 |
| Exemple 93 | 0,786 |
| Exemple 94 | 0,557 |
| Exemple 95 | 0,177 |
| Exemple 96 | 0,955 |
| Exemple 97 | 0,040 |
| Exemple 98 | 0,663 |
| Exemple 99 | 0,018 |
| Exemple 100 | < 0,019 |
| Exemple 101 | <0,019 |

## EXEMPLE 103

L'activité des composés selon l'invention peut également être évaluée par la capacité desdits composés à inhiber la croissance en agar de clones issus de lignées tumorales humaines. Par exemple des cellules de la lignée de carcinome colique humain HCT116, fournies par l'ATCC, sont mises à pousser en monocouche dans un milieu de culture, Dubelcco modifié Eagle, contenant 2 mM de L-glutamine, 200 U/ml de pénicilline, 200 μg/ml de streptomycine complémenté par 10% en volumes de sérum de veau foetal inactivé à la chaleur. Les cellules en croissance exponentielle sont trypsinisées, lavées au PBS et diluées à une concentration finale de 5000 cellules/ml dans du milieu de culture complet. Les inhibiteurs à tester, ou le solvant de contrôle sont alors ajoutés, sous un volume de 50 μl, à 2,5 ml de suspension de cellules, préparée précédemment, on ajoute ensuite 0,4 ml d'une solution d'agar, Noble Difco, maintenue à 45°C puis on mélange. Le milieu ainsi obtenu est immédiatement jeté dans des boites de Pétri, maintenu cinq minutes à 4°C puis mis à incuber à 37°C sous une atmosphère de 5% de $CO_2$. Les nombre de clones cellulaires (> 50 cellules) est compté après douze jours d'incubation à 37°C sous une atmosphère de 5% de $CO_2$. Chaque inhibiteur est testé en duplicate aux concentrations finales en agar de 10, 1, 0,1 0,01 et 0,001 μg/ml. Les résultats sont exprimésen pourcentage d'inhibition de clonogénicité par rapport aux contrôles non traités. Les doses inhibitrices $IC_{50}$ sont déterminées graphiquement à partir des moyennes semi-logarithmiques des valeurs obtenues pour chaque concentration.

Les produits selon l'invention inhibent de 50% la farnésylation de la protéine Ras a des concentrations comprises entre 0.1 nM et 100 μM.

**EXEMPLE 104**

L'activité anti-tumorale des composés selon l'invention peut également être mise en évidence par essai *in vivo* chez la souris, selon les tests et méthodologies habituellement utilisées (Corbett et al. Cancer Research, 42, 1707-1715 (1982) ; Corbett et al. Cancer Treatment report, 66, 1187-1200 (1982) ; Corbett et al. 40, 2660-2680, (1977)).

On étudie l'influence des composés selon l'invention administrés chez des souris sur la croissance de carcinomes d'origine humaine greffés en sous-cutané chez ces souris.

Au jour 0 de l'expérience, les souris reçoivent un fragment tumoral (30-60 mg) sous la peau en bilatéral à l'aide d'un trocart. L'implantation bilatérale des tumeurs assure un poids tumoral par souris plus uniforme et permet ainsi de réduire le nombre d'animaux par groupe. Les souris sont ensuite réparties dans les différents groupes traités et non traités.

Le produit est mis en suspension dans une solution aqueuse à des concentrations allant de 0,01 à 1200 mg/ml, correspondant à des doses de 0,1 à 10000 mg/kg.

Les traitements ou leur excipient éventuel sont administrés tous les jours par voie orale, sous un volume maximal de 0,2 ml, la durée d'administration dépendant du temps de doublement de la tumeur.

Les tumeurs sont mesurées 2 à 3 fois par semaine, à l'aide d'un pied à coulisse, selon deux mesures en mm qui sont ensuite converties en poids tumoral selon la formule suivante :

$$\text{poids tumoral (mg)} = \frac{\text{Longueur (mm) x largeur}^2\ (\text{mm}^2)}{2}$$

Un produit actif est un produit qui permet de limiter la croissance de la tumeur après son administration.

L'exemple suivant peut être cité à titre illustratif et non limitatif de la présente invention ;

Une stabilisation tumorale a été obtenue à la dose 400 mg/kg/adm. du produit de l'exemple 3 administré par voie orale sur des souris greffées en sous-cutané avec la lignée de carcinome colique humain HCT116 fournie par l'ATCC (Brattain et al. Cancer Research, 41, 1751-1756 (1981))

**EXEMPLE 105**

EXEMPLE A

On prépare, selon la technique habituelle, des gélules dosées à 50 mg de produit actif ayant la composition suivante :

- Produit actif 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

EXEMPLE B

On prépare selon la technique habituelle des comprimés dosés à 50 mg de produit actif ayant la composition suivante :

- Produit actif 50 mg
- Lactose 104 mg
- Cellulose 40 mg
- Polyvidone 10 mg
- Carboxyméthylamidon sodique 22 mg
- Talc 10 mg

- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

EXEMPLE C

On prépare une solution injectable contenant 50 mg de produit actif ayant la composition suivante :

- Produit actif 50 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml

## Revendications

1. Composés de formule générale I

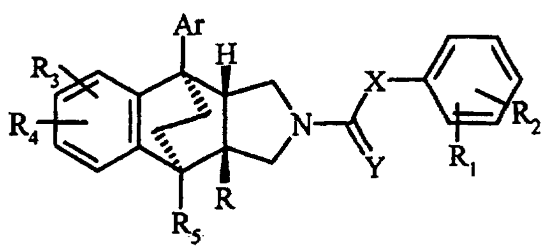

(I)

dans laquelle:

- Ar représente
    - un radical phényle substitué par un ou plusieurs atomes ou radicaux, identiques ou différents, choisis parmi les atomes d'halogène et les radicaux alcoyles contenant 1 à 4 atomes de carbone, tel que méthyle, alcényles contenant 2 à 4 atomes de carbone, hydroxy, mercapto, alcoylthio, alcoylsulfonyle ou alcoylsulfinyle, amino, alcoylamino ou dialcoylamino, formyle, alcoylcarbonyle, carboxy, alcoxycarbonyle, carbamoyle, alcoylcarbamoyle ou dialcoylcarbamoyle, cyano ou trifluorométhyle, alcoxy contenant 1 à 4 atomes de carbone, tel que méthoxy, dont la portion alcoyle est éventuellement perhalogénée, tel que trifluorométhoxy, ou
    - un radical phényle condensé à un hétérocycle de 4 à 7 chaînons contenant un ou plusieurs hétéroatomes choisis parmi les atomes d'oxygène, d'azote et de soufre,
    - un radical aromatique polycyclique,
    - un radical aromatique hétérocyclique de 5 à 12 chaînons incorporant un ou plusieurs hétéroatomes choisis parmi les atomes d'oxygène, d'azote et de soufre, lié au cycle condensé par une liaison carbone-carbone,

  avec pour l'ensemble de ces radicaux, alcoyl contenant 1 à 4 atomes de carbone,
- R représente un radical carboxy, ou un radical -COOMe, ou encore un radical - $CON(R_7)(R_8)$ pour lequel lorsque $R_7$ représente un atome d'hydrogène, $R_8$ représente un radical méthyle substitué par un radical 2-, 3- ou 4- pyridyle, ou le N-oxyde de pyridine
- $R_1$ et $R_2$, représentent un atome d'hydrogène et l'autre des symboles représente un radical méthoxy.
- ou bien $R_3$ et $R_4$ représentent chacun un atome d'hydrogène, ou bien l'un des symboles $R_3$ ou $R_4$ représente un atome d'hydrogène et l'autre des symboles $R_3$ ou $R_4$ représente un radical méthoxy.
- $R_5$ représente un atome d'hydrogène ou un radical alcoyle, un radical alcoylthio, avec pour la définition de $R_5$, alcoyle contenant 1 à 4 atomes de carbone;

- X représente un groupement méthylène ou vinyldiyle et
- Y représente un atome d'oxygène ou de soufre,

sous forme racémique ou leurs isomères optiques ainsi que les sels du produit de formule générale (I).

2. Composés selon la revendication 1 **caractérisés en ce que** Ar représente un radical 2,3-dihydro-1,4-benzodioxin-6-yle, 2,3-dihydrobenzofuran-5-yl, 2,3-dihydrobenzopyran-6-yle, 1- ou 2-naphtyle, 5-indanyle, ou 1,2,3,4-tétrahydronapht-6-yle ou un radical phényle substitué en position 4 par un radical méthyle, trifluorométhyle ou méthoxy.

3. Composés selon l'une quelconque des revendications précédentes **caractérisés en ce que** Ar représente un radical 2,3-dihydro-1,4-benzodioxin-6-yle ou 2,3-dihydrobenzofuran-5-yl, ou un radical phényle substitué en position 4 par un radical méthyle, trifluorométhyle ou méthoxy.

4. Composés selon l'une quelconque des revendications précédentes **caractérisés en ce que** $R_5$ représente un atome d'hydrogène ou un radical méthyle.

5. Composés selon l'une quelconque des revendications précédentes **caractérisés en ce que** Y représente un atome d'oxygène.

6. Composés selon l'une quelconque des revendications précédentes **caractérisés en ce qu'**ils se présentent sous forme dextrogyre.

7. Composé **caractérisé en ce qu'**il s'agit d'un composé choisi isolément parmi

   4,9-éthano-2-[2-(2-méthoxyphényl)propènoyl]-9-(4-méthylphényl)-2,3,3a,4,9,9a-hexahydro-1H-benzo[f]isoindole-3a-carboxylate de méthyle-(3aRS,4SR,9SR,9aRS) acide 4,9-éthano-2-(2-méthoxyphényl)propènoyl-9-(4-méthylphényl)-2,3,3a,4,9,9a-hexahydro-1H-benzo[f]isoindole-3a-carboxylique-(3aRS,4SR,9SR,9aRS)

   acide 4,9-éthano-2-[(2-méthoxyphényl)acétyl]-9-(4-méthylphényl)-2,3,3a,4,9,9a-hexahydro-1H-benzo[f]isoindole-3a-carboxylique-(3aRS,4SR,9SR,9aRS),

   acide 4,9-éthano-9-(4-méthoxyphényl)-2-[(2-méthoxyphényl)acétyl]-2,3,3a,4,9,9a-hexahydro-1H-benzo[f]isoindole-3a-carboxylique (3aRS,4SR,9SR,9aRS),

   acide 4,9-éthano-2-[(2-méthoxyphényl)acétyl]-9-(4-méthylsulfanylphényl)-2,3,3a,4, 9,9a-hexahydro-1H-benzo[f]isoindole-3a-carboxylique (3aRS,4SR,9SR,9aRS),

   acide 4,9-éthano-9-(4-fluorophényl)-2-[(2-méthoxyphényl)acétyl]-2,3,3a,4,9,9a-hexahydro-1H-benzo[f]isoindole-3a-carboxylique-(3aRS,4SR,9SR,9aRS),

   acide 4,9-éthano-2-[(2-méthoxyphényl)acétyl]-9-(3-méthylphényl)-2,3,3a,4,9,9a-hexahydro-1H-benzo[f]isoindole-3a-carboxylique-(3aRS,4SR,9SR,9aRS),

   acide 4,9-éthano-9-(3-méthoxyphényl)-2-[(2-méthoxyphényl)acétyl]-2,3,3a,4,9,9a-hexahydro-1H-benzo[f]isoindole-3a-carboxylique-(3aRS,4SR,9SR,9aRS),

   9-(3,4-diméthylphényl)-4,9-éthano-2-[(2-méthoxyphényl)acétyl]-2,3,3a,4,9,9a-hexahydro-1H-benze[f]isoindole-3a-carboxylate de méthyle-(3aRS,4SR,9SR,9aRS), acide 9-(3,4-diméthylphényl)-4,9-éthano-2-[(2-méthoxyphényl)acétyl]-2,3,3a,4,9,9a-hexahydro-1H-benzo[f]isoindole-3a-carboxylique-(3aRS,4SR,9SR,9aRS),

   3a-N-benzylcarbamoyl-4,9-éthano-2-[2-(2-méthoxyphényl)propènoyl]-9-(4-méthylphényl)-2,3,3a,4,9,9a-hexahydro-1H-benzo[f]isoindole-(3aRS,4SR,9SR,9aRS), 3a-carbamoyl-4,9-éthano-2-[2-(2-méthoxyphényl)propènoyl]-9-(4-méthylphényl)-2,3,3a,4,9,9a-hexahydro-1H-benzo[f]isoindole-(3aRS,4SR,9SR,9aRS),

   4,9-éthano-2-[2-(2-méthoxyphényl)propènoyl]-9-(4-méthylphényl)-2,3,3 a,4,9,9a-hexahydro-1H-bonze[f]isoindole-3a-hydroxamate de benzyle-(3aRS,4SR,9SR,9aRS),

acide 4,9-éthano-2-[2-(2-méthoxyphényl)propènoyl]-9-(4-méthylphényl)-,3,3a,4,9,9a-hexahydro-1H-benzo[f]isoindole-3a-hydroxamique-(3aRS,4SR,9SR,9aRS)

4,9-éthano-2-[2-(2-méthoxyphényl)propènoyl]-9-(4-méthylphényl)-2,3,3a,4,9,9a-hexahydro-1H-benzo[f]isoindole-3a-N-(3-pyridylméthyl)-carboxamide-(3aRS,4SR, 9SR,9aRS)

4,9-éthano-2-[2-(2-méthoxyphényl)propènoyl]-9-(4-méthylphényl)-2,3,3a,4,9,9a-hexahydro-1H-benzo[f]isoindole-3a-N-(4-pyridylméthyl)-carboxamide-(3aRS,4SR,9SR,9aRS)

4,9-éthano-2-[2-(2-méthoxyphényl)propènoyl]-9-(4-méthylphényl)-2,3,3a,4,9,9a-hexahydro-1H-benzo[f]isoindole-3a-hydroxamate de méthyle-(3aRS,4SR,9SR,9aRS)

4,9-éthano-2-[2-(2-méthoxyphényl)propènoyl]-9-(4-méthylphényl)-2,3,3a,4,9,9a-hexahydro-1H-benzo[f]isoindole-3a-N',N'-diméthylcarbohydrazide-(3aRS,4SR,9SR,9aRS)

4,9-éthano-2-[2-(2-méthoxyphényl)propènoyl]-9-(4-méthylphényl)-2,3,3a,4,9,9a-hexahydro-1H-benzo[f]isoindole-3a-N'-phénylcarbohydrazide-(3aRS,4SR,9SR,9aRS) 4,9-éthano-2-[2-(2-méthoxyphényl)propènoyl]-9-(4-méthylphényl)-2,3,3a,4,9,9a-hexahydro-1H-benzo[f]isoindole-3a-N',N'-pentaméthylènecarbohydrazide-(3aRS,4SR,9SR,9aRS)

4,9-éthano-2-[2-(méthoxyphényl)propènoyl]-9-(4-méthylphényl)-3a-phénylsulfonylaminocarbonyl-2,3,3a,4,9,9a-hexahydro-1H-benzo[f]isoindole-(3aRS,4SR,9SR,9aRS)

4,9-éthano-2-[2-(2-méthoxyphényl)propènoyl]-9-(4-méthylphényl)-2,3,3a,4,9,9a-hexahydro-1H-benzo[f]isoindole-3a-N-(N-oxo-3pyridyl)méthyl-carboxamide-(3aRS,4SR,9SR,9aRS)

4,9-éthano-2-[2-(2-méthoxyphényl)propènoyl]-9-(4-méthylphényl)-2,3,3a,4,9,9a-hexahydro-1H-benzo[f]isoindole-3a-N'-(4-méthoxyphényl)carbohydrazide-(3aRS,4SR,9SR,9aRS)

4,9-éthano-2-[2-(2-méthoxyphényl)propènoyl]-9-(4-méthylphényl)-2,3,3a,4,9,9a-hexahydro-1H-benzo[f]isoindole-3a-N'-méthyl,N'-phénylcarbohydrazide-(3aRS,4SR,9SR,9aRS)

4,9-éthano-2-[2-(2-méthoxyphényl)propènoyl]-9-(4-méthylphényl)-2,3,3a,4,9,9a-hexahydro-1H-benzo[f]isoindole-3a-N'-(2-méthylphényl)carbohydrazide-(3aRS,4SR,9SR,9aRS)

9-(2,3-dihydro-1,4-benzodioxin-6-yl)-4,9-éthano-2-[2-(2-méthoxy phényl)propènoyl]-2,3,3a,4,9,9a-hexahydro-1H-benzo[f]isoindole-3a-carboxylate de méthyle-(3aRS,4SR,9SR,9aRS)

acide 9-(2,3-dihydro-1,4-benzodioxin-6-yl)-4,9-éthano-2-[2-(2-méthoxyphényl) propènoyl]-2,3,3a,4,9,9a-hexahydro-1H-benzo[f]isoindole-3a-carboxylique-(3aRS,4SR,9SR,9aRS)

9-(2,3-dihydro-1,4-benzodioxin-6-yl)-4,9-éthano-2-[2-(2-méthoxy phényl)propènoyl)]- 2,3,3a,4,9,9a-hexahydro-1H-benzo[f]isoindole-3a-[N-(3-pyridylméthyl)carboxamide-(3aRS,4SR,9SR,9aRS)

9-(2,3-dihydro-1,4-benzodioxin-6-yl)-4,9-éthano-2-[2-(2-méthoxy phényl)propènoyl)]-2,3,3a,4,9,9a-hexahydro-1H-benzo[f]isoindole-3a-N-(2-thiényhnéthyl)carboxamide-(3aRS,4SR,9SR,9aRS)

acide 4,9-éthano-2-[(2-méthoxyphényl)acétyl]-9-(3,4,5-triméthylphényl)-2,3,3a,4,9,9a-hexahydro-1H-benzo[f]isoindole-3a-carboxylique-(3a*RS*,4*SR*,9*SR*,9a*RS*)

acide 4,9-éthano-2-[2-(2-méthoxyphényl)propènoyl]-9-(2-méthyl-phényl)-2,3,3a,4,9,9a-hexahydro-1H-benzo[f]isoindole-3a-carboxylique-(3aRS, 4SR,9SR,9aRS)

4,9-éthano-2-[2-(2-méthoxyphényl)propènoyl]-9-(4-trifluorométhylphényl)-2,3,3a,4,9,9a-hexahydro-1H-benzo[f]isoindole-3a-carboxylate de méthyle-(3aRS,4SR,9SR,9aRS)

acide 4,9-éthano-2-[2-(2-méthoxyphényl)propènoyl]-9-(4-trifluorométhyl-phényl)-2,3,3a,4,9,9a-hexahydro-1H-benzo[f]isoindole-3a-carboxylique-(3aRS, 4SR,9SR,9aRS)

4,9-éthano-2-[2-(2-méthoxyphényl)propènoyl]-9-(4-trifluorométhylphényl)-2,3,3a,4,9,9a-hexahydro-1H-benzo[f]isoindole-3a-N-(4-pyridylméthyl)-carboxamide-(3aRS,4SR,9SR,9aRS)

4,9-éthano-2-[2-(2-méthoxyphényl)propènoyl]-9-(4-trifluorométhylphényl)-2,3,3a,4,9,9a-hexahydro-1H-benzo[f]isoindole-3a-N'-benzoyl-carbohydrazide-(3aRS,4SR,9SR,9aRS)

4,9-éthano-2-[2-(2-méthoxyphényl)propènoyl]-9-(4-trifluorométhylphényl)-2,3,3a,4,9,9a-hexahydro-1H-benzo[f]isoindole-3a-N'-phényl-carbohydrazide-(3aRS,4SR,9SR,9aRS)

4,9-éthano-2-[2-(2-méthoxyphényl)propènoyl]-9-(2-naphtyl)-2,3,3a,4,9,9a-hexahydro-1H-benzo[f]isoindole-3a-carboxylate de méthyle-(3aRS,4SR,9SR,9aRS)

acide 4,9-éthano-2-[2-(2-méthoxyphényl)propènoyl]-9-(2-naphtyl)-2,3,3a,4,9,9a-hexahydro-1H-benzo[f]isoindole-3a-carboxylique-(3aRS,4SR,9SR, 9aRS)

4,9-éthano-2-[2-(2-méthoxyphényl)propènoyl]-9-(5-méthyl-2-thiényl)-2,3,3a,4,9,9a-hexahydro-1H-benzo[f]isoindole-3a-carboxylate de méthyle-(3aRS,4SR,9SR,9aRS)

acide 4,9-éthano-2-[2-(2-méthoxyphényl)propènoyl]-9-(5-méthyl-2-thiényl)-2,3,3a,4,9,9a-hexahydro-1H-benzo[f]isoindole-3a-carboxylique-(3aRS,4SR, 9SR,9aRS)

9-(4-bromophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS, 4SR, 9SR,9aRS)

acide 9-(4-bromophényl)-4,9-éthano-2-[2-(2-méthoxyphényl)propènoyl]-2,3,3a,4,9,9a-hexahydro-1H-benzo[f]isoindole-3a-carboxylique-(3aRS,4SR,9SR, 9aRS)

acide 9-(3,4-dichlorophényl)-4,9-éthano-2-[2-(2-méthoxyphényl) propènoyl]-2,3,3a,4,9,9a-hexahydro-1H-benzo[f]isoindole-3a-carboxylique-(3aRS, 4SR,9SR,9aRS)

4,9-éthano-2-[2-(2-méthoxyphényl)propènoyl]-9-(4-chlorophényl)-2,3,3a,4,9,9a-hexahydro-1H-benzo[f]isoindole-3a-carboxylate de méthyle-(3aRS, 4SR,9SR,9aRS)

acide 9-(4-chlorophényl)-4,9-éthano-2-[2-(2-méthoxyphényl) propènoyl]-2,3,3a,4,9,9a-hexahydro-1H-benzo[f]isoindole-3a-carboxylique-(3aRS, 4SR,9SR,9aRS)

acide 4,9-éthano-2-[2-(2-méthoxyphényl)propènoyl]-9-(4-méthoxy-3-méthylphényl)-2,3,3a,4,9,9a-hexahydro-1H-benzo[f]isoindole-3a-carboxylique-(3aRS, 4SR,9SR,9aRS)

4,9-éthano-2-[2-(2-méthoxyphényl)propènoyl]-9-(3-indolyl)-2,3,3a,4,9,9a-hexahydro-1H-benzo[f]isoindole-3a-carboxylate de méthyle-(3aRS, 4SR,9SR,9aRS)

acide 4,9-éthano-9-(4-isopropylphényl)-2-[(2-méthoxyphényl)acétyl]-2,3,3a,4,9,9a-hexahydro-1H-benzo[f]isoindole-3a-carboxylique-(3aRS,4SR,9SR, 9aRS)

acide 4,9-éthano-9-(4-isopropylphényl)-2-[2-(2-méthoxyphényl)propenoyl]-2,3,3a,4,9,9a-hexahydro-1H-benzo[f]isoindole-3a-carboxylique-(3aRS, 4SR,9SR, 9aRS)

4,9-éthano-2-[2-(2-méthoxyphényl)propènoyl]-9-(3-thiényl)-2,3,3a,4,9,9a-hexahydro-1H-benzo[f]isoindole-3a-carboxylate de méthyle-(3aRS, 4SR, 9SR,9aRS)

4,9-éthano-9-(4-éthylphényl)-2-[2-(2-méthoxyphényl)propènoyl]-2,3,3a,4,9,9a-hexahydro-1H-benzo[f]isoindole-3a-carboxylate de méthyle-(3aRS, 4SR, 9SR,9aRS)

acide 4,9-éthano-9-(4-éthylphényl)-2-[2-(2-méthoxyphényl) propènoyl]-2,3,3a,4,9,9a-hexahydro-1H-benzo[f]isoindole-3a-carboxylique-(3aRS,4SR,9SR, 9aRS)

9-(2,3-dihydrobenzofuran-5-yl)-4,9-éthano-2-[2-(2-méthoxy phényl)propènoyl]-2,3,3a,4,9,9a-hexahydro-1H-benzo[f]isoindole-3a-carboxylate de méthyle-(3aRS,4SR, 9SR, 9aRS)

acide 9-(2,3-dihydrobenzofuran-5-yl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR, 9SR, 9aRS)

9-(2,3-dihydrobenzofuran-5-yl)-4,9-éthano-2-[2-(2-méthoxy phényl)propènoyl)]-2,3,3a,4,9,9a-hexahydro-1H-benzo[f]isoindole-3a-N-(3-pyridylméthyl)carboxamide-(3aRS,4SR,9SR,9aRS)

4,9-éthano-9-(4-fluorophényl)-2-[2-(2-méthoxyphényl)propènoyl]- 2,3,3a,4,9,9a-hexahydro-1H-benzo[f]isoindole-3a-carboxylate de méthyle-(3aRS, 4SR,9SR,9aRS)

acide 4,9-éthano-9-(4-fluorophényl)--2-[2-(2-méthoxyphényl) propènoyl2,3,3a,4,9,9a-hexahydro-1H-benzo[f] isoindole-3a-carboxylique-(3aRS, 4SR,9SR,9aRS)

9-(4-chloro-3-fluorophényl)-4,9-éthano-2-[2-(2-méthoxyphényl) propènoyl2,3,3a,4,9,9a-hexahydro-1H-benzo[f]isoindole-3a-carboxylate de méthyle-(3aRS, 4SR,9SR,9aRS)

acide 9-(4-chloro-3-fluorophényl)-4,9-éthano-2-[2-(2-méthoxyphényl)propènoyl]-2,3,3a,4,9,9a-hexahydro-1H-benzo[f]isoindole-3a-carboxylique-(3aRS, 4SR,9SR,9aRS)

4,9-éthano-2-[2-(2-méthoxyphényl)propènoyl]-9-(3-méthylphényl)-2,3,3a,4,9,9a-hexahydro-1H-benzo[f] isoindole-3a-carboxylate de méthyle-(3aRS,4SR,9SR,9aRS)

acide 4,9-éthano-2-[2-(2-méthoxyphényl)propènoyl]-9-(3-méthylphényl)-2,3,3a,4,9,9a-hexahydro-1H-benzo [f]isoindole-3a-carboxylique-(3aRS, 4SR,9SR,9aRS)

9-(1,3-benzodioxol-5-yl)-4,9-éthano-2-[2-(2-méthoxyphényl) propènoyl]2,3,3a,4,9,9a-hexahydro-1H-benzo[f] isoindole-3a-carboxylate de méthyle-(3aRS,4SR,9SR,9aRS) 9-(3,4-diméthylphényl)-4,9-éthano-2[2-(2-méthoxy phényl)propènoyl]-2,3,3a,4,9,9a-hexahydro-1H-benzo[f]isoindole-3a-carboxylate de méthyle-(3aRS, 4SR,9SR,9aRS)

acide-9-(3,4-diméthylphényl)-4,9-éthano-2[2-(2-méthoxyphényl) propènoyl]-2,3,3a,4,9,9a-hexahydro-1H-benze[f]isoindole-3a-carboxylique-(3aRS, 4SR,9SR,9aRS)

4,9-éthano-9-(4-méthoxyphényl)-2-[2-(2-méthoxyphényl)propènoyl]-2,3,3a,4,9,9a-hexahydro-1H-benzo[f] isoindole-3a-carboxylate de méthyle-(3aRS,4SR,9SR,9aRS)

acide 4,9-éthano-9-(4-méthoxyphényl)-2-[2-(2-méthoxyphényl) propènoyl]-2,3,3a,4,9,9a-hexahydro-1H-benzo[f]isoindole-3a-carboxylique-(3aRS, 4SR,9SR,9aRS)

4,9-éthano-9-(4-méthoxyphényl)-2-[2-(2-méthoxyphényl)propènoyl]-2,3,3a,4,9,9a-hexahydro-1H-benzo[f] isoindole-3a-N-(3-pyridylméthyl)-carboxamide-(3aRS,4SR,9SR,9aRS)

4,9-éthano-9-(4-méthoxyphényl)-2-[2-(2-méthoxyphényl)propènoyl]- 2,3,3a,4,9,9a-hexahydro-1H-benzo[f] isoindole-3a-N'-phénylcarbohydrazide-(3aRS,4SR,9SR,9aRS)

acide 4,9-éthano-]-9-(4-méthoxyphényl)-2-[2-(2-méthoxyphényl)propènoyl-2,3,3a,4,9,9a-hexahydro-1H-benzo[f]isoindole-3a-hydroxamique-(3aRS,4SR,9SR,9aRS)

4,9-éthano-9-(4-méthoxyphényl)-2-[2-(2-méthoxyphényl)propènoyl]-2,3,3a,4,9,9a-hexahydro-1H-benzo[f] isoindole-3a-N'-(3-pyridyl)carbohydrazide-(3aRS,4SR,9SR,9aRS)

4,9-éthano-9-(4-méthoxyphényl)-2-[2-(2-méthoxyphényl)propènoyl]-2,3,3a,4,9,9a-hexahydro-1H-benzo[f] isoindole-3a-N-(3-thiénylméthyl)-carboxamide-(3aRS,4SR,9SR,9aRS)

acides 2-{4,9-éthano-9-(4-méthoxyphényl)-2-[2-(2-méthoxyphényl)propènoyl]-2,3,3a,4,9,9a-hexahydro-1H-benzo[f]isoindole-3a-carbonylamino-(3aRS,4SR,9SR,9aRS)}phénylacétiques-(RS) et (SR)

acide 4,9-éthano-2-[2-(2-méthoxyphényl)propènoyl]-9-(4-trifluorométhoxyphényl)-2,3,3a,4,9,9,9a-hexahydro-1H-benzo[f]isoindole-3a-carboxylique-(3aRS, 4SR, 9SR,9aRS)

9-(3-bromophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS, 4SR, 9SR,9aRS)

acide 9-(3-bromophényl)-4,9-éthano-2-[2-(2-méthoxyphényl) propènoyl]-2,3,3a,4,9,9a-hexahydro-1H-benzo[f]isoindole-3a-carboxylique-(3aRS,4SR,9SR, 9aRS)

4,9-éthano-9(3-fluorophényl-2-[2-(2-méthoxyphényl)propènoyl]-2,3,3a,4,9,9a-hexahydro-1H-benzo[flisoindole-3a-carboxylate de méthyle-(3aRS, 4SR,9SR,9aRS) acide 4,9-éthano-9-(3-fluorophényl)-2-[2-(2-méthoxyphényl)propènoyl])-2,3,3a,4,9,9a-hexahydro-1H-benzo[f]-isoindole-3a-carboxylique-(3aRS, 4SR, 9SR, 9aRS)

4,9-éthano-9-(3-fluorophényl)-2-[2-(2-méthoxyphényl)propènoyl)]- 2,3,3a,4,9,9a-hexahydro-1H-benzo[f]isoindole-3a-[N-(3-pyridylméthyl)carboxamide-(3aRS,4SR,9SR,9aRS)

9-(3-chlorophényl)-4,9-éthano-2-[2-(2-méthoxyphényl)propènoyl]-2,3,3a,4,9,9a-hexahydro-1H-benzo[f]isoindole-3a-carboxylate de méthyle-(3aRS,4SR,9SR,9aRS) acide 9-(3-chlorophényl)-4,9-éthano-2-[2-(2-méthoxyphényl) propènoyl]-2,3,3a,4,9,9a-hexahydro-1H-benzo[f]isoindole-3a-carboxylique-(3aRS, 4SR,9SR, 9aRS)

9-(3-chlorophényl)-4,9-éthano-2-[2-(2-méthoxyphényl)propènoyl)]-2,3,3 a,4,9,9a-hexahydro-1H-benzo[f]isoindole 3a-[N-(3-pyridylméthyl)carboxamide-(3aRS,4SR,9SR,9aRS)

9-(3-N,N-diméthylaminophényl)-4,9-éthano-2-[2-(2-méthoxyphényl)propènoyl]-2,3,3a,4,9,9,9a-hexahydro-1H-benzo[f]isoindole-3a-carboxylate de méthyle-(3aRS, 4SR, 9SR,9aRS)

acide 9-(3-N,N-diméthylaminophényl)-4,9-éthano-2-[2-(2-méthoxyphényl) propènoyl]-2,3,3a,4,9,9,9a-hexahydro- 1H-benzo[f]isoindole-3a-carboxylique-(3aRS,4SR,9SR, 9aRS)

chlorhydrate de 9-(3-aminophényl)-4,9-éthano-2-[2-(2-méthoxyphényl) propènoyl]-2,3,3a,4,9,9,9a-hexahydro-1H-benzo[f]isoindole-3a-carboxylate de méthyle-(3aRS, 4SR,9SR,9aRS)

9-(4-N,N-diméthylaminophényl)-4,9-éthano-2-[2-(2-méthoxyphényl)propènoyl]-2,3,3a,4,9,9,9a-hexahydro-1H-benzo[f]isoindole-3a-carboxylate de méthyle-(3aRS, 4SR,9SR,9aRS)

acide 9-(4-cyanophényl)-4,9-éthano-2-[2-(2-méthoxyphényl)propènoyl]-2,3,3a,4,9,9,9a-hexahydro-1H-benzo[f]isoindole-3a-carboxylique-(3aRS,4SR,9SR, 9aRS)

9-(3-cyanophényl)-4,9-éthano-2-[2-(2-méthoxyphényl) propènoyl]-2,3,3a,4,9,9,9a-hexahydro-1H-benzo[f]isoindole-3a-carboxylate de méthyle-(3aRS,4SR,9SR,9aRS)

acide 4,9-éthano-9-(3-hydroxy-4-méthoxyphényl)-2-[2-(2-méthoxyphényl)propènoyl]-2,3,3a,4,9,9a-hexahydro-1H-benzo[f]isoindole-3a-carboxylique-(3aRS,4SR,9SR,9aRS)

4,9-éthano-5-méthoxy-2-[2-(2-méthoxyphényl)-propènoyl]-9-(4-méthylphényl)-2,3,3a,4,9,9a-hexahydro-1H-benzo[f]isoindole-3a-carboxylate de méthyle-(3aRS,4SR, 9SR,9aRS)

acide 4,9-éthano-5-méthoxy-2-[2-(2-méthoxyphényl)-propènoyl]-9-(4-méthylphényl)-2,3,3a,4,9,9a-hexahydro-1H-benzo[f]isoindole-3a-carboxylique-(3aRS,4SR, 9SR,9aRS)

acide 4,9-éthano-2[2-(2-méthoxyphényl)-2-propènoyl]-4-méthyl-9-(4-méthoxyphényl)-2,3,3a,4,9,9a-hexahydro-1H-benzo[f]isoindole-3a-carboxylique-(3aRS,4SR,9SR,9aRS)

sous forme racémique ou leurs isomères optiques ainsi que leurs sels.

**8.** Composé **caractérisé en ce qu'**il s'agit de

acide 4,9-éthano-2[2-(2-méthoxyphényl)propènoyl]-9-(4-méthylphényl)-2,3,3a,4,9,9a-hexahydro-1H-benzo[f] isoindole-3a-carboxylique-(3aRS,4SR, 9SR,9aRS)
sous forme racémique ou ses isomères optiques, ainsi que ses sels.

**9.** Composé **caractérisé en ce qu'**il s'agit de
4,9-éthano-2-[2-(2-méthoxyphényl)propènoyl]-9-(4-méthylphényl)-2,3,3a,4,9,9a-hexahydro-1H-benzo[f]isoindole-3a-N-(3-pyridylméthyl)-carboxamide-(3aRS,4SR, 9SR,9aRS)
sous forme racémique ou ses isomères optiques, ainsi que ses sels.

**10.** Composé **caractérisé en ce qu'**il s'agit de
9-(2,3-dihydro-1,4-benzodioxin-6-yl)-4,9-éthano-2-[2-(2-méthoxy phényl)propènoyl)]- 2,3,3a,4,9,9a-hexahydro-1H-benzo[f] isoindole-3a-[N-(3-pyridylméthyl)carboxamide-(3aRS,4SR,9SR,9aRS)
sous forme racémique ou ses isomères optiques, ainsi que ses sels.

**11.** Composé **caractérisé en ce qu'**il s'agit de
acide 4,9-éthano-2-[2-(2-méthoxyphényl)propènoyl]-9-(4-trifluorométhyl-phényl)-2,3,3a,4,9,9a-hexahydro-1H-benzo[f]isoindole-3a-carboxylique-(3aRS, 4SR,9SR,9aRS)
sous forme racémique ou ses isomères optiques, ainsi que ses sels.

**12.** Composé **caractérisé en ce qu'**il s'agit de
9-(2,3-dihydrobenzofuran-5-yl)-4,9-éthano-2-[2-(2-méthoxyphényl)propènoyl]-2,3,3a,4,9,9a-hexahydro-1H-benzo[f]isoindole-3a-N-(3 -pyridylméthyl)carboxamide-(3aRS,4SR, 9SR, 9aRS)
sous forme racémique ou ses isomères optiques, ainsi que ses sels.

**13.** Composé **caractérisé en ce qu'**il s'agit de
acide 4,9-éthano-9-(4-méthoxyphényl)-2-[2-(2-méthoxyphényl) propènoyl]-2,3,3a,4,9,9a-hexahydro-1H-benzo[f] isoindole-3a-carboxylique-(3aRS, 4SR,9SR,9aRS)
sous forme racémique ou ses isomères optiques, ainsi que ses sels.

**14.** Composé **caractérisé en ce qu'**il s'agit de
acide 4,9-éthano-5-méthoxy-2-[2-(2-méthoxyphényl)-propènoyl]-9-(4-méthylphényl)-2,3,3a,4,9,9a-hexahydro-1H-benzo[f]isoindole-3a-carboxylique-(3aRS,4SR, 9SR,9aRS)
sous forme racémique ou ses isomères optiques, ainsi que ses sels.

**15.** Composé **caractérisé en ce qu'**il s'agit de
acide 4,9-éthano-2[2-(2-méthoxyphényl)-2-propènoyl]-4-méthyl-9-(4-méthoxyphényl)-2,3,3a,4,9,9a-hexahydro-1H-benzo[f]isoindole-3a-carboxylique-(3aRS,4SR,9SR,9aRS)
sous forme racémique ou ses isomères optiques, ainsi que ses sels.

**16.** Composé **caractérisé en ce qu'**il s'agit de
énantiomère dextrogyre de l'acide 4,9-éthano-2[2-(2-méthoxyphényl)propènoyl]-9-(4-méthylphényl)-2,3,3a, 4,9,9a-hexahydro-1H-benzo[f]isoindole-3a-carboxylique-(3aRS,4SR, 9SR,9aRS)

**17.** Composé **caractérisé en ce qu'**il s'agit de
énantiomère dextrogyre du 9-(2,3-dihydro-1,4-benzodioxin-6-yl)-4,9-éthano-2-[2-(2-méthoxy phényl)propènoyl)]-2,3,3a,4,9,9a-hexahydro-1H-benzo[f] isoindole-3a-[N-(3-pyridylméthyl)carboxamide-(3aRS,4SR,9SR,9aRS)

**18.** Procédé de préparation d'un composé de formule générale (I) selon l'une quelconque des revendications 1 à 17 pour lequel Y représente un atome d'oxygène ou de soufre et X représente un groupement méthylène ou vinyldiyle **caractérisé en ce que** l'on fait agir un acide de formule générale :

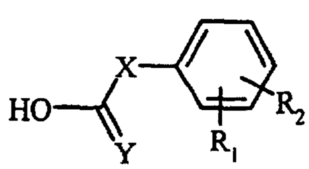

(II)

dans laquelle $R_1$ et $R_2$ sont définis selon la revendication 1, X tel que défini précédemment et Y représente un atome d'oxygène ou de soufre, de son ester méthylique ou d'un dérivé de cet acide tel qu'un halogénure ou l'anhydride, sur un produit de formule générale :

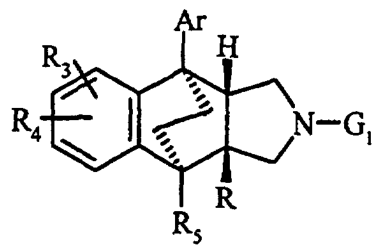

(III)

dans laquelle Ar, R, $R_3$, $R_4$ et $R_5$ sont définis selon la revendication 1 et $G_1$ représente un atome d'hydrogène.

**19.** Procédé de préparation d'un composé de formule générale (III)

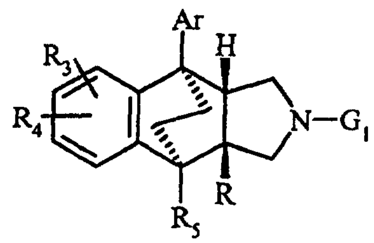

(III)

dans laquelle $R_3$, $R_4$, $R_5$, Ar et R sont tels que définis en revendication 1 et G1 représente un radical benzyle, à partir de composés de formule générale (IX):

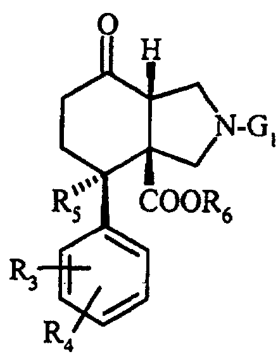

(IX)

dans laquelle $R_3$, $R_4$, $R_5$, $R_6$ et $G_1$ sont tels que définis précédemment **caractérisée en ce que** on passe par la formation d'un intermédiaire de formule générale (XV)

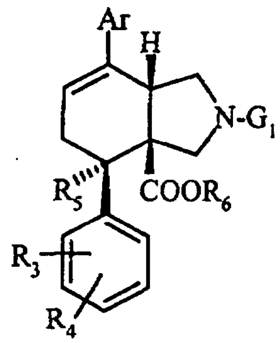

(XV)

dans laquelle $R_3$, $R_4$, $R_5$, $R_6$, Ar et $G_1$ sont tels que définis précédemment, et dont la transformation par cyclisation intramoléculaire de type Friedl-Crafts conduit au produit (III) telle que définie en revendication 18.

**20.** Composé **caractérisé en ce qu'**il est représenté par la formule générale XV

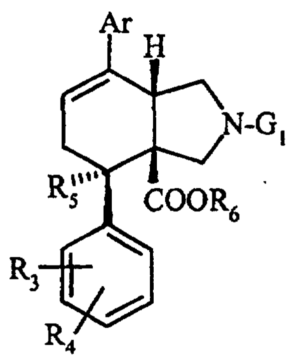

(XV)

dans laquelle $R_3$, $R_4$, $R_5$ et Ar sont tels que définis en revendication 1, $G_1$ représente un radical benzyle et $R_6$ représente un radical alcoyle contenant 1 à 3 atomes de carbone sous forme racémique ainsi que leurs isomères optiques.

**21.** Procédé de préparation d'un composé de formule générale (XV) selon la revendication 19 **caractérisé en ce que** l'on procède sur un composé de formule générale (IX)

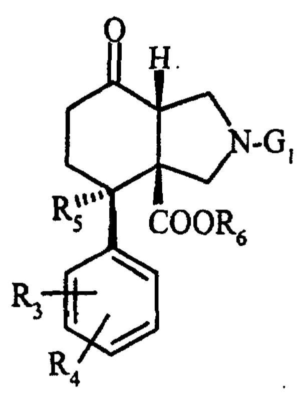

(IX)

dans laquelle $R_3$, $R_4$, $R_5$ et Ar sont tels que définis en revendication 1, $G_1$ représente un radical benzyle et $R_6$ représente un radical alcoyle contenant 1 à 3 atomes de carbone, à sa transformation, en position 7, soit en dérivé iodoéthylènique, par une réaction de Barton, soit en triflate d'énol puis à une réaction de couplage au palladium,

dite réaction de Suzuki avec un acide aryle boronique ou dite réaction de Stille avec un arylstannane

**22.** Procédé de préparation d'un composé de formule générale (III) selon la revendication 18, dans lequel Ar représente de préférence

- un noyau phényle substitué en position para, méta-para ou méta-para-méta' par des groupements donneur en électrons,
- un radical hétérocyclique riche en électrons ou
- un radical hétérocyclique convenablement substitué par des groupements donneurs d'électrons

**caractérisé en ce que** l'on fait agir un hydrocarbure aromatique ou hétérocyclique Ar-H, selon des réactions de cyclisation intermoléculaire puis intramoléculaire de type Friedel-Crafts, sur un composé de formule générale (IX) dans laquelle $R_3$, $R_4$, $R_5$ et Ar sont tels que définis en revendication 1, $G_i$ représente un radical benzyle et $R_6$ représente un radical alcoyle contenant 1 à 3 atomes de carbone dans un solvant organique en présence d'un excès d'acide fort ou d'un acide de Lewis.

**23.** Procédé de préparation d'un produit de formule générale (III)

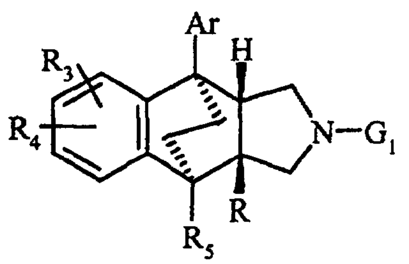

(III)

dans laquelle $R_3$, $R_4$, $R_5$, Ar sont tels que définis en revendication 1, G1 représente un radical benzyle et R représente un radical carboxy ou un radical de formule générale $COOR_6$ et $R_6$ représente un radical alcoyle contenant 1 à 3 atomes de carbone, **caractérisé en ce que** l'on procède par cyclisation intramoléculaire de type Friedel-Crafts par action de l'acide trifluorométhanesulfonique sur un produit de formule générale (VIII):

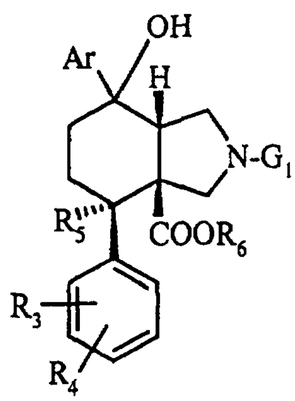

(VIII)

dans laquelle :

Ar, $R_3$, $R_4$, et $R_5$ sont tels que définis en revendication 1, $G_1$ représente un radical benzyle, $R_6$ représente un radical alcoyle contenant 1 à 3 atomes de carbone, suivie du remplacement du groupe $G_1$ par un atome d'hydrogène soit par hydrogénolyse puis éventuellement du remplacement de l'atome d'hydrogène par un radical *tert*-butoxycarbonyle, par action de l'anhydride *tert*-butoxycarbonyle dans un solvant organique, ou par un radical benzyloxycarbonyle, par action du chlorure de benzyloxycarbonyle, soit par action d'un chloroformiate d'alcoyle, suivie de l'hydrolyse acide du carbamate intermédiairement formé, éventuellement suivie de la saponification du produit obtenu.

**24.** Procédé de préparation d'un composé de formule générale (VIII) dans laquelle Ar, $R_3$, $R_4$, $R_5$, $R_6$ et $G_1$ sont tels que définis en revendication 23 **caractérisé en ce que** l'on procède à partir d'un composé de formule générale (IX)

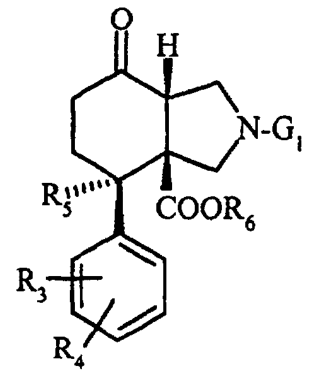

(IX)

dans laquelle $R_3$, $R_4$, $R_5$, $R_6$ et $G_1$ sont tels que définis en revendication 21,
soit par action d'un dérivé organomagnésien de formule générale Ar-Mg-X dans laquelle Ar est tel que défini précédemment et X représente un atome d'halogène, en présence de chlorure de cérium (III) anhydre dans un solvant organique à une température comprise entre 0°C et le reflux du mélange réactionnel,
soit par action d'un organolithien de formule générale Ar-Li dans laquelle Ar est tel que défini en revendication en opérant dans un solvant organique, à une température comprise entre -78° et -20°C.

**25.** Procédé de préparation d'un composé de formule générale (IX) tel que défini en revendication 21 ou 24, dans laquelle dans laquelle $R_3$, $R_4$, $R_5$ et Ar sont tels que définis en revendication 1, $G_1$ représente un radical benzyle et $R_6$ représente un radical alcoyle contenant 1 à 3 atomes de carbone, **caractérisé en ce que** l'on fait agir une N-trialcoylsilylméthyl-N-alcoxyméthyl- amine portant un groupement protecteur de la fonction amine sur un dérivé d'une cyclohexènone de formule générale :

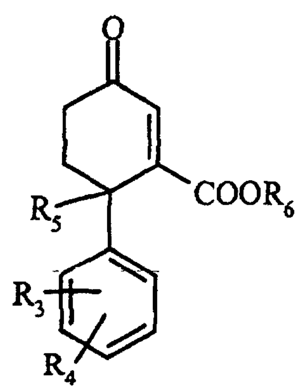

(X)

dans laquelle $R_3$, $R_4$ et $R_5$ sont tels que définis en revendication 1 et $R_6$ représente un radical alcoyle contenant 1 à 3 atomes de carbone.

**26.** Composition pharmaceutique **caractérisée en ce qu'**elle contient au moins un produit selon l'une des revendications précédentes 1 à 17 en association avec un ou plusieurs diluants ou adjuvants pharmaceutiquement acceptables qu'ils soient inertes ou biologiquement actifs.

**27.** Utilisation des composés selon l'une des revendications précédentes 1 à 17 pour la préparation de compositions pharmaceutiques utiles pour inhiber la protéine Farnésyle transférase.

**28.** Utilisation des composés selon l'une des revendications 1 à 17 pour la préparation de compositions pharmaceutiques pour le traitement des maladies liées à des proliférations cellulaires malignes ou bégnines, des cellules de divers tissus et/ou organes, comprenant les tissus musculaires, osseux ou conjonctifs, la peau, le cerveau, les

poumons, les organes sexuels, les systèmes lymhatiques ou rénaux, les cellules mammaires ou sanguines, le foie, l'appareil digestif, le pancréas et les glandes thyroïdes ou adrénales et incluant les pathologies suivantes : le psoriasis, la résténose, les tumeurs solides, le sarcome de Kaposi, les carcinomes, le cholangiocarcinome, le choriocarcinome, le neuroblastome, la tumeur de Wilms, la maladie de Hodgkin, les mélanomes, les tératocarcinomes, les gliomes, les myélomes multiples, les leucémies lymphocitaires chroniques, les lymphomes granulocitaires aigus ou chroniques, et les cancers tels que les cancers du pancréas, du colon, du poumon, de l'ovaire, du sein, du cerveau, de la prostate, du foie, de l'estomac, de la vessie ou des testicules.

**29.** Utilisation des composés de formule générale (I) selon l'une des revendications 1 à 17 pour la préparation de compositions pharmaceutiques utiles pour le traitement des maladies liées à des proliférations cellulaires par inhibition de la farnésyle transférase

**30.** Utilisation des composés de formule générale (I) selon l'une des revendications 1 à 17 pour la préparation de compositions pharmaceutiques pour le traitement de cancers.

**31.** Utilisation du composé de la revendication 16 pour la préparation de compositions pharmaceutiques pour le traitement de cancers.

**32.** Utilisation des composés de formule générale (I) selon l'une des revendications 1 à 17 pour la préparation de compositions pharmaceutiques pour le traitement du cancer du colon.

**33.** Utilisation des composés de formule générale (I) selon l'une des revendications 1 à 17 pour la préparation de compositions pharmaceutiques pour le traitement du cancer du pancréas.

**34.** Utilisation du composé de la revendication 16 pour la préparation de compositions pharmaceutiques pour le traitement du cancer du colon.

**35.** Utilisation du composé de la revendication 16 pour la préparation de compositions pharmaceutiques pour le traitement du cancer du pancréas.

**36.** Associations d'un produit selon l'une des revendications précédentes 1 à 17 avec un ou plusieurs composés compatibles et pharmacologiquement actifs et/ou avec un traitement radiothérapique afin d'obtenir un effet thérapeutique synergique.

**Patentansprüche**

**1.** Verbindungen der allgemeinen Formel I

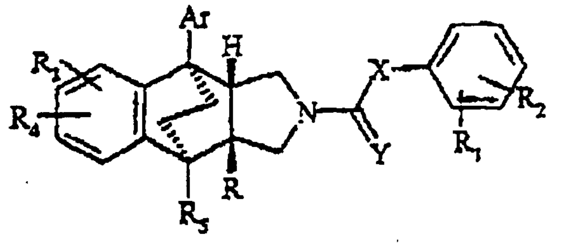

(I)

worin:

- Ar

    - einen Phenylrest, substituiert durch ein oder mehrere Atom(e) oder Rest(e), die gleich oder verschieden sind, ausgewählt aus Halogenatomen und Alkylresten mit 1 bis 4 Kohlenstoffatomen, wie Methyl, Alkenyl mit 2 bis 4 Kohlenstoffatomen, Hydroxy, Mercapto, Alkylthio, Alkylsulfonyl oder Alkylsulfinyl, Amino, Alkylamino oder Dialkylamino, Formyl, Alkylcarbonyl, Carboxy, Alkoxycarbonyl, Carbamoyl, Alkylcarbamoyl

oder Dialkylcarbamoyl, Cyano oder Trifluormethyl, Alkoxy mit 1 bis 4 Kohlenstoffatomen, wie Methoxy, dessen Alkylteil gegebenenfalls perhalogeniert ist, wie Trifluormethoxy, oder

- einen Phenylrest, kondensiert an einen Heterozyklus mit 4 bis 7 Kettengliedern, enthaltend ein oder mehrere Heteroatome, ausgewählt aus den Atomen Sauerstoff, Stickstoff und Schwefel,
- einen aromatischen polycyclischen Rest,
- einen aromatischen heterocyclischen Rest mit 5 bis 12 Kettengliedern, in die ein oder mehrere Heteroatom (e), ausgewählt aus den Atomen Sauerstoff, Stickstoff und Schwefel, eingebaut sind, gebunden an einen kondensierten Ring durch eine Kohlenstoff-Kohlenstoff-Bindung,

bedeutet, wobei für die Gesamtheit der Reste, Alkyl 1 bis 4 Kohlenstoffatome enthält,

- R einen Carboxyrest oder einen Rest -COOMe oder auch einen Rest CON($R_7$) ($R_8$) bedeutet, worin , wenn $R_7$ ein Wasserstoffatom bedeutet, $R_8$ einen Methylrest substituiert durch einen 2-, 3- oder 4-Pyridylrest oder Pyridin-N-oxid bedeutet,
- $R_1$ und $R_2$ ein Wasserstoffatom bedeuten und das andere der Symbole einen Methoxyrest bedeutet,
- oder auch $R_3$ und $R_4$ jeweils ein Wasserstoffatom bedeuten oder auch eines der Symbole $R_3$ oder $R_4$ ein Wasserstoffatom und das andere der Symbole $R_3$ oder $R_4$ einen Methoxyrest bedeutet,
- $R_5$ ein Wasserstoffatom oder einen Alkylrest, einen Alkylthiorest, worin für die Definition von $R_5$ Alkoyl 1 bis 4 Kohlenstoffatome enthält, bedeutet,
- X eine Methylen- oder Vinyldiylgruppe bedeutet und
- Y ein Sauerstoff- oder Schwefelatom bedeutet,

in racemischer Form oder ihre optischen Isomere, wie die Salze des Produkts der allgemeinen Formel (I).

2. Verbindungen nach Anspruch 1, **dadurch gekennzeichnet, dass** Ar einen 2,3-Dihydro-1,4-benzodioxin-6-yl-, 2,3-Dihydrobenzofuran-5-yl-, 2,3-Dihydrobenzopyran-6-yl-, 1- oder 2-Naphthyl-, 5-Indanyl- oder 1,2,3,4-Tetrahydronaphth-6-ylrest oder einen Phenylrest, der in Position 4 durch einen Methylrest, Trifluormethylrest oder Methoxyrest substituiert ist, bedeutet.

3. Verbindungen nach einem der vorstehenden Ansprüche, **dadurch gekennzeichnet, dass** Ar einen 2,3-Dihydro-1,4-benzodioxin-6-ylrest oder 2,3-Dihydrobenzofuran-5-ylrest oder einen Phenylrest, substituiert in Position 4 durch einen Methylrest, Trifluormethylrest oder Methoxyrest, bedeutet.

4. Verbindungen nach einem der vorstehenden Ansprüche, **dadurch gekennzeichnet, dass** $R_5$ ein Wasserstoffatom oder einen Methylrest bedeutet.

5. Verbindungen nach einem der vorstehenden Ansprüche, **dadurch gekennzeichnet, dass** Y ein Sauerstoffatom bedeutet.

6. Verbindungen nach einem der vorstehenden Ansprüche, **dadurch gekennzeichnet, dass** sie in rechtsdrehender Form vorliegen.

7. Verbindung, **dadurch gekennzeichnet, dass** es sich um die Verbindung, ausgewählt isoliert aus:

(3aRS,4SR,9SR,9aRS)-4,9-Ethano-2-[2-(2-methoxyphenyl)propenoyl]-9-(4-methylphenyl)-2,3,3a,4,9,9a-hexahydro-1H-benzo-[f]isoindol-3a-methylcarboxylat,
(3aRS,4SR,9SR,9aRS)-4,9-Ethano-2-(2-methoxyphenyl)propenyl-9-(4-methylphenyl)-2,3,3a,4,9,9a-hexyhydro-1H-benzo[f]isoindol-3a-carbonsäure,
(3aRS,4SR,9SR,9aRS)-4,9-Ethano-2-[(2-methoxyphenyl)acetyl]-9-(4-methylphenyl)-2,3,3a,4,9,9a-hexahydro-1H-benzo[f]isoindol-3a-carbonsäure,
(3aRS,4SR,9SR,9aRS)-4,9-Ethano-9-(4-methoxyphenyl)-2-[(2-methoxyphenyl)acetyl]-2,3,3a,4,9,9a-hexahydro-1H-benzo[f]-isoindol-3a-carbonsäure,
(3aRS,4SR,9SR,9aRS)-4,9-Ethano-2-[(2-methoxyphenyl)acetyl]-9-(4-methylsulfanylphenyl)-2,3,3a,4,9,9a-hexahydro-1H-benzo-[f] isoindol-3a-carbonsäure,
(3aRS,4SR,9SR,9aRS)-4,9-Ethano-9- [4-fluorphenyl)-2-[(2-methoxyphenyl)acetyl]-2,3,3a,4,9,9a-hexahydro-1H-benzo[f]-isoindol-3a-carbonsäure,
(3aRS,4SR,9SR,9aRS)-4,9-Ethano-2-[(2-methoxyphenyl)acetyl]-9-(3-methylphenyl)-2,3,3a,4,9,9a-hexahydro-1H-benzo[f]isoindol-3a-carbonsäure,
(3aRS,4SR,9SR,9aRS)-4,9-Ethano-9-(3-methoxyphenyl)-2-[(2-methoxyphenyl)acetyl]-2,3,3a,4,9,9a-hexahydro-1H-benzo[f]-isoindol-3a-carbonsäure,
(3aRS,4SR,9SR,9aRS)-9-(3,4-Dimethylphenyl)-4,9-ethano-2-[(2-methoxyphenyl)acetyl]-2,3,3a,4,9,9a-hexahydro-1H-benzo[f]-isoindol-3a-methylcarboxylat,
(3aRS,4SR,9SR,9aRS)-9-(3,4-Dimethylphenyl)-4,9-ethano-2-[(2-methoxyphenyl)acetyl]-2,3,3a,4,9a-hexahydro-1H-benzo[f]isoindol-3a-carbonsäure,
(3aRS,4SR,9SR,9aRS)-3a-N-Benzylcarbamoyl-4,9-ethano-2-[2-(2-methoxyphenyl)propenoyl]-9-(4-methylphenyl)-2,3,3a,4,9,9a-hexahydro-1H-benzo[f]isoindol,
(3aRS,4SR,9SR,9aRS)-3a-Carbamoyl-4,9-ethano-2-[2-(2-methoxyphenyl)propenoyl]-9-(4-methylphenyl)-2,3,3a,4,9,9a-hexahydro-1H-benzo[f]isoindol,
(3aRS,4SR,9SR,9aRS)-4,9-Ethano-2-[2-(2-methoxyphenyl)propenoyl]-9-(4-methylphenyl)-2,3,3a,4,9,9a-hexahydro-1H-benzo [f]isoindol-3a-benzylhydroxamat,
(3aRS,4SR,9SR,9aRS)-4,9-Ethano-2-[2-(2-methoxyphenyl)-propenoyl]-9-(4-methylphenyl)-2,3,3a,4,9,9a-hexahydro-1H-benzo [f]isoindol-3a-hydroxamsäure,
(3aRS,4SR,9SR,9aRS)-4,9-Ethano-2-[2-(2-methoxyphenyl)propenoyl] -9-(4-methylphenyl)-2,3,3a,4,9,9a-hexahydro-1H-benzo [f]isoindol-3a-N-(3-pyridylmethyl)carboxamid,
(3aRS,4SR,9SR,9aRS)-4,9-Ethano-2-[2-(2-methoxyphenyl)-propenoyl]-9-(4-methylphenyl)-2,3,3a,4,9,9a-hexahydro-1H-benzo[f]isoindol-3a-N-(4-pyridylmethyl)carboxamid,
(3aRS,4SR,9SR,9aRS)-4,9-Ethano-2- [2-(2-methoxyphenyl)-propenoyl]-9-(4-methylphenyl)-2,3,3a,4,9,9a-hexahydro-1H-benzo [f]isoindol-3a-methylhydroxamat,
(3aRS,4SR,9SR,9aRS)-4,9-Ethano-2-[2-(2-methoxyphenyl)-propenoyl]-9-(4-methylphenyl)-2,3,3a,4,9,9a-hexahydro-1H-benzo[f]isoindol-3a-N',N'-dimethylcarbohydrazid,
(3aRS,4SR,9SR,9aRS)-4,9-Ethano-2-[2-(2-methoxyphenyl)-propenoyl]-9-(4-methylphenyl)-2,3,3a,4,9,9a-hexahydro-1H-benzo[f]isoindol-3a-N'-phenylcarbohydrazid,
(3aRS,4SR,9SR,9aRS)-4,9-Ethano-2-[2-(2-methoxyphenyl)-propenoyl]-9-(4-methylphenyl)-2,3,3a,4,9,9a-hexahydro-1H-benzo[f]isoindol-3a-N',N'-pentamethylencarbohydrazid,
(3aRS,4SR, 9SR, 9aRS)-4,9-Ethano-2-[2-(2-methoxyphenyl)-propenoyl]-9-(4-methylphenyl)-3a-phenylsulfonylaminocarbonyl-2,3,3a,4,9,9a-hexahydro-1H-benzo[f]isoindol,
(3aRS,4SR,9SR,9aRS)-4,9-Ethano-2-[2-(2-methoxyphenyl)-propenoyl]-9-(4-methylphenyl)-2,3,3a,4,9,9a-hexahydro-1H-benzo[f]isoindol-3a-N-(N-oxo-3-pyridyl)methylcarboxamid,
(3aRS,4SR,9SR,9aRS)-4,9-Ethano-2-[2-(2-methoxyphenyl)-propenoyl]-9-(4-methylphenyl)-2,3,3a,4,9,9a-hexahydro-1H-benzo[f]isoindol-3a-N'-(4-methoxyphenyl)carbohydrazid,
(3aRS,4SR,9SR,9aRS)-4,9-Ethano-2-[2-(2-methoxyphenyl)-propenoyl]-9-(4-methylphenyl)-2,3,3a,4,9,9a-hexahydro-1H-benzo [f]isoindol-3a-N'-methyl-N'-phenylcarbohydrazid,
(3aRS,4SR,9SR,9aRS)-4,9-Ethano-2-[2-(2-methoxyphenyl)-propenoyl]-9-(4-methylphenyl)-2,3,3a,4,9,9a-hexahydro-1H-benzo[f]isoindol-3a-N'-(2-methylphenyl)carbohydrazid,
(3aRS,4SR,9SR,9aRS)-9-(2,3-Dihydro-1,4-benzodioxin-6-yl)-4,9-ethano-2-[2-(2-methoxyphenyl)propenoyl]-2,3,3a,4,9,9a-hexahydro-1H-benzo[f]isoindol-3a-methylcarboxylat,
(3aRS,4SR,9SR,9aRS)-9-(2,3-Dihydro-1,4-benzodioxin-6-yl)-4,9-ethano-2-[2-(2-methoxyphenyl)propenoyl]-2,3,3a,4,9,9a-hexahydro-1H-benzo[f]isoindol-3a-carbonsäure,
(3aRS,4SR,9SR,9aRS)-9-(2,3-Dihydro-1,4-benzodioxin-6-yl)-4,9-ethano-2-[2-(2-methoxyphenyl)propenoyl]-2,3,3a,4,9,9a-hexahydro-1H-benzo[f]isoindol-3a-[N-(3-pyridylmethyl)-carboxamid,
(3aRS,4SR,9SR,9aRS)-9-(2,3-Dihydro-1,4-benzodioxin-6-yl)-4,9-ethano-2-[2-(2-methoxyphenyl)propenoyl]-2,3,3a,4,9,9a-hexahydro-1H-benzo[f]isoindol-3a-N-(2-thienylmethyl)-carboxamid,
(3aRS,4SR,9SR,9aRS)-4,9-Ethano-2-[(2-methoxyphenyl)acetyl]-9-(3,4,5-trimethylphenyl)-2,3,3a,4,9,9a-hexahydro-1H-benzo[f]isoindol-3a-carbonsäure,
(3aRS,4SR,9SR,9aRS)-4,9-Ethano-2-[2-(2-methoxyphenyl)propenoyl]-9-(2-methylphenyl)-2,3,3a,4,9,9a-hexahydro-1H-benzo[f]isoindol-3a-carbonsäure,
(3aRS,4SR,9SR,9aRS)-4,9-Ethano-2- [2-(2-methoxyphenyl)propenoyl]-9-(4-trifluormethylphenyl)-2,3,3a,4,9,9a-hexahydro-1H-benzo[f]isoindol-3a-methylcarboxylat,
(3aRS,4SR,9SR,9aRS)-4,9-Ethano-2-[2-(2-methoxyphenyl)propenoyl]-9-(4-trifluormethylphenyl)-2,3,3a,4,9,9a-hexahydro-1H-benzo[f]isoindol-3a-carbonsäure,
(3aRS,4SR,9SR,9aRS)-4,9-Ethano-2-[2-(2-methoxyphenyl)propenoyl]-9-(4-trifluormethylphenyl)-2,3,3a,4,9,9a-hexahydro-1H-benzo[f]isoindol-3a-N-(4-pyridylmethyl)carboxamid,
(3aRS,4SR,9SR,9aRS)-4,9-Ethano-2-[2-(2-methoxyphenyl)propenoyl]-9-(4-trifluormethylphenyl)-2,3,3a,4,9,9a-hexahydro-1H-benzo[f]isoindol-3a-N'-benzoylcarbohydrazid,
(3aRS,4SR,9SR,9aRS)-4,9-Ethano-2-[2-(2-methoxyphenyl)propenoyl]-9-(4-trifluormethylphenyl)-2,3,3a,4,9,9a-hexahydro-1H-benzo[f]isoindol-3a-N'-phenylcarbohydrazid,
(3aRS,4SR,9SR,9aRS)-4,9-Ethano-2-[2-(2-methoxyphenyl)propenoyl]-9-(2-naphthyl)-2,3,3a,4,9,9a-hexahydro-1H-benzo[f]isoindol-3a-methylcarboxylat,
(3aRS,4SR,9SR,9aRS)-4,9-Ethano-2-[2-(2-methoxyphenyl)propenoyl]-9-(2-naphthyl)-2,3,3a,4,9,9a-hexahydro-1H-benzo[f]isoindol-3a-carbonsäure,
(3aRS,4SR,9SR,9aRS)-4,9-Ethano-2-[2-(2-methoxyphenyl)propenoyl]-9-(5-methyl-2-thienyl)-2,3,3a,4,9,9a-hexahydro-1H-benzo[f]isoindol-3a-methylcarboxylat,
(3aRS,4SR,9SR,9aRS)-4,9-Ethano-2-[2-(2-methoxyphenyl)propenoyl]-9-(5-methyl-2-thienyl)-2,3,3a,4,9,9a-hexahydro-1H-benzo[f]isoindol-3a-carbonsäure,
(3aRS,4SR,9SR,9aRS)-9-(4-Bromphenyl)-4,9-ethano-2-[2-(2-methoxyphenyl)propenoyl]-2,3,3a,4,9,9a-hexahydro-1H-benzo[f]isoindol-3a-methylcarboxylat,
(3aRS,4SR,9SR,9aRS)-9-(4-Bromphenyl)-4,9-ethano-2-[2-(2-methoxyphenyl)propenoyl]-2,3,3a,4,9,9a-hexahydro-1H-benzo[f]isoindol-3a-carbonsäure,
(3aRS,4SR,9SR,9aRS)-9-(3,4-Dichlorphenyl)-4,9-ethano-2-[2-(2-methoxyphenyl)propenoyl]-2,3,3a,4,9,9a-hexahydro-1H-benzo [f]isoindol-3a-carbonsäure,
(3aRS,4SR,9SR,9aRS) -4,9-ethano-2-[2-(2-methoxyphenyl)propenoyl]-9-(4-chlorphenyl)-2,3,3a,4,9,9a-hexahydro-1H-benzo [f]isoindol-3a-methylcarboxylat,
(3aRS,4SR,9SR,9aRS)-9-(4-Chlorphenyl)-4,9-ethano-2-[2-(2-methoxyphenyl)propenoyl]-2,3,3a,4,9,9a-hexahydro-1H-benzo[f]isoindol-3a-carbonsäure,
(3aRS,4SR,9SR,9aRS)-4,9-Ethano-2-[2-(2-methoxyphenyl)propenoyl]-9-(4-methoxy-3-methylphenyl)-2,3,3a,4,9,9a-hexahydro-1H-benzo [f] isoindol-3a-carbonsäure,
(3aRS,4SR,9SR,9aRS)-4,9-Ethano-2-[(2-methoxyphenyl)propenoyl]-9-(3-indoyl)-2,3,3a,4,9,9a-hexahydro-1H-benzo-[f]isoindol-3a-methylcarboxylat,
(3aRS,4SR,9SR,9aRS)-4,9-Ethano-9-(4-isopropylphenyl)-2-[2-(2-methoxyphenyl)acetyl]-2,3,3a,4,9,9a-hexahydro-1H-benzo-[f]isoindol-3a-carbonsäure,
(3aRS,4SR,9SR,9aRS)-4,9-Ethano-9-(4-isopropylphenyl)-2-[(2-methoxyphenyl)propenoyl]-2,3,3a,4,9,9a-hexahydro-1H-benzo[f]isoindol-3a-carbonsäure,
(3aRS,4SR,9SR,9aRS)-4,9-Ethano-2-[2-(2-methoxyphenyl)-propenoyl)-9-(3-thienyl)-2,3,3a,4,9,9a-hexahydro-1H-benzo[f]isoindol-3a-methylcarboxylat,
(3aRS,4SR,9SR,9aRS)-4,9-Ethano-9-(4-ethylphenyl)-2-[2-(2-methoxyphenyl)propenoyl)-2,3,3a,4,9,9a-hexahydro-1H-benzo[f]isoindol-3a-methylcarboxylat,
(3aRS,4SR,9SR,9aRS)-4,9-Ethano-9-(4-ethylphenyl)-2-[2-(2-methoxyphenyl)propenoyl)-2,3,3a,4,9,9a-hexahydro-1H-benzo[f]isoindol-3a-carbonsäure,
(3aRS,4SR,9SR,9aRS)-9-(2,3-Dihydrobenzofuran-5-yl)-4,9-ethano-2-[2-(2-methoxyphenyl)propenoyl)-2,3,3a,4,9,9a-hexahydro-1H-benzo[f]isoindol-3a-methylcarboxylat,
(3aRS,4SR,9SR,9aRS)-9-(2,3-Dihydrobenzofuran-5-yl)-4,9-ethano-2-[2-(2-methoxyphenyl)propenoyl)-2,3,3a,4,9,9a-hexahydro-1H-benzo[f]isoindol-3a-methylcarbonsäure,
(3aRS,4SR,9SR,9aRS)-9-(2,3-Dihydrobenzofuran-5-yl)-4,9-ethano-2-[2-(2-methoxyphenyl)propenoyl)-2,3,3a,4,9,9a-hexahydro-1H-benzo[f]isoindol-3a-N-(3-pyridylmethyl)carboxamid,
(3aRS,4SR,9SR,9aRS)-4,9-Ethano-9-(4-fluorphenyl)-2-[2-(2-methoxyphenyl)propenoyl)-2,3,3a,4,9,9a-hexahydro-1H-benzo[f]isoindol-3a-methylcarboxylat,
(3aRS,4SR,9SR,9aRS)-4,9-Ethano-9-(4-fluorphenyl)-2-[2-(2-methoxyphenyl)propenoyl)-2,3,3a,4,9,9a-hexahydro-1H-benzo[f]isoindol-3a-carbonsäure,
(3aRS,4SR,9SR,9aRS)-9-(4-Chlor-3-fluorphenyl)-4,9-ethano-2-[2-(2-methoxyphenyl)propenoyl)-2,3,3a, 4,9,9a-hexahydro-1H-benzo[f]isoindol-3a-methylcarboxylat,
(3aRS,4SR,9SR,9aRS)-9-(4-Chlor-3-fluorphenyl)-4,9-ethano-2-[2-(2-methoxyphenyl)propenoyl)-2,3,3a, 4,9,9a-hexahydro-1H-benzo [f]isoindol-3a-carbonsäure,
(3aRS,4SR,9SR,9aRS)-4,9-Ethano-2-[2-(2-methoxyphenyl)-propenoyl)-9-(3-methylphenyl)-2,3,3a,4,9,9a-hexahydro-1H-benzo[f]isoindol-3a-methylcarboxylat,
(3aRS,4SR,9SR,9aRS)-4,9-Ethano-2-[2-(2-methoxyphenyl)-propenoyl)-9-(3-methylphenyl)-2,3,3a,4,9,9a-hexahydro-1H-benzo[f]isoindol-3a-carbonsäure,
(3aRS,4SR,9SR,9aRS)-9-(1,3-Benzodioxol-5-yl)-4,9-ethano-2-[2-(2-methoxyphenyl)propenoyl)-2,3,3a, 4,9,9a-hexahydro-1H-benzo[f]isoindol-3a-methylcarboxylat,
(3aRS,4SR,9SR,9aRS)-9-(3,4-Dimethylphenyl))-4,9-ethano-2-[2-(2-methoxyphenyl)propenoyl)-2,3,3a, 4,9,9a-hexahydro-1H-benzo[f]isoindol-3a-methylcarboxylat,
(3aRS,4SR,9SR,9aRS)-9-(3,4-Dimethylphenyl))-4,9-ethano-2-[2-(2-methoxyphenyl)propenoyl)-2,3,3a, 4,9,9a-hexahydro-1H-benzo[f]isoindol-3a-carbonsäure,
(3aRS,4SR,9SR,9aRS)-4,9-Ethano-9-(4-methoxyphenyl)-2-[2-(2-methoxyphenyl)propenoyl)-2,3,3a,4,9,9a-hexahydro-1H-benzo-[f]isoindol-3a-methylcarboxylat,
(3aRS,4SR,9SR,9aRS)-4,9-Ethano-9-(4-methoxyphenyl)-2- [2-(2-methoxyphenyl)propenoyl)-2,3,3a,4,9,9a-hexahydro-1H-benzo-[f]isoindol-3a-carbonsäure,
(3aRS,4SR,9SR,9aRS)-4,9-Ethano-9-(4-methoxyphenyl)-2-[2-(2-methoxyphenyl)propenoyl)-2,3,3a,4,9,9a-hexahydro-1H-benzo-[f]isoindol-3a-N-(3-pyridylmethyl)carboxamid,
(3aRS,4SR,9SR,9aRS)-4,9-Ethano-9-(4-methoxyphenyl)-2-[2-(2-methoxyphenyl)propenoyl)-2,3,3a,4,9,9a-hexahydro-1H-benzo-[f]isoindol-3a-N'-phenylcarbohydrazid,
(3aRS,4SR,9SR,9aRS)-4,9-Ethano-9-(4-methoxyphenyl)-2-[2-(2-methoxyphenyl)propenoyl)-2,3,3a,4,9,9a-hexahydro-1H-benzo-[f]isoindol-3a-hydroxamsäure,
(3aRS,4SR,9SR,9aRS)-4,9-Ethano-9-(4-methoxyphenyl)-2-[2-(2-methoxyphenyl)propenoyl)-2,3,3a,4,9,9a-hexahydro-1H-benzo-[f]isoindol-3a-N'-(3-pyridyl)carbohydrazid,
(3aRS,4SR,9SR,9aRS)-4,9-Ethano-9-(4-methoxyphenyl)-2-[2-(2-methoxyphenyl)propenoyl)-2,3,3a,4,9,9a-hexahydro-1H-benzo-[f]isoindol-3a-N-(3-thienylmethyl)carboxamid,
(3aRS,4SR,9SR,9aRS)-2-{4,9-Ethano-9-(4-methoxyphenyl)-2-[2-(2-methoxyphenyl)propenoyl)-2,3,3a,4,9,9a-hexahydro-1H-benzo[f]isoindol-3a-carbonylamino}(RS)- und -(SR)-phenylessigsäure,
(3aRS,4SR,9SR,9aRS)-4,9-Ethano-2-[2-(2-methoxyphenyl)-propenoyl)-9-(4-trifluormethoxyphenyl)-2,3,3a,4,9,9a-hexahydro-1H-benzo[f]isoindol-3a-carbonsäure,
(3aRS,4SR,9SR,9aRS)-9-(3-Bromphenyl)-4,9-ethano-2-[2-(2-methoxyphenyl)propenoyl) -2,3,3a,4,9,9a-hexahydro-1H-benzo-[f]isoindol-3a-methylcarboxylat,
(3aRS, 4SR, 9SR, 9aRS) -9-(3-Bromphenyl) -4,9-ethano-2- [2-(2-methoxyphenyl)propenoyl) -2,3,3a,4,9,9a-hexahydro-1H-benzo-[f]isoindol-3a-carbonsäure,
(3aRS,4SR,9SR,9aRS)-4,9-Ethano-9-(3-fluorphenyl-2-[2-(2-methoxyphenyl)propenoyl)-2,3,3a,4,9,9a-hexahydro-1H-benzo-[f]isoindol-3a-methylcarboxylat,
(3aRS,4SR,9SR,9aRS)-4,9-Ethano-9-(3-fluorphenyl-2-[2-(2-methoxyphenyl)propenoyl)-2,3,3a,4,9,9a-hexahydro-1H-benzo-[f]isoindol-3a-carbonsäure,
(3aRS,4SR,9SR,9aRS)-4,9-Ethano-9-(3-fluorphenyl-2-[2-(2-methoxyphenyl)propenoyl)-2,3,3a,4,9,9a-hexahydro-1H-benzo-[f] isoindol-3a- [N-(3-pyridylmethyl)]carboxamid,
(3aRS,4SR,9SR,9aRS)-9-(3-Chlorphenyl)-4,9-ethano-2-[2-(2-methoxyphenyl)propenoyl)-2,3,3a,4,9,9a-hexahydro-1H-benzo-[f]isoindol-3a-methylcarboxylat,
(3aRS,4SR,9SR,9aRS)-9-(3-Chlorphenyl)-4,9-ethano-2-[2-(2-methoxyphenyl)propenoyl)-2,3,3a,4,9,9a-hexahydro-1H-benzo-[f]isoindol-3a-carbonsäure,
(3aRS,4SR,9SR,9aRS)-9-(3-Chlorphenyl)-4,9-ethano-2-[2-(2-methoxyphenyl)propenoyl)-2,3,3a,4,9,9a-hexahydro-1H-benzo-[f]isoindol-3a-[N-(3-pyridylmethyl)]carboxamid,
(3aRS,4SR,9SR,9aRS)-9-(3-N,N-Dimethylaminophenyl)-4,9-ethano-2-[2-(2-methoxyphenyl)propenoyl)-2,3,3a,4,9,9a-hexahydro-1H-benzo[f]isoindol-3a-methylcarboxylat,
(3aRS,4SR,9SR,9aRS)-9-(3-N,N-Dimethylaminophenyl)-4,9-ethano-2- [2-(2-methoxyphenyl)propenoyl)-2,3,3a,4,9,9a-hexahydro-1H-benzo[f]isoindol-3a-carbonsäure,
(3aRS,4SR,9SR,9aRS)-9-(3-Aminophenyl)-4,9-ethano-2-[2-(2-methoxyphenyl)propenoyl)-2,3,3a,4,9,9a-hexahydro-1H-benzo-[f]isoindol-3a-methylcarboxylat-chlorhydrat,
(3aRS,4SR,9SR,9aRS)-9-(4-N,N-Dimethylaminophenyl)-4,9-ethano-2-[2-(2-methoxyphenyl)propenoyl)-2,3,3a,4,9,9a-hexahydro-1H-benzo[f]isoindol-3a-methylcarboxylat,
(3aRS,4SR,9SR,9aRS)-9-(4-Cyanophenyl)-4,9-ethano-2-[2-(2-methoxyphenyl)propenoyl)-2,3,3a,4,9,9a-hexahydro-1H-benzo-[f]isoindol-3a-carbonsäure,
(3aRS,4SR,9SR,9aRS)-9-(3-Cyanophenyl)-4,9-ethano-2-[2-(2-methoxyphenyl)propenoyl)-2,3,3a,4,9,9a-hexahydro-1H-benzo-[f]isoindol-3a-methylcarboxylat,
(3aRS,4SR,9SR,9aRS)-4,9-Ethano-9-(3-hydroxy-4-methoxyphenyl)-2-[2-(2-methoxyphenyl)propenoyl)-2,3,3a,4,9,9a-hexahydro-1H-benzo[f]isoindol-3a-carbonsäure,
(3aRS,4SR,9SR,9aRS)-4,9-Ethano-5-methoxy-2-[2-(2-methoxyphenyl)propenoyl]-9-(4-methylphenyl)-2,3,3a,4,9,9a-hexahydro-1H-benzo[f]isoindol-3a-methylcarboxylat,
(3aRS,4SR,9SR,9aRS)-4,9-Ethano-5-methoxy-2-[2-(2-methoxyphenyl)propenoyl]-9-(4-methylphenyl)-2,3,3a,4,9,9a-hexahydro-1H-benzo[f]isoindol-3a-carbonsäure,
(3aRS,4SR,9SR,9aRS)-4,9-Ethano-2-[2-(2-methoxyphenyl)-2-propenoyl]-4-methyl-9-(4-methoxyphenyl)-2,3,3a,4,9,9a-hexahydro-1H-benzo[f]isoindol-3a-carbonsäure,

in racemischer Form oder in Form ihrer optischen Isomeren sowie ihrer Salze, handelt.

**8.** Verbindung, nämlich (3aRS,4SR,9SR,9aRS)-4,9-Ethano-2-[2-(2-methoxyphenyl)propenoyl]-9-(4-methylphenyl)-2,3,3a,4,9,9a-hexahydro-1H-benzo[f]isoindol-3a-carbonsäure in racemischer Form oder in Form ihrer optischen Isomeren sowie die Salze davon.

**9.** Verbindung, nämlich (3aRS,4SR,9SR,9aRS)-4,9-Ethano-2-[2-(2-methoxyphenyl)propenoyl]-9-(4-methylphenyl)-2,3,3a,4,9,9a-hexahydro-1H-benzo[f]isoindol-3a-N-(3-pyridylmethyl)carboxamid in racemischer Form oder in Form ihrer optischen Isomeren sowie die Salze davon.

**10.** Verbindung, nämlich (3aRS,4SR,9SR,9aRS)-9-(2,3-Dihydro-1,4-benzodioxin-6-yl)-4,9-ethano-2-[2-(2-methoxyphenyl)propenoyl) ]-2,3,3a,4,9,9a-hexahydro-1H-benzo[f]isoindol-3a-[N-(3-pyridylmethyl)carboxamid in racemischer Form oder in Form ihrer optischen Isomeren sowie die Salze davon.

**11.** Verbindung, nämlich (3aRS,4SR,9SR,9aRS)-4,9-Ethano-2-[2-(-methoxyphenyl)propenoyl)]-9-(4-trifluormethylphenyl)-2,3,3a,4,9,9a-hexahydro-1H-benzo[f]isoindol-3a-carbonsäure in racemischer Form oder in Form ihrer optischen Isomeren sowie die Salze davon.

**12.** Verbindung, nämlich (3aRS,4SR,9SR,9aRS)-9-(2,3-Dihydrobenzofuran-5-yl) -4,9-ethano-2-[2-(2-methoxyphenyl)-propenoyl)]-2,3,3a,4,9,9a-hexahydro-1H-benzo[f]isoindol-3a-N-(3-pydridylmethyl)carboxamid in racemischer Form oder in Form ihrer optischen Isomeren sowie die Salze davon.

**13.** Verbindung, nämlich (3aRS,4SR,9SR,9aRS)-4,9-Ethano-9-(4-methoxyphenyl)-2-[2-(2-methoxyphenyl)propenoyl)]-2,3,3a,4,9,9a-hexahydro-1H-benzo[f]isoindol-3a-carbonsäure in racemischer Form oder in Form ihrer optischen Isomeren sowie die Salze davon.

**14.** Verbindung, nämlich (3aRS,4SR,9SR,9aRS)-4,9-Ethano-5-methoxy-2- [2-(2-methoxyphenyl)propenoyl)]-9-(4-methylphenyl)-2,3,3a,4,9,9a-hexahydro-1H-benzo[f]isoindol-3a-carbonsäure in racemischer Form oder in Form ihrer optischen Isomeren sowie die Salze davon.

**15.** Verbindung, nämlich (3aRS,4SR,9SR,9aRS)-4,9-Ethano-2-[2-(2-methoxyphenyl) propenoyl)]-4-methyl-9-'(4-methoxyphenyl)-2,3,3a,4,9,9a-hexahydro-1H-benzo[f]isoindol-3a-carbonsäure in racemischer Form oder in Form ihrer optischen Isomeren sowie die Salze davon.

**16.** Verbindung, nämlich das rechtsdrehende Enantiomere von (3aRS,4SR,9SR,9aRS)-4,9-Ethano-2-[2-(2-methoxyphenyl)propenoyl)]-9-(4-methylphenyl)-2,3,3a,4,9,9a-hexahydro-1H-benzo[f]isoindol-3a-carbonsäure.

**17.** Verbindung, nämlich das rechtsdrehende Enantiomere von (3aRS,4SR,9SR,9aRS)-9-(2,3-Dihydro-1,4-benzodioxin-6-yl)-4,9-ethano-2-[2-(2-methoxyphenyl)propenoyl)]-2,3,3a,4,9,9a-hexahydro-1H-benzo[f]isoindol-3a-[N-(3-pydridylmethyl)-carboxamid.

**18.** Verfahren zur Herstellung einer Verbindung der allgemeinen Formel (I) nach einem der Ansprüche 1 bis 17, worin Y ein Sauerstoffatom oder ein Schwefelatom bedeutet und X eine Methylengruppierung oder Vinyldiylgruppierung bedeutet, **dadurch gekennzeichnet, dass** man eine Säure der allgemeinen Formel:

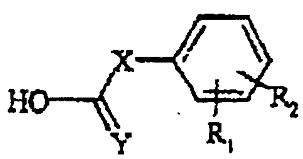

(II)

worin $R_1$ und $R_2$ wie in Anspruch 1 definiert sind, X wie vorstehend definiert ist und Y ein Sauerstoff- oder Schwefelatom bedeutet, seinen Methylester oder ein Derivat dieser Säure, wie ein Halogenid oder Anhydrid, mit einer Verbindung der allgemeinen Formel:

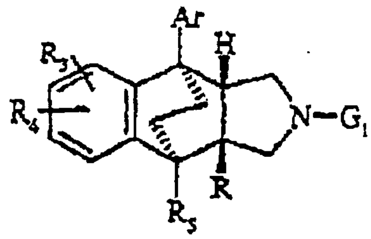

(III)

worin Ar, R, $R_3$, $R_4$ und $R_5$ wie Anspruch 1 definiert sind und $G_1$ ein Wasserstoffatom bedeutet, umsetzt.

**19.** Verfahren zur Herstellung einer Verbindung der allgemeinen Formel (III)

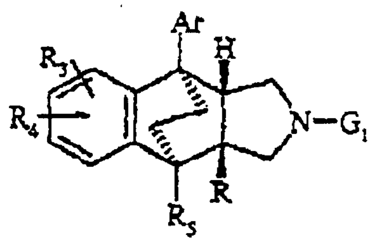

(III)

worin $R_3$, $R_4$, $R_5$, Ar und R wie in Anspruch 1 definiert sind und $G_1$ einen Benzylrest bedeutet, ausgehend von Verbindungen der allgemeinen Formel (IX):

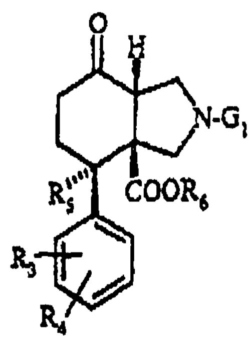

(IX)

worin $R_3$, $R_4$, $R_5$, $R_6$ und $G_1$ wie vorstehend definiert sind, **dadurch gekennzeichnet, dass** man ein Zwischenprodukt der allgemeinen Formel (XV)

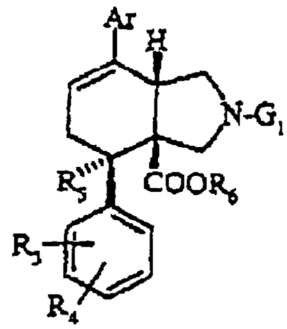

(XV) .

bildet, worin $R_3$, $R_4$, $R_5$, $R_6$, Ar und $G_1$ wie vorstehend definiert sind und dessen Transformation durch intramolekulare Zyklisierung vom Friedl-Crafts-Typ zu einem Produkt (III), wie in Anspruch 18 definiert, durchführt.

**20.** Verbindung, **dadurch gekennzeichnet, dass** sie durch die allgemeine Formel XV

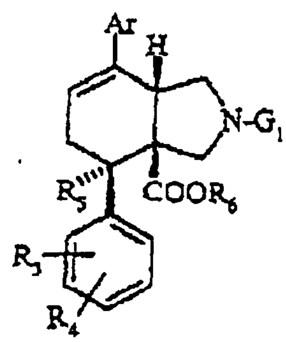

(XV)

wiedergegeben ist, worin $R_3$, $R_4$, $R_5$ und Ar wie in Anspruch 1 definiert sind, $G_1$ einen Benzylrest bedeutet und $R_6$ einen Alkylrest mit 1 bis 3 Kohlenstoffatomen bedeutet, in racemischer Form sowie ihre optischen Isomeren.

**21.** Verfahren zur Herstellung einer Verbindung der allgemeinen Formel (XV) nach Anspruch 19, **dadurch gekennzeichnet, dass** man über eine Verbindung der allgemeinen Formel (IX)

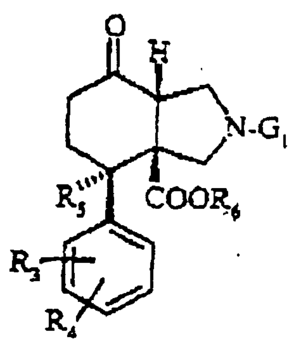

(IX)

worin $R_3$, $R_4$, $R_5$ und Ar wie in Anspruch 1 definiert sind, $G_1$ einen Benzylrest bedeutet und $R_6$ einen Alkylrest mit 1 bis 3 Kohlenstoffatomen bedeutet, deren Transformation in Position 7 durchführt, entweder über ein Iodethylenderivat durch eine Barton-Reaktion oder ein Enoltriflat, anschließend eine Kupplung mit Palladium, eine sog. Suzuki-Reaktion, mit einer Arylboronsäure oder sog. Stille-Reaktion mit einem Arylstannan durchführt.

**22.** Verfahren zur Herstellung einer Verbindung der allgemeinen Formel (III) nach Anspruch 18, worin Ar bevorzugt

- einen in der Position para, meta-para oder meta-para-meta' durch Elektronendonorgruppierung substituierten Phenylrest,
- einen elektronenreichen heterocyclischen Rest oder
- einen in geeigneter Weise durch Elektronendonorgruppierungen substituierten heterocyclischen Rest bedeutet,

**dadurch gekennzeichnet, dass** man einen aromatischen oder einen heterocyclischen Kohlenwasserstoff Ar-H nach intramolekularen Zyklisierungsreaktionen umsetzt, dann nach intramolekularen Zyklisierungsreaktionen vom Friedel-Crafts-Typ mit einer Verbindung der allgemeinen Formel (IX), worin $R_3$, $R_4$, $R_5$ und Ar wie in Anspruch 1 definiert sind, $G_1$ einen Benzylrest bedeutet und $R_6$ einen Alkylrest mit 1 bis 3 Kohlenstoffatomen bedeutet, in einem organischen Lösungsmittel in Gegenwart eines Überschusses an einer starken Säure oder einer Lewis-Säure umsetzt.

**23.** Verfahren zur Herstellung einer Verbindung der allgemeinen Formel (III)

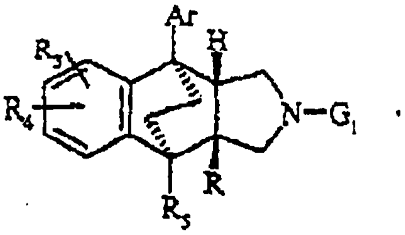 (III)

worin $R_3$, $R_4$, $R_5$, Ar wie in Anspruch 1 definiert sind, $G_1$ einen Benzylrest bedeutet und R einen Carboxyrest oder einen Rest der allgemeinen Formel $COOR_6$ bedeutet und $R_6$ einen Alkylrest mit 1 bis 3 Kohlenstoffatomen bedeutet, **dadurch gekennzeichnet, dass** man über eine intramolekulare Zyklisierung vom Friedel-Crafts-Typ durch Einwirkung von Trifluormethansulfonsäure auf ein Produkt der allgemeinen Formel (VIII):

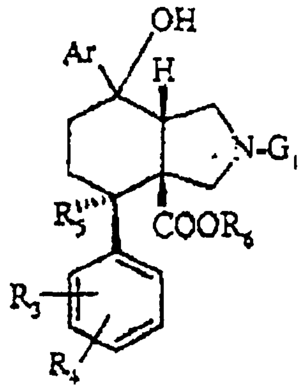 (VIII)

arbeitet, worin:

Ar, $R_3$, $R_4$ und $R_5$ wie in Anspruch 1 definiert sind, $G_1$ einen Benzylrest bedeutet, $R_6$ einen Alkylrest mit 1 bis 3 Kohlenstoffatomen bedeutet, anschließend die Gruppe $G_1$ durch ein Wasserstoffatom ersetzt, entweder durch Hydrogenolyse, dann gegebenenfalls durch Ersatz des Wasserstoffatoms durch einen tert-Butoxycarbonylrest durch Einwirkung von tert-Butoxycarbonylanhydrid in einem organischen Lösungsmittel oder durch einen Benzyloxycarbonylrest durch Wirkung von Benzyloxycarbonylchlorid, oder durch Wirkung eines Alkylchlorformiats, gefolgt von sauerer Hydrolyse des intermediär gebildeten Carbamats, gegebenenfalls gefolgt von einer Verseifung des so erhaltenen Produkts.

**24.** Verfahren zur Herstellung einer Verbindung der allgemeinen Formel (VIII), worin Ar, $R_3$, $R_4$, $R_5$, $R_6$ und $G_1$ wie in Anspruch 23 definiert sind, **dadurch gekennzeichnet, dass** man, ausgehend von einer Verbindung der allgemeinen Formel (IX)

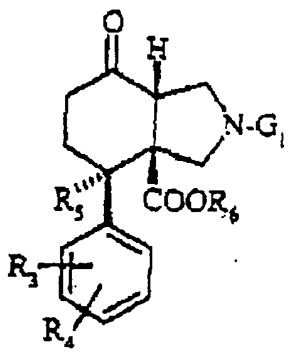

(IX)

worin $R_3$, $R_4$, $R_5$, $R_6$ und $G_1$ wie in Anspruch 21 definiert sind, arbeitet,
entweder durch Wirkung eines Organomagnesiumderivats der allgemeinen Formel Ar-Mg-X, worin Ar, wie vorstehend definiert ist, und X ein Halogenatom bedeutet, in Gegenwart von wasserfreiem Cer(III)-anhydrid in einem organischen Lösungsmittel bei einer Temperatur zwischen 0°C und der Rückflusstemperatur des Reaktionsgemisches,
oder durch Wirkung eines Organolithiums der allgemeinen Formel Ar-Li, worin Ar wie im Anspruch definiert ist, wobei man in einem organischen Lösungsmittel bei einer Temperatur zwischen -78°C und -20°C arbeitet.

**25.** Verfahren zur Herstellung einer Verbindung der allgemeinen Formel (IX), wie in Anspruch 21 oder 24 definiert, worin $R_3$, $R_4$, $R_5$ und Ar wie in Anspruch 1 definiert sind, $G_1$ einen Benzylrest bedeutet und $R_6$ einen Alkylrest mit 1 bis 3 Kohlenstoffatomen bedeutet, **dadurch gekennzeichnet, dass** man ein N-Trialkoylsilylmethyl-N-alkoxymethylamin mit einer Schutzgruppe der Aminfunktion mit einem Derivat eines Cyclohexenons der allgemeinen Formel:

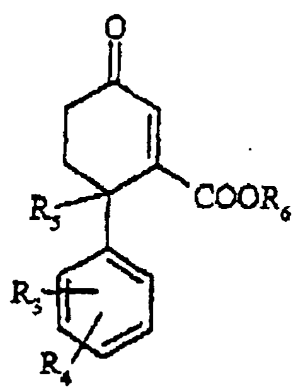

(X)

worin $R_3$, $R_4$ und $R_5$ wie in Anspruch 1 definiert sind und $R_6$ einen Alkylrest mit 1 bis 3 Kohlenstoffatomen bedeutet, umsetzt.

**26.** Pharmazeutische Zusammensetzung, **dadurch gekennzeichnet, dass** sie mindestens eine Verbindung nach einem der vorstehenden Ansprüche 1 bis 17 zusammen mit einem oder mehreren Verdünnungsmitteln oder pharmazeutisch verträglichen Adjuvantien, die inert oder biologisch aktiv sein können, umfasst.

**27.** Verwendung der Verbindungen nach einem der vorstehenden Ansprüche 1 bis 17 zur Herstellung von pharmazeutischen Zusammensetzungen, die zur Hemmung des Proteins von Farnesyltransferase nützlich sind.

**28.** Verwendung von Verbindungen nach einem der Ansprüche 1 bis 17 zur Herstellung von pharmazeutischen Zusammensetzungen zur Behandlung von Erkrankungen im Zusammenhang mit malignen oder benignen Zellproliferationen, Zellen verschiedener Gewebe und/oder Organe, umfassend Muskelgewebe, Knochengewebe oder

Bindegewebe, Haut, Gehirn, Lungen, Sexualorganen, lymphatische Systeme oder Nieren, Brust- oder Blutzellen, Leber, Verdauungsapparat, Pankreas und Schilddrüsen oder Nebennieren und umfassend die folgenden Pathologien: Psoriasis, Restenose, feste Tumore, Kaposisarkom, Karzinome, Cholangiokarzinom, Chorionkarzinom, Neuroblastom, Wilms-Tumor, Hodgkin-Erkrankung, Melanome, Teratokarzinome, Gliome, multiple Myelome, chronische lymphozytäre Leukämieformen, akute oder chronische granulozytäre Lymphome und Krebsarten, wie Pankreaskrebs, Kolonkrebs, Lungenkrebs, Eierstockkrebs, Brustkrebs, Hirntumor, Prostatakrebs, Leberkrebs, Magenkrebs oder Blasen- oder Hodenkrebs.

**29.** Verwendung von Verbindungen der allgemeinen Formel (I) nach einem der Ansprüche 1 bis 17 zur Herstellung von pharmazeutischen Zusammensetzungen, die zur Behandlung von Krankheiten im Zusammenhang mit Zellproliferationen durch Hemmung der Farnesyltransferase nützlich sind.

**30.** Verwendung der Verbindungen der allgemeinen Formel (I) nach einem der Ansprüche 1 bis 17 zur Herstellung von pharmazeutischen Zusammensetzungen zur Behandlung von Krebs.

**31.** Verwendung der Verbindung nach Anspruch 16 zur Herstellung von pharmazeutischen Zusammensetzungen zur Behandlung von Krebs.

**32.** Verwendung der Verbindungen der allgemeinen Formel (I) nach einem der Ansprüche 1 bis 17 zur Herstellung von pharmazeutischen Zusammensetzungen zur Behandlung von Kolonkrebs.

**33.** Verwendung von Verbindungen der allgemeinen Formel (I) nach einem der Ansprüche 1 bis 17 zur Herstellung von pharmazeutischen Zusammensetzungen zur Behandlung von Pankreaskrebs.

**34.** Verwendung der Verbindung nach Anspruch 16 zur Herstellung von pharmazeutischen Zusammensetzungen zur Behandlung von Kolonkrebs.

**35.** Verwendung einer Verbindung nach Anspruch 16 zur Herstellung von pharmazeutischen Zusammensetzungen zur Behandlung von Pankreaskrebs.

**36.** Assoziationen eines Produkts nach einem der vorstehenden Ansprüche 1 bis 17 mit einer oder mehreren kompatiblen und pharmakologisch aktiven Verbindungen und/oder mit einer Radiotherapie zum Erhalt eines synergistischen therapeutischen Effekts.

**Claims**

**1.** Compounds of general formula I

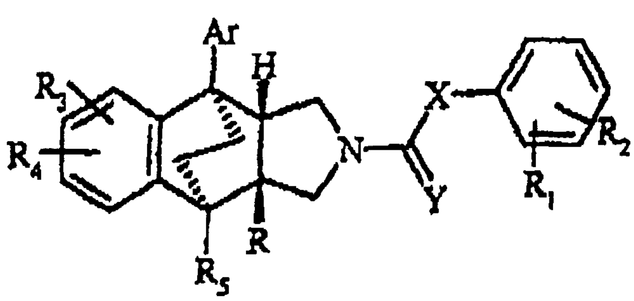

(I)

in which:

- Ar represents
  - a phenyl radical substituted by one or more atoms or radicals, which are identical or different, chosen from halogen atoms and the following radicals: alkyl containing 1 to 4 carbon atoms, such as methyl, alkenyl containing 2 to 4 carbon atoms, hydroxyl, mercapto, alkylthio, alkylsulphonyl or alkyl-sulphinyl, amino,

alkylamino or dialkylamino, formyl, alkylcarbonyl, carboxyl, alkoxycarbonyl, carbamoyl, alkylcarbamoyl or dialkylcarbamoyl, cyano or trifluoromethyl, or alkoxy containing 1 to 4 carbon atoms, such as methoxy, the alkyl portion of which is optionally perhalogenated, such as trifluoromethoxy, or

- a phenyl radical condensed with a 4- to 7-membered heterocycle containing one or more heteroatoms chosen from oxygen, nitrogen and sulphur atoms,

- a polycyclic aromatic radical,

- a 5- to 12-membered heterocyclic aromatic radical incorporating one or more heteroatoms chosen from oxygen, nitrogen and sulphur atoms, bonded to the condensed ring via a carbon-carbon bond,
with, for all of these radicals, alkyl containing 1 to 4 carbon atoms,

- R represents a carboxyl radical or a -COOMe radical or alternatively a -CON($R_7$)($R_8$) radical in which, when $R_7$ represents a hydrogen atom, $R_8$ represents a methyl radical substituted by a 2-, 3- or 4- pyridyl radical or the N-oxide of pyridine,

- $R_1$ and $R_2$ represent a hydrogen atom and the other of the symbols represents a methoxy radical,

- or else $R_3$ and $R_4$ each represent a hydrogen atom or else one of the $R_3$ or $R_4$ symbols represents a hydrogen atom and the other of the $R_3$ or $R_4$ symbols represents a methoxy radical,

- $R_5$ represents a hydrogen atom or an alkyl radical or an alkylthio radical, with, for the definition of $R_5$, alkyl containing 1 to 4 carbon atoms,

- X represents a methylene or vinyldiyl group, and

- Y represents an oxygen or sulphur atom,

in the racemic form or their optical isomers, as well as the salts of the product of general formula (I).

**2.** Compounds according to Claim 1, **characterized in that** Ar represents a 2,3-dihydro-1,4-benzodioxin-6-yl, 2,3-dihydrobenzofuran-5-yl, 2, 3-dihydrobenzopyran-6-yl, 1- or 2-naphthyl, 5-indanyl or 1,2,3,4-tetrahydronaphth-6-yl radical or a phenyl radical substituted at the 4 position by a methyl, trifluoromethyl or methoxy radical.

**3.** Compounds according to any one of the preceding claims, **characterized in that** Ar represents a 2,3-dihydro-1,4-benzodioxin-6-yl or 2,3-dihydrobenzofuran-5-yl radical or a phenyl radical substituted at the 4 position by a methyl, trifluoromethyl or methoxy radical.

**4.** Compounds according to any one of the preceding claims, **characterized in that** $R_5$ represents a hydrogen atom or a methyl radical.

**5.** Compounds according to any one of the preceding claims, **characterized in that** Y represents an oxygen atom.

**6.** Compounds according to any one of the preceding claims, **characterized in that** they are provided in the dextrorotatory form.

**7.** Compound, **characterized in that** it is a compound chosen individually from

methyl (3aRS,4SR,9SR,9aRS)-4,9-ethano-2-[2-(2-methoxy-phenyl)propenoyl]-9-(4-methylphenyl)-2,3,3a,4,9,9a-hexahydro-1H-benzo[f]isoindole-3a-carboxylate,
(3aRS,4SR,9SR,9aRS)-4,9-ethano-2-(2-methoxyphenyl)-propenoyl-9-(4-methylphenyl)-2,3,3a, 4, 9,9a-hexahydro-1H-benzo[f]isoindole-3a-carboxylic acid,
(3aRS,4SR,9SR,9aRS)-4,9-ethano-2-[(2-methoxyphenyl)-acetyl]-9-(4-methylphenyl)-2,3,3a,4,9,9a-hexahydro-1H-benzo[f]isoindole-3a-carboxylic acid,
(3aRS,4SR,9SR,9aRS)-4,9-ethano-9-(4-methoxyphenyl)-2-[(2-methoxyphenyl)acetyl]-2,3,3a,4,9,9a-hexahydro-1H-benzo[f]isoindole-3a-carboxylic acid,
(3aRS,4SR,9SR,9aRS)-4,9-ethano-2-[(2-methoxyphenyl)-acetyl]-9-(4-methylsulphanylphenyl)-2,3,3a,4,9,9a-hexahydro-1H-benzo[f]isoindole-3a-carboxylic acid,
(3aRS,4SR,9SR,9aRS)-4,9-ethano-9-(4-fluorophenyl)-2-[(2-methoxyphenyl)acetyl]-2,3,3a,4,9,9a-hexahydro-1H-benzo[f]isoindole-3a-carboxylic acid,
(3aRS,4SR,9SR,9aRS)-4,9-ethano-2-[(2-methoxyphenyl) -acetyl]-9-(3-methylphenyl)-2, 3, 3a, 4, 9, 9a-hexahydro-1H-benzo[f]isoindole-3a-carboxylic acid,
(3aRS,4SR,9SR,9aRS)-4, 9-ethano-9-(3-methoxyphenyl) -2-[(2-methoxyphenyl)acetyl]-2,3,3a,4,9,9a-hexahydro-1H-benzo[f]isoindole-3a-carboxylic acid,
methyl (3aRS,4SR,9SR,9aRS)-9-(3,4-dimethylphenyl)-4, 9-ethano-2-[(2-methoxyphenyl)acetyl]-2,3,3a, 4,9,9a-hexahydro-1H-benzo[f]isoindole-3a-carboxylate,
(3aRS,4SR,9SR,9aRS)-9-(3,4-dimethylphenyl)-4,9-ethano-2- [(2-methoxyphenyl)acetyl]-2,3,3a,4,9,9a-hexahydro-1H-benzo[f]isoindole-3a-carboxylic acid,
(3aRS, 4SR, 9SR, 9aRS)-3a-N-benzylcarbamoyl-4, 9-ethano-2-[2-(2-methoxyphenyl)propenoyl]-9-(4-methylphenyl)2,3,3a,4,9,9a-hexahydro-1H-benzo [f] isoindole,
(3aRS,4SR,9SR,9aRS)-3a-carbamoyl-4, 9-ethano-2- [2-(2-methoxyphenyl)propenoyl]-9-(4-methylphenyl)-2,3,3a,4,9,9a-hexahydro-1H-benzo[f] isoindole,
benzyl (3aRS,4SR,9SR,9aRS)-4,9-ethano-2-[2-(2-methoxy-phenyl)propenoyl]-9-(4-methylphenyl)-2,3,3a, 4,9,9a-hexahydro-1H-benzo[f]isoindole-3a-hydroxamate,
(3aRS,4SR,9SR,9aRS)-4,9-ethano-2-[2-(2-methoxyphenyl)-propenoyl] -9-(4-methylphenyl)-2,3,3a,4,9,9a-hexahydro-1H-benzo[f]isoindole-3a-hydroxamic acid,
(3aRS,4SR,9SR,9aRS)-4, 9-ethano-2-[2-(2-methoxyphenyl)propenoyl]-9-(4-methylphenyl)-2,3,3a,4,9,9a-hexahydro-1H-benzo[f]isoindole-3a-N-(3-pyridylmethyl)-carboxamide,
(3aRS,4SR,9SR,9aRS)-4,9-ethano-2-[2-(2-methoxyphenyl)propenoyl]-9-(4-methylphenyl)-2,3,3a,4,9,9a-hexahydro-1H-benzo[f]isoindole-3a-N-(4-pyridylmethyl)-carboxamide,
methyl (3aRS, 4SR, 9SR, 9aRS) -4, 9-ethano-2- [2-(2-methoxyphenyl)propenoyl]-9-(4-methylphenyl)-2, 3,3a, 4,9,9a-hexahydro-1H-benzo[f]isoindole-3a-hydroxamate,
(3aRS,4SR,9SR,9aRS)-4,9-ethano-2-[2-(2-methoxyphenyl)propenoyl]-9-(4-methylphenyl)-2,3,3a,4,9,9a-hexahydro-1H-benzo[f]isoindole-3a-N',N'-dimethylcarbohydrazide,
(3aRS,4SR,9SR,9aRS)-4,9-ethano-2-[2-(2-methoxyphenyl)propenoyl]-9-(4-methylphenyl)-2,3,3a,4,9,9a-hexahydro-1H-benzo [f] isoindole-3a-N'-phenylcarbohydrazide,
(3aRS,4SR,9SR,9aRS)-4,9-ethano-2-[2-(2-methoxyphenyl)propenoyl]-9-(4-methylphenyl)-2,3,3a,4,9,9a-hexahydro-1H-benzo[f]isoindole-3a-N',N'-pentamethylene-carbohydrazide,
(3aRS, 4SR, 9SR, 9aRS) -4, 9-ethano-2- [2-(methoxyphenyl)propenoyl]-9-(4-methylphenyl)-3a-phenylsulphonylaminocarbonyl-2,3,3a,4,9,9a-hexahydro-1H-benzo[f]isoindole,
(3aRS,4SR,9SR,9aRS)-4, 9-ethano-2-[2-(2-methoxyphenyl)propenoyl]-9-(4-methylphenyl)-2,3,3a,9,9,9a-hexahydro-1H-benzo[f]isoindole-3a-N-(N-oxo-3-pyridyl)methylcarboxamide,
(3aRS,4SR,9SR,9aRS)-4,9-ethano-2-[2-(2-methoxyphenyl)propenoyl]-9-(4-methylphenyl)-2,3,3a,4,9,9a-hexahydro-1H-benzo[f]isoindole-3a-N'-(4-methoxyphenyl)- carbohydrazide,
(3aRS,4SR,9SR,9aRS)-4,9-ethano-2-[2-(2-methoxyphenyl)propenoyl]-9-(4-methylphenyl)-2,3,3a,4,9,9a-hexahydro-1H-benzo[f]isoindole-3a-N'-methyl-N'-phenylcarbohydrazide,
(3aRS,4SR,9SR,9aRS)-4, 9-ethano-2-[2-(2-methoxyphenyl)propenoyl]-9-(4-methylphenyl)-2,3,3a,4,9,9a-hexahydro-1H-benzo[f]isoindole-3a-N'-(2-methyl-phenyl) carbohydrazide,
methyl (3aRS,4SR,9SR,9aRS)-9-(2,3-dihydro-1,4-benzodioxin-6-yl)-4,9-ethano-2-[2-(2-methoxyphenyl)-propenoyl]-2,3,3a,4,9,9a-hexahydro-1H-benzo[f]isoindole-3a-carboxylate,
(3aRS,4SR,9SR,9aRS)-9-(2,3-dihydro-1,4-benzodioxin-6-yl) -4, 9-ethano-2-[2-(2-methoxyphenyl)propenoyl]-2,3,3a,4,9,9a-hexahydro-1H-benzo[f]isoindole-3a-carboxylic acid,
(3aRS,4SR,9SR,9aRS)-9-(2, 3-dihydro-1, 4-benzodioxin-6-yl)-4,9-ethano-2-[2-(2-methoxyphenyl)propenoyl]-2,3,3a,4,9,9a-hexahydro-1H-benzo[f]isoindole-3a-N-(3-pyridylmethyl)carboxamide,
(3aRS,4SR,9SR,9aRS)-9-(2, 3-dihydro-1,4-benzodioxin-6-yl)-4, 9-ethano-2-[2-(2-methoxyphenyl)propenoyl]-2,3,3a,4,9,9a-hexahydro-1H-benzo[f]isoindole-3a-N-(2-thienylmethyl)carboxamide,
(3aRS,4SR,9SR,9aRS)-4,9-ethano-2-[2-(2-methoxyphenyl)-acetyl]-9-(3,4,5-trimethylphenyl)-2,3,3a,4,9,9a-hexahydro-1H-benzo[f]isoindole-3a-carboxylic acid,
(3aRS, 4SR, 9SR, 9aRS)-4,9-ethano-2- [2-(2-methoxyphenyl)-propenoyl]-9-(2-methylphenyl)-2,3,3a,4,9,9a-hexahydro-1H-benzo[f]isoindole-3a-carboxylic acid,
methyl (3aRS,4SR,9SR,9aRS)-4,9-ethano-2-[2-(2-methoxyphenyl)propenoyl]-9-(4-trifluoromethylphenyl)-2,3,3a,4,9,9a-hexahydro-1H-benzo [f]isoindole-3a-carboxylate,
(3aRS,4SR,9SR,9aRS)-4,9-ethano-2-[2-(2-methoxyphenyl)-propenoyl]-9-(4-trifluoromethylphenyl)-2,3,3a, 4,9,9a-hexahydro-1H-benzo[f]isoindole-3a-carboxylic acid,
(3aRS,4SR,9SR,9aRS)-4,9-ethano-2-[2-(2-methoxyphenyl)-propenoyl]-9-(4-trifluoromethylphenyl)-2,3,3a,4,9,9a-hexahydro-1H-benzo[f]isoindole-3a-N-(4-pyridylmethyl)carboxamide,
(3aRS,4SR,9SR,9aRS)-4,9-ethano-2-[2-(2-methoxyphenyl)-propenoyl)-9-(4-trifluoromethylphenyl)-2,3,3a, 4,9,9a-hexahydro-1H-benzo[f]isoindole-3a-N'-benzoylcarbohydrazide,
(3aRS,4SR,9SR,9aRS)-4, 9-ethano-2-[2-(2-methoxyphenyl)-propenoyl]-9-(4-trifluoromethylphenyl)-2,3,3a, 4,9,9a-hexahydro-1H-benzo[f]isoindole-3a-N'-phenylcarbohydrazide,
methyl (3aRS,4SR,9SR,9aRS)-4,9-ethano-2-[2-(2-methoxyphenyl)propenoyl]-9-(2-naphthyl)-2,3,3a,4,9, 9a-hexahydro-1H-benzo[f]isoindole-3a-carboxylate,
(3aRS,4SR,9SR,9aRS) -4, 9-ethano-2- [2-(2-methoxyphenyl)-propenoyl]-9-(2-naphthyl)-2,3,3a,4,9,9a-hexahydro-1H-benzo[f]isoindole-3a-carboxylic acid,
methyl (3aRS,4SR,9SR,9aRS)-4,9-ethano-2-[2-(2-methoxyphenyl)propenoyl]-9-(5-methyl-2-thienyl)-2,3,3a, 4,9,9a-hexahydro-1H-benzo[f]isoindole-3a-carboxylate,
(3aRS,4SR,9SR,9aRS)-4,9-ethano-2-[2-(2-methoxyphenyl)-propenoyl]-9-(5-methyl-2-thienyl)-2,3,3a,4,9,9a-hexahydro-1H-benzo[f]isoindole-3a-carboxylic acid,
methyl (3aRS,4SR,9SR,9aRS)-9-(4-bromophenyl)-4,9-ethano-2- [2-(2-methoxyphenyl)propenoyl]-2,3,3a, 4,9,9a-hexahydro-1H-benzo[f]isoindole-3a-carboxylate,
(3aRS, 4SR, 9SR, 9aRS) -9-(4-bromophenyl)-4,9-ethano-2-[2-(2-methoxyphenyl)propenoyl]-2,3,3a, 4,9,9a-hexahydro-1H-benzo[f]isoindole-3a-carboxylic acid,
(3aRS,4SR,9SR,9aRS)-9-(3,4-dichlorophenyl)-4,9-ethano-2-[2-(2-methoxyphenyl)propenoyl]-2,3,3a,4,9,9a-hexahydro-1H-benzo[f]isoindole-3a-carboxylic acid,
methyl (3aRS,4SR,9SR,9aRS)-4,9-ethano-2-[2-(2-methoxyphenyl)propenoyl]-9-(4-chlorophenyl)-2,3,3a, 4,9,9a-hexahydro-1H-benzo[f]isoindole-3a-carboxylate,
(3aRS,4SR,9SR,9aRS)-9-(4-chlorophenyl)-4, 9-ethano-2- [2-(2-methoxyphenyl)propenoyl-2,3,3a, 4,9,9a-hexahydro-1H-benzo[f]isoindole-3a-carboxylic acid,
(3aRS,4SR,9SR,9aRS)-4,9-ethano-2-[2-(2-methoxyphenyl)propenoyl]-9-(4-methoxy-3-methylphenyl)-2,3,3a,4,9,9a-hexahydro-1H-benzo[f]isoindole-3a-carboxylic acid,
methyl (3aRS,4SR,9SR,9aRS)-4,9-ethano-2-[2-(2-methoxyphenyl)propenoyl]-9-(3-indolyl)-2,3,3a,4,9,9a-hexahydro-1H-benzo[f]isoindole-3a-carboxylate,
(3aRS,4SR,9SR,9aRS)-4,9-ethano-9-(4-isopropylphenyl)-2-(2-methoxyphenyl)acetyl]-2,3,3a,4,9,9a-hexahydro-1H-benzo[f]isoindole-3a-carboxylic acid,
(3aRS,4SR,9SR,9aRS)-4,9-ethano-9-(4-isopropylphenyl)-2-[2-(2-methoxyphenyl)propenoyl]-2,3,3a,4,9,9a-hexahydro-1H-benzo[f]isoindole-3a-carboxylic acid,
methyl (3aRS,9SR,9SR,9aRS)-4,9-ethano-2-[2-(2-methoxyphenyl)propenoyl]-9-(3-thienyl)-2, 3, 3a, 4, 9, 9a-hexahydro-1H-benzo[f]isoindole-3a-carboxylate,
methyl (3aRS,4SR,9SR,9aRS)-4,9-ethano-9-(4-ethylphenyl)-2-[2-(2-methoxyphenyl)propenoyl]-2,3,3a, 4,9,9a-hexahydro-1H-benzo[f] isoindole-3a-carboxylate,
(3aRS,4SR,9SR,9aRS)-4,9-ethano-9-(4-ethylphenyl)-2-[2-(2-methoxyphenyl)propenoyl]-2,3,3a,4,9,9a-hexahydro-1H-benzo[f]isoindole-3a-carboxylic acid,
methyl (3aRS, 4SR, 9SR, 9aRS) -9-(2, 3-dihydrobenzofuran-5-yl)-4,9-etbano-2-[2-(2-methoxyphenyl)propenoyl]-2, 3, 3a, 4,9,9a-hexahydro-1H-benzo[f]isoindole-3a-carboxylate,
methyl (3aRS, 4SR, 9SR, 9aRS) -9-(2,3-dihydrobenzofuran-5-yl)-4,9-ethano-2-[2-(2-methoxyphenyl)propenoyl] -2,3,3a,4,9,9a-hexahydro-1H-benzo[f]isoindole-3a-carboxylate acid,
(3aRS,4SR,9SR,9aRS)-9-(2, 3-dihydrobenzofuran-5-yl)-4,9-ethano-2-[2-(2-methoxyphenyl)propenoyl]-2, 3,3a,4,9,9a-hexahydro-1H-benzo[f]isoindole-3a-N-(3-pyridylmethyl)-carboxamide,
methyl (3aRS,4SR,9SR,9aRS)-4,9-ethano-9-(4-fluorophenyl)-2-[2-(2-methoxyphenyl)propenoyl]-2,3,3a, 4,9,9a-hexahydro-1H-benzo[f]isoindole-3a-carboxylate,
(3aRS,4SR,9SR,9aRS)-4, 9-ethano-9-(4-fluorophenyl)-2-[2-(2-methoxyphenyl)propenoyl]-2,3,3a,4,9, 9a-hexahydro-1H-benzo[f]isoindole-3a-carboxylic acid,
methyl (3aRS,4SR,9SR,9aRS)-9-(4-chloro-3-fluorophenyl) -4,9-ethano-2- [2-(2-methoxyphenyl)propenoyl]-2,3,3a,9,9,9a-hexahydro-1H-benzo[f]isoindole-3a-carboxylate,
(3aRS,4SR,9SR,9aRS)-9-(4-chloro-3-fluorophenyl)-4, 9-ethano-2- [2-(2-methoxyphenyl)propenoyl]-2,3,3a, 4,9,9a-hexahydro-1H-benzo[f]isoindole-3a-carboxylic acid,
methyl (3aRS,4SR,9SR,9aRS)-4,9-ethano-2-[2-(2-methoxyphenyl)propenoyl]-9-(3-methylphenyl)-2,3,3a, 4,9,9a-hexahydro-1H-benzo[f]isoindole-3a-carboxylate,
(3aRS,4SR,9SR,9aRS)-4,9-ethano-2-[2-(2-methoxyphenyl)propenoyl]-9-(3-methylphenyl)-2,3,3a,4,9,9a-hexahydro-1H-benzo[f]isoindole-3a-carboxylic acid,
methyl (3aRS,4SR,9SR,9aRS)-9-(1,3-benzodioxol-5-yl)-4,9-ethano-2-[2-(2-methoxyphenyl)propenoyl]-2,3,3a,4,9,9a-hexahydro-1H-benzo[f]isoindole-3a-carboxylate,
methyl (3aRS,4SR,9SR,9aRS)-9-(3,4-dimethylphenyl)-4,9-ethano-2-[2-(2-methoxyphenyl)propenoyl]-2,3,3a,

4,9,9a-hexahydro-1H-benzo[f]isoindole-3a-carboxylate,
(3aRS,4SR,9SR,9aRS)-9-(3,4-dimethylphenyl)-4,9-ethano-2-[2-(2-methoxyphenyl)propenoyl]-2,3,3a,4,9,9a-hexahydro-1H-benzo[f]isoindole-3a-carboxylic acid,
methyl (3aRS,4SR,9SR,9aRS)-4,9-ethano-9-(4-methoxy-phenyl)-2-[2-(2-methoxyphenyl)propenoyl]-2,3,3a,4,9,9a-hexahydro-1H-benzo[f]isoindole-3a-carboxylate,
(3aRS, 4SR, 9SR, 9aRS)-4, 9-ethano-9-(4-methoxyphenyl)-2-[2-(2-methoxyphenyl)propenoyl]-2,3,3a,4,9,9a-hexahydro-1H-benzo[f]isoindole-3a-carboxylic acid,
(3aRS,4SR,9SR,9aRS)-4,9-ethano-9-(4-methoxyphenyl)-2-[2-(2-methoxyphenyl)propenoyl]-2,3,3a,4,9,9a-hexahydro-1H-benzo[f]isoindole-3a-N-(3-pyridylmethyl)carboxamide,
(3aRS,4SR,9SR,9aRS)-4,9-ethano-9-(4-methoxyphenyl) -2-[2-(2-methoxyphenyl)propenoyl]-2,3,3a,4,9,9a-hexahydro-1H-benzo[f]isoindole-3a-N'-phenylcarbohydrazide,
(3aRS,4SR,9SR,9aRS)-4,9-ethano-9-(4-methoxyphenyl)-2-[2-(2-methoxyphenyl)propenoyl]-2,3,3a,4,9,9a-hexahydro-1H-benzo[f]isoindole-3a-hydroxamic acid,
(3aRS,4SR,9SR,9aRS)-4,9-ethano-9-(4-methoxyphenyl)-2-[2-(2-methoxyphenyl)propenoyl]-2,3,3a,4,9,9a-hexahydro-1H-benzo[f]isoindole-3a-N'-(3-pyridyl)carbohydrazide,
(3aRS,4SR,9SR,9aRS)-4,9-ethano-9-(4-methoxyphenyl)-2-[2-(2-methoxyphenyl)propenoyl]-2,3,3a,4,9,9a-hexahydro-1H-benzo[f]isoindole-3a-N-(3-thienylmethyl)carboxamide,
(RS)- and (SR) -2-{(3aRS,4SR,9SR,9aRS)-4,9-ethano-9-(4-methoxyphenyl) -2- [2-(2-methoxyphenyl)propenoyl]-2,3,3a,4,9,9a-hexahydro-1H-benzo [f]isoindolyl-3a-carbonylamino]phenylacetic acids,
(3aRS,4SR,9SR,9aRS)-4,9-ethano-2-[2-(2-methoxy-phenyl)propenoyl]-9-(4-trifluoromethoxyphenyl)-2,3,3a,4,9,9a-hexahydro-1H-benzo[f]isoindole-3a-carboxylic acid,
methyl (3aRS,4SR,9SR,9aRS)-9-(3-bromophenyl)-4,9-ethano-2-[2-(2-methoxyphenyl)propenoyl]-2,3,3a,4,9,9a-hexahydro-1H-benzo[f]isoindole-3a-carboxylate,
(3aRS,4SR,9SR,9aRS)-9-(3-bromophenyl)-4,9-ethano-2-[2-(2-methoxyphenyl)propenoyl]-2,3,3a,4,9,9a-hexahydro-1H-benzo[f]isoindole-3a-carboxylic acid,
methyl (3aRS,4SR,9SR,9aRS)-4,9-ethano-9-(3-fluorophenyl)-2-[2-(2-methoxyphenyl)propenoyl]-2,3,3a,4,9,9a-hexahydro-1H-benzo[f]isoindole-3a-carboxylate,
(3aRS,4SR,9SR,9aRS)-4,9-ethano-9-(3-fluorophenyl)-2- [2-(2-methoxyphenyl)propenoyl]-2,3,3a,4,9,9a-hexahydro-1H-benzo[f]isoindole-3a-carboxylic acid, (3aRS,4SR,9SR,9aRS)-4,9-ethano-9-(3-fluorophenyl)-2-[2-(2-methoxyphenyl)propenoyl]-2,3,3a,4,9,9a-hexahydro-1H-benzo[f]isoindole-3a-N-(3-pyridylmethyl)carboxamide,
methyl (3aRS,4SR,9SR,9aRS)-9-(3-chlorophenyl)-4,9-ethano-2-[2-(2-methoxyphenyl)propenoyl]-2,3,3a,4,9,-9a-hexahydro-1H-benzo[f]isoindole-3a-carboxylate,
(3aRS,4SR,9SR,9aRS) -9-(3-chlorophenyl) -4, 9-ethano-2- [2-(2-methoxyphenyl)propenoyl]-2,3,3a, 4,9,9a-hexahydro-1H-benzo[f]isoindole-3a-carboxylic acid,
(3aRS,4SR,9SR,9aRS)-9-(3-chlorophenyl) -4, 9-ethano-2-[2-(2-methoxyphenyl)propenoyl]-2,3,3a,4,9,9a-hexahydro-1H-benzo[f]isoindole-3a-N-(3-pyridylmethyl)carboxamide,
methyl (3aRS,4SR,9SR,9aRS)-9-(3-N,N-dimethylaminophenyl)-4,9-ethano-2-[2-(2-methoxyphenyl)-propenoyl]-2,3,3a,4,9,9a-hexahydro-1H-benzo[f]-isoindole-3a-carboxylate,
(3aRS,4SR,9SR,9aRS)-9-(3-N,N-dimethylaminophenyl)-4, 9-ethano-2- [2-(2-methoxyphenyl)propenoyl]-2,3,3a,4, 9,9a-hexahydro-1H-benzo[f]isoindole-3a-carboxylic acid,
methyl (3aRS, 4SR, 9SR, 9aRS) -9-(3-aminophenyl)-4, 9-ethano-2-[2-(2-methoxyphenyl)propenoyl]-2,3,3a,4,9,9a-hexahydro-1H-benzo[f]isoindole-3a-carboxylate hydrochloride,
methyl (3aRS, 4SR, 9SR, 9aRS) -9-(4-N,N-dimethylaminophenyl) -4, 9-ethano-2- [2-(2-methoxyphenyl)propenoyl]-2,3,3a,4,9,9a-hexahydro-1H-benzo[f] isoindole-3a-carboxylate,
(3aRS,4SR,9SR,9aRS)-9-(4-cyanophenyl)-4,9-ethano-2-[2-(2-methoxyphenyl)propenoyl]-2,3,3a,4,9,9a-hexahydro-1H-benzo[f]isoindole-3a-carboxylic acid,
methyl (3aRS,4SR,9SR,9aRS)-9-(3-cyanophenyl)-4,9-ethano-2-[2-(2-methoxyphenyl)propenoyl]-2,3,3a,4,9,9a-hexahydro-1H-benzo[f]isoindole-3a-carboxylate,
(3aRS, 4SR, 9SR, 9aRS) -4,9-ethano-9-(3-hydroxy-4-methoxyphenyl)-2-[2-(2-methoxyphenyl)propenoyl]-2,3,3a,4,9,9a-hexahydro-1H-benzo[f]isoindole-3a-carboxylic acid,
methyl (3aRS,4SR,9SR,9aRS)-4, 9-ethano-5-methoxy-2-[2-(2-methoxyphenyl)propenoyl]-9-(4-methylphenyl)-2, 3, 3a,4,9,9a-hexahydro-1H-benzo [f]isoindole-3a-carboxylate,
(3aRS,4SR,9SR,9aRS)-4,9-ethano-5-methoxy-2-[2-(2-methoxyphenyl)propenoyl]-9-(4-methylphenyl)-2,3,3a,4,9,9a-hexahydro-1H-benzo[f]isoindole-3a-carboxylic acid,
(3aRS,4SR,9SR, 9aRS)-4, 9-ethano-2- [2-(2-methoxyphenyl) -propenoyl]-4-methyl-9-(4-methoxyphenyl)-2,3,3a,4,9,9a-hexahydro-1H-benzo[f]isoindole-3a-carboxylic acid,

in the racemic form or their optical isomers, as well as their salts.

8. Compound, **characterized in that** it is
(3aRS,4SR,9SR,9aRS)-4,9-ethano-2-[2-(2-methoxyphenyl)-propenoyl]-9-(4-methylphenyl)-2,3,3a,4,9,9a-hexahydro-1H-benzo [f]isoindole-3a-carboxylic acid
in the racemic form or its optical isomers, as well as its salts.

9. Compound, **characterized in that** it is
(3aRS, 4SR, 9SR, 9aRS)-4,9-ethano-2- [2-(2-methoxyphenyl)propenoyl]-9-(4-methylphenyl)-2,3,3a,4,9,9a-hexahydro-1H-benzo[f]isoindole-3a-N-(3-pyridylmethyl)carboxamide
in the racemic form or its optical isomers, as well as its salts.

10. Compound, **characterized in that** it is
(3aRS,4SR,9SR,9aRS)-9-(2,3-dihydro-1,4-benzodioxin-6-yl)-4,9-ethano-2-[2-(2-methoxyphenyl)propenoyl]-2,3,3a,4,9,9a-hexahydro-1H-benzo [f]isoindole-3a-N-(3-pyridylmethyl)carboxamide
in the racemic form or its optical isomers, as well as its salts.

11. Compound, **characterized in that** it is
(3aRS,4SR,9SR,9aRS)-4,9-ethano-2-[2-(2-methoxyphenyl)propenoyl]-9-(4-trifluoromethylphenyl)-2,3,3a,4,9,9a-hexahydro-1H-benzo[f]isoindole-3a-carboxylic acid
in the racemic form or its optical isomers, as well as its salts.

12. Compound, **characterized in that** it is
(3aRS,4SR,9SR,9aRS)-9-(2,3-dihydrobenzofuran-5-yl) -4,9-ethano-2- [2-(2-methoxyphenyl)propenoyl]-2,3,3a,4,9,9a-hexahydro-1H-benzo[f]isoindole-3a-N-(3-pyridylmethyl)carboxamide
in the racemic form or its optical isomers, as well as its salts.

13. Compound, **characterized in that** it is
(3aRS,4SR,9SR,9aRS)-4,9-ethano-9-(4-methoxyphenyl)-2-[2-(2-methoxyphenyl)propenoyl]-2,3,3a,4,9,9a-hexahydro-1H-benzo[f]isoindole-3a-carboxylic acid
in the racemic form or its optical isomers, as well as its salts.

14. Compound, **characterized in that** it is
(3aRS,4SR,9SR,9aRS)-4,9-ethano-5-methoxy-2-[2-(2-methoxyphenyl)propenoyl]-9-(4-methylphenyl)-2,3,3a,4,9,9a-hexahydro-1H-benzo[f]isoindole-3a-carboxylic acid
in the racemic form or its optical isomers, as well as its salts.

15. Compound, **characterized in that** it is
(3aRS,4SR,9SR,9aRS)-4, 9-ethano-2- [2-(2-methoxyphenyl)-propenoyl]-4-methyl-9-(4-methoxyphenyl)-2,3,3a,4,9,9a-hexahydro-1H-benzo[f]isoindole-3a-carboxylic acid
in the racemic form or its optical isomers, as well as its salts.

16. Compound, **characterized in that** it is
the dextrorotatory enantiomer of (3aRS,4SR,9SR,9aRS)-4,9-ethano-2-[2-(2-methoxyphenyl)propenoyl]-9-(4-methylphenyl)-2,3,3a,4,9,9a-hexahydro-1H-benzo[f]isoindole-3a-carboxylic acid.

17. Compound, **characterized in that** it is
the dextrorotatory enantiomer of (3aRS,4SR,9SR,9aRS)-9-(2,3-dihydro-1, 4-benzodioxin-6-yl)-4,9-ethano-2-[2-(2-methoxyphenyl)propenoyl]-2,3,3a,4,9,9a-hexahydro-1H-benzo[f]isoindole-3a-N-(3-pyridylmethyl)carboxamide.

18. Process for the preparation of a compound of general formula (I) according to any one of Claims 1 to 17, in which Y represents an oxygen or sulphur atom and X represents a methylene or vinyldiyl group, **characterized in that** an acid of general formula:

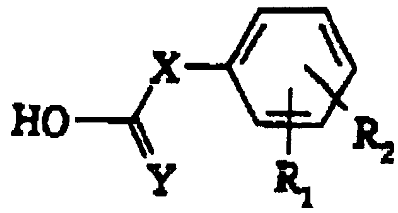

(II)

in which $R_1$ and $R_2$ are defined according to Claim 1, X is as defined above and Y represents an oxygen or sulphur atom, its methyl ester or a derivative of this acid, such as a halide or the anhydride, is reacted with a product of general formula:

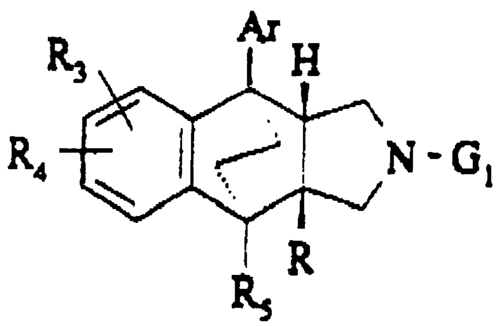

(III)

in which Ar, R, $R_3$, $R_4$ and $R_5$ are defined according to Claim 1 and $G_1$ represents a hydrogen atom.

**19.** Process for the preparation of a compound of general formula (III)

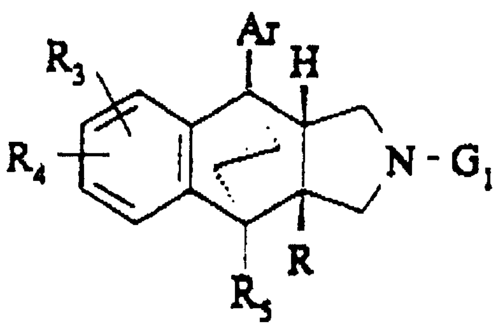

(III)

in which $R_3$, $R_4$, $R_5$, Ar and R are as defined in Claim 1 and $G_1$ represents a benzyl radical, from compounds of general formula (IX):

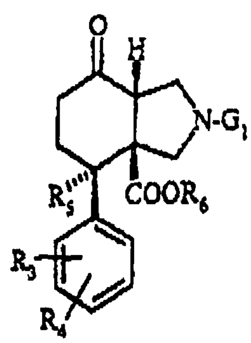

(IX)

in which $R_3$, $R_4$, $R_5$, $R_6$ and $G_1$ are as defined above, **characterized in that** an intermediate of general formula (XV)

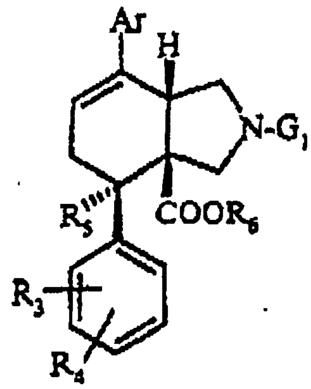

(XV)

in which $R_3$, $R_4$, $R_5$, $R_6$, Ar and $G_1$ are as defined above, is formed and the conversion of which by intramolecular cyclization of Friedel-Crafts type results in the product (III) as defined in Claim 18.

**20.** Compound, **characterized in that** it is represented by the general formula XV

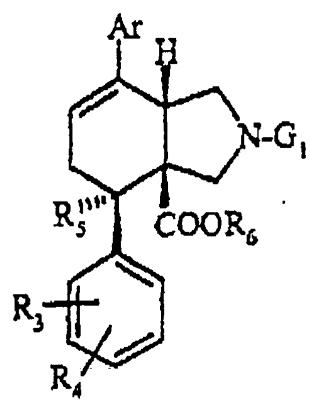

(XV)

in which $R_3$, $R_4$, $R_5$ and Ar are as defined in Claim 1, $G_1$ represents a benzyl radical and $R_6$ represents an alkyl radical containing 1 to 3 carbon atoms, in the racemic form, as well as its optical isomers.

**21.** Process for the preparation of a compound of general formula (XV) according to Claim 19, **characterized in that** a compound of general formula (IX)

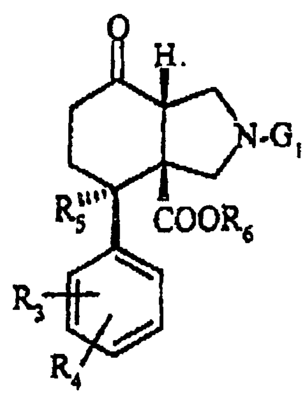

(IX)

in which $R_3$, $R_4$, $R_5$ and Ar are as defined in Claim 1, $G_1$ represents a benzyl radical and $R_6$ represents an alkyl

radical containing 1 to 3 carbon atoms, is converted at the 7 position, either into an iodoethylene derivative, by a Barton reaction, or into an enol triflate, and is then subjected to a palladium coupling reaction, the so-called Suzuki reaction with an arylboronic acid or the so-called Stille reaction with an arylstannane.

**22.** Process for the preparation of a compound of general formula (III) according to Claim 18, in which Ar preferably represents

- a phenyl nucleus substituted at the para, metapara or meta-para-meta' position by electron-donating groups,
- an electron-rich heterocyclic radical or
- a heterocyclic radical suitably substituted by electron-donating groups,

**characterized in that** an aromatic or heterocyclic hydrocarbon Ar-H is reacted, according to intermolecular and then intramolecular cyclization reactions of Friedel-Crafts type, with a compound of general formula (IX), in which $R_3$, $R_4$, $R_5$ and Ar are as defined in Claim 1, $G_1$ represents a benzyl radical and $R_6$ represents an alkyl radical containing 1 to 3 carbon atoms, in an organic solvent in the presence of an excess of strong acid or of a Lewis acid.

**23.** Process for the preparation of a product of general formula (III)

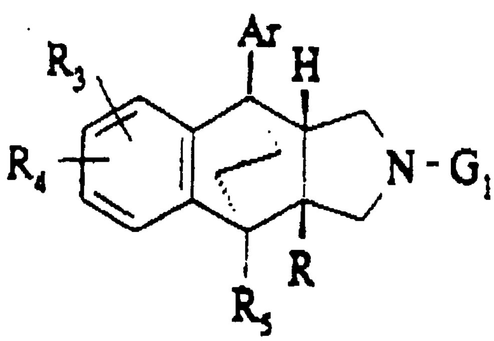

(III)

in which $R_3$, $R_4$, $R_5$ and Ar are as defined in Claim 1, $G_1$ represents a benzyl radical and R represents a carboxyl radical or a radical of general formula $COOR_6$ and $R_6$ represents an alkyl radical containing 1 to 3 carbon atoms, **characterized in that** intramolecular cyclization of Friedel-Crafts type is carried out by the action of trifluoromethanesulphonic acid on a product of general formula (VIII):

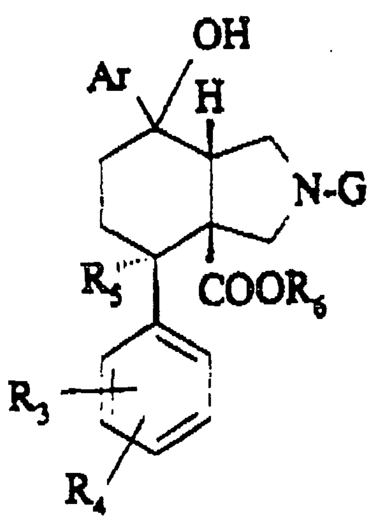

(VIII)

in which:

Ar, $R_3$, $R_4$ and $R_5$ are as defined in Claim 1, $G_1$ represents a benzyl radical and $R_6$ represents an alkyl radical containing 1 to 3 carbon atoms, followed by replacement of the $G_1$ group by a hydrogen atom, either by hydrogenolysis and then optionally by replacement of the hydrogen atom by a *tert*-butoxycarbonyl radical, by reaction with *tert*-butoxycarbonyl anhydride in an organic solvent, or by a benzyloxycarbonyl radical, by reaction with benzyloxycarbonyl chloride; or by reaction with an alkyl chloroformate, followed by acid hydrolysis of the carbamate formed as an intermediate, optionally followed by saponification of the product obtained.

**24.** Process for the preparation of a compound of general formula (VIII), in which Ar, $R_3$, $R_4$, $R_5$, $R_6$ and $G_1$ are as defined in Claim 23, **characterized in that** it is carried out from a compound of general formula (IX)

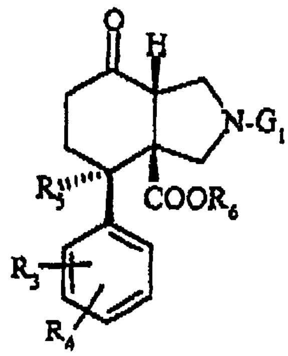

(IX)

in which $R_3$, $R_4$, $R_5$, $R_6$ and $G_1$ are as defined in Claim 21,
either by reaction with an organomagnesium derivative of general formula Ar-Mg-X, in which Ar is as defined above and X represents a halogen atom, in the presence of anhydrous cerium(III) chloride in an organic solvent at a temperature of between 0°C and the reflux temperature of the reaction mixture,
or by reaction with an organolithium derivative of general formula Ar-Li, in which Ar is as defined in Claim 1, the reaction being carried out in an organic solvent at a temperature of between -78° and -20°C.

**25.** Process for the preparation of a compound of general formula (IX) as defined in Claim 21 or 24, in which $R_3$, $R_4$, $R_5$ and Ar are as defined in Claim 1, $G_1$ represents a benzyl radical and $R_6$ represents an alkyl radical containing 1 to 3 carbon atoms, **characterized in that** an N-trialkylsilylmethyl-N-(alkoxymethyl)amine, carrying a protective group for the amine functional group, is reacted with a derivative of a cyclohexenone of general formula:

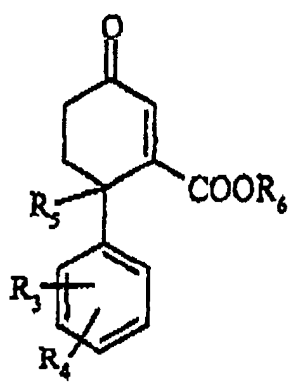

(X)

in which $R_3$, $R_4$ and $R_5$ are as defined in Claim 1 and $R_6$ represents an alkyl radical containing 1 to 3 carbon atoms.

**26.** Pharmaceutical composition, **characterized in that** it contains at least one product according to one of the preceding Claims 1 to 17 in combination with one or more pharmaceutically acceptable diluents or adjuvants, whether inert or biologically active.

**27.** Use of the compounds according to one of the preceding Claims 1 to 17 in the preparation of pharmaceutical

compositions useful in inhibiting the protein farnesyl transferase.

**28.** Use of the compounds according to one of Claims 1 to 17 in the preparation of pharmaceutical compositions for the treatment of diseases related to cell proliferations, malignant or benign, of cells of various tissues and/or organs, comprising muscle, bone or connective tissues, the skin, the brain, the lungs, the sexual organs, the lymphatic or renal systems, mammary or blood cells, the liver, the digestive system, the pancreas and the thyroid or adrenal glands, and including the following pathologies: psoriasis, restenosis, solid tumours, Kaposi's sarcoma, carcinomas, cholangiocarcinoma, choriocarcinoma, neuroblastoma, Wilms' tumour, Hodgkin's disease, melanomas, teratocarcinomas, gliomas, multiple myelomas, chronic lymphocytic leukaemias, acute or chronic granulocytic lymphomas, and cancers, such as cancers of the pancreas, colon, lung, ovary, breast, brain, prostate, liver, stomach, bladder or testicles.

**29.** Use of the compounds of general formula (I) according to one of Claims 1 to 17 in the preparation of pharmaceutical compositions useful in the treatment of diseases related to cell proliferations by inhibition of farnesyl transferase.

**30.** Use of the compounds of general formula (I) according to one of Claims 1 to 17 in the preparation of pharmaceutical compositions for the treatment of cancers.

**31.** Use of the compound of Claim 16 in the preparation of pharmaceutical compositions for the treatment of cancers.

**32.** Use of the compounds of general formula (I) according to one of Claims 1 to 17 in the preparation of pharmaceutical compositions for the treatment of cancer of the colon.

**33.** Use of the compounds of general formula (I) according to one of Claims 1 to 17 in the preparation of pharmaceutical compositions for the treatment of cancer of the pancreas.

**34.** Use of the compound of Claim 16 in the preparation of pharmaceutical compositions for the treatment of cancer of the colon.

**35.** Use of the compound of Claim 16 in the preparation of pharmaceutical compositions for the treatment of cancer of the pancreas.

**36.** Combinations of a product according to one of the preceding Claims 1 to 17 with one or more compatible and pharmacologically active compounds and/or with a radiotherapy treatment, in order to obtain a synergic therapeutic effect.